# EUROPEAN PATENT APPLICATION

(11) **EP 4 768 044 A2**
(43) Date of publication of application: **01.07.2026**
(21) Application number: 26162693.1
(22) Date of filing: 29.04.2022
(51) Int. Cl.: A61K 45/06

(54) **19-NOR C3,3-DISUBSTITUTED C21 -N-PYRAZOLYL STEROID FOR USE IN TREATING MAJOR DEPRESSIVE DISORDER AND POSTPARTUM DEPRESSION**

(30) Priority: 29.04.2021 US 202163181743 P; 04.06.2021 US 202163197025 P; 15.06.2021 US 202163210810 P; 31.08.2021 US 202163239096 P; 03.12.2021 US 202163285812 P; 14.12.2021 US 202163289506 P; 14.12.2021 US 202163289520 P; 11.01.2022 US 202263298601 P
(62) Divisional of application: 22724335.9
(71) Applicant: Sage Therapeutics, LLC, Rockville, MD 20850 (US)
(72) Inventor: LASSER, Robert Alfonso, Cambridge, MA 02142 (US); DOHERTY, James, Cambridge, MA 02142 (US); JONAS, Jeffrey Martin, Cambridge, MA 02142 (US); KANES, Stephen Jay, Cambridge, MA 02142 (US); GUNDUZ-BRUCE, Handan, Cambridge, MA 02142 (US); COLQUHOUN, Helen Anne, Cambridge, MA 02142 (US); ARNOLD, Ryan, Cambridge, MA 02142 (US); BONTHAPALLY, Vijayveer, Cambridge, MA 02142 (US); DUNBAR, Joi Lisa, Cambridge, MA 02142 (US); ADIWIJAYA, Bambang Senoaji, Cambridge, MA 02142 (US)
(74) Representative: Coles, Andrea Birgit

(57) **Abstract**

The present disclosure relates to methods of treating major depressive disorder (MDD) with elevated anxiety in a subject in need thereof, by administering a therapeutically effective amount of Compound **(1),** or a pharmaceutically acceptable salt thereof. The disclosure also relates to methods of treating postpartum depression (PPD) with elevated anxiety in a subject in need thereof, by administering a therapeutically effective amount of Compound **(1),** or a pharmaceutically acceptable salt thereof.

## Description

### CROSS-REFERENCE TO RELATED APPLICATIONS

This application claims the benefit of U.S. Provisional Application No. 63/181,743, filed on April 29, 2021; U.S. Provisional Application No. 63/197,025, filed on June 4, 2021; U.S. Provisional Application No. 63/210,810, filed on June 15, 2021; U.S. Provisional Application No. 63/239,096, filed on August 31, 2021; U.S. Provisional Application No. 63/285,812, filed on December 3, 2021; U.S. Provisional Application No. 63/289,506, filed on December 14, 2021; U.S. Provisional Application No. 63/289,520, filed on December 14, 2021; and U.S. Provisional Application No. 63/298,601, filed on January 11, 2022. The entire contents of the aforementioned applications are incorporated herein by reference in their entireties.

### FIELD OF THE INVENTION

The present disclosure is directed to methods of treating major depressive disorder (MDD) with elevated anxiety in a subject in need thereof, by administering a therapeutically effective amount of Compound **(1),** or a pharmaceutically acceptable salt thereof. The disclosure is also directed to methods of treating postpartum depression (PPD) with elevated anxiety in a subject in need thereof, by administering a therapeutically effective amount of Compound **(1),** or a pharmaceutically acceptable salt thereof.

### BACKGROUND

GABA, γ-aminobutyric acid, has a profound influence on overall brain excitability because up to 40% of the neurons in the brain utilize GABA as a neurotransmitter. GABA interacts with its recognition site on the GRC (GABA receptor complex) to facilitate the flow of chloride ions down an electrochemical gradient of the GRC into the cell. An intracellular increase in the levels of this anion causes hyperpolarization of the transmembrane potential, rendering the neuron less susceptible to excitatory inputs (i.e., reduced neuron excitability). In other words, the higher the chloride ion concentration in the neuron, the lower the brain excitability (the level of arousal). It is well-documented that the GRC is responsible for the mediation of anxiety, seizure activity, and sedation. Thus, GABA and drugs that act like GABA (*e.g.,* the therapeutically useful barbiturates and benzodiazepines (BZs), such as Valium^{®}) produce their therapeutically useful effects by interacting with specific regulatory sites on the GRC.

Accumulated evidence has indicated that the GRC contains a distinct site for neuroactive steroids (Lan, N. C. et al., Neuwchem. Res. 16:347-356 (1991)). Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232: 1004-1007 (1986); Harrison, N. L. et al., J. Pharmacol. Exp. Ther. 241:346-353 (1987)).

### SUMMARY OF THE INVENTION

In one aspect, the disclosure provides a method of treating major depressive disorder (MDD) with elevated anxiety in a subject in need thereof, comprising administering a therapeutically effective amount of Compound **(1):**

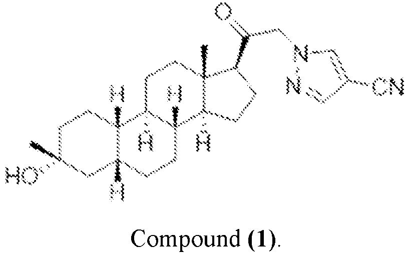

In one aspect, the disclosure provides method of treating major depressive disorder (MDD) with elevated anxiety in a subject in need thereof, comprising administering a therapeutically effective amount of a pharmaceutically acceptable salt of Compound **(1):**

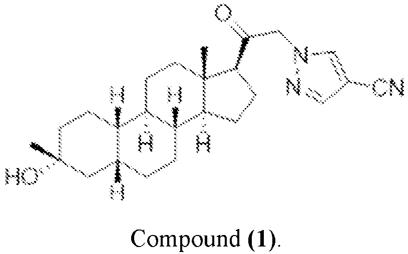

In some embodiments, Compound **(1),** or the pharmaceutically acceptable salt of Compound **(1),** is administered once a day for about 14 days or about 2 weeks. In some embodiments, Compound **(1)** is administered at a dose of about 20 mg to about 55 mg. In some embodiments, Compound **(1)** is administered at a dose of about 50 mg. In some embodiments, Compound **(1)** is administered at a dose of about 30 mg or about 40 mg.

In some embodiments, the pharmaceutically acceptable salt of Compound **(1)** is administered at a dose equivalent to about 20 mg to about 55 mg of the free base compound. In some embodiments, the pharmaceutically acceptable salt of Compound **(1)** is administered at a dose equivalent to about 50 mg of the free base compound. In some embodiments, the pharmaceutically acceptable salt of Compound **(1)** is administered at a dose equivalent to about 30 mg or about 40 mg of the free base compound.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally. In some embodiments, Compound **(1),** or the pharmaceutically acceptable salt of Compound **(1),** is administered with food. In some embodiments, Compound **(1),** or the pharmaceutically acceptable salt of Compound **(1),** is administered once a day at night.

In some embodiments, Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ.

In some embodiments, Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.

In some embodiments, Compound **(1),** or the pharmaceutically acceptable salt of Compound **(1),** is re-administered to the subject in response to a recurrence of depression symptoms after completion of the initial treatment. In some embodiments, there is at least a 6 week interval between the last dose of the initial treatment and the first dose of the re-administration. In some embodiments, each of the initial treatment and re-administration occurs for about 14 days or about 2 weeks.

In some embodiments, the method further comprises administration of a second therapeutic agent.

In some embodiments, the subject is treatment naive.

In some embodiments, the subject has been on a stable dose of an additional antidepressant for at least 30 days or for at least 60 days prior to the administration of Compound **(1)** or the pharmaceutically acceptable salt of Compound **(1).**

In some embodiments, MDD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).** In some embodiments, MDD with elevated anxiety is characterized by a HAM-D total score of 24 or greater and a HAM-A total score of 17 or greater prior to the administration of Compound **(1),** or the pharmaceutically acceptable salt of Compound **(1).** In some embodiments, MDD with elevated anxiety is characterized by a HAM-D total score of 24 or greater and a HAM-D Anxiety/Somatization subscale score of 7 or greater prior to the administration of Compound **(1),** or the pharmaceutically acceptable salt of Compound **(1).**

In some embodiments, the subject exhibits a reduction in HAM-D total score, HAM-A total score, HAM-D Anxiety/Somatization subscale score, or a combination thereof, from baseline. In some embodiments, the subject exhibits a reduction of at least 14 points in HAM-D total score on Day 15 after administration of Compound **(1),** or the pharmaceutically acceptable salt of Compound **(1).** In some embodiments, the subject exhibits a reduction of at least 12 points in HAM-A total score on Day 15 after administration of Compound **(1),** or the pharmaceutically acceptable salt of Compound **(1).**

In another aspect, the disclosure provides a method of treating postpartum depression (PPD) with elevated anxiety in a subject in need thereof, comprising administering a therapeutically effective amount of Compound **(1):**

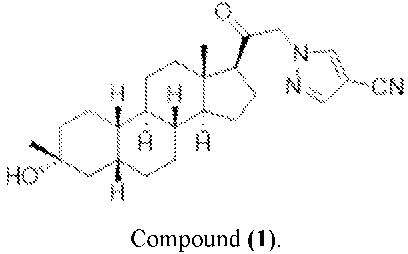

In one aspect, the disclosure provides method of treating postpartum depression (PPD) with elevated anxiety in a subject in need thereof, comprising administering a therapeutically effective amount of a pharmaceutically acceptable salt of Compound **(1):**

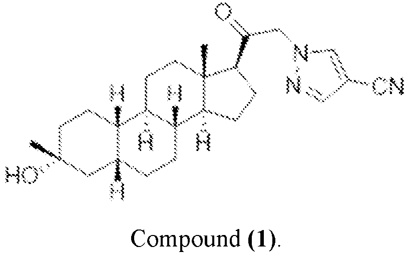

In some embodiments, Compound **(1),** or the pharmaceutically acceptable salt of Compound **(1),** is administered once a day for about 14 days or about 2 weeks. In some embodiments, Compound **(1)** is administered at a dose of about 20 mg to about 55 mg. In some embodiments, Compound **(1)** is administered at a dose of about 50 mg. In some embodiments, Compound **(1)** is administered at a dose of about 30 mg or about 40 mg.

In some embodiments, the pharmaceutically acceptable salt of Compound **(1)** is administered at a dose equivalent to about 20 mg to about 55 mg of the free base compound. In some embodiments, the pharmaceutically acceptable salt of Compound **(1)** is administered at a dose equivalent to about 50 mg of the free base compound. In some embodiments, the pharmaceutically acceptable salt of Compound **(1)** is administered at a dose equivalent to about 30 mg or about 40 mg of the free base compound.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally. In some embodiments, Compound **(1),** or the pharmaceutically acceptable salt of Compound **(1),** is administered with food. In some embodiments, Compound **(1),** or the pharmaceutically acceptable salt of Compound **(1),** is administered once a day at night.

In some embodiments, Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ.

In some embodiments, Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.

In some embodiments, Compound **(1),** or the pharmaceutically acceptable salt of Compound **(1),** is re-administered to the subject in response to a recurrence of depression symptoms after completion of initial treatment. In some embodiments, there is at least a 6 week interval between the last dose of the initial treatment and the first dose of the re-administration. In some embodiments, each of the initial treatment and re-administration occurs for about 14 days or about 2 weeks.

In some embodiments, the method further comprises administration of a second therapeutic agent.

In some embodiments, the subject is treatment naive.

In some embodiments, the subject has been on a stable dose of an additional antidepressant for at least 30 days or for at least 60 days prior to the administration of Compound **(1),** or the pharmaceutically acceptable salt of Compound **(1).**

In some embodiments, PPD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).** In some embodiments, PPD with elevated anxiety is characterized by a HAM-D total score of 26 or greater and a HAM-A total score of 17 or greater prior to the administration of Compound **(1),** or the pharmaceutically acceptable salt of Compound **(1).** In some embodiments, PPD with elevated anxiety is characterized by a HAM-D total score of 26 or greater and a HAM-D Anxiety/Somatization subscale score of 7 or greater prior to the administration of Compound **(1),** or the pharmaceutically acceptable salt of Compound **(1).**

In some embodiments, the subject exhibits a reduction in HAM-D total score, HAM-A total score, HAM-D Anxiety/Somatization subscale score, or a combination thereof, from baseline.

In one aspect, the disclosure provides a method of treating major depressive disorder (MDD) with elevated anxiety in a subject in need thereof, comprising administering about 30 mg to about 50 mg of Compound **(1):**

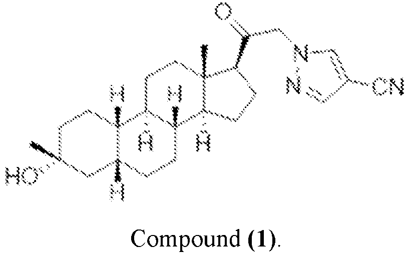

In one aspect, the disclosure provides a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound:

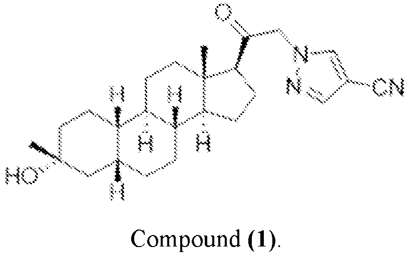

In one aspect, the disclosure provides a method of treating postpartum depression (PPD) with elevated anxiety in a subject in need thereof, comprising administering about 30 mg to about 50 mg of Compound **(1):**

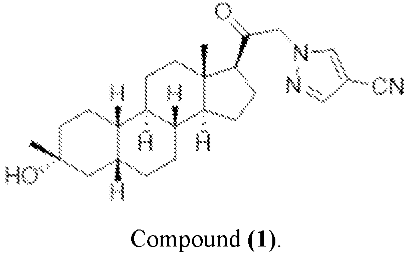

In one aspect, the disclosure provides a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound:

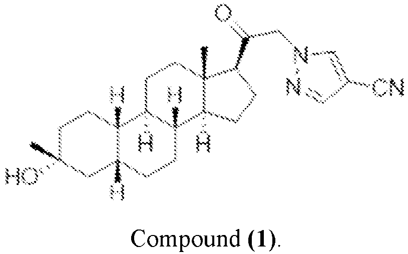

In one aspect, the disclosure provides a method of treating major depressive disorder (MDD) with elevated anxiety in a subject in need thereof, comprising administering about 30 mg to about 50 mg of Compound **(1)** once a day for about 14 days or about 2 weeks:

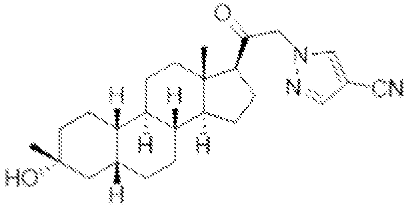

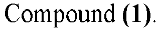

In one aspect, the disclosure provides a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks:

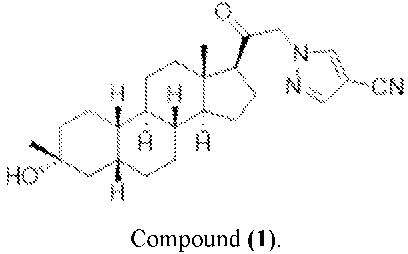

In one aspect, the disclosure provides a method of treating postpartum depression (PPD) with elevated anxiety in a subject in need thereof, comprising administering about 30 mg to about 50 mg of Compound **(1)** once a day for about 14 days or about 2 weeks:

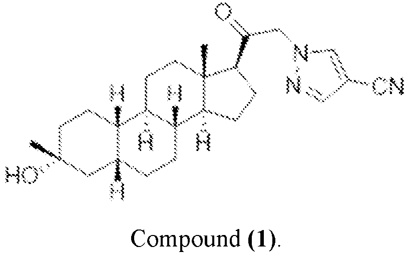

In one aspect, the disclosure provides a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks:

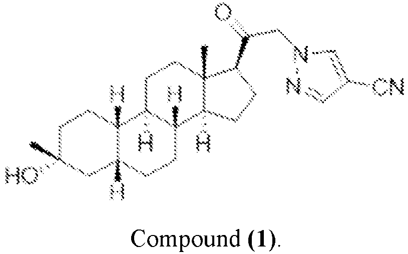

In one aspect, the disclosure provides a method of treating major depressive disorder (MDD) with elevated anxiety in a subject in need thereof, comprising administering about 30 mg to about 50 mg of Compound **(1)** once a day for about 14 days or about 2 weeks: 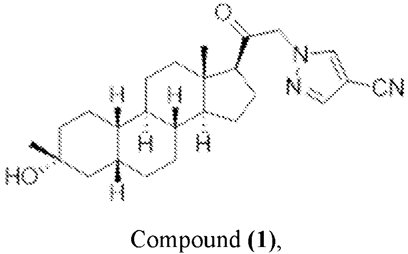 wherein the subject is treatment naïve.

In one aspect, the disclosure provides a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 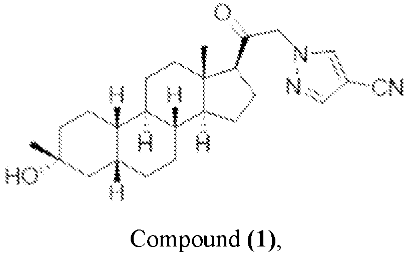 wherein the subject is treatment naïve.

In one aspect, the disclosure provides a method of treating postpartum depression (PPD) with elevated anxiety in a subject in need thereof, comprising administering about 30 mg to about 50 mg of Compound **(1)** once a day for about 14 days or about 2 weeks: 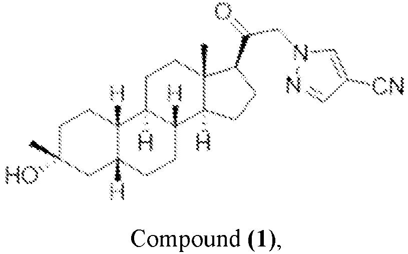 wherein the subject is treatment naïve.

In one aspect, the disclosure provides a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 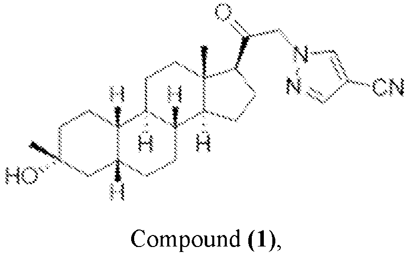 wherein the subject is treatment naïve.

In one aspect, the disclosure provides a method of treating major depressive disorder (MDD) with elevated anxiety in a subject in need thereof, comprising administering about 30 mg to about 50 mg of Compound **(1)** once a day for about 14 days or about 2 weeks: 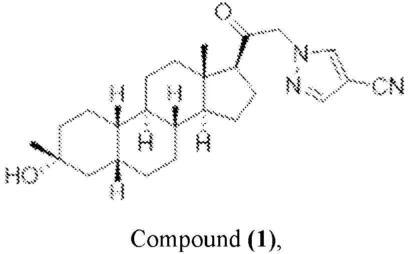 wherein the subject has been on a stable dose of an additional antidepressant for at least 60 days prior to the administration of Compound **(1).**

In one aspect, the disclosure provides a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 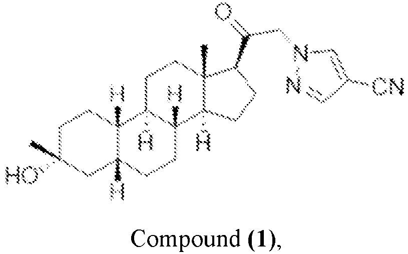 wherein the subject has been on a stable dose of an additional antidepressant for at least 60 days prior to the administration of the pharmaceutically acceptable salt of Compound **(1).**

In one aspect, the disclosure provides a method of treating postpartum depression (PPD) with elevated anxiety in a subject in need thereof, comprising administering about 30 mg to about 50 mg of Compound **(1)** once a day for about 14 days or about 2 weeks: 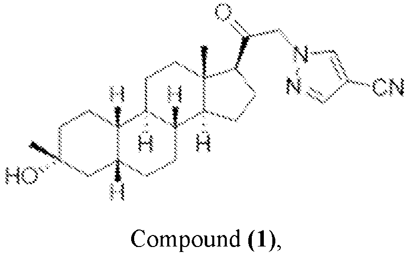 wherein the subject has been on a stable dose of an additional antidepressant for at least 30 days prior to the administration of Compound **(1).**

In one aspect, the disclosure provides a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 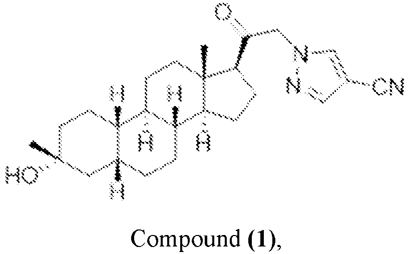 wherein the subject has been on a stable dose of an additional antidepressant for at least 30 days prior to the administration the pharmaceutically acceptable salt of Compound **(1).**

In one aspect, the disclosure provides a method of treating postpartum depression (PPD) with elevated anxiety in a subject in need thereof, comprising administering about 30 mg to about 50 mg of Compound **(1)** once a day for about 14 days or about 2 weeks: 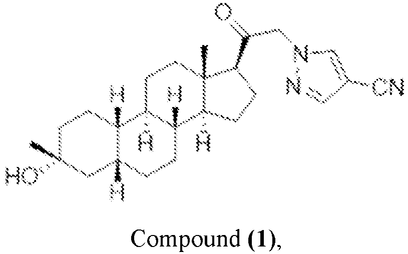 wherein the subject has been on a stable dose of an additional antidepressant for at least 60 days prior to the administration of Compound **(1).**

In one aspect, the disclosure provides a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 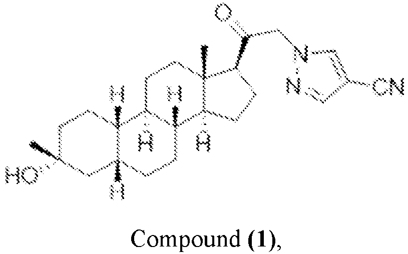 wherein the subject has been on a stable dose of an additional antidepressant for at least 60 days prior to the administration the pharmaceutically acceptable salt of Compound **(1).**

In some embodiments, Compound **(1)** is administered at a dose of about 50 mg or the pharmaceutically acceptable salt of Compound **(1)** is administered at a dose equivalent to about 50 mg of the free base compound. In some embodiments, Compound **(1)** is administered at a dose of about 40 mg or the pharmaceutically acceptable salt of Compound **(1)** is administered at a dose equivalent to about 40 mg of the free base compound. In some embodiments, Compound **(1)** is administered at a dose of about 30 mg or the pharmaceutically acceptable salt of Compound **(1)** is administered at a dose equivalent to about 30 mg of the free base compound.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally. In some embodiments, Compound **(1),** or the pharmaceutically acceptable salt of Compound **(1),** is administered with food. In some embodiments, Compound **(1),** or the pharmaceutically acceptable salt of Compound **(1),** is administered once a day at night.

In some embodiments, the subject is treatment naive.

In some embodiments, the subject has been on a stable dose of an additional antidepressant for at least 30 days or for at least 60 days prior to the administration of Compound **(1),** or the pharmaceutically acceptable salt of Compound **(1).**

In some embodiments, the method further comprises administration of a second therapeutic agent.

In some embodiments, Compound **(1),** or the pharmaceutically acceptable salt of Compound **(1),** is re-administered to the subject in response to a recurrence of depression symptoms after completion of an initial treatment. In some embodiments, there is at least a 6 week interval between the last dose of the initial treatment and the first dose of the re-administration. In some embodiments, the re-administration occurs for about 14 days or about 2 weeks.

In some embodiments, MDD with elevated anxiety or PPD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1)** or the pharmaceutically acceptable salt of Compound **(1).** In some embodiments, MDD with elevated anxiety or PPD with elevated anxiety is characterized by a HAM-D total score of 24 or greater and a HAM-A total score of 17 or greater prior to the administration of Compound **(1)** or the pharmaceutically acceptable salt of Compound **(1).** In some embodiments, MDD with elevated anxiety or PPD with elevated anxiety is characterized by a HAM-D total score of 24 or greater and a HAM-D Anxiety/Somatization subscale score of 7 or greater prior to the administration of Compound **(1)** or the pharmaceutically acceptable salt of Compound **(1).**

In some embodiments, the subject exhibits a reduction in HAM-D total score, HAM-A total score, HAM-D Anxiety/Somatization subscale score, or a combination thereof, from baseline. In some embodiments, the subject exhibits a reduction of at least 14 points in HAM-D total score on Day 15 after administration of Compound **(1)** or the pharmaceutically acceptable salt of Compound **(1).** In some embodiments, the subject exhibits a reduction of at least 12 points in HAM-A total score on Day 15 after administration of Compound **(1)** or the pharmaceutically acceptable salt of Compound **(1).**

### BRIEF DESCRIPTION OF THE DRAWINGS

**FIG. 1** depicts an exemplary study design for treating MDD with Compound **(1).**
**FIG. 2** shows HAMD-17 total score least squares (LS) mean change from baseline at Day 15 and other timepoints.
**FIG. 3** shows the mean of percent Day 15 CFB retained at subsequent visits. Percent retention of Day 15 HAMD-17 total score reduction from baseline is the change from baseline in HAMD-17 total score at post-Day 15 visits as percentage of change from baseline at Day 15 and was evaluated in Day 15 HAMD-17 responders only in the Compound **(1)** treatment group (≥50% change in HAMD-17 total score at Day 15 versus baseline).
**FIG. 4** shows LS mean difference of HAMD-17 subgroups at Day 15.
**FIG. 5** shows CGI-S LS mean change from baseline at Day 15 and other timepoints.
**FIG. 6** shows CGI - Improvement Response at Day 15 and other timepoints.
**FIG. 7** shows CGI - Improvement scores at Day 15 for the clinical trial study of Example 1 and three other clinical trials conducted by Applicant. Phase 3 Study* is a Phase 3 clinical trial study conducted by Applicant (ClinicalTrials.gov identifier/NCT number: NCT03864614). Phase 3 Study** is a Phase 3 clinical trial study conducted by Applicant (ClinicalTrials.gov identifier/NCT number: NCT0672175). Phase 2 Study*** is a Phase 2 clinical trial study conducted by Applicant (ClinicalTrials.gov identifier/NCT number: NCT03000530).
**FIG. 8** shows MADRS total score change from baseline at Day 15 and other timepoints.
**FIG. 9** shows that treatment difference in HAMD-17 and MADRS Total Scores at Day 15 significantly favored Compound **(1).**
**FIG. 10A** shows HAMD-17 Response at Day 15 and other timepoints.
**FIG. 10B** shows HAMD-17 Remission at Day 15 and other timepoints.
**FIG. 11** shows MADRS Response and Remission rates at Days 8 and 15.
**FIG. 12** shows HAM-A total score change from baseline at Day 15 and other timepoints.
**FIG. 13** shows HAMD-17 Anxiety/Somatization score change from baseline at Day 15 and other timepoints.
**FIG. 14A** shows improvement in both Anxiety and Depressive Symptoms favor Compound **(1)** compared with Placebo at Day 8.
**FIG. 14B** shows improvement in both Anxiety and Depressive Symptoms favor Compound **(1)** compared with Placebo at Day 15.
**FIG. 15A** shows HAMD-17 LS mean change from baseline of Compound **(1)-**treated patients having MDD with elevated anxiety and Compound **(1)-**treated patients having MDD without elevated anxiety.
**FIG. 15B** shows HAM-A LS mean change from baseline of Compound (**1**)-treated patients having MDD with elevated anxiety and Compound **(1)-**treated patients having MDD without elevated anxiety.
**FIG. 16** shows pooled HAMD-17 LS mean change from baseline of Compound (**1**)-treated patients having MDD without elevated anxiety and Compound (**1**)-treated patients having MDD with elevated anxiety.
**FIG. 17** shows pooled mean HAMD-17 total score of Compound (**1**)-treated patients having MDD without elevated anxiety and Compound (**1**)-treated patients having MDD with elevated anxiety.
**FIG. 18A** shows pooled mean SF-36v2 scores of Compound (**1**)-treated patients having MDD without elevated anxiety
**FIG. 18B** shows pooled mean SF-36v2 scores of Compound (**1**)-treated patients having MDD with elevated anxiety.
**FIG. 19** is a line graph showing the improvement in depressive symptoms achieved with Compound **(1)** vs placebo based on the change from baseline in HAMD-17 scores.
**FIG. 20A** is a bar graph showing the concurrent improvement of depressive and anxiety symptoms with Compound **(1)** vs placebo based on HAMD-17 and HAM-A scores.
**FIG. 20B** is a bar graph showing the concurrent improvement of depressive and anxiety symptoms with Compound **(1)** vs placebo based on MADRS and HAM-A scores.
**FIG. 21** is a bar graph showing the proportion of patients with sustained concurrent depression and anxiety improvement.
**FIG. 22A** is a bar graph comparing NNT vs placebo for Response and Remission.
**FIG. 22B** is a bar graph comparing NNT vs placebo for sustained Remission.
**FIG. 23A** is a line graph showing the change from baseline in HAMD-17 A/S.
**FIG. 23B** is a line graph showing the change from baseline in EPDS-3A.
**FIG. 24A** is a bar graph showing HAM-A Response rates.
**FIG. 24B** is a bar graph showing HAM-A Remission rates.
**FIG. 24C** is a bar graph showing HAMD-17 A/S Response rates.
**FIG. 25A** is a line graph showing the improvement in symptoms of insomnia based on the change from baseline in HAMD-17-Ins scores.
**FIG. 25B** is a line graph showing the improvement in symptoms of insomnia based on the change from baseline in MADRS-Ins scores.
**FIG. 26A** is a line graph showing the PHQ-9 change from baseline.
**FIG. 26B** shows the correlations with HAM-D in the pooled population.
**FIG. 27** are bar graphs showing EPDS remission percentages.
**FIG. 28** shows the correlation between HAMD-17 and EPDS scores by visit.
**FIG. 29A** is a bar graph comparing improvements in SF-36 Domains and summary Scores.
**FIG. 29B** is a bar graph comparing Proportion Meeting SF-36 Population Norm Values at baseline, Day 15, and Day 45.
**FIG. 30A** shows the study design for the dose selection phase of the study of Example 7. For the dose selection phase, Compound **(1)** doses did not exceed 90 mg. *Sentinel dosing was employed for each cohort, with one participant randomized to receive Compound **(1)** and one to receive placebo on Day 1. The remaining 6 participants were dosed approximately 24 hours later after tolerability was confirmed.
**FIG. 30B** shows the study design for the main study phase of the study of Example 7. †Randomization ratio was 1:1:1:1:1:1. ‡Doses were determined based on the results of the dose selection phase. R = randomization.
**FIG. 31A** shows the patient disposition for the dose selection phase of the study of Example 7.
**FIG. 31B** shows the patient disposition for the treatment phase of the study of Example 7.
**FIG. 32** shows Mean Drug Liking VAS Emax over time (modified completer set, n = 60). Drug liking VAS was assessed using the question, "At this moment, my liking for this drug is" where responses ranged from 0 (strong disliking) to 100 (strong liking) with a score of 50 being neutral. Emax = maximum effect; VAS = visual analog scale.
**FIG. 33** shows paired differences in Drug Liking VAS Emax (modified completer set, n = 60). Drug Liking (at this moment) was assessed by "At this moment, my liking for this drug is," where responses ranged from 0 (strong disliking) to 100 (strong liking), with 50 being the neutral point. CI = confidence interval; Emax = maximum effect; VAS = visual analog scale.
**FIG. 34A** shows paired differences in Overall Drug Liking VAS Emax (modified completer set, n = 60). Overall Drug Liking was assessed by "Overall, my liking for this drug is," where responses ranged from 0 (strong disliking) to 100 (strong liking) with 50 being the neutral point. CI = confidence interval; Emax = maximum effect; VAS = visual analog scale.
**FIG. 34B** shows paired differences in Take Drug Again VAS Emax (modified completer set, n = 60). Take Drug Again was assessed by "I would take this drug again", where responses ranged from 0 (definitely not) to 100 (definitely so) with 50 being the neutral point. CI = confidence interval; Emax = maximum effect; VAS = visual analog scale.
**FIG. 35** shows Mean Alertness/Drowsiness VAS over time (modified completer set, n = 60). Alertness/drowsiness VAS was assessed using the question, "At this moment, my mental state is" where responses ranged from 0 (very drowsy) to 100 (very alert) with a score of 50 being neutral. VAS = visual analog scale.
**FIG. 36** shows Mean Any Drug effect VAS Emax over time (modified completer set, n = 60). Any Drug effect VAS was assessed using the question, "At this moment, my liking for this drug is" where responses ranged from 0 (strong disliking) to 100 (strong liking) with a score of 50 being neutral. Emax = maximum effect; VAS = visual analog scale.
**FIG. 37** shows Mean Compound **(1)** plasma concentration vs time profile by treatment following a single-dose administration (PK set, n = 71).

### DETAILED DESCRIPTION

### I. DEFINITIONS

As used herein, "Compound **(1)"** refers to the compound having the formula (or structure):

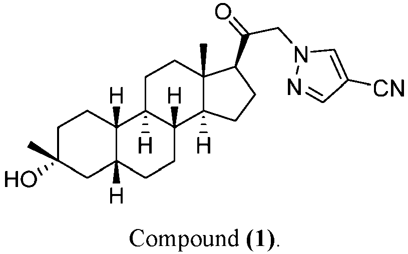

Compound **(1)** is also known as zuranolone, 3α-hydroxy-3β-methyl-21-(4-cyanopyrazol-1-yl)-5β-19-norpregnan-20-one, and by its IUPAC name: 1-(2-((3R,5R,8R,9R,10S,13S,14S,17S)-3-hydroxy-3,13-dimethylhexadecahydro-1H-cyclopenta[a]phenanthren-17-yl)-2-oxoethyl)-1H-pyrazole-4-carbonitrile (CAS Registry Number 1632051-40-1). A method of chemically synthesizing Compound **(1),** was described in U.S. Patent No. 9,512,165 and PCT Application Publication No. WO 2014/169833; the entire contents of the aforementioned applications are incorporated herein by reference in their entireties. Several crystalline forms of Compound **(1)** and methods of preparing said forms were described in U.S. Patent No. 11,236,121; U.S. Patent Application Publication No. US 2019/0177359; and PCT Application Publication No. WO 2018/039378; the entire contents of the aforementioned applications are incorporated herein by reference in their entireties. Pharmaceutical compositions of Compound **(1)** and methods of preparing said compositions were described in PCT Application Publication No. WO 2022/020363A9 and in US Application No. 17/579,541; the entire contents of the aforementioned applications are each incorporated herein by reference in its entirety.

Compound **(1) is** a neuroactive steroid that has been shown to be a positive allosteric modulator of GABA_{A} receptors that target synaptic and extrasynaptic GABA_{A} receptors. As a positive allosteric modulator of GABA_{A} receptors, Compound **(1)** serves as a therapeutic agent to treat CNS related disorders, *e.g.*, depression, postpartum depression and major depressive disorder and to treat neurological conditions, *e.g*., essential tremor, epilepsy, and Parkinson's disease.

As used herein, "crystalline" refers to a solid phase of a given chemical entity having well-defined 3-dimensional structural order. The atoms, ions, and/or molecules are arranged in a regular, periodic manner within a repeating 3-dimensional lattice. In various embodiments, a crystalline material may comprise one or more discreet crystalline forms.

As used herein, the terms "crystalline form", "crystalline solid form," "crystal form," "solid form," and related terms refer to crystalline modifications comprising a given substance (*e.g*., Compound **(1)),** including single-component crystal forms and multiple-component crystal forms, and including, but not limited to, polymorphs, solvates, hydrates, and salts.

The term "substantially crystalline" refers to forms that may be at least a particular weight percent crystalline. Particular weight percentages may include 70%, 75%, 80%, 85%, 87%, 88%, 89%, 90%, 91 %, 92%, 93%, 94%, 95%, 96%, 97%, 98%, 99%, 99.5%, 99.9%, or any percentage between 70% and 100%. In some embodiments, the particular weight percent of crystallinity is at least 90%. In some embodiments, the particular weight percent of crystallinity is at least 95%. In some embodiments, Compound **(1)** can be a substantially crystalline sample of any of the crystalline forms described herein (*e.g*., crystalline Forms A and C) and/or PCT Application Publication No. WO 2018/039378; the entire contents of the aforementioned application are incorporated herein by reference in its entirety.

The term "substantially pure" relates to the composition of a specific crystalline form (*e.g.,* a crystalline form of Compound **(1))** that may be at least a particular weight percent free of impurities and/or other solid forms. Particular weight percentages may include 70%, 75%, 80%, 85%, 90%, 95%, 99%, or any percentage between 70% and 100%. In some embodiments, Compound **(1)** can be a substantially pure sample of any of the crystalline forms described herein, (*e.g*., crystalline Forms A and C). In some embodiments, Compound **(1)** can be substantially pure Form A. In some embodiments, Compound **(1)** can be substantially pure Form C.

As used herein, "XRPD" refers to X-ray powder diffraction. An XRPD pattern is an x-y graph with 2Q (diffraction angle) plotted on the x-axis and intensity plotted on the y-axis. These are the diffraction peaks which may be used to characterize a crystalline material. The diffraction peaks are usually represented and referred to by their position on the x-axis rather than the intensity of the diffraction peaks on the y-axis because diffraction peak intensity can be particularly sensitive to sample orientation (see Pharmaceutical Analysis, Lee & Web, pp. 255- 257 (2003)). Thus, intensity is not typically used by those of skill in the art to characterize a crystalline material. As with any data measurement, there may be variability in XRPD data. In addition to the variability in diffraction peak intensity, there may also be variability in the position of the diffraction peaks on the x-axis. This variability can, however, typically be accounted for when reporting the positions of diffraction peaks for purposes of characterization. Such variability in the position of diffraction peaks along the x-axis may be derived from several sources. One such source can be sample preparation. Samples of the same crystalline material prepared under different conditions may yield slightly different diffractograms. Factors such as particle size, moisture content, solvent content, temperature, and orientation may all affect how a sample diffracts X-rays. Another source of variability comes from instrument parameters. Different X-ray powder diffractometers operate using different parameters and may lead to slightly different diffraction patterns from the same crystalline material. Likewise, different software packages process XRPD data differently and this may also lead to variability. These and other sources of variability are known to those of ordinary skill in the art. Due to such sources of variability, the values of each X-ray diffraction peak may be preceded with the term "about" or proceeded with an appropriate range defining the experimental variability (*e.g*., ± 0.1°, ± 0.2°, ± 0.3°, ± 0.4°, ± 0.5°, etc.).

The term "characteristic peaks" when referring to the peaks in an XRPD pattern of a crystalline form of a given chemical entity (*e.g.,* a crystalline form of Compound **(1))** refers to a collection of specific diffraction peaks whose values span a range of 2θ values (*e.g*., 0° to 40°) that are, as a whole, unique to that specific crystalline form.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: **(1)** acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counter-ion of an acidic functional group. Such cations are exemplified by sodium, potassium, calcium, magnesium, ammonium, tetraalkylammonium cations, and the like. See, e.g., Berge, et al., J. Pharm. Sci. (1977) 66(1): 1-79.

The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March' s Advanced Organic Chemistry, 5th Edition, John Wiley & Sons, Inc., New York, 2001; Larock, Comprehensive Organic Transformations,VCH Publishers, Inc., New York, 1989; and Carruthers, Some Modern Methods of Organic Synthesis, 3rd Edition, Cambridge University Press, Cambridge, 1987.

Where the use of the term "about" is before a quantitative value, the present teachings also include the specific quantitative value itself, unless specifically stated otherwise. As used herein, the term "about" refers to a ±10% variation from the nominal value unless otherwise indicated or inferred.

The terms "disease", "disorder", and "condition" are used interchangeably herein.

As used herein, the term "dose equivalent" means a bioequivalent dose. For example, the dose equivalent of a pharmaceutically acceptable salt of Compound **(1)** for a 50 mg dose of Compound **(1)** is the amount of the pharmaceutically acceptable salt (by weight) needed to provide a bioequivalent dose to the 50 mg dose of the free base of Compound **(1).**

As used herein, an "effective amount" of a compound (or pharmaceutically acceptable salt thereof) refers to an amount sufficient to elicit the desired biological response, *e.g.,* to treat a CNS-related disorder, *e.g.,* depression, *e.g.,* major depressive disorder (MDD) with elevated anxiety or postpartum depression (PPD) with elevated anxiety. As will be appreciated by those of ordinary skill in this art, the effective amount of a compound (or pharmaceutically acceptable salt thereof) of the invention may vary depending on such factors as the desired biological endpoint, the pharmacokinetics of the compound, the disease being treated, the mode of administration, and the age, weight, health, and condition of the subject. An effective amount encompasses therapeutic and prophylactic treatment.

As used herein, an "episodic dosing regimen" is a dosing regimen wherein a compound or a composition comprising a compound is administered to a subject for a finite period of time in response to the diagnosis of a disorder or symptom thereof, *e.g.,* a diagnosis or symptom of depression or an episode of major depressive disorder. In some embodiments, the major depressive disorder is moderate major depressive disorder. In some embodiments, the major depressive disorder is severe major depressive disorder. In some embodiments, the compound is formulated as individual dosage units, each unit comprising Compound **(1)** and one or more suitable pharmaceutical excipient. In some embodiments, the episodic dosing regimen has a duration of a plurality of weeks, *e.g*., about 8 weeks. In contrast with chronic administration as defined herein, episodic dosing of a compound occurs over a finite period of time, *e.g.,* from about 2 weeks to about 8 weeks, in response to a diagnosis or recurrence of a disorder, *e.*g., depression, or a symptom thereof. In some embodiments, episodic dosing occurs once per day across a plurality of weeks, *e.g*., from about 2 weeks to about 6 weeks. In one embodiment, the episodic dosing has a duration of two weeks. In some embodiments, more than one episodic dosing regimen, but no more than 3 episodic dosing regimens, is administered to the subject, *e.g*., two or more episodic regimens over a period of 12 months.

As used herein, the term "modulation" refers to the inhibition or potentiation of GABA_{A} receptor function. A "modulator" (*e.g.,* a compound or pharmaceutically acceptable salt thereof that modulates GABA_{A} receptor function) may be, for example, an agonist, partial agonist, antagonist, or partial antagonist of the GABA_{A} receptor.

"MDD with elevated anxiety" or "MDD with anxious distress" are used interchangeably and refer to subjects with MDD who present elevated anxiety as a symptom of their depression. In some embodiments, MDD with elevated anxiety is characterized by a HAM-D Anxiety/Somatization Subscale score of at least 7 at baseline (i.e. prior to administration of Compound (1) or a pharmaceutically acceptable salt thereof). In some embodiments, MDD with elevated anxiety is characterized by a HAM-A total score of at least 17 at baseline (i.e. prior to administration of Compound (1) or a pharmaceutically acceptable salt thereof). In some embodiments, MDD with elevated anxiety is characterized by a HAM-A total score of at least 18 at baseline. In some embodiments, MDD with elevated anxiety is characterized by a HAM-A total score of at least 20 at baseline. "PPD with elevated anxiety" or "PPD with anxious distress" are used interchangeably and refer to subjects with PPD who present elevated anxiety as a symptom of their depression. In some embodiments, PPD with elevated anxiety is characterized by a HAM-D Anxiety/Somatization Subscale score of at least 7 at baseline (i.e. prior to administration of Compound (1) or a pharmaceutically acceptable salt thereof). In some embodiments, PPD with elevated anxiety is characterized by a HAM-A total score of at least 17 at baseline (i.e. prior to administration of Compound (1) or a pharmaceutically acceptable salt thereof). In some embodiments, PPD with elevated anxiety is characterized by a HAM-A total score of at least 18 at baseline. In some embodiments, PPD with elevated anxiety is characterized by a HAM-A total score of at least 20 at baseline.

In other embodiments, "elevated anxiety" is characterized by a HAM-A score based on the HAM-A anxiety items and somatic items. In some embodiments, "elevated anxiety" is characterized by a HAM-A score based on the HAM-A anxiety items. In some embodiments, "elevated anxiety" is characterized by a HAM-D score based on the following HAM-D items: psychic anxiety, somatic anxiety, GI somatic symptoms, and/or general somatic symptoms. In some embodiments, "elevated anxiety" is characterized by a HAM-D score based on the following HAM-D item: psychic anxiety. In some embodiments, "elevated anxiety" is characterized by a HAM-D score based predominately on the items evaluating somatic symptoms of depression. In some embodiments, "elevated anxiety" is characterized by a HAM-D score based predominately on the items evaluating anxiety symptoms of depression. In some embodiments, "elevated anxiety" is characterized by a HAM-D Anxiety/Somatization Subscale score based predominately on the items evaluating somatic symptoms of depression. In some embodiments, "elevated anxiety" is characterized by a HAM-D Anxiety/Somatization Subscale score based predominately on the items evaluating anxiety symptoms of depression. In some embodiments, "elevated anxiety" is characterized by a MADRS score based predominately on the items evaluating the somatic symptoms of depression. In some embodiments, "elevated anxiety" is characterized by a MADRS score based predominately on the items evaluating the anxiety symptoms of depression.

As used herein, and unless otherwise specified, a "therapeutically effective amount" of a compound (or pharmaceutically acceptable salt thereof) is an amount sufficient to provide a therapeutic benefit in the treatment of a disease, disorder or condition, or to delay or minimize one or more symptoms associated with the disease, disorder or condition. A therapeutically effective amount of a compound (or pharmaceutically acceptable salt thereof) means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

In an alternate embodiment, the present disclosure contemplates administration of Compound **(1)** or a pharmaceutically acceptable salt or a pharmaceutically acceptable composition thereof, as a prophylactic before a subject begins to suffer from the specified disease, disorder or condition. As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, "solid dosage form" means a pharmaceutical dose(s) in solid form, e.g., tablets, capsules, granules, powders, sachets, reconstitutable powders, dry powder inhalers and chewables.

A "subject" or "patient" is a human (*e.g.,* a male or female of any age group, *e.g.,* a pediatric subject (*e.g.,* infant, child, adolescent) or adult subject (*e.g.,* young adult, middle-aged adult or senior adult)).

As used herein, and unless otherwise specified, the terms "treat," "treating" and "treatment" contemplate an action that occurs while a subject is suffering from the specified disease, disorder or condition, which reduces the severity of the disease, disorder or condition (or any symptom thereof), or retards or slows the progression of the disease, disorder or condition ("therapeutic treatment"), and also contemplates a prophylactic action that occurs before a subject begins to suffer from the specified disease, disorder or condition.

As used herein, "treatment naïve" refers to a subject that has not been previously treated with the additional antidepressant within the current depressive episode. "Treatment naive" also refers to a subject that has not taken any antidepressant within at least 30 days prior or within at least 60 days prior to the start of treatment (*e.g*., Day 1). In some embodiments, the treatment naive subject has not taken any antidepressant within at least 30 days prior to the start of treatment. In some embodiments, the treatment naive subject has not taken any antidepressant within at least 60 days prior to the start of treatment.

As used herein, the term "unit dosage form" is defined to refer to the form in which Compound **(1)** is administered to the subject. In some embodiments, the unit dosage form can be, for example, a pill, capsule, or tablet. In some embodiments, the unit dosage form is a capsule. In some embodiments, the typical amount of Compound **(1)** in a unit dosage form useful in the disclosure is about 10 mg to about 100 mg, about 20 mg to about 55 mg, or about 30 mg to about 50 mg (*e.g.,* about, 10 mg, about 15 mg, about 20 mg, about 25 mg, about 30 mg, about 35 mg, about 40 mg, about 45 mg, about 50 mg, or about 55 mg).

In some embodiments, the unit dosage form comprises about 40 mg of Compound **(1)** and is in the form of a capsule. In other embodiments, the unit dosage form comprises about 50 mg Compound **(1)** and is in the form of a capsule. In another embodiment, the unit dosage form comprises about 45 mg Compound **(1)** and is in the form of a capsule. In some embodiments, capsules which comprise about 40 mg, about 45 mg, or about 50 mg of Compound **(1)** are administered to a subject once per day. In some embodiments, two or more capsules together comprise the 40 mg of Compound **(1).** In some embodiments, two or more capsules together comprises the 45 mg of Compound **(1).** In some embodiments, two or more capsules together comprises the 50 mg of Compound **(1)**

In other embodiments, the unit dosage form comprises about 20 mg of Compound **(1)** and is in the form of a capsule. In other embodiments, the unit dosage form comprises about 10 mg of Compound **(1)** and is in the form of a capsule. In other embodiments, the unit dosage form comprises about 15 mg of Compound **(1)** and is in the form of a capsule. In other embodiments, the unit dosage form comprises about 25 mg of Compound **(1)** and is in the form of a capsule. In some embodiments, one or more capsules that comprise about 30 mg or 45 mg of Compound **(1),** are administered to a subject once per day. In some embodiments, three capsules together comprise the 30 mg of Compound **(1).** In some embodiments, three capsules together comprises the 45 mg of Compound **(1).**

In some embodiments, administering Compound **(1)** improves cognitive function. In some embodiments, the cognitive function refers to a collection of mental tasks and functions, including but not limited to: memory (*e*.*g*., semantic, episodic, procedural, priming, or working); orientation; language; problem solving; visual perception, construction, and integration; planning; organizational skills; selective attention; inhibitory control; and ability to mentally manipulate information. In one embodiment, the cognitive function is one or more selected from the group consisting of memory (*e.g*., semantic, episodic, procedural, priming, or working); orientation; language; problem solving; visual perception, construction, and integration; planning; organizational skills; selective attention; inhibitory control; and ability to mentally manipulate information. Measures of cognitive functioning include assessment tools designed to measure, for example: (a) general intelligence, (b) nonverbal intelligence, (c) achievement, (d) attention/executive functioning, (e) memory and learning, (f) visual-motor and motor functioning and (g) language.

Any change in cognitive function, for example, over time or through treatment, can be monitored by using one or more of these well-established tests at two or more time points and comparing the results. The phrase "improves cognitive function", as referred to herein, means a positive change in the ability of the subject to perform a symbolic operation, for example, to perceive, remember, create a mental image, have clarity of thought, be aware, to reason, think or judge. The positive change can be measured using any of the aforementioned tests on two or more occasions, for example, a first occasion to measure baseline cognitive function and a second occasion to measure cognitive function following a period of time (in which treatment may have been administered).

### II. METHODS OF TREATMENT

### MDD with Elevated Anxiety

In one aspect, the present disclosure is directed to methods of treating major depressive disorder (MDD) with elevated anxiety. The diagnosis and severity of the major depressive disorder treated by the methods described herein can be characterized as defined by the Diagnostic and Statistical Manual of Mental Disorders, 5th Edition (DSM-5).

### Depressive Disorders

Depressive disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder. The common feature of all of these disorders is the presence of sad, empty, or irritable mood, accompanied by somatic and cognitive changes that significantly affect the individual's capacity to function. What differs among them are issues of duration, timing, or presumed etiology.

Major depressive disorder represents the classic condition in this group of disorders. It is characterized by discrete episodes of at least 2 weeks' duration (although most episodes last considerably longer) involving clear-cut changes in affect, cognition, and neurovegetative functions and inter-episode remissions. A discrete episode of major depressive disorder may be referred to as a "major depressive episode" or "depressive episode".

### Major Depressive Disorder (MDD)

Major depressive disorder is generally known in the art.

In some embodiments, MDD is also known as depression or clinical depression and it is a mood disorder that causes a persistent feeling of sadness and loss of interest. MDD affects how a subject may feel, think, and behave, and can lead to a variety of emotional and physical problems.

In some embodiments, MDD is defined and diagnosed according to the DSM-5, for example, MDD is diagnosed according to Criterion A, as described below.

**Criterion A.** Five (or more) of the following symptoms have been present during the same 2-week period and represent a change from previous functioning; at least one of the symptoms is either (1) depressed mood or (2) loss of interest or pleasure.
1. Depressed mood most of the day, nearly every day, as indicated by either subjective report (*e.g.,* feels sad, empty, hopeless) or observation made by others (*e.g.,* appears tearful). (Note: In children and adolescents, can be irritable mood.)
2. Markedly diminished interest or pleasure in all, or almost all, activities most of the day, nearly every day (as indicated by either subjective account or observation).
3. Significant weight loss when not dieting or weight gain (*e.g.,* a change of more than 5% of body weight in a month), or decrease or increase in appetite nearly every day (Note: In children, consider failure to make expected weight gain.)
4. Insomnia or hypersomnia nearly every day.
5. Psychomotor agitation or retardation nearly every day (observable by others, not merely subjective feelings of restlessness or being slowed down).
6. Fatigue or loss of energy nearly every day.
7. Feelings of worthlessness or excessive or inappropriate guilt (which may be delusional) nearly every day (not merely self-reproach or guilt about being sick).
8. Diminished ability to think or concentrate, or indecisiveness, nearly every day (either by subjective account or as observed by others).
9. Recurrent thoughts of death (not just fear of dying), recurrent suicidal ideation without a specific plan, or a suicide attempt or a specific plan for committing suicide.

Criteria B-E, described below, are additional descriptions of MDD and may be considered for describing or diagnosing MDD, but are not required.

**Criterion B.** The symptoms cause clinically significant distress or impairment in social, occupational, or other important areas of functioning.

**Criterion C.** The episode is not attributable to the physiological effects of a substance or to another medical condition.

Criteria A-C can represent a major depressive episode.

**Criterion D.** The occurrence of the major depressive episode is not better explained by schizoaffective disorder, schizophrenia, schizophreniform disorder, delusional disorder, or other specified and unspecified schizophrenia spectrum and other psychotic disorders.

**Criterion E.** There has never been a manic episode or a hypomanic episode.

In some embodiments, a major depressive episode (MDE) is a period characterized by the symptoms of MDD as described above.

In some embodiments, MDD is a clinical course that is characterized by one or more major depressive episodes (MDE) in a subject.

In some embodiments, MDD is diagnosed according to Criteria A-C, as described above. In some embodiments, MDD is diagnosed according to Criteria A-E, as described above.

### Diagnostic Features

The criterion symptoms for major depressive disorder must be present nearly every day to be considered present, with the exception of weight change and suicidal ideation. Depressed mood must be present for most of the day, in addition to being present nearly every day. Often insomnia or fatigue is the presenting complaint, and failure to probe for accompanying depressive symptoms will result in underdiagnosis. Sadness may be denied at first but may be elicited through interview or inferred from facial expression and demeanor. With individuals who focus on a somatic complaint, clinicians should determine whether the distress from that complaint is associated with specific depressive symptoms. Fatigue and sleep disturbance are present in a high proportion of cases; psychomotor disturbances are much less common but are indicative of greater overall severity, as is the presence of delusional or near-delusional guilt.

The essential feature of a major depressive episode is a period of at least 2 weeks during which there is either depressed mood or the loss of interest or pleasure in nearly all activities (Criterion A above). In children and adolescents, the mood may be irritable rather than sad. The individual must also experience at least four additional symptoms drawn from a list that includes changes in appetite or weight, sleep, and psychomotor activity; decreased energy; feelings of worthlessness or guilt; difficulty thinking, concentrating, or making decisions; or recurrent thoughts of death or suicidal ideation or suicide plans or attempts. To count toward a major depressive episode, a symptom must either be newly present or must have clearly worsened compared with the person's pre-episode status. The symptoms must persist for most of the day, nearly every day, for at least 2 consecutive weeks. The episode must be accompanied by clinically significant distress or impairment in social, occupational, or other important areas of functioning. For some individuals with mild episodes, functioning may appear to be normal but requires markedly increased effort.

Sleep disturbance may take the form of either difficulty sleeping or sleeping excessively (Criterion A4). When insomnia is present, it typically takes the form of middle insomnia (i.e., waking up during the night and then having difficulty returning to sleep) or terminal insomnia (i.e., waking too early and being unable to return to sleep). Initial insomnia (i.e., difficulty falling asleep) may also occur. Individuals who present with over-sleeping (hypersomnia) may experience prolonged sleep episodes at night or increased daytime sleep. Sometimes the reason that the individual seeks treatment is for the disturbed sleep.

### Major Depressive Disorder with Elevated Anxiety/Anxious Distress

The "anxious distress" identifier for MDD, as defined by the DSM-5, indicates the presence of at least two of the following symptoms during the majority of days of a major depressive episode or persistent depressive disorder (dysthymia):
1. Feeling keyed up or tense.
2. Feeling unusually restless.
3. Difficulty concentrating because of worry.
4. Fear that something awful may happen.
5. Feeling that the individual might lose control of himself or herself.

"Anxious distress" and "elevated anxiety" are used interchangeably herein.

Severity is defined as:
Mild: Two symptoms.
Moderate: Three symptoms.
Moderate-severe: Four or five symptoms.
Severe: Four or five symptoms and with motor agitation.

Anxious distress has been noted as a prominent feature of both bipolar and major depressive disorder in both primary care and specialty mental health settings. High levels of anxiety have been associated with higher suicide risk, longer duration of illness, and greater likelihood of treatment nonresponse.

Accordingly, one aspect of the present disclosure is directed to a method of treating major depressive disorder (MDD) with elevated anxiety in a subject in need thereof, comprising administering a therapeutically effective amount of Compound **(1):**

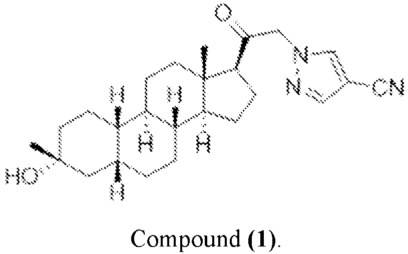

Another aspect of the disclosure provides a method of treating major depressive disorder (MDD) with elevated anxiety in a subject in need thereof, comprising administering a therapeutically effective amount of a pharmaceutically acceptable salt of Compound **(1):**

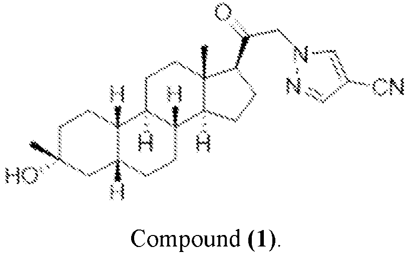

In some embodiments, Compound **(1),** or the pharmaceutically acceptable salt of Compound **(1),** is administered once a day for about 14 days or about 2 weeks. In some embodiments, Compound **(1),** or the pharmaceutically acceptable salt of Compound **(1),** is administered once a day for about 14 days. In some embodiments, Compound **(1),** or the pharmaceutically acceptable salt of Compound **(1),** is administered once a day for about 2 weeks.

In some embodiments, Compound **(1)** is administered at a dose of about 10 mg to about 100 mg. In some embodiments, Compound **(1)** is administered at a dose of about 15 mg to about 75 mg. In some embodiments, Compound **(1)** is administered at a dose of about 20 mg to about 60 mg. In some embodiments, Compound **(1)** is administered at a dose of about 20 mg to about 55 mg. In some embodiments, Compound **(1)** is administered at a dose of about 30 mg to about 50 mg. In some embodiments, Compound **(1)** is administered at a dose of about 45 mg to about 55 mg. In some embodiments, Compound **(1)** is administered at a dose of about 20 mg, about 25 mg, about 30 mg, about 35 mg, about 40 mg, about 45 mg, about 50 mg, about 55 mg, or about 60 mg. In some embodiments, Compound **(1)** is administered at a dose of about 50 mg. In some embodiments, Compound **(1)** is administered at a dose of about 40 mg. In some embodiments, Compound **(1)** is administered at a dose of about 30 mg.

In some embodiments, Compound **(1)** is administered at a dose of about 10 mg to about 100 mg once a day. In some embodiments, Compound **(1)** is administered at a dose of about 15 mg to about 75 mg once a day. In some embodiments, Compound **(1)** is administered at a dose of about 20 mg to about 60 mg once a day. In some embodiments, Compound **(1)** is administered at a dose of about 20 mg to about 55 mg once a day. In some embodiments, Compound **(1)** is administered at a dose of about 30 mg to about 50 mg once a day. In some embodiments, Compound **(1)** is administered at a dose of about 45 mg to about 55 mg once a day. In some embodiments, Compound **(1)** is administered at a dose of about 20 mg, about 25 mg, about 30 mg, about 35 mg, about 40 mg, about 45 mg, about 50 mg, about 55 mg, or about 60 mg once a day. In some embodiments, Compound **(1)** is administered at a dose of about 50 mg once a day. In some embodiments, Compound **(1)** is administered at a dose of about 40 mg once a day. In some embodiments, Compound **(1)** is administered at a dose of about 30 mg once a day.

In some embodiments, Compound **(1)** is administered at a dose of about 20 mg to about 55 mg once a day for about 2 weeks or about 14 days. In some embodiments, Compound **(1)** is administered at a dose of about 30 mg to about 50 mg once a day for about 2 weeks or about 14 days. In some embodiments, Compound **(1)** is administered at a dose of about 45 mg to about 55 mg once a day for about 2 weeks or about 14 days. In some embodiments, Compound **(1)** is administered at a dose of about 50 mg once a day for less than 2 weeks. In some embodiments, Compound **(1)** is administered at a dose of about 50 mg once a day for about 2 weeks. In some embodiments, Compound **(1)** is administered at a dose of about 50 mg once a day for about 14 days. In some embodiments, Compound **(1)** is administered at a dose of about 40 mg once a day for less than 2 weeks. In some embodiments, Compound **(1)** is administered at a dose of about 40 mg once a day for about 2 weeks. In some embodiments, Compound **(1)** is administered at a dose of about 40 mg once a day for about 14 days. In some embodiments, Compound **(1)** is administered at a dose of about 30 mg once a day for less than 2 weeks. In some embodiments, Compound **(1)** is administered at a dose of about 30 mg once a day for about 2 weeks. In some embodiments, Compound **(1)** is administered at a dose of about 30 mg once a day for about 14 days.

In other embodiments, the pharmaceutically acceptable salt of Compound **(1)** is administered at a dose equivalent of about 10 mg to about 100 mg of the free base compound. In some embodiments, the pharmaceutically acceptable salt of Compound **(1)** is administered at a dose equivalent of about 15 mg to about 75 mg of the free base compound. In some embodiments, the pharmaceutically acceptable salt of Compound **(1)** is administered at a dose equivalent of about 20 mg to about 60 mg of the free base compound. In some embodiments, the pharmaceutically acceptable salt of Compound **(1)** is administered at a dose equivalent of about 20 mg to about 55 mg of the free base compound. In some embodiments, the pharmaceutically acceptable salt of Compound **(1)** is administered at a dose equivalent of about 30 mg to about 50 mg of the free base compound. In some embodiments, the pharmaceutically acceptable salt of Compound **(1)** is administered at a dose equivalent of about 45 mg to about 55 mg of the free base compound. In some embodiments, the pharmaceutically acceptable salt of Compound **(1)** is administered at a dose equivalent of about 20 mg, about 25 mg, about 30 mg, about 35 mg, about 40 mg, about 45 mg, about 50 mg, about 55 mg, or about 60 mg of the free base compound. In some embodiments, the pharmaceutically acceptable salt of Compound **(1)** is administered at a dose equivalent of about 50 mg of the free base compound. In some embodiments, the pharmaceutically acceptable salt of Compound **(1)** is administered at a dose equivalent of about 40 mg of the free base compound. In some embodiments, the pharmaceutically acceptable salt of Compound **(1)** is administered at a dose equivalent of about 30 mg of the free base compound.

In some embodiments, the pharmaceutically acceptable salt of Compound **(1)** is administered at a dose equivalent of about 10 mg to about 100 mg of the free base compound once a day. In some embodiments, the pharmaceutically acceptable salt of Compound **(1)** is administered at a dose equivalent of about 15 mg to about 75 mg of the free base compound once a day. In some embodiments, the pharmaceutically acceptable salt of Compound **(1)** is administered at a dose equivalent of about 20 mg to about 60 mg of the free base compound once a day. In some embodiments, the pharmaceutically acceptable salt of Compound **(1)** is administered at a dose equivalent of about 20 mg to about 55 mg of the free base compound once a day. In some embodiments, the pharmaceutically acceptable salt of Compound **(1)** is administered at a dose equivalent of about 30 mg to about 50 mg of the free base compound once a day. In some embodiments, the pharmaceutically acceptable salt of Compound **(1)** is administered at a dose equivalent of about 45 mg to about 55 mg of the free base compound once a day. In some embodiments, the pharmaceutically acceptable salt of Compound **(1)** is administered at a dose equivalent of about 20 mg, about 25 mg, about 30 mg, about 35 mg, about 40 mg, about 45 mg, about 50 mg, about 55 mg, or about 60 mg of the free base compound once a day. In some embodiments, the pharmaceutically acceptable salt of Compound **(1)** is administered at a dose equivalent of about 50 mg of the free base compound once a day. In some embodiments, the pharmaceutically acceptable salt of Compound **(1)** is administered at a dose equivalent of about 40 mg of the free base compound once a day. In some embodiments, the pharmaceutically acceptable salt of Compound **(1)** is administered at a dose equivalent of about 30 mg of the free base compound once a day.

In some embodiments, the pharmaceutically acceptable salt of Compound **(1)** is administered at a dose equivalent of about 20 mg to about 55 mg of the free base compound once a day for about 2 weeks or about 14 days. In some embodiments, the pharmaceutically acceptable salt of Compound **(1)** is administered at a dose equivalent of about 30 mg to about 50 mg of the free base compound once a day for about 2 weeks or about 14 days. In some embodiments, the pharmaceutically acceptable salt of Compound **(1)** is administered at a dose equivalent of about 45 mg to about 55 mg of the free base compound once a day for about 2 weeks or about 14 days. In some embodiments, the pharmaceutically acceptable salt of Compound **(1)** is administered at a dose equivalent of about 50 mg of the free base compound once a day for less than 2 weeks. In some embodiments, the pharmaceutically acceptable salt of Compound **(1)** is administered at a dose equivalent of about 50 mg of the free base compound once a day for about 2 weeks. In some embodiments, the pharmaceutically acceptable salt of Compound **(1)** is administered at a dose equivalent of about 50 mg of the free base compound once a day for about 14 days. In some embodiments, the pharmaceutically acceptable salt of Compound **(1)** is administered at a dose equivalent of about 40 mg of the free base compound once a day for less than 2 weeks. In some embodiments, the pharmaceutically acceptable salt of Compound **(1)** is administered at a dose equivalent of about 40 mg of the free base compound once a day for about 2 weeks. In some embodiments, the pharmaceutically acceptable salt of Compound **(1)** is administered at a dose equivalent of about 40 mg of the free base compound once a day for about 14 days. In some embodiments, the pharmaceutically acceptable salt of Compound **(1)** is administered at a dose equivalent of about 30 mg of the free base compound once a day for less than 2 weeks. In some embodiments, the pharmaceutically acceptable salt of Compound **(1)** is administered at a dose equivalent of about 30 mg of the free base compound once a day for about 2 weeks. In some embodiments, the pharmaceutically acceptable salt of Compound **(1)** is administered at a dose equivalent of about 30 mg of the free base compound once a day for about 14 days.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally.

In some embodiments, Compound **(1),** or the pharmaceutically acceptable salt of Compound **(1),** is administered chronically.

In some embodiments, Compound **(1),** or the pharmaceutically acceptable salt of Compound **(1),** is administered in one or more capsules. In some embodiments, the therapeutically effective amount is administered across two capsules. In some embodiments, the therapeutically effective amount is administered across three capsules.

In some embodiments, Compound **(1),** or the pharmaceutically acceptable salt of Compound **(1),** is administered with food. In some embodiments, Compound **(1),** or the pharmaceutically acceptable salt of Compound **(1),** is administered with fat-containing food. Examples of fat-containing food include nuts, peanut butter, avocado, eggs, and cheese. In some embodiments, Compound **(1),** or the pharmaceutically acceptable salt of Compound **(1),** is administered at night with fat-containing food (*e.g*., within 1 hour of an evening meal which contains fat, or with a fat-containing snack).

In some embodiments, the subject is administered Compound **(1),** or the pharmaceutically acceptable salt of Compound **(1),** at night. In some embodiments, the subject is administered Compound **(1),** or the pharmaceutically acceptable salt of Compound **(1),** no later than 1 hour before the patient sleeps. In some embodiments, the subject is administered Compound **(1),** or the pharmaceutically acceptable salt of Compound **(1),** no later than 15 minutes before the patient sleeps. In some embodiments, the subject is administered Compound **(1),** or the pharmaceutically acceptable salt of Compound **(1),** once a day at night. In some embodiments, the subject is administered Compound **(1),** or the pharmaceutically acceptable salt of Compound **(1),** once a day no later than 1 hour before the patient sleeps. In some embodiments, the subject is administered Compound **(1),** or the pharmaceutically acceptable salt of Compound **(1),** once a day no later than 15 minutes before the patient sleeps.

In some embodiments, Compound **(1)** is in a crystalline form. In some embodiments, the crystalline form of Compound **(1)** is any crystalline form disclosed in PCT Application Publication No. WO 2018/039378; the entire contents of the aforementioned application are incorporated herein by reference in its entirety.

In some embodiments, Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ. In some embodiments, Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ. In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.

In some embodiments, the crystalline form of Compound **(1)** comprises a mixture of two or more crystalline forms.

In some embodiments, Compound **(1),** or the pharmaceutically acceptable salt of Compound **(1),** is re-administered to the subject in response to a recurrence of depression symptoms after completion of the initial treatment. In some embodiments, there is at least a 6 week interval between the last dose of the initial treatment and the first dose of the re-administration. In some embodiments, each of the initial treatment and re-administration occurs for about 14 days or about 2 weeks.

In some embodiments, the method administers a second therapeutic agent.

In some embodiments, the subject is treatment naive. In some embodiments, the subject has not received any antidepressant treatment within at least 30 days prior to the administration of Compound **(1),** or the pharmaceutically acceptable salt of Compound **(1).** In some embodiments, the subject has not received any antidepressant treatment within at least 60 days prior to the administration of Compound **(1),** or the pharmaceutically acceptable salt of Compound **(1).**

In some embodiments, the subject has been on a stable dose of an additional antidepressant for at least 30 days or for at least 60 days prior to the administration of Compound **(1),** or the pharmaceutically acceptable salt of Compound **(1).** In some embodiments, the subject has been on a stable dose of an additional antidepressant for at least 60 days prior to the administration of Compound **(1),** or the pharmaceutically acceptable salt of Compound **(1).** In some embodiments, the subject has been on a stable dose of an additional antidepressant for at least 30 days prior to the administration of Compound **(1),** or the pharmaceutically acceptable salt of Compound **(1).**

In some embodiments, MDD with elevated anxiety is characterized by a Hamilton Rating Scale for Anxiety (HAM-A) total score of 17 or greater, 18 or greater, 19 or greater, or 20 or greater. In some embodiments, MDD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).** In some embodiments, MDD with elevated anxiety is characterized by a Hamilton Rating Scale for Anxiety (HAM-A) total score of 17 or greater. In some embodiments, MDD with elevated anxiety is characterized by a Hamilton Rating Scale for Anxiety (HAM-A) total score of 18 or greater. In some embodiments, MDD with elevated anxiety is characterized by a Hamilton Rating Scale for Anxiety (HAM-A) total score of 19 or greater. In some embodiments, MDD with elevated anxiety is characterized by a Hamilton Rating Scale for Anxiety (HAM-A) total score of 20 or greater. In some embodiments, MDD with elevated anxiety is characterized by a Hamilton Rating Scale for Depression (HAM-D) Anxiety/Somatization subscale score of 7 or greater.

In some embodiments, MDD with elevated anxiety is characterized by a HAM-D total score of 24 or greater and a HAM-A total score of 17 or greater prior to the administration of Compound **(1),** or the pharmaceutically acceptable salt of Compound **(1).** In some embodiments, MDD with elevated anxiety is characterized by a HAM-D total score of 24 or greater and a HAM-A total score of 18 or greater prior to the administration of Compound **(1),** or the pharmaceutically acceptable salt of Compound **(1).** In some embodiments, MDD with elevated anxiety is characterized by a HAM-D total score of 24 or greater and a HAM-A total score of 19 or greater prior to the administration of Compound **(1),** or the pharmaceutically acceptable salt of Compound **(1).** In some embodiments, MDD with elevated anxiety is characterized by a HAM-D total score of 24 or greater and a HAM-A total score of 20 or greater prior to the administration of Compound **(1),** or the pharmaceutically acceptable salt of Compound **(1).** In some embodiments, MDD with elevated anxiety is characterized by a HAM-D total score of 24 or greater and a HAM-D Anxiety/Somatization subscale score of 7 or greater prior to the administration of Compound **(1),** or the pharmaceutically acceptable salt of Compound **(1).**

In some embodiments, MDD with elevated anxiety is characterized by a HAM-D total score of 20 or greater, a MADRS total score of 28 or greater, and a HAM-A total score of 17 or greater (*e.g.,* 18 or greater, 19 or greater, or 20 or greater) prior to the administration of Compound **(1),** or the pharmaceutically acceptable salt of Compound **(1).** In some embodiments, MDD with elevated anxiety is characterized by a HAM-D total score of 20 or greater, a MADRS total score of 28 or greater, and a HAM-D Anxiety/Somatization subscale score of 7 or greater prior to the administration of Compound **(1),** or the pharmaceutically acceptable salt of Compound **(1).**

In other embodiments, "elevated anxiety" is characterized by a HAM-A score based on the HAM-A anxiety items and somatic items. In some embodiments, "elevated anxiety" is characterized by a HAM-A score based on the HAM-A anxiety items. In some embodiments, "elevated anxiety" is characterized by a HAM-D score based on the following HAM-D items: psychic anxiety, somatic anxiety, GI somatic symptoms, and/or general somatic symptoms. In some embodiments, "elevated anxiety" is characterized by a HAM-D score based on the following HAM-D item: psychic anxiety. In some embodiments, "elevated anxiety" is characterized by a HAM-D score based predominately on the items evaluating somatic symptoms of depression. In some embodiments, "elevated anxiety" is characterized by a HAM-D score based predominately on the items evaluating anxiety symptoms of depression. In some embodiments, "elevated anxiety" is characterized by a HAM-D Anxiety/Somatization Subscale score based predominately on the items evaluating somatic symptoms of depression. In some embodiments, "elevated anxiety" is characterized by a HAM-D Anxiety/Somatization Subscale score based predominately on the items evaluating anxiety symptoms of depression. In some embodiments, "elevated anxiety" is characterized by a MADRS score based predominately on the items evaluating the somatic symptoms of depression. In some embodiments, "elevated anxiety" is characterized by a MADRS score based predominately on the items evaluating the anxiety symptoms of depression.

In some embodiments, the subject exhibits a reduction in HAM-D total score, HAM-A total score, HAM-D Anxiety/Somatization subscale score, or a combination thereof, from baseline. In some embodiments, the subject exhibits a reduction of at least 14 points in HAM-D total score on Day 15 after administration of Compound **(1),** or the pharmaceutically acceptable salt of Compound **(1).** In some embodiments, the subject exhibits a reduction of at least 12 points in HAM-A total score on Day 15 after administration of Compound **(1),** or the pharmaceutically acceptable salt of Compound **(1).**

In some embodiments, the method provides therapeutic effect *(e.g.,* as measured by reduction in Hamilton Depression Total Score (HAM-D)) within about 45, about 21, about 15, about 8, or about 3 days. In some embodiments, the therapeutic effect is a decrease from baseline in HAM-D total score at the end of a treatment period (*e.g.*, about 45, about 21, about 15, about 8, or about 3 days after beginning administration of Compound **(1),** or the pharmaceutically acceptable salt of Compound **(1)).** In some embodiments, the decrease from baseline in HAM-D total score is from severe (*e.g*., HAM-D total score of 24 or greater; or a score of 26 or greater) to symptom-free, *i.e.* remission of depression (*e.g.,* HAM-D total score of 7 or lower). In some embodiments, the decrease from baseline in HAM-D total score is from severe (*e.g*., HAM-D total score of 24 or greater; or a total score of 26 or greater) to normal or mild depression (*e.g*., HAM-D total score of 7 or lower; or HAM-D total score of 18-13).

In some embodiments, the method provides therapeutic effect (*e.g.,* as measured by reduction in Hamilton Depression Subscale Scores (HAM-D subscale)) within about 45, about 21, about 15, about 8, or about 3 days. In some embodiments, the HAM-D subscale scores are Core Depression, Bech-6, Maier, and/or Anxiety scores. In some embodiments, the HAM-D Core Depression subscale score LS mean decrease from baseline at day 15 is at least about 1-3. In some embodiments, the HAM-D Bech-6 subscale score LS mean decrease from baseline at day 15 is at least about 3. In some embodiments, the HAM-D Maier subscale score LS mean decrease from baseline at day 15 is at least about 2.5. In some embodiments, the HAM-D anxiety subscale score LS mean decrease from baseline at day 15 is at least about 0.5.

In some embodiments, the method provides therapeutic effect (*e.g.,* as measured by reduction in Montgomery-Asberg Depression Rating Scale (MADRS)) within about 45, about 21, about 15, about 8, or about 3 days or less. The Montgomery-Åsberg Depression Rating Scale (MADRS) is a ten-item diagnostic questionnaire (regarding apparent sadness, reported sadness, inner tension, reduced sleep, reduced appetite, concentration difficulties, lassitude, inability to feel, pessimistic thoughts, and suicidal thoughts) which psychiatrists use to measure the severity of depressive episodes in patients with mood disorders. 0-6 indicates normal/symptom absent; 7-19 indicates mild depression; 20-34 indicates moderate depression; and >34 indicates severe depression. In some embodiments, the therapeutic effect is a decrease from baseline in MADRS score at the end of a treatment period (*e.g*., about 45, about 21, about 15, about 8, or about 3 days or less). In some embodiments, the decrease from baseline in MADRS score is from severe (*e.g.,* MADRS score of 30 or greater) to symptom-free (*e.g.,* MADRS score of 20 or lower). For example, the mean change from baseline in MADRS total score from treatment with Compound **(1),** or the pharmaceutically acceptable salt of Compound **(1)** is about -15, -20, -25, -30, while the mean change from baseline in MADRS total score from treatment with placebo is about -15, -10, -5.

In some embodiments, the method provides therapeutic effect (*e.g.,* as measured by reduction in Clinical Global Impression-Improvement Scale (CGI)) within about 45, about 21, about 15, about 8, or about 3 days or less. In some embodiments, the therapeutic effect is a CGI score of 2 or less.

In some embodiments, the method provides therapeutic effect (*e.g.,* as measured by reduction in Hamilton Anxiety Score (HAM-A)) within about 45, about 21, about 15, about 8, or about 3 days. HAM-A is scored where <17 indicates mild severity, 18-24 mild to moderate severity and 25-30 moderate to severe. In some embodiments, the therapeutic effect is a decrease from baseline in HAM-A score at the end of a treatment period (*e.g.*, about 45, about 21, about 15, about 8, or about 3 days after beginning administration of Compound **(1),** or the pharmaceutically acceptable salt of Compound **(1)).** In some embodiments, the decrease from baseline in HAM-A score is from severe (*e.g*., HAM-A score of 25 or greater) to symptom-free (*e.g.,* HAM-A score of 17 or lower). In some embodiments, the decrease from baseline in HAM-A score is from severe (*e.g.,* HAM-A score of 25 or greater) to mild (*e.g.,* HAM-A score of 24 or lower).

In some embodiments, the method provides therapeutic effect (*e.g.,* as measured by improvements in SF-36 scores) within about 45, about 21, about 15, about 8, or about 3 days. SF-36 Physical Functioning Score. The SF-36 is a short-form health survey with 36 questions used to evaluate health-related quality of life (Ware, 1996). In some embodiments, the Short Form-36 (SF-36v2) assesses health related quality of life (HRQoL) for 8 domains (Physical-Functioning [PF]; Role-Physical [RP]; Bodily Pain [BP]; General Health [GH]; Vitality [V]; Social-Functioning [SF]; Role-Emotional [RE]; Mental Health [MH]). In some embodiments, the therapeutic effect is a decrease from baseline in each domain of the SG-36v2 at the end of the treatment period. In some embodiments, the (*e.g.,* about 45, about 21, about 15, about 8, or about 3 days after beginning administration of Compound **(1),** or the pharmaceutically acceptable salt of Compound **(1)).**

### PPD with Elevated Anxiety

In another aspect, the present disclosure is directed to methods of postpartum depression (PPD) with elevated anxiety.

Postpartum depression (PPD), also called postnatal depression, is a type of mood disorder associated with childbirth. Postpartum depression (PPD) is generally known in the art.

PPD is identified as the most common psychiatric illness to occur in the puerperium (O'Hara MW, Wisner KL. Best Pract Res Clin Obstet Gynaecol. 2014;28(1):3-12); and it can occur during the third trimester or after giving birth. If untreated, PPD can have devastating consequences for the woman and her family. In some embodiments, PPD is characterized by significant functional impairment for the mother due to sadness and depressed mood, loss of interest in daily activities, changes in eating and sleeping habits, fatigue and decreased energy, inability to concentrate, and feelings of worthlessness, shame, or guilt. Postpartum depression also carries an increased risk for suicide, which is the leading cause of maternal death following childbirth in developed countries.

Professional health organizations differ in their definition of PPD onset. For example, the American Psychiatric Association characterizes PPD as having an onset during pregnancy or within 4 weeks of delivery (DSM-5). The American College of Obstetricians and Gynecologists characterizes PPD as having an onset during pregnancy or within 12 months postpartum (ACOG Updated Dec 2021). The World Health Organization characterizes PPD as having an onset within 12 months postpartum (International Classification of Diseases 10th edition (ICD-10)). Accordingly, in some embodiments, the diagnosis of the PPD treated by the methods described herein can be characterized as defined by the DSM-5. In some embodiments, the diagnosis of the PPD treated by the methods described herein can be characterized as defined by the ACOG. In some embodiments, the diagnosis of the PPD treated by the methods described herein can be characterized as defined by the ICD-10.

In some embodiments, the diagnosis of the PPD with elevated anxiety treated by the methods described herein can be characterized as defined by the Diagnostic and Statistical Manual of Mental Disorders, 5^{th} Edition (DSM-5), that is as a MDD with peripartum onset and with anxious distress specifiers, as described below.

### Depressive Disorders

Depressive disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder. The common feature of all of these disorders is the presence of sad, empty, or irritable mood, accompanied by somatic and cognitive changes that significantly affect the individual's capacity to function. What differs among them are issues of duration, timing, or presumed etiology.

Major depressive disorder represents the classic condition in this group of disorders. It is characterized by discrete episodes of at least 2 weeks' duration (although most episodes last considerably longer) involving clear-cut changes in affect, cognition, and neurovegetative functions and inter-episode remissions. A discrete episode of major depressive disorder may be referred to as a "major depressive episode" or "depressive episode".

### Major Depressive Disorder (MDD)

In some embodiments, MDD is also known as depression or clinical depression and it is a mood disorder that causes a persistent feeling of sadness and loss of interest.

In some embodiments, MDD is defined and diagnosed according to the DSM-5, for example, MDD is diagnosed according to Criterion A, as described below.

**Criterion A.** Five (or more) of the following symptoms have been present during the same 2-week period and represent a change from previous functioning; at least one of the symptoms is either **(1)** depressed mood or (2) loss of interest or pleasure.
1. Depressed mood most of the day, nearly every day, as indicated by either subjective report (*e.g.,* feels sad, empty, hopeless) or observation made by others (*e.g.,* appears tearful). (Note: In children and adolescents, can be irritable mood.)
2. Markedly diminished interest or pleasure in all, or almost all, activities most of the day, nearly every day (as indicated by either subjective account or observation).
3. Significant weight loss when not dieting or weight gain (*e.g.,* a change of more than 5% of body weight in a month), or decrease or increase in appetite nearly every day (Note: In children, consider failure to make expected weight gain.)
4. Insomnia or hypersomnia nearly every day.
5. Psychomotor agitation or retardation nearly every day (observable by others, not merely subjective feelings of restlessness or being slowed down).
6. Fatigue or loss of energy nearly every day.
7. Feelings of worthlessness or excessive or inappropriate guilt (which may be delusional) nearly every day (not merely self-reproach or guilt about being sick).
8. Diminished ability to think or concentrate, or indecisiveness, nearly every day (either by subjective account or as observed by others).
9. Recurrent thoughts of death (not just fear of dying), recurrent suicidal ideation without a specific plan, or a suicide attempt or a specific plan for committing suicide.

Criteria B-E, described below, are additional descriptions of MDD and may be considered for describing or diagnosing MDD, but are not required.

**Criterion B.** The symptoms cause clinically significant distress or impairment in social, occupational, or other important areas of functioning.

**Criterion C.** The episode is not attributable to the physiological effects of a substance or to another medical condition.

Criteria A-C can represent a major depressive episode.

**Criterion D.** The occurrence of the major depressive episode is not better explained by schizoaffective disorder, schizophrenia, schizophreniform disorder, delusional disorder, or other specified and unspecified schizophrenia spectrum and other psychotic disorders.

**Criterion E.** There has never been a manic episode or a hypomanic episode.

In some embodiments, a major depressive episode (MDE) is a period characterized by the symptoms described above.

In some embodiments, MDD is a clinical course that is characterized by one or more major depressive episodes (MDE) in a subject.

In some embodiments, MDD is diagnosed according to Criteria A-C, as described above. In some embodiments, MDD is diagnosed according to Criteria A-E, as described above.

### Diagnostic Features

The criterion symptoms for major depressive disorder must be present nearly every day to be considered present, with the exception of weight change and suicidal ideation. Depressed mood must be present for most of the day, in addition to being present nearly every day. Often insomnia or fatigue is the presenting complaint, and failure to probe for accompanying depressive symptoms will result in underdiagnosis. Sadness may be denied at first but may be elicited through interview or inferred from facial expression and demeanor. With individuals who focus on a somatic complaint, clinicians should determine whether the distress from that complaint is associated with specific depressive symptoms. Fatigue and sleep disturbance are present in a high proportion of cases; psychomotor disturbances are much less common but are indicative of greater overall severity, as is the presence of delusional or near-delusional guilt.

The essential feature of a major depressive episode is a period of at least 2 weeks during which there is either depressed mood or the loss of interest or pleasure in nearly all activities (Criterion A above). In children and adolescents, the mood may be irritable rather than sad. The individual must also experience at least four additional symptoms drawn from a list that includes changes in appetite or weight, sleep, and psychomotor activity; decreased energy; feelings of worthlessness or guilt; difficulty thinking, concentrating, or making decisions; or recurrent thoughts of death or suicidal ideation or suicide plans or attempts. To count toward a major depressive episode, a symptom must either be newly present or must have clearly worsened compared with the person's pre-episode status. The symptoms must persist for most of the day, nearly every day, for at least 2 consecutive weeks. The episode must be accompanied by clinically significant distress or impairment in social, occupational, or other important areas of functioning. For some individuals with mild episodes, functioning may appear to be normal but requires markedly increased effort.

Sleep disturbance may take the form of either difficulty sleeping or sleeping excessively (Criterion A4). When insomnia is present, it typically takes the form of middle insomnia (i.e., waking up during the night and then having difficulty returning to sleep) or terminal insomnia (i.e., waking too early and being unable to return to sleep). Initial insomnia (i.e., difficulty falling asleep) may also occur. Individuals who present with over-sleeping (hypersomnia) may experience prolonged sleep episodes at night or increased daytime sleep. Sometimes the reason that the individual seeks treatment is for the disturbed sleep.

### With Peripartum Onset (Specifier for Depressive Disorders per the DSM-5)

This specifier can be applied to the current or, if full criteria are not currently met for a major depressive episode, most recent episode of major depression if onset of mood symptoms occurs during pregnancy or in the 4 weeks following delivery.

Mood episodes can have their onset either during pregnancy or postpartum. Although the estimates differ according to the period of follow-up after delivery, between 3% and 6% of women will experience the onset of a major depressive episode during pregnancy or in the weeks or months following delivery. Fifty percent of "postpartum" major depressive episodes actually begin prior to delivery. Thus, these episodes are referred to collectively as peripartum episodes. Women with peripartum major depressive episodes often have severe anxiety and even panic attacks. Prospective studies have demonstrated that mood and anxiety symptoms during pregnancy, as well as the "baby blues," increase the risk for a post partum major depressive episode. Peripartum-onset mood episodes can present either with or without psychotic features. Infanticide is most often associated with postpartum psychotic episodes that are characterized by command hallucinations to kill the infant or delusions that the infant is possessed, but psychotic symptoms can also occur in severe post partum mood episodes without such specific delusions or hallucinations.

### With Elevated Anxiety/Anxious Distress (Specifier for Depressive Disorders per the DSM-5)

The DSM-5 defines the "anxious distress" specifier as the presence of at least two of the following symptoms during the majority of days of a major depressive episode (MDD) or persistent depressive disorder (dysthymia):
1. Feeling keyed up or tense.
2. Feeling unusually restless.
3. Difficulty concentrating because of worry.
4. Fear that something awful may happen.
5. Feeling that the individual might lose control of himself or herself.

Severity is defined as:
Mild: Two symptoms.
Moderate: Three symptoms.
Moderate-severe: Four or five symptoms.
Severe: Four or five symptoms and with motor agitation.

Anxious distress has been noted as a prominent feature of both bipolar and major depressive disorder in both primary care and specialty mental health settings. High levels of anxiety have been associated with higher suicide risk, longer duration of illness, and greater likelihood of treatment nonresponse.

Accordingly, one aspect of the present disclosure is directed to a method of treating postpartum depression (PPD) with elevated anxiety in a subject in need thereof, comprising administering a therapeutically effective amount of Compound **(1):**

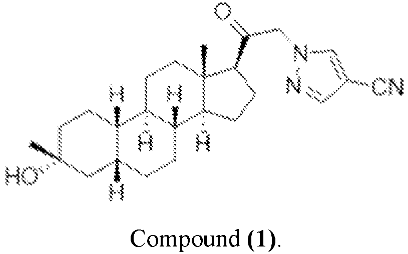

Another aspect of the disclosure provides a method of postpartum depression (PPD) with elevated anxiety in a subject in need thereof, comprising administering a therapeutically effective amount of a pharmaceutically acceptable salt of Compound **(1):**

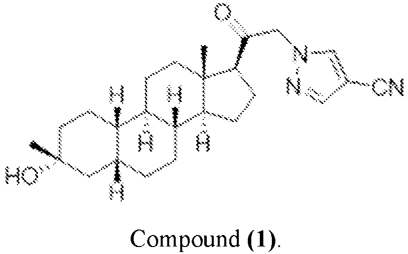

In some embodiments, Compound **(1),** or the pharmaceutically acceptable salt of Compound **(1),** is administered once a day for about 14 days or about 2 weeks. In some embodiments, Compound **(1),** or the pharmaceutically acceptable salt of Compound **(1),** is administered once a day for about 14 days. In some embodiments, Compound **(1),** or the pharmaceutically acceptable salt of Compound **(1),** is administered once a day for about 2 weeks.

In some embodiments, Compound **(1)** is administered at a dose of about 10 mg to about 100 mg. In some embodiments, Compound **(1)** is administered at a dose of about 15 mg to about 75 mg. In some embodiments, Compound **(1)** is administered at a dose of about 20 mg to about 60 mg. In some embodiments, Compound **(1)** is administered at a dose of about 20 mg to about 55 mg. In some embodiments, Compound **(1)** is administered at a dose of about 30 mg to about 50 mg. In some embodiments, Compound **(1)** is administered at a dose of about 45 mg to about 55 mg. In some embodiments, Compound **(1)** is administered at a dose of about 20 mg, about 25 mg, about 30 mg, about 35 mg, about 40 mg, about 45 mg, about 50 mg, about 55 mg, or about 60 mg. In some embodiments, Compound **(1)** is administered at a dose of about 50 mg. In some embodiments, Compound **(1)** is administered at a dose of about 40 mg. In some embodiments, Compound **(1)** is administered at a dose of about 30 mg.

In some embodiments, Compound **(1)** is administered at a dose of about 10 mg to about 100 mg once a day. In some embodiments, Compound **(1)** is administered at a dose of about 15 mg to about 75 mg once a day. In some embodiments, Compound **(1)** is administered at a dose of about 20 mg to about 60 mg once a day. In some embodiments, Compound **(1)** is administered at a dose of about 20 mg to about 55 mg once a day. In some embodiments, Compound **(1)** is administered at a dose of about 30 mg to about 50 mg once a day. In some embodiments, Compound **(1)** is administered at a dose of about 45 mg to about 55 mg once a day. In some embodiments, Compound **(1)** is administered at a dose of about 20 mg, about 25 mg, about 30 mg, about 35 mg, about 40 mg, about 45 mg, about 50 mg, about 55 mg, or about 60 mg once a day. In some embodiments, Compound **(1)** is administered at a dose of about 50 mg once a day. In some embodiments, Compound **(1)** is administered at a dose of about 40 mg once a day. In some embodiments, Compound **(1)** is administered at a dose of about 30 mg once a day.

In some embodiments, Compound **(1)** is administered at a dose of about 20 mg to about 55 mg once a day for about 2 weeks or about 14 days. In some embodiments, Compound **(1)** is administered at a dose of about 30 mg to about 50 mg once a day for about 2 weeks or about 14 days. In some embodiments, Compound **(1)** is administered at a dose of about 45 mg to about 55 mg once a day for about 2 weeks or about 14 days. In some embodiments, Compound **(1)** is administered at a dose of about 50 mg once a day for less than 2 weeks. In some embodiments, Compound **(1)** is administered at a dose of about 50 mg once a day for about 2 weeks. In some embodiments, Compound **(1)** is administered at a dose of about 50 mg once a day for about 14 days. In some embodiments, Compound **(1)** is administered at a dose of about 40 mg once a day for less than 2 weeks. In some embodiments, Compound **(1)** is administered at a dose of about 40 mg once a day for about 2 weeks. In some embodiments, Compound **(1)** is administered at a dose of about 40 mg once a day for about 14 days. In some embodiments, Compound **(1)** is administered at a dose of about 30 mg once a day for less than 2 weeks. In some embodiments, Compound **(1)** is administered at a dose of about 30 mg once a day for about 2 weeks. In some embodiments, Compound **(1)** is administered at a dose of about 30 mg once a day for about 14 days.

In other embodiments, the pharmaceutically acceptable salt of Compound **(1)** is administered at a dose equivalent of about 10 mg to about 100 mg of the free base compound. In some embodiments, the pharmaceutically acceptable salt of Compound **(1)** is administered at a dose equivalent of about 15 mg to about 75 mg of the free base compound. In some embodiments, the pharmaceutically acceptable salt of Compound **(1)** is administered at a dose equivalent of about 20 mg to about 60 mg of the free base compound. In some embodiments, the pharmaceutically acceptable salt of Compound **(1)** is administered at a dose equivalent of about 20 mg to about 55 mg of the free base compound. In some embodiments, the pharmaceutically acceptable salt of Compound **(1)** is administered at a dose equivalent of about 30 mg to about 50 mg of the free base compound. In some embodiments, the pharmaceutically acceptable salt of Compound **(1)** is administered at a dose equivalent of about 45 mg to about 55 mg of the free base compound. In some embodiments, the pharmaceutically acceptable salt of Compound **(1)** is administered at a dose equivalent of about 20 mg, about 25 mg, about 30 mg, about 35 mg, about 40 mg, about 45 mg, about 50 mg, about 55 mg, or about 60 mg of the free base compound. In some embodiments, the pharmaceutically acceptable salt of Compound **(1)** is administered at a dose equivalent of about 50 mg of the free base compound. In some embodiments, the pharmaceutically acceptable salt of Compound **(1)** is administered at a dose equivalent of about 40 mg of the free base compound. In some embodiments, the pharmaceutically acceptable salt of Compound **(1)** is administered at a dose equivalent of about 30 mg of the free base compound.

In some embodiments, the pharmaceutically acceptable salt of Compound **(1)** is administered at a dose equivalent of about 10 mg to about 100 mg of the free base compound once a day. In some embodiments, the pharmaceutically acceptable salt of Compound **(1)** is administered at a dose equivalent of about 15 mg to about 75 mg of the free base compound once a day. In some embodiments, the pharmaceutically acceptable salt of Compound **(1)** is administered at a dose equivalent of about 20 mg to about 60 mg of the free base compound once a day. In some embodiments, the pharmaceutically acceptable salt of Compound **(1)** is administered at a dose equivalent of about 20 mg to about 55 mg of the free base compound once a day. In some embodiments, the pharmaceutically acceptable salt of Compound **(1)** is administered at a dose equivalent of about 30 mg to about 50 mg of the free base compound once a day. In some embodiments, the pharmaceutically acceptable salt of Compound **(1)** is administered at a dose equivalent of about 45 mg to about 55 mg of the free base compound once a day. In some embodiments, the pharmaceutically acceptable salt of Compound **(1)** is administered at a dose equivalent of about 20 mg, about 25 mg, about 30 mg, about 35 mg, about 40 mg, about 45 mg, about 50 mg, about 55 mg, or about 60 mg of the free base compound once a day. In some embodiments, the pharmaceutically acceptable salt of Compound **(1)** is administered at a dose equivalent of about 50 mg of the free base compound once a day. In some embodiments, the pharmaceutically acceptable salt of Compound **(1)** is administered at a dose equivalent of about 40 mg of the free base compound once a day. In some embodiments, the pharmaceutically acceptable salt of Compound **(1)** is administered at a dose equivalent of about 30 mg of the free base compound once a day.

In some embodiments, the pharmaceutically acceptable salt of Compound **(1)** is administered at a dose equivalent of about 20 mg to about 55 mg of the free base compound once a day for about 2 weeks or about 14 days. In some embodiments, the pharmaceutically acceptable salt of Compound **(1)** is administered at a dose equivalent of about 30 mg to about 50 mg of the free base compound once a day for about 2 weeks or about 14 days. In some embodiments, the pharmaceutically acceptable salt of Compound **(1)** is administered at a dose equivalent of about 45 mg to about 55 mg of the free base compound once a day for about 2 weeks or about 14 days. In some embodiments, the pharmaceutically acceptable salt of Compound **(1)** is administered at a dose equivalent of about 50 mg of the free base compound once a day for less than 2 weeks. In some embodiments, the pharmaceutically acceptable salt of Compound **(1)** is administered at a dose equivalent of about 50 mg of the free base compound once a day for about 2 weeks. In some embodiments, the pharmaceutically acceptable salt of Compound **(1)** is administered at a dose equivalent of about 50 mg of the free base compound once a day for about 14 days. In some embodiments, the pharmaceutically acceptable salt of Compound **(1)** is administered at a dose equivalent of about 40 mg of the free base compound once a day for less than 2 weeks. In some embodiments, the pharmaceutically acceptable salt of Compound **(1)** is administered at a dose equivalent of about 40 mg of the free base compound once a day for about 2 weeks. In some embodiments, the pharmaceutically acceptable salt of Compound **(1)** is administered at a dose equivalent of about 40 mg of the free base compound once a day for about 14 days. In some embodiments, the pharmaceutically acceptable salt of Compound **(1)** is administered at a dose equivalent of about 30 mg of the free base compound once a day for less than 2 weeks. In some embodiments, the pharmaceutically acceptable salt of Compound **(1)** is administered at a dose equivalent of about 30 mg of the free base compound once a day for about 2 weeks. In some embodiments, the pharmaceutically acceptable salt of Compound **(1)** is administered at a dose equivalent of about 30 mg of the free base compound once a day for about 14 days.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally.

In some embodiments, Compound **(1),** or the pharmaceutically acceptable salt of Compound **(1),** is administered chronically.

In some embodiments, Compound **(1),** or the pharmaceutically acceptable salt of Compound **(1),** is administered in one or more capsules. In some embodiments, the therapeutically effective amount is administered across two capsules. In some embodiments, the therapeutically effective amount is administered across three capsules.

In some embodiments, Compound **(1),** or the pharmaceutically acceptable salt of Compound **(1),** is administered with food. In some embodiments, Compound **(1),** or the pharmaceutically acceptable salt of Compound **(1),** is administered with fat-containing food. Examples of fat-containing food include nuts, peanut butter, avocado, eggs, and cheese. In some embodiments, Compound **(1),** or the pharmaceutically acceptable salt of Compound **(1),** is administered at night with fat-containing food (*e.g*., within 1 hour of an evening meal which contains fat, or with a fat-containing snack).

In some embodiments, the subject is administered Compound **(1),** or the pharmaceutically acceptable salt of Compound **(1),** at night. In some embodiments, the subject is administered Compound **(1),** or the pharmaceutically acceptable salt of Compound **(1),** no later than 1 hour before the patient sleeps. In some embodiments, the subject is administered Compound **(1),** or the pharmaceutically acceptable salt of Compound **(1),** no later than 15 minutes before the patient sleeps. In some embodiments, the subject is administered Compound **(1),** or the pharmaceutically acceptable salt of Compound **(1),** once a day at night. In some embodiments, the subject is administered Compound **(1),** or the pharmaceutically acceptable salt of Compound **(1),** once a day no later than 1 hour before the patient sleeps. In some embodiments, the subject is administered Compound **(1),** or the pharmaceutically acceptable salt of Compound **(1),** once a day no later than 15 minutes before the patient sleeps.

In some embodiments, Compound **(1)** is in a crystalline form. In some embodiments, the crystalline form of Compound **(1)** is any crystalline form disclosed in PCT Application Publication No. WO 2018/039378; the entire contents of the aforementioned application are incorporated herein by reference in its entirety.

In some embodiments, Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ. In some embodiments, Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.

In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ. In some embodiments, Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.

In some embodiments, the crystalline form of Compound **(1)** comprises a mixture of two or more crystalline forms.

In some embodiments, Compound **(1),** or the pharmaceutically acceptable salt of Compound **(1),** is re-administered to the subject in response to a recurrence of depression symptoms after completion of the initial treatment. In some embodiments, there is at least a 6 week interval between the last dose of the initial treatment and the first dose of the re-administration. In some embodiments, each of the initial treatment and re-administration occurs for about 14 days or about 2 weeks.

In some embodiments, the method administers a second therapeutic agent.

In some embodiments, the subject is treatment naive. In some embodiments, the subject has not received any antidepressant treatment within at least 30 days prior to the administration of Compound **(1),** or the pharmaceutically acceptable salt of Compound **(1).** In some embodiments, the subject has not received any antidepressant treatment within at least 60 days prior to the administration of Compound **(1),** or the pharmaceutically acceptable salt of Compound **(1).**

In some embodiments, the subject has been on a stable dose of an additional antidepressant for at least 30 days or for at least 60 days prior to the administration of Compound **(1),** or the pharmaceutically acceptable salt of Compound **(1).** In some embodiments, the subject has been on a stable dose of an additional antidepressant for at least 30 days prior to the administration of Compound **(1),** or the pharmaceutically acceptable salt of Compound **(1).** In some embodiments, the subject has been on a stable dose of an additional antidepressant for at least 60 days prior to the administration of Compound **(1),** or the pharmaceutically acceptable salt of Compound **(1).**

In some embodiments, PPD with elevated anxiety is characterized by a Hamilton Rating Scale for Anxiety (HAM-A) total score of 17 or greater, 18 or greater, 19 or greater, or 20 or greater, or by a Hamilton Rating Scale for Depression (HAM-D) Anxiety/Somatization subscale score of 7 or greater, prior to the administration of Compound **(1),** or the pharmaceutically acceptable salt of Compound (1). In some embodiments, PPD with elevated anxiety is characterized by a Hamilton Rating Scale for Anxiety (HAM-A) total score of 17 or greater. In some embodiments, PPD with elevated anxiety is characterized by a Hamilton Rating Scale for Anxiety (HAM-A) total score of 18 or greater. In some embodiments, PPD with elevated anxiety is characterized by a Hamilton Rating Scale for Anxiety (HAM-A) total score of 19 or greater. In some embodiments, PPD with elevated anxiety is characterized by a Hamilton Rating Scale for Anxiety (HAM-A) total score of 20 or greater. In some embodiments, PPD with elevated anxiety is characterized by a Hamilton Rating Scale for Depression (HAM-D) Anxiety/Somatization subscale score of 7 or greater.

In some embodiments, PPD with elevated anxiety is characterized by a HAM-D total score of 24 or greater and a HAM-A total score of 17 or greater prior to the administration of Compound (1), or the pharmaceutically acceptable salt of Compound (1). In some embodiments, PPD with elevated anxiety is characterized by a HAM-D total score of 24 or greater and a HAM-A total score of 18 or greater prior to the administration of Compound (1), or the pharmaceutically acceptable salt of Compound (1). In some embodiments, PPD with elevated anxiety is characterized by a HAM-D total score of 24 or greater and a HAM-A total score of 19 or greater prior to the administration of Compound (1), or the pharmaceutically acceptable salt of Compound (1). In some embodiments, PPD with elevated anxiety is characterized by a HAM-D total score of 24 or greater and a HAM-A total score of 20 or greater prior to the administration of Compound (1), or the pharmaceutically acceptable salt of Compound (1). In some embodiments, PPD with elevated anxiety is characterized by a HAM-D total score of 24 or greater and a HAM-D Anxiety/Somatization subscale score of 7 or greater prior to the administration of Compound (1), or the pharmaceutically acceptable salt of Compound (1).

In some embodiments, PPD with elevated anxiety is characterized by a HAM-D total score of 26 or greater and a HAM-A total score of 17 or greater prior to the administration of Compound (1), or the pharmaceutically acceptable salt of Compound (1). In some embodiments, PPD with elevated anxiety is characterized by a HAM-D total score of 26 or greater and a HAM-A total score of 18 or greater prior to the administration of Compound (1), or the pharmaceutically acceptable salt of Compound (1). In some embodiments, PPD with elevated anxiety is characterized by a HAM-D total score of 26 or greater and a HAM-A total score of 19 or greater prior to the administration of Compound (1), or the pharmaceutically acceptable salt of Compound (1). In some embodiments, PPD with elevated anxiety is characterized by a HAM-D total score of 26 or greater and a HAM-A total score of 20 or greater prior to the administration of Compound (1), or the pharmaceutically acceptable salt of Compound (1). In some embodiments, PPD with elevated anxiety is characterized by a HAM-D total score of 26 or greater and a HAM-D Anxiety/Somatization subscale score of 7 or greater prior to the administration of Compound (1), or the pharmaceutically acceptable salt of Compound (1).

In other embodiments, "elevated anxiety" is characterized by a HAM-A score based on the HAM-A anxiety items and somatic items. In some embodiments, "elevated anxiety" is characterized by a HAM-A score based on the HAM-A anxiety items. In some embodiments, "elevated anxiety" is characterized by a HAM-D score based on the following HAM-D items: psychic anxiety, somatic anxiety, GI somatic symptoms, and/or general somatic symptoms. In some embodiments, "elevated anxiety" is characterized by a HAM-D score based on the following HAM-D item: psychic anxiety. In some embodiments, "elevated anxiety" is characterized by a HAM-D score based predominately on the items evaluating somatic symptoms of depression. In some embodiments, "elevated anxiety" is characterized by a HAM-D score based predominately on the items evaluating anxiety symptoms of depression. In some embodiments, "elevated anxiety" is characterized by a HAM-D Anxiety/Somatization Subscale score based predominately on the items evaluating somatic symptoms of depression. In some embodiments, "elevated anxiety" is characterized by a HAM-D Anxiety/Somatization Subscale score based predominately on the items evaluating anxiety symptoms of depression. In some embodiments, "elevated anxiety" is characterized by a MADRS score based predominately on the items evaluating the somatic symptoms of depression. In some embodiments, "elevated anxiety" is characterized by a MADRS score based predominately on the items evaluating the anxiety symptoms of depression.

In some embodiments, the subject exhibits a reduction in HAM-D total score, HAM-A total score, HAM-D Anxiety/Somatization subscale score, or a combination thereof, from baseline. In some embodiments, the subject exhibits a reduction of at least 14 points in HAM-D total score on Day 15 after administration of Compound (1), or the pharmaceutically acceptable salt of Compound (1). In some embodiments, the subject exhibits a reduction of at least 12 points in HAM-A total score on Day 15 after administration of Compound (1), or the pharmaceutically acceptable salt of Compound (1).

In some embodiments, the method provides a therapeutic effect (*e.g.,* as measured by reduction in Hamilton Depression Score (HAMD-17)) within about 45, about 21, about 15, about 8, or about 3 days. In some embodiments, the therapeutic effect is a decrease from baseline in HAMD-17 score at the end of a treatment period (*e.g*., about 45, about 21, about 15, about 8, or about 3 days after beginning administration of Compound (1), or the pharmaceutically acceptable salt of Compound (1)). In some embodiments, the decrease from baseline in HAMD-17 score is from severe (*e.g*., HAMD-17 score of 24 or greater; or a score of 26 or greater) to symptom-free, i.e. Remission of depression (*e*.*g*., HAMD-17 score of 7 or lower). In some embodiments, the decrease from baseline in HAMD-17 score is from severe (*e.g*., HAMD-17 score of 24 or greater; or a score of 26 or greater) to normal or mild depression (*e.g.,* HAMD-17 score of 7 or lower; or HAMD-17 score of 18-13).

In some embodiments, the method provides a therapeutic effect (*e.g.,* as measured by reduction in Hamilton Anxiety Rating Scale score (HAM-A)) within about 45, about 21, about 15, about 8, or about 3 days. In some embodiments, the therapeutic effect is a decrease from baseline in HAM-A score at the end of a treatment period (*e.g*., about 45, about 21, about 15, about 8, or about 3 days after beginning administration of Compound **(1),** or the pharmaceutically acceptable salt of Compound **(1)).** In some embodiments, the decrease from baseline in HAM-A score is from severe (*e.g*., HAM-A score of 25 or greater) to symptom-free, i.e. Remission of anxiety (*e.g*., HAM-A score of 7 or lower). In some embodiments, the decrease from baseline in HAM-A score is from severe (*e.g*., HAM-A score of 25 or greater) to normal or mild anxiety (*e.g*., HAM-A score of 18-24).

In some embodiments, the method provides therapeutic effect (*e.g.,* as measured by reduction in Montgomery-Asberg Depression Rating Scale (MADRS)) within about 45, about 21, about 15, about 8, or about 3 days or less. The Montgomery-Åsberg Depression Rating Scale (MADRS) is a ten-item diagnostic questionnaire (regarding apparent sadness, reported sadness, inner tension, reduced sleep, reduced appetite, concentration difficulties, lassitude, inability to feel, pessimistic thoughts, and suicidal thoughts) which psychiatrists use to measure the severity of depressive episodes in patients with mood disorders. 0-6 indicates normal/symptom absent; 7-19 indicates mild depression; 20-34 indicates moderate depression; and >34 indicates severe depression. In some embodiments, the therapeutic effect is a decrease from baseline in MADRS score at the end of a treatment period (*e.g*., about 45, about 21, about 15, about 8, or about 3 days or less). In some embodiments, the decrease from baseline in MADRS score is from severe (*e.g.,* MADRS score of 30 or greater) to symptom-free (*e.g.,* MADRS score of 20 or lower). For example, the mean change from baseline in MADRS total score from treatment with of Compound **(1),** or the pharmaceutically acceptable salt of Compound **(1)** is about -15, -20, -25, -30, while the mean change from baseline in MADRS total score from treatment with placebo is about -15, -10, -5.

In some embodiments, the method provides therapeutic effect (*e.g.,* as measured by reduction in Clinical Global Impression-Improvement Scale (CGI)) within about 45, about 21, about 15, about 8, or about 3 days or less. In some embodiments, the therapeutic effect is a CGI score of 2 or less.

In some embodiments, the method provides therapeutic effect (*e.g.,* as measured by reduction in Hamilton Anxiety/Somatization Score (HAMD-17 A)/S) within about 45, about 21, about 15, about 8, or about 3 days.

In some embodiments, the method provides therapeutic effect (*e.g.,* as measured by reduction in Edinburgh Postnatal Depression Scale (EPDS)) within about 45, about 21, about 15, or about 8 days. In some embodiments, the therapeutic effect is an improvement measured by the EPDS.

In some embodiments, the method increases the subject's generic health status (*e.g*., as measured by increase in Medical Outcomes Study 36-Item Short Form Survey Instrument version 2 (SF-36v2)) within about 45, about 21, about 15, or about 3 days. In some embodiments, the method increases the subject's generic health status as measured by at least five domains of the SF-36v2 survey. In some embodiments, the five domains are social functioning, mental health, physical functioning, role physical, and bodily pain. In some embodiments, the method increases the mental health component summary score as measured by SF-36v2.

In some embodiments, the method provides therapeutic effect (*e.g.,* as measured by reduction in Hamilton Depression Score for the insomnia questions only (HAMD-17-Ins)) within about 45, about 21, about 15, about 8, or about 3 days. In some embodiments, the therapeutic effect is a decrease from baseline in HAMD-17-Ins score at the end of a treatment period (*e.g.,* about 45, about 21, about 15, about 8, or about 3 days after beginning administration of Compound **(1),** or the pharmaceutically acceptable salt of Compound **(1)).** In some embodiments, the decrease from baseline in HAMD-17-Ins score is from 1 - 4 points, *e.g.,* a decrease of about 1 point from baseline, a decrease of about 2 points from baseline, a decrease of about 3 points from baseline, or a decrease of about 4 points from baseline.

Another aspect of the disclosure includes a method of treating major depressive disorder (MDD) with elevated anxiety in a subject in need thereof, comprising administering about 30 mg to about 50 mg of Compound **(1):**

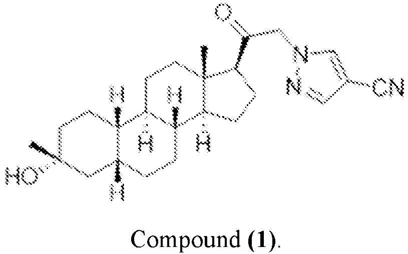

Another aspect of the disclosure includes a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound:

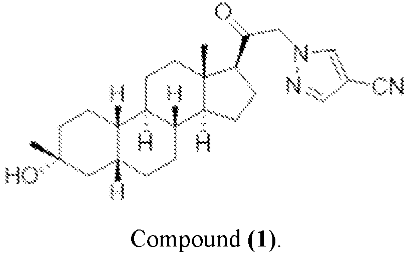

Another aspect of the disclosure includes a method of treating postpartum depression (PPD) with elevated anxiety in a subject in need thereof, comprising administering about 30 mg to about 50 mg of Compound **(1):**

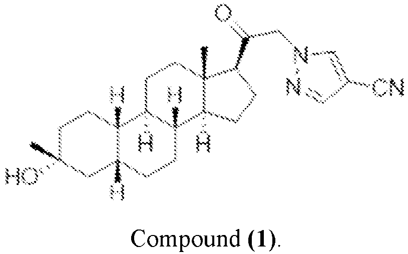

Another aspect of the disclosure includes a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound:

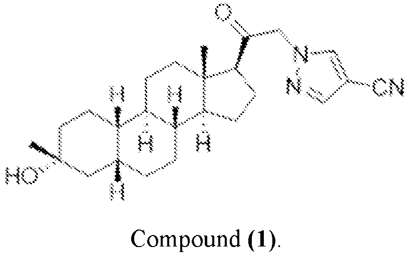

Another aspect of the disclosure includes a method of treating major depressive disorder (MDD) with elevated anxiety in a subject in need thereof, comprising administering about 30 mg to about 50 mg of Compound **(1)** once a day for about 14 days or about 2 weeks:

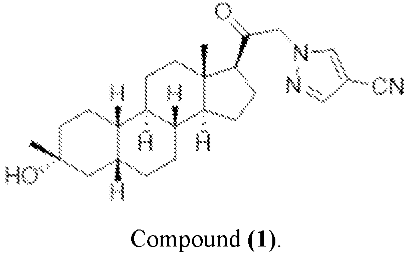

Another aspect of the disclosure includes a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks:

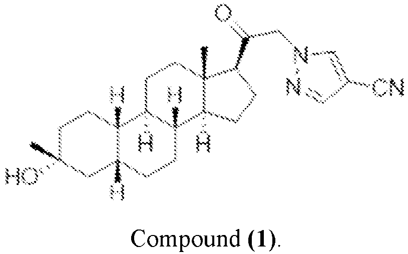

Another aspect of the disclosure includes a method of treating postpartum depression (PPD) with elevated anxiety in a subject in need thereof, comprising administering about 30 mg to about 50 mg of Compound **(1)** once a day for about 14 days or about 2 weeks:

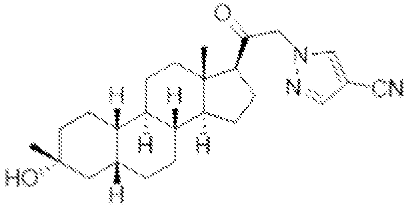

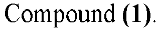

Another aspect of the disclosure includes a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks:

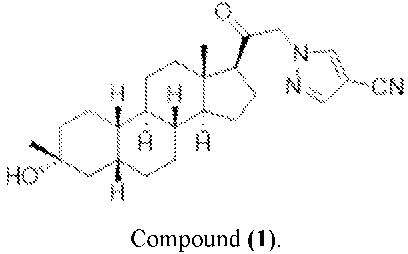

Another aspect of the disclosure includes a method of treating major depressive disorder (MDD) with elevated anxiety in a subject in need thereof, comprising administering about 30 mg to about 50 mg of Compound **(1)** once a day for about 14 days or about 2 weeks: 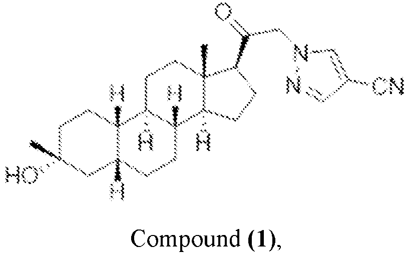 wherein the subject is treatment naïve.

Another aspect of the disclosure includes a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 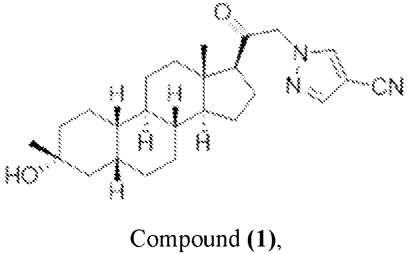 wherein the subject is treatment naive.

Another aspect of the disclosure includes a method of treating postpartum depression (PPD) with elevated anxiety in a subject in need thereof, comprising administering about 30 mg to about 50 mg of Compound **(1)** once a day for about 14 days or about 2 weeks: 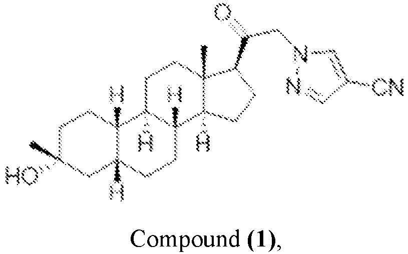 wherein the subject is treatment naïve.

Another aspect of the disclosure includes a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 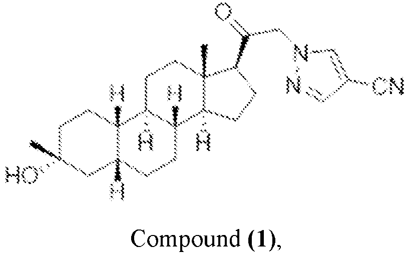 wherein the subject is treatment naïve.

Another aspect of the disclosure includes a method of treating major depressive disorder (MDD) with elevated anxiety in a subject in need thereof, comprising administering about 30 mg to about 50 mg of Compound **(1)** once a day for about 14 days or about 2 weeks: 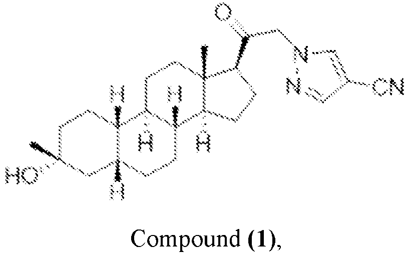 wherein the subject has been on a stable dose of an additional antidepressant for at least 60 days prior to the administration of Compound **(1).**

Another aspect of the disclosure includes a method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 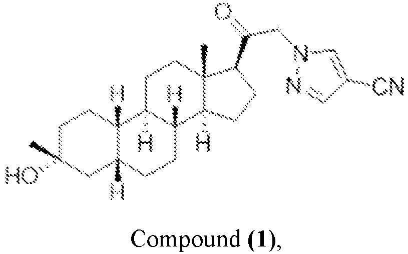 wherein the subject has been on a stable dose of an additional antidepressant for at least 60 days prior to the administration of the pharmaceutically acceptable salt of Compound **(1).**

Another aspect of the disclosure includes a method of treating postpartum depression (PPD) with elevated anxiety in a subject in need thereof, comprising administering about 30 mg to about 50 mg of Compound **(1)** once a day for about 14 days or about 2 weeks: 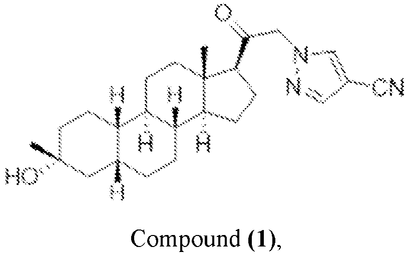 wherein the subject has been on a stable dose of an additional antidepressant for at least 30 days prior to the administration of Compound **(1).**

Another aspect of the disclosure includes a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 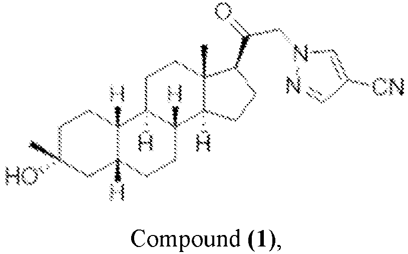 wherein the subject has been on a stable dose of an additional antidepressant for at least 30 days prior to the administration the pharmaceutically acceptable salt of Compound **(1).**

Another aspect of the disclosure includes a method of treating postpartum depression (PPD) with elevated anxiety in a subject in need thereof, comprising administering about 30 mg to about 50 mg of Compound **(1)** once a day for about 14 days or about 2 weeks: 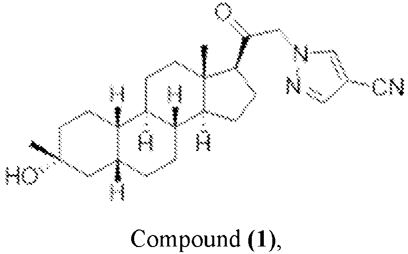 wherein the subject has been on a stable dose of an additional antidepressant for at least 60 days prior to the administration of Compound **(1).**

Another aspect of the disclosure includes a method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 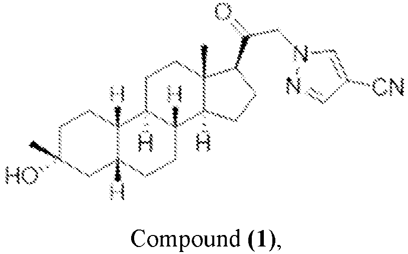 wherein the subject has been on a stable dose of an additional antidepressant for at least 60 days prior to the administration the pharmaceutically acceptable salt of Compound **(1).**

In some embodiments, Compound **(1)** is administered at a dose of about 50 mg or the pharmaceutically acceptable salt of Compound **(1)** is administered at a dose equivalent to about 50 mg of the free base compound.

In some embodiments, Compound **(1)** is administered at a dose of about 40 mg or the pharmaceutically acceptable salt of Compound **(1)** is administered at a dose equivalent to about 40 mg of the free base compound.

In some embodiments, Compound **(1)** is administered at a dose of about 30 mg or the pharmaceutically acceptable salt of Compound **(1)** is administered at a dose equivalent to about 30 mg of the free base compound.

In some embodiments, Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally. In some embodiments, Compound **(1),** or the pharmaceutically acceptable salt of Compound **(1),** is administered orally.

In some embodiments, Compound **(1),** or the pharmaceutically acceptable salt of Compound **(1),** is administered with food. In some embodiments, Compound **(1),** or the pharmaceutically acceptable salt of Compound **(1),** is administered with fat-containing food. Examples of fat-containing food include nuts, peanut butter, avocado, eggs, and cheese. In some embodiments, Compound **(1),** or the pharmaceutically acceptable salt of Compound **(1),** is administered at night with fat-containing food (*e*.*g*., within 1 hour of an evening meal which contains fat, or with a fat-containing snack).

In some embodiments, the subject is administered Compound **(1),** or the pharmaceutically acceptable salt of Compound **(1),** at night. In some embodiments, the subject is administered Compound **(1),** or the pharmaceutically acceptable salt of Compound **(1),** no later than 1 hour before the patient sleeps. In some embodiments, the subject is administered Compound **(1),** or the pharmaceutically acceptable salt of Compound **(1),** no later than 15 minutes before the patient sleeps.

In some embodiments, the subject is treatment naïve. In some embodiments, the subject has not received any antidepressant treatment within at least 30 days prior to the start of the initial treatment course. In some embodiments, the subject has not received any antidepressant treatment within at least 60 days prior to the start of the initial treatment course.

In some embodiments, the subject has been on a stable dose of an additional antidepressant for at least 30 days or for at least 60 days prior to the administration of Compound **(1),** or the pharmaceutically acceptable salt of Compound **(1).** In some embodiments, the subject has been on a stable dose of an additional antidepressant for at least 30 days prior to the administration of Compound **(1),** or the pharmaceutically acceptable salt of Compound **(1).** In some embodiments, the subject has been on a stable dose of an additional antidepressant for at least 60 days prior to the administration of Compound **(1),** or the pharmaceutically acceptable salt of Compound **(1).**

In some embodiments, the method further comprises administration of a second therapeutic agent.

In some embodiments, Compound **(1),** or the pharmaceutically acceptable salt of Compound **(1),** is re-administered to the subject in response to a recurrence of depression symptoms after completion of an initial treatment. In some embodiments, there is at least a 6 week interval between the last dose of the initial treatment and the first dose of the re-administration. In some embodiments, the re-administration occurs for about 14 days or about 2 weeks.

In some embodiments, MDD with elevated anxiety or PPD with elevated anxiety is characterized by a Hamilton Rating Scale for Anxiety (HAM-A) total score of 17 or greater, 18 or greater, 19 or greater, or 20 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).** In some embodiments, MDD with elevated anxiety or PPD with elevated anxiety is characterized by a Hamilton Rating Scale for Anxiety (HAM-A) total score of 17 or greater prior to the administration of Compound **(1),** or the pharmaceutically acceptable salt of Compound **(1).** In some embodiments, MDD with elevated anxiety or PPD with elevated anxiety is characterized by a Hamilton Rating Scale for Anxiety (HAM-A) total score of 18 or greater prior to the administration of Compound **(1),** or the pharmaceutically acceptable salt of Compound **(1).** In some embodiments, MDD with elevated anxiety or PPD with elevated anxiety is characterized by a Hamilton Rating Scale for Anxiety (HAM-A) total score of 19 or greater prior to the administration of Compound **(1),** or the pharmaceutically acceptable salt of Compound **(1).** In some embodiments, MDD with elevated anxiety or PPD with elevated anxiety is characterized by a Hamilton Rating Scale for Anxiety (HAM-A) total score of 20 or greater prior to the administration of Compound **(1),** or the pharmaceutically acceptable salt of Compound **(1).** In some embodiments, MDD with elevated anxiety or PPD with elevated anxiety is characterized by a Hamilton Rating Scale for Depression (HAM-D) Anxiety/Somatization subscale score of 7 or greater, prior to the administration of Compound **(1),** or the pharmaceutically acceptable salt of Compound **(1).**

In some embodiments, MDD with elevated anxiety or PPD with elevated anxiety is characterized by a HAM-D total score of 24 or greater and a HAM-A total score of 17 or greater prior to the administration of Compound **(1),** or the pharmaceutically acceptable salt of Compound **(1).** In some embodiments, MDD with elevated anxiety or PPD with elevated anxiety is characterized by a HAM-D total score of 24 or greater and a HAM-A total score of 18 or greater prior to the administration of Compound **(1),** or the pharmaceutically acceptable salt of Compound **(1).** In some embodiments, MDD with elevated anxiety or PPD with elevated anxiety is characterized by a HAM-D total score of 24 or greater and a HAM-A total score of 19 or greater prior to the administration of Compound **(1),** or the pharmaceutically acceptable salt of Compound **(1).** In some embodiments, MDD with elevated anxiety or PPD with elevated anxiety is characterized by a HAM-D total score of 24 or greater and a HAM-A total score of 20 or greater prior to the administration of Compound **(1),** or the pharmaceutically acceptable salt of Compound **(1).**

In some embodiments, MDD with elevated anxiety or PPD with elevated anxiety is characterized by a HAM-D total score of 24 or greater and a HAM-D Anxiety/Somatization subscale score of 7 or greater prior to the administration of Compound **(1),** or the pharmaceutically acceptable salt of Compound **(1).**

In some embodiments, MDD with elevated anxiety or PPD with elevated anxiety is characterized by a HAM-D total score of 26 or greater and a HAM-A total score of 17 or greater (*e.g.,* 18 or greater, 19 or greater, or 20 or greater) prior to the administration of Compound **(1),** or the pharmaceutically acceptable salt of Compound **(1).** In some embodiments, MDD with elevated anxiety or PPD with elevated anxiety is characterized by a HAM-D total score of 26 or greater and a HAM-D Anxiety/Somatization subscale score of 7 or greater prior to the administration of Compound **(1),** or the pharmaceutically acceptable salt of Compound **(1).**

In some embodiments, MDD with elevated anxiety or PPD with elevated anxiety is characterized by a HAM-D total score of 20 or greater, a MADRS total score of 28 or greater, and a HAM-A total score of 17 or greater (*e.g.,* 18 or greater, 19 or greater, or 20 or greater) prior to the administration of Compound **(1),** or the pharmaceutically acceptable salt of Compound **(1).** In some embodiments, MDD with elevated anxiety or PPD with elevated anxiety is characterized by a HAM-D total score of 20 or greater, a MADRS total score of 28 or greater, and a HAM-D Anxiety/Somatization subscale score of 7 or greater prior to the administration of Compound **(1),** or the pharmaceutically acceptable salt of Compound **(1).**

In other embodiments, "elevated anxiety" is characterized by a HAM-A score based on the HAM-A anxiety items and somatic items. In some embodiments, "elevated anxiety" is characterized by a HAM-A score based on the HAM-A anxiety items. In some embodiments, "elevated anxiety" is characterized by a HAM-D score based on the following HAM-D items: psychic anxiety, somatic anxiety, GI somatic symptoms, and general somatic symptoms. In some embodiments, "elevated anxiety" is characterized by a HAM-D score based on the following HAM-D item: psychic anxiety. In some embodiments, "elevated anxiety" is characterized by a HAM-D score based predominately on the items evaluating somatic symptoms of depression. In some embodiments, "elevated anxiety" is characterized by a HAM-D score based predominately on the items evaluating anxiety symptoms of depression. In some embodiments, "elevated anxiety" is characterized by a HAM-D Anxiety/Somatization Subscale score based predominately on the items evaluating somatic symptoms of depression. In some embodiments, "elevated anxiety" is characterized by a HAM-D Anxiety/Somatization Subscale score based predominately on the items evaluating anxiety symptoms of depression. In some embodiments, "elevated anxiety" is characterized by a MADRS score based predominately on the items evaluating the somatic symptoms of depression. In some embodiments, "elevated anxiety" is characterized by a MADRS score based predominately on the items evaluating the anxiety symptoms of depression.

In some embodiments, the subject exhibits a reduction in HAM-D total score, HAM-A total score, HAM-D Anxiety/Somatization subscale score, or a combination thereof, from baseline.

In some embodiments, the subject exhibits a reduction of at least 14 points in HAM-D total score on Day 15 after administration of Compound **(1),** or the pharmaceutically acceptable salt of Compound **(1).** In some embodiments, the subject exhibits a reduction of at least 12 points in HAM-A total score on Day 15 after administration of Compound **(1),** or the pharmaceutically acceptable salt of Compound **(1).**

### III. PHARMACEUTICAL COMPOSITIONS

Another aspect of the disclosure provides a pharmaceutical composition comprising Compound **(1)** (also referred to as the "active ingredient"), and a pharmaceutically acceptable excipient for use in the methods described herein. In another aspect, the disclosure provides a pharmaceutical composition comprising a pharmaceutically acceptable salt of the active ingredient and a pharmaceutically acceptable excipient for use in the methods described herein. In certain embodiments, the pharmaceutical composition comprises an effective amount of the active ingredient or a pharmaceutically acceptable salt of the active ingredient. In certain embodiments, the pharmaceutical composition comprises a therapeutically effective amount of the active ingredient or a pharmaceutically acceptable salt of the active ingredient. In some embodiments, the pharmaceutical composition of Compound **(1)** is any pharmaceutical composition disclosed in PCT Application Publication No. WO 2022/020363A9; the entire contents of the aforementioned application are incorporated herein by reference in its entirety.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration. In some embodiments, the pharmaceutical composition is administered orally.

The pharmaceutical compositions of the present disclosure may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g.*, through an IV drip) allows a much faster delivery which quickly raises the concentration of the active ingredient in the blood to an effective level. In other embodiments, the pharmaceutical composition may be administered as a continuous infusion, *e.g.,* by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

The above-described components for orally administrable, injectable or topically administrable compositions are merely representative. Other materials, as well as processing techniques and the like, are set forth in Part 8 of Remington's Pharmaceutical Sciences, 17th edition, 1985, Mack Publishing Company, Easton, Pennsylvania, which is incorporated herein by reference.

The compositions of the present disclosure can also be administered in sustained release forms or from sustained release drug delivery systems. A description of representative sustained release materials can be found in *Remington's Pharmaceutical Sciences.*

Although the descriptions of pharmaceutical compositions provided herein are principally directed to pharmaceutical compositions that are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

Another aspect of the disclosure includes a method of treating major depressive disorder in a subject in need thereof, comprising administering to the subject about 40 mg of Compound **(1)** once a day for 14 days. In one aspect, the disclosure includes a method of treating major depressive disorder in a subject in need thereof, comprising administering to the subject about 50 mg Compound **(1)** once a day for 14 days.

In embodiments of these aspects, the major depressive disorder is severe major depressive disorder. In some embodiments, the subject exhibits a reduction in depression-related symptoms. In some embodiments, the reduction in depression-related symptoms is characterized by a reduction in HAM-D score from baseline. In some embodiments, the major depressive disorder is characterized by a HAM-D total score of at least 22 prior to treatment. In some embodiments, the major depressive disorder is characterized by a HAM-D total score of at least 24 prior to treatment. In some embodiments, the major depressive disorder is characterized by a HAM-D total score of at least 25 prior to treatment. In some embodiments, the major depressive disorder is characterized by a HAM-D total score of at least 26 prior to treatment. In some embodiments, the major depressive disorder is characterized by a MADRS score of at least 28 prior to treatment. In some embodiments, the major depressive disorder is characterized by a MADRS score of at least 32 prior to treatment.

Another aspect of the disclosure includes a method of treating depression in a subject in need thereof, the method comprising the steps of (i) administering once daily to the subject about 40 mg of Compound **(1)** once a day for 14 days; and (ii) re-administering once daily to the subject about 30 mg of Compound **(1)** for 15 days in response to a recurrence of depression symptoms, provided there is at least a 6 week interval between administration of Compound **(1)** to the subject and re-administration of Compound **(1)** to the subject. In one aspect, the disclosure includes a method of treating depression in a subject in need thereof, the method comprising the steps of (i) administering once daily to the subject about 50 mg of Compound **(1)** once a day for 14 days; and (ii) re-administering once daily to the subject about 50 mg of Compound **(1)** for 15 days in response to a recurrence of depression symptoms, provided there is at least a 6 week interval between administration of Compound **(1)** to the subject and re-administration of Compound **(1)** to the subject.

In embodiments of these aspects, the depression is major depressive disorder or severe major depressive disorder. In some embodiments, the subject exhibits a reduction in depression-related symptoms. In some embodiments, the reduction in depression-related symptoms is characterized by a reduction in HAM-D score from baseline. In some embodiments, the major depressive disorder is characterized by a HAM-D total score of at least 20 prior to treatment. In some embodiments, the major depressive disorder is characterized by a HAM-D total score of at least 22 prior to treatment. In some embodiments, the major depressive disorder is characterized by a HAM-D total score of at least 24 prior to treatment. In some embodiments, the major depressive disorder is characterized by a HAM-D total score of at least 25 prior to treatment. In some embodiments, the major depressive disorder is characterized by a HAM-D total score of at least 26 prior to treatment. In some embodiments, the major depressive disorder is characterized by a MADRS score of at least 28 prior to treatment. In some embodiments, the major depressive disorder is characterized by a MADRS score of at least 29 prior to treatment. In some embodiments, the major depressive disorder is characterized by a MADRS score of at least 30 prior to treatment. In some embodiments, the major depressive disorder is characterized by a MADRS score of at least 3 1 prior to treatment. In some embodiments, the major depressive disorder is characterized by a MADRS score of at least 32 prior to treatment. In some embodiments, the major depressive disorder is characterized by a HAM-D total score of at least 20 and a MADRS score of at least 28 prior to treatment.

In some embodiments, the HAM-D score is HAM-D total score. In some embodiments, the HAM-D score is a HAM-D subscale score selected from the group consisting of Core Depression, Bech-6 and Maier HAM-D subscale score.

In some embodiments, the method improves general health status in the subject. In some embodiments, the improvement in general health status is characterized by a reduction in at least one domain SF-36v2 score from baseline. In some embodiments, the at least one domain is physical-functioning (PF), role-physical (RP), bodily pain (BP), general health (GH), vitality (V), social-functioning (SF), role-emotional (RE), or mental health (MH).

Another aspect of the disclosure includes a method of simultaneously treating depression and anxiety in a subject in need thereof, comprising administering to the subject about 40 mg of Compound **(1)** once a day for 14 days. In one aspect, the disclosure includes a method of simultaneously treating depression and anxiety in a subject in need thereof, comprising administering to the subject about 50 mg of Compound **(1)** once a day for 14 days.

In embodiments of these aspects, the depression is a major depressive disorder. In some embodiments, the major depressive disorder is severe major depressive disorder. In some embodiments, the subject exhibits a reduction in anxiety- and depression-related symptoms. In some embodiments, the reduction in depression-related symptoms is characterized by a reduction in HAM-D score from baseline. In some embodiments, the HAM-D score is HAM-D total score. In some embodiments, the HAM-D score is a HAM-D subscale score. In some embodiments, the HAM-D subscale score is Core Depression, Bech-6, or Maier score. In some embodiments, the anxiety-related symptoms is characterized by a reduction in HAM-A score from baseline. In some embodiments, the anxiety-related symptoms is characterized by a reduction in HAM-D anxiety subscale score from baseline. In some embodiments, the subject scores a HAM-D total score of at least 20 prior to treatment. In some embodiments, the subject scores a HAM-D total score of at least 21 prior to treatment. In some embodiments, the subject scores a HAM-D total score of at least 22 prior to treatment. In some embodiments, the subject scores a HAM-D total score of at least 24 prior to treatment. In some embodiments, the subject scores a HAM-D total score of at least 25 prior to treatment. In some embodiments, the subject scores a HAM-D total score of at least 26 prior to treatment. In some embodiments, the subject scores a MADRS total score of at least 28 prior to treatment. In some embodiments, the subject scores a MADRS total score of at least 32 prior to treatment. In some embodiments, the subject scores a HAM-D total score of at least 20 and a MADRS total score of at least 28 prior to treatment.

In some embodiments, the subject has a reduction from baseline of at least 13 points in HAM-D score and a reduction of at least 10 points in HAM-A score on Day 15 after the start of treatment. In some embodiments, the subject has a reduction from baseline of at least 14 points in HAM-D score and a reduction of at least 10 points in HAM-A score on Day 15 after the start of treatment. In some embodiments, the subject has a reduction from baseline of at least 16 points in MADRS score and a reduction of at least 10 points in HAM-A score on Day 15 after the start of treatment.

Another aspect of the disclosure includes a method of simultaneously treating a major depressive disorder and anxiety in a subject in need thereof, the method comprising the steps of (i) administering once daily to the subject about 40 mg of Compound **(1)** once a day for 14 days; and (ii) re-administering once daily to the subject about 30 mg of Compound **(1)** for 15 days in response to a recurrence of depression symptoms, provided there is at least a 6 week interval between administration of Compound **(1)** to the subject and re-administration of Compound **(1)** to the subject. In one aspect, the disclosure includes a method of simultaneously treating a major depressive disorder and anxiety in a subject in need thereof, the method comprising the steps of (i) administering once daily to the subject about 50 mg of Compound **(1)** once a day for 14 days; and (ii) re-administering once daily to the subject about 50 mg of Compound **(1)** for 15 days in response to a recurrence of depression symptoms, provided there is at least a 6 week interval between administration of Compound **(1)** to the subject and re-administration of Compound **(1)** to the subject.

In embodiments of these aspects, the major depressive disorder is severe major depressive disorder. In some embodiments, the subject exhibits a reduction in anxiety- and depression-related symptoms. In some embodiments, the reduction in depression-related symptoms is characterized by a reduction in HAM-D score from baseline. In some embodiments, the HAM-D score is HAM-D total score. In some embodiments, the HAM-D score is a HAM-D subscale score. In some embodiments, the HAM-D subscale score is Core Depression, Bech-6, or Maier score.

In some embodiments, the anxiety-related symptoms is characterized by a reduction in HAM-A score from baseline. In some embodiments, the anxiety-related symptoms is characterized by a reduction in HAM-D anxiety subscale score from baseline.

In some embodiments, the subject scores a HAM-D score of at least 20 prior to treatment. In some embodiments, the subject scores a HAM-D score of at least 21 prior to treatment. In some embodiments, the subject scores a HAM-D score of at least 22 prior to treatment. In some embodiments, the subject scores a HAM-D score of at least 24 prior to treatment. In some embodiments, the subject scores a HAM-D score of at least 25 prior to treatment. In some embodiments, the subject scores a HAM-D score of at least 26 prior to treatment. In some embodiments, the subject scores a MADRS score of at least 28 prior to treatment. In some embodiments, the subject scores a MADRS score of at least 32 prior to treatment. In some embodiments, the subject scores a HAM-D score of at least 20 and a MADRS score of at least 28 prior to treatment.

In some embodiments, the subject has a reduction from baseline of at least 13 points in HAM-D score and a reduction of at least 10 points in HAM-A score on Day 15 after the start of treatment. In some embodiments, the subject has a reduction from baseline of at least 14 points in HAM-D score and a reduction of at least 10 points in HAM-A score on Day 15 after the start of treatment. In some embodiments, the subject has a reduction from baseline of at least 16 points in MADRS score and a reduction of at least 10 points in HAM-A score on Day 15 after the start of treatment.

Another aspect of the disclosure includes a method of simultaneously treating depression and anxiety in a subject in need thereof, the method comprising administering to the subject a therapeutically effective amount of Compound **(1),** or a pharmaceutically acceptable salt thereof, using an episodic dosing regimen to simultaneously treat depression and anxiety in the subject.

In embodiments of this aspect, the episodic dosing regimen has a duration of about 2 to about 8 weeks. In some embodiments, the episodic dosing regimen has a duration of about 2 to about 6 weeks. In some embodiments, the episodic dosing regimen has a duration of about 2 to about 4 weeks. In some embodiments, the episodic dosing regimen has a duration of about 2 weeks or 14 days. In some embodiments, the episodic dosing regimen has a duration of 14 days.

In some embodiments, the subject exhibits a response to the episodic dosing regimen, wherein the response is indicated by greater than or equal to about 50% reduction from baseline in the subject's HAMD-17 total score and HAM-A total score. In some embodiments, the subject is evaluated for recurrence, or reappearance of depression symptoms. In some embodiments, the method comprises a plurality of episodic dosing regimens. In some embodiments, the episodic dosing regimens are spaced apart by at least a 6 week interval.

Another aspect of the disclosure includes a method of simultaneously treating depression and anxiety in a subject in need thereof, the method comprising the steps of: (i) administering once daily to the subject a therapeutically effective amount Compound **(1)** for about two weeks; and (ii) re-administering once daily to the subject a therapeutically effective amount of Compound **(1)** for about two weeks in response to a recurrence of depression symptoms, provided there is at least a 6 week interval between administration of Compound **(1)** to the subject and re-administration of Compound **(1)** to the subject.

In embodiments of this aspect, Compound **(1)** is re-administered to the subject for about 4 weeks. In some embodiments, Compound **(1)** is re-administered to the subject for about 2 weeks. In some embodiments, the interval between administration of Compound **(1)** to the subject and re-administration of Compound **(1)** to the subject is about 45 days. In some embodiments, the interval between administration of Compound **(1)** to the subject and re-administration of Compound **(1)** to the subject is about 8 weeks.

In some embodiments, the subject is a woman, and the depression is postpartum depression (PPD). In some embodiments, the subject is a woman and is about six months postpartum or less. In some embodiments, the PPD is defined as a major depressive episode with an onset at a point in time from the subject's 3rd trimester until 4 weeks postpartum.

In some embodiments, the subject scored 24 or greater on a HAMD-17 test prior to the administration of Compound **(1).** In some embodiments, the subject scored 26 or greater on a HAMD-17 test prior to the administration of Compound **(1).** In some embodiments, the subject scores 10 or less on a HAMD-17 test and 10 or less on a HAM-A test on day 15 after the start of the episodic dosing regimen. In some embodiments, the subject scores 7 or less on a HAMD-17 test and 7 or less on a HAM-A test on day 15 after the start of the episodic dosing regimen. In some embodiments, the subject scores 13 or less on a MADRS test and 10 or less on a HAM-A test on day 15 after the start of the episodic dosing regimen. In some embodiments, the subject scores 10 or less on a MADRS test and 7 or less on a HAM-A test on day 15 after the start of the episodic dosing regimen. In some embodiments, the subject scores 10 or less on a HAMD-17 test and 10 or less on a HAM-A test on day 45 after the start of the episodic dosing regimen. In some embodiments, the subject scores 7 or less on a HAMD-17 test and 7 or less on a HAM-A test on day 45 after the start of the episodic dosing regimen. In some embodiments, the subject scores 13 or less on a MADRS test and 10 or less on a HAM-A test on day 45 after the start of the episodic dosing regimen. In some embodiments, the subject scores 10 or less on a MADRS test and 7 or less on a HAM-A test on day 45 after the start of the episodic dosing regimen. In some embodiments, the subject is between about 18 and about 65 years of age. In some embodiments, the subject is between about 18 and about 45 years of age.

In some embodiments, the therapeutically effective amount is from about 25 mg to about 35 mg of Compound **(1).** In some embodiments, the therapeutically effective amount is about 30 mg of Compound **(1).** In some embodiments, the subject is administered the therapeutically effective amount of Compound **(1)** once per day. In some embodiments, the amount of Compound **(1)** administered to the subject is reduced in the occurrence of a severe adverse effect. In some embodiments, Compound **(1)** is administered in the evening. In some embodiments, Compound **(1)** is administered with food. In some embodiments, Compound **(1)** is in a capsule. In some embodiments, method further comprising administration of a second therapeutic agent.

Another aspect of the disclosure includes a method of simultaneously treating depression and anxiety in a subject in need thereof, using a kit comprising: a plurality of individual dosage units comprising Compound **(1),** and an instruction set, wherein the instruction set describes a method for administering the dosage units to the subject using an episodic dosing regimen.

In embodiments of this aspect, the episodic dosing regimen has a duration of about 2 to about 8 weeks. In some embodiments, the episodic dosing regimen has a duration of about 2 to about 6 weeks. In some embodiments, the episodic dosing regimen has a duration of about 2 to about 4 weeks. In some embodiments, the episodic dosing regimen has a duration of about 2 weeks. In some embodiments, the episodic dosing regimen has a duration of 2 weeks. In some embodiments, the subject is a woman and has been diagnosed with postpartum depression.

Another aspect of the disclosure includes a kit comprising a plurality of therapeutically efficacious dosages of Compound **(1)** and an instruction set describing a method of administering the dosages using an episodic dosing regimen for simultaneously treating depression and anxiety.

In embodiments of this aspect, the dosages are individual dosage units of Compound **(1).** In some embodiments, an individual dosage unit comprises from about 25 mg to about 35 mg of Compound **(1).** In some embodiments, an individual dosage unit comprises about 30 mg of Compound **(1).** In some embodiments, the episodic dosing regimen has a duration of about 2 to about 8 weeks. In some embodiments, the episodic dosing regimen has a duration of about 2 to about 6 weeks. In some embodiments, the episodic dosing regimen has a duration of about 2 to about 4 weeks. In some embodiments, the episodic dosing regimen has a duration of about 2 weeks or 14 days. In some embodiments, the episodic dosing regimen has a duration of 2 weeks. In some embodiments, the depression is postpartum depression (PPD).

In some embodiments, the instruction set is printed on a suitable material. In some embodiments, the individual dosage units are capsules or tablets. In some embodiments, the individual dosage unit is a capsule. In some embodiments, the individual dosage unit is a capsule of size 1, 2, 3, or 4. In some embodiments, the capsule is size 1.

In some embodiments, the method improves cognitive function in the subject. In some embodiments, the method provides no cognitive impairment in the subject.

In some embodiments, the subject also experiences insomnia prior to the administration of Compound **(1).** In some embodiments, the subject scores at least 3 points less on a HAMD-17-Ins test on day 3, after the start of the episodic dosing regimen, compared to the subject's HAMD-17-Ins test score prior to the administration of Compound **(1).** In some embodiments, the subject scores at least 2 points less on a MADRS-Ins test on day 3, after the start of the episodic dosing regimen, compared to the subject's MADRS-Ins test score prior to the administration of Compound **(1).** In some embodiments, the subject scores at least 3 points less on a HAMD-17-Ins test on day 15, after the start of the episodic dosing regimen, compared to the subject's HAMD-17-Ins test score prior to the administration of Compound **(1).** In some embodiments, the subject scores at least 2 points less on a MADRS-Ins test on day 15, after the start of the episodic dosing regimen, compared to the subject's MADRS-Ins test score prior to the administration of Compound **(1).** In some embodiments, the subject scores at least 3 points less on a HAMD-17-Ins test on day 45, after the start of the episodic dosing regimen, compared to the subject's HAMD-17-Ins test score prior to the administration of Compound **(1).** In some embodiments, the subject scores at least 2 points less on a MADRS-Ins test on day 45, after the start of the episodic dosing regimen, compared to the subject's MADRS-Ins test score prior to the administration of Compound **(1).**

In some embodiments, the subject scores at least 5 points less on a HAMD-17-A/S test on day 15, after the start of the episodic dosing regimen, compared to the subject's HAMD-17-A/S test score prior to the administration of Compound **(1).**

In some embodiments, the subject scores at least 2 points less on an EPDS-3A test on day 15, after the start of the episodic dosing regimen, compared to the subject's EPDS-3A test score prior to the administration of Compound **(1).**

In some embodiments, the subject scores at least 5 points less on a HAMD-17-A/S test on day 45, after the start of the episodic dosing regimen, compared to the subject's HAMD-17-A/S test score prior to the administration of Compound **(1).**

In some embodiments, the subject scores at least 3 points less on an EPDS-3A test on day 45, after the start of the episodic dosing regimen, compared to the subject's EPDS-3A test score prior to the administration of Compound **(1).**

In some embodiments, the methods described herein improve general health status in the subject.

In some embodiments, the subject scores at least 10 points higher in five domains of a SF-36v2 test on day 15, after the start of the episodic dosing regimen, compared to the subject's SF-36v2 test score prior to the administration of Compound **(1).** In some embodiments, the subject scores at least 10 points higher in five domains of a SF-36v2 test on day 45, after the start of the episodic dosing regimen, compared to the subject's SF-36v2 test score prior to the administration of Compound **(1).** In some embodiments, the five domains of the SF-36v2 test are social functioning, mental health, physical functioning, role physical, bodily pain, and mental health component summary.

### EXAMPLES

**Example 1. A Phase 3, Multicenter, Double-Blind, Randomized, Placebo-Controlled Study Evaluating the Efficacy of Compound (1) in the Treatment of Adult Subjects with Major Depressive Disorder.**

**List of Abbreviations**

| | |
|---|---|
| ADR | Adverse drug reaction |
| AE | adverse event |
| AUC | Area under the curve |
| C_{avg} | Average plasma concentration |
| CGI-I | Clinical Global Impression - Improvement |
| CGI-S | Clinical Global Impression - Severity |
| Cmax | Maximum plasma concentration |
| CRF | Case report form |
| CS | clinically significant |
| C-SSRS | Columbia Suicide Severity Rating Scale |
| CYP | cytochrome P450 |
| DSM-5 | Diagnostic and Statistical Manual of Mental Disorders, Fifth Edition |
| ECG | Electrocardiogram |
| eCRF | electronic case report form |
| EOT | end of treatment |
| ET | early termination |
| FSH | follicle stimulating hormone |
| GABA | γ-aminobutyric acid |
| GEE | Generalized estimating equation |
| HAM-A | Hamilton Rating Scale for Anxiety |
| HAM-D | Hamilton Rating Scale for Depression |
| HCV | hepatitis C virus |
| HIV | human immunodeficiency virus |
| ICF | informed consent form |
| ID | identification |
| IRB | institutional review board |
| IRT | interactive response technology |
| ISI | Insomnia Severity Index |
| MADRS | Montgomery-Åsberg Depression Rating Scale |
| MDD | major depressive disorder |
| mFAS | Modified full analysis set |
| MGH ATRQ | Massachusetts General Hospital Antidepressant Treatment Response Questionnaire |
| MMRM | Mixed effects model for repeated measures |
| MTD | Maximum tolerated dose |
| NCS | not clinically significant |
| PCS | Potentially clinically significant |
| PHQ-9 | 9-item Patient Health Questionnaire |
| PK | pharmacokinetic(s) |
| PRO | Patient-reported outcome |
| PWC-20 | 20-item Physician Withdrawal Checklist |
| PSQ | patient status questionnaire |
| QTcF | QT corrected according to Fridericia's formula |
| SAE | serious adverse event |
| SAP | statistical analysis plan |
| SCID-5-CT | Structured Clinical Interview for Diagnostic and DSM-5 Clinical Trial Version |
| SD | standard deviation |
| SDS | Sheehan Disability Scale |
| SF-36v2 | 36-item Short Form version 2 |
| SUSAR | suspected, unexpected, serious adverse reactions |
| TEAE | treatment-emergent adverse event |
| WHO | World Health Organization |

**Title of Study:** A Phase 3, Multicenter, Double-Blind, Randomized, Placebo-Controlled Study Evaluating the Efficacy of Compound **(1)** in the Treatment of Adult Subjects with Major Depressive Disorder

### ClinicalTrials.gov Identifier: NCT04442490

Planned Duration of Subject Participation: Up to 70 days (up to 28-day Screening Period, 14-day Double-blind Treatment Period, and a 28-day double-blind Follow-up Period).

### Introduction

Major depressive disorder (MDD) is a complex and heterogeneous disorder and one of the leading causes of disability in the US and worldwide. In 2019, 19.4 million adults in the US had experienced a major depressive episode within the past year and approximately 12.8 million received treatment. Patients with MDD experience depressive episodes that are associated with serious functional impairment; in the US, approximately 60% of adults experiencing a major depressive episode in the past year also experienced severe or very severe impairment in at least one role domain (social, occupational, educational, or family) as measured by the Sheehan Disability Scale. The goals of treatment for MDD are remission of symptoms, prevention of relapse and recurrence, and improvement in quality of life by alleviating functional impairment.

The standard-of-care therapies for MDD, such as selective serotonin reuptake inhibitors, often require weeks or months to improve depressive symptoms. (Rush AJ, et al. Acute and longer-term outcomes in depressed outpatients requiring one or several treatment steps: a STAR*D report. Am J Psychiatry 2006; 163: 1905-1917. 2006). These therapies are also associated with several adverse side effects such as suicidal ideation, weight gain, sexual dysfunction, cognitive impairment, insomnia, anxiety, and sedation, which may result in discontinuation of therapy. (Gelenberg AJ FM, Markowitz JC. APA Practice Guideline for the Treatment of Patients With Major Depressive Disorder. Am J Psychiatry 2010: 1-152). Variable treatment response and adverse side effects result in patients with MDD often requiring multiple changes in antidepressant therapy (ADT) to achieve remission of symptoms. In the Sequenced Treatment Alternatives to Relieve Depression (STAR*D, NCT00021528) study, remission rates in patients with MDD declined when multiple regimens of ADT were required (first treatment 36.8% remission vs 13.0% remission for fourth treatment). The delay in treatment response and frequent need for therapy change with standard-of-care therapies is a barrier to treatment and fuels an extended time of disability and a delay in functional recovery. There is an unmet need for treatment options that have a rapid onset of action, a high response and remission rate, and minimize the adverse side effects commonly seen with standard-of-care ADTs.

To address this unmet need, new therapies with novel mechanisms of action are being developed. For example, esketamine, a drug that modulates the activity of the excitatory neurotransmitter glutamate, in the SUSTAIN-1 trial (NCT02493868), demonstrated efficacy for treatment-resistant depression. γ-aminobutyric acid (GABA) is the primary inhibitory neurotransmitter in the brain and serves an important role in adaptive signaling by balancing excitatory glutamate neurotransmission. Levels of GABA are reduced in the plasma and cerebrospinal fluid of patients with depression. However, the therapeutic potential of modulating GABA signaling for the treatment of MDD is underexplored.

Inhibitory signaling through GABA is influenced endogenously by neuroactive steroids such as the progesterone metabolite allopregnanolone. Allopregnanolone potentiates both phasic and tonic inhibitory neurotransmission through modulation of synaptic and extrasynaptic GABA type A receptors (GABAARs), respectively. This is distinct from the mechanism of benzodiazepines, which only target phasic inhibition and synaptic GABAARs. Tonic and phasic inhibition mediate transient and prolonged GABAergic signaling, and targeting both therapeutically may achieve both rapid onset and sustained effects of treatment and have potential for the treatment of depression.

### Objectives

Primary: Evaluated the efficacy of Compound **(1)** in the treatment of major depressive disorder (MDD) compared to placebo.

Secondary: Assessed patient-reported outcome (PRO) measures as they relate to health-related quality of life and depressive symptoms.

Safety Objective: evaluated the safety and tolerability of Compound **(1).**

Other Objective(s): assessed the PK of Compound **(1)** using a population PK approach.

### Endpoints

Primary Endpoint:
- The primary endpoint of this study was the change from baseline in the 17-item HAM-D total score at Day 15

Key Secondary Endpoints:
- Change from baseline in CGI-S at Day 15
- Change from baseline in HAM-D total score at Day 8, Day 3, and Day 42
Key secondary endpoints tested sequentially with a significance level of 0.05 at each step (formal testing stopped if the p-value at any step was >0.05)

Other Secondary Endpoints:
- HAM-D Response (≥50% reduction in HAM-D total score since baseline) at Day 15 and Day 42
- HAM-D Remission (HAM-D ≤7) at Day 15 and Day 42
- CGI-I Response, defined as "much improved" or "very much improved", at Day 15
- Change from baseline in MADRS total score at Day 15
- Change from baseline in HAM-A total score at Day 15
- Time to first HAM-D Response
- Change from baseline in PRO measures of health-related quality of life, as assessed by responses to the SF-36v2, and of depressive symptoms as assessed by the PHQ-9

Safety Endpoints:
- Incidence and severity of adverse events/serious adverse events
- Changes from baseline in clinical laboratory measures, vital signs, and electrocardiograms (ECGs)
- Suicidal ideation and behavior using the Columbia Suicide Severity Rating Scale (C-SSRS)
- Potential withdrawal symptoms using the 20-item Physician Withdrawal Checklist (PWC-20)

Other endpoints:
- PK parameters (*e.g.,* clearance) and exposure estimates (*e.g.,* area under the curve over a dosing interval, maximum plasma concentration) as assessed via population PK methods.

### Study Description

This was a randomized, double-blind, parallel-group, placebo-controlled study in subjects with MDD. The diagnosis of MDD was made according to Structured Clinical Interview for Diagnostic and DSM-5 Clinical Trial Version (SCID-5-CT) performed by a qualified healthcare professional.

The study had a Screening Period of up to 28 days, a 14-day Treatment Period, and a 28-day double-blind follow-up period.

The Screening Period began with the signing of the ICF at the Screening Visit; the ICF must have been signed prior to beginning any screening activities. At the time of providing informed consent for the study, subjects were also asked to authorize that their unique subject identifiers be entered into a registry (www.subjectregistry.com) with the intent of identifying subjects who may meet exclusion criteria due to participation in another clinical study.

Subjects underwent preliminary screening procedures at the Screening Visit to determine eligibility, including completion of the HAM-D and CGI-S.

Antidepressants were permitted, provided subjects were on a stable dose for at least 60 days prior to Day 1 and agreed to continue on the stable dose through the follow-up period (Day 42). Initiation of new antidepressants or any other medications that could have potentially have an impact on efficacy or safety endpoints were not allowed between screening and completion of the Day 42 assessments.

Eligible subjects were stratified based on use of antidepressant treatment (current/stable or not treated/withdrawn ≥60 days) and randomized within each stratum to one of 2 treatment groups (Compound **(1)** - 50 mg or matching placebo) in a 1:1 ratio. Subjects self-administered study drug once daily at approximately 8 PM with fat-containing food (*e.g.,* within 1 hour of an evening meal which contains fat or with a fat-containing snack), on an outpatient basis, for 14 days. Subjects returned to the study center during the treatment and follow-up periods as outlined in Table 1.

During the treatment period, subjects were be able to receive study drug as long as there were no dose limiting safety/tolerability concerns. Subjects who couldn't tolerate 50 mg received 40 mg for the remainder of the treatment period. At the discretion of the Investigator, subjects who couldn't tolerate the 40-mg dose could be discontinued from study drug.

### Number of Subjects

Up to 575 subjects were randomized and dosed to obtain sufficient evaluable subjects for all analyses.

### Treatment Assignment

Subjects were randomly assigned to a treatment group on Day 1 and were stratified based on use of antidepressant treatment (current/stable or not treated/withdrawn ≥60 days) at baseline. Randomization was performed within each stratum in a 1:1 ratio to receive Compound **(1)** 50 mg or matching placebo.

### Dose Adjustment Criteria

Subjects were able to receive study drug as long as there were no dose limiting safety/tolerability concerns. Subjects who couldn't tolerate 50 mg received 40 mg for the remainder of the treatment period. At the discretion of the Investigator, subjects who couldn't tolerate the 40-mg dose could be discontinued from study drug.

### Subject Inclusion Criteria

Qualified subjects had to meet all of the following criteria:
1. Subject has signed an ICF prior to any study-specific procedures being performed.
2. Subject was a male or female between 18 and 64 years of age, inclusive.
3. Subject was in good physical health and has no clinically significant findings, as determined by the Investigator, on physical examination, 12-lead ECG, or clinical laboratory tests.
4. Subject agreed to adhere to the study requirements, including not participating in night shift work.
5. Subject had a diagnosis of MDD as diagnosed by SCID-5-CT, with symptoms that have been present for at least a 4-week period.
6. Subject had a HAM-D total score ≥24 at screening and Day 1 (prior to dosing).
7. Subjects taking antidepressants must have been taking these medications at the same dose for at least 60 days prior to Day 1. Subjects who stopped taking antidepressants within 60 days must have stopped for longer than 5 half-lives of the antidepressant prior to Day 1. Subjects receiving psychotherapy must have been receiving therapy on a regular schedule for at least 60 days prior to Day 1.
8. Subject was willing to delay start of other antidepressant or antianxiety medications and any new pharmacotherapy regimens, including as-needed benzodiazepine anxiolytics and sleep aids, until after completion of the Day 42 visit.
9. Female subject agreed to use one of the following methods of contraception during the treatment period and for 30 days following the last dose of study drug, unless she was postmenopausal (defined as no menses for 12 months without an alternative medical cause and confirmed by follicle stimulating hormone [FSH] >40 mIU/mL), surgically sterile (hysterectomy or bilateral oophorectomy), or did not engage in sexual relations which carry a risk of pregnancy:
   - Combined (estrogen and progestogen containing) oral, intravaginal, or transdermal hormonal contraception associated with inhibition of ovulation.
   - Oral, injectable, or implantable progestogen-only hormonal contraception associated with inhibition of ovulation.
   - Intrauterine device
   - Intrauterine hormone-releasing system
   - Bilateral tubal ligation/occlusion
   - Vasectomized partner
10. Male subject agreed to use an acceptable method of effective contraception during the treatment period and for 5 days after receiving the last dose of the study drug, unless the subject did not engage in sexual relations which carry a risk of pregnancy. Acceptable methods of effective contraception for males included vasectomy, or a condom with or without spermicide used together with highly effective female contraception methods if the female partner was of child-bearing potential (see Inclusion Criteria #9 for acceptable contraception methods).
11. Male subject were willing to abstain from sperm donation the treatment period and for 5 days after receiving the last dose of the study drug.
12. Subject agreed to refrain from drugs of abuse and alcohol for the duration of the study.

### Subject Exclusion Criteria

Subjects who met any of the following criteria were disqualified from participation in the study:
1. Subject was at significant risk of suicide, as judged by the Investigator, or had attempted suicide associated with the current episode of MDD.
2. Subject had onset of the current depressive episode during pregnancy or 4 weeks postpartum, or the subject had presented for screening during the 6-month postpartum period.
3. Subject had a recent history or active clinically significant manifestations of metabolic, hepatic, renal, hematological, pulmonary, cardiovascular, gastrointestinal, musculoskeletal, dermatological, urogenital, neurological, or eyes, ears, nose, and throat disorders, or any other acute or chronic condition that, in the Investigator's opinion, would limit the subject's ability to complete or participate in this clinical study. A BMI ≤18 or ≥45 kg/m2 was exclusionary; a BMI of 40 to 44.9 kg/m2, inclusive, at Screening was subject to a broader evaluation of medical comorbidities as described above.
4. Subject had treatment-resistant depression, defined as persistent depressive symptoms despite treatment with adequate doses of antidepressants within the current major depressive episode (excluding antipsychotics) from two different classes for at least 4 weeks of treatment. Massachusetts General Hospital Antidepressant Treatment Response Questionnaire (MGH ATRQ) will be used for this purpose.
5. Subject had vagus nerve stimulation, electroconvulsive therapy, or has taken ketamine within the current major depressive episode.
6. Subject had a known allergy to Compound **(1),** allopregnanolone, or related compounds.
7. Subject had a positive pregnancy test at screening or on Day 1 prior to the start of study drug administration or, if breastfeeding at Screening or on Day 1 (prior to administration of study drug), she did not agree to temporarily cease giving breast milk to her child(ren) from just prior to receiving study drug on Day 1 until 7 days after the last dose of study drug.
8. Subject had detectable hepatitis B surface antigen (HBsAg), anti-hepatitis C virus (HCV) and positive HCV viral load, or human immunodeficiency virus (HIV) antibody at screening.
9. Subject had a clinically significant abnormal 12-lead ECG at the screening or baseline visits. NOTE: mean QT interval calculated using the Fridericia method (QTcF) of >450 msec in males or >470 msec in females will be the basis for exclusion from the study.
10. Subject had active psychosis per Investigator assessment.
11. Subject had a medical history of seizures.
12. Subject had a medical history of bipolar disorder, schizophrenia, and/or schizoaffective disorder.
13. Subject had a history of mild, moderate, or severe substance use disorder (including benzodiazepines) diagnosed using DSM-5 criteria in the 12 months prior to screening.
14. Subject had exposure to another investigational medication or device within 30 days prior to screening.
15. Subject had previously participated in a Compound **(1)** or a SAGE-547 (brexanolone) clinical trial.
16. Used any known strong inhibitors of cytochrome P450 (CYP)3A4 within 28 days or five half-lives (whichever is longer) or consumed grapefruit juice, grapefruit, or Seville oranges, or products containing these within 14 days prior to the first dose of study drug.
17. Used any strong CYP3A inducer, such as rifampin, carbamazepine, enzalutamide, mitotane, phenytoin, or St John's Wort, within 28 days prior to the first dose of study drug.
18. Subject had a positive drug and/or alcohol screen at screening or on Day 1 prior to dosing.
19. Subject planned to undergo elective surgery before completion of the Day 42 visit.
20. Subject was taking benzodiazepines, barbiturates, or GABAA modulators (e.g., eszopiclone, zopiclone, zaleplon, and zolpidem) at Day -28, or had been using these agents daily or near-daily (≥4 times per week) for more than 1 year. Subject was taking any benzodiazepine or GABA modulator with a half-life of ≥48 hours (e.g., diazepam) from 60 days prior to Day 1.
21. Subject was taking non-GABA anti-insomnia medications (e.g., melatonin, Benadryl [anti-histamines], trazodone), or first or second generation (typical/atypical) antipsychotics at Day -14.
22. Subject had been diagnosed with and/or treated for any type of cancer (excluding basal cell carcinoma and melanoma in situ) within the past year prior to Screening.
23. Subject had a history of sleep apnea.
24. Subject had gastric bypass surgery, a gastric sleeve or lap band, or had any related procedures that interfere with gastrointestinal transit.
25. Subject was taking psychostimulants (*e.g.,* methylphenidate, amphetamine) or opioids, regularly or as-needed, at Day -28.

### Study Drug

Subjects self-administered Compound **(1)** (50 mg or 40 mg [for dose adjustments only as permitted]) or matching placebo orally once daily at approximately 8 PM with food for 14 days. The 50-mg and 40-mg doses was administered as 2 capsules per dose (50 mg, administered as one 30 mg-capsule and one 20-mg capsule, and 40-mg, administered as two 20-mg capsules). Placebo was also administered as 2 capsules to maintain the blind.

### Prior Medications, Concomitant Medications, and Restrictions

### Prior and Concomitant Medications and/or Supplements

The start and end dates, route, dose/units, frequency, and indication for all medications and/or supplements taken within 30 days prior to Screening and throughout the duration of the study were recorded. In addition, psychotropic medications taken 6 months prior to Screening were recorded.

Any medication and/or supplement determined necessary for the welfare of the subject could be given at the discretion of the Investigator at any time during the study.

Antidepressants that had been taken at the same dose for at least 60 days prior to Day 1 were permitted if the subject intended to continue the stable dose through Day 42.

The following medications intended for contraception were permitted for female subjects:
- Combined (estrogen and progestogen containing) oral, intravaginal, or transdermal hormonal contraception associated with inhibition of ovulation
- Oral, injectable, or implantable progestogen-only hormonal contraception associated with inhibition of ovulation
- Intrauterine device
- Intrauterine hormone-releasing system

### Prohibited Medications

The following specific classes of medications were prohibited:
- Initiation of new psychotropic medications through the Day 42 visit
- Initiation of new antidepressant therapy from 60 days prior to Day 1 through the Day 42 visit
- Use of any benzodiazepines, barbiturates, GABAA modulators, GABA-containing agents from Day -28 through the Day 42 visit (from Day -60 for benzodiazepine or GABA modulators with a half-life of ≥48 hours)
- Chronic or as-needed psychostimulants (*e.g.,* methylphenidate, amphetamine) or opioids from Day -28 through the Day 42 visit
- First generation (typical) antipsychotics (*e.g*., haloperidol, perphenazine) and second generation (atypical) antipsychotics (*e.g*., aripiprazole, quetiapine) from Day -14 through the Day 42 visit
- Use of any non-GABA anti-insomnia medications (*e.g*., melatonin, Benadryl [anti-histamines], trazodone, low dose quetiapine, mirtazapine, etc.) from Day -14 through the Day 42 visit
- Exposure to another investigational medication or device from 30 days prior to Screening through the Day 42 visit
- Any known strong inhibitors CYP3A4 from Day -28 or 5 half-lives prior to Day 1 (whichever is longer) through the treatment period
- Use of any strong CYP3A inducer, such as rifampin, carbamazepine, enzalutamide, mitotane, phenytoin, or St John's Wort from Day -28 through the treatment period.

### Other Restrictions

The consumption of grapefruit juice, grapefruit, or Seville oranges, or products containing these was prohibited throughout the treatment period.

Consumption of alcohol or use of drugs of abuse was discouraged throughout the duration of the study.

Female subjects who were lactating or actively breastfeeding must have stopped giving breast milk to the baby(ies) starting on Day 1 until 7 days after the last dose of study drug.

Elective surgeries or procedures were prohibited through the Day 42 visit.

Subjects must not have participated in night shift work.

Subjects who were feeling sedated, somnolent, and/or dizzy were to refrain from driving or engaging in any activity requiring alertness.

Subjects receiving psychotherapy on a regular schedule for at least 60 days prior to Day 1 were permitted if the subject intended to continue the stable dose through the follow-up period (Day 42).

### Description of Study Drug

Compound **(1)** was available as hard gelatin capsules containing a white to off-white powder. In addition to the specified amount of Compound **(1)** Drug Substance, active Compound **(1)** Capsules contained croscarmellose sodium, mannitol, silicified microcrystalline cellulose (SMCC), colloidal silicon dioxide, and sodium stearyl fumarate as excipients. Capsules were available in 20-mg and 30-mg dose strengths.

Matching placebo capsules were hard gelatin capsules containing only the excipients listed for the active capsule.

### Study Drug Packaging and Labeling

Compound **(1)** capsules and matched placebo capsules were provided to the clinic pharmacist and/or designated site staff responsible for dispensing the study drug in appropriately labeled, subject-specific kits containing sealed unit doses. Each unit dose consisted of 2 capsules.

Additional information regarding the packaging and labeling was provided in the Pharmacy Manual.

### Statistical methods:

A detailed description of the statistical analyses to be performed in the study was provided in the Statistical Analysis Plan (SAP). Separate SAPs were generated for each part of the study. The SAP for each study part was finalized and approved prior to treatment unblinding of each respective part.

### General Considerations

For the purpose of all safety and efficacy analyses where applicable, baseline was defined as the last measurement prior to the start of study drug administration.

Continuous endpoints were summarized descriptively with n, mean, standard deviation, median, minimum, and maximum. In addition, change from baseline values were calculated at each time point and summarized descriptively. For categorical endpoints, descriptive summaries included counts and percentages.

Analysis Sets
- The Randomized Set was defined as all subjects who were randomized.
- The Safety Set was defined as all subjects administered study drug.
- The Full Analysis Set was defined as all randomized subjects in the Safety Set with a valid baseline HAM-D total score at least 1 post-baseline HAM-D total score.
- The Modified Full Analysis Set (mFAS) was defined as all participants in the FAS with a total HAM-D score ≥ 26 at baseline.
- The PK Set was defined as all subjects in the Safety Set with at least 1 plasma concentration.

### Determination of Sample Size

Assuming a two-sided alpha level of 0.05, a sample size of 216 evaluable subjects would provide 90% power to detect a placebo-adjusted treatment difference of approximately 4 points in the primary endpoint, change from baseline in HAM-D total score at Day 15, assuming a standard deviation (SD) of 9 points. Assuming a 10% dropout rate and a 1:1 randomization ratio within each stratum (antidepressant use at baseline, yes or no), approximately 240 total randomized subjects were required to obtain 216 evaluable subjects. Evaluable subjects were defined as those randomized subjects who received study drug and had valid baseline and at least 1 post-baseline HAM-D assessment. To provide more power for analyses of key secondary endpoints, up to 575 subjects were randomized.

### Analysis of Primary Endpoint

The estimand for the primary efficacy analysis was the mean change from baseline in HAM-D total score at Day 15. This was analyzed using a mixed effects model for repeated measures (MMRM); the model included treatment, baseline HAM-D total score, stratification factor, assessment time point, and time point-by-treatment as explanatory variables. All explanatory variables were treated as fixed effects. All post-baseline time points were included in the model. The main comparison was between Compound **(1)** and placebo at the 15-day time point. Model-based point estimates (*e.g*., least squares [LS] means, 95% confidence intervals, and p-values) were reported where applicable. An unstructured covariance structure was used to model the within-subject errors. If there was a convergence issue with the unstructured covariance model, Toeplitz, or Autoregressive **(1)** [AR(**1**)] covariance structure was used, following this sequence until convergence was achieved. If the model still did not converge with AR(**1**) structure, no results were reported. When the covariance structure was not UN, the sandwich estimator for the variance covariance matrix was derived, using the EMPIRICAL option in the PROC MIXED statement in SAS.

### Analysis of Secondary Endpoints

Similar to those methods described above for the primary endpoint, an MMRM was used for the analysis of the change from baseline in other time points in HAM-D total score, MADRS total score, HAM-A total score, SF-36v2 score, PHQ-9 score, and selected individual items and/or subscale scores in HAM-D for change from baseline.

Generalized estimating equation (GEE) methods were used for the analysis of HAM-D Response (defined as ≥50% reduction from baseline in HAM-D total score) and HAM-D Remission (defined as HAM-D total score of ≤7.0). GEE models included terms for treatment, baseline score, stratification factor, assessment time point, and time point-by-treatment as explanatory variables. The comparison of interest was the difference between Compound **(1)** and matching placebo at the 15-day time point. Model-based point estimates (*e.g.,* odds ratios), 95% confidence intervals, and p-values were reported.

A GEE method was also used for the analysis of CGI-I Response including terms for treatment, baseline CGI-S score, stratification factor, assessment time point, and time point-by-treatment as explanatory variables.

### Safety Analysis

Safety and tolerability of study drug was evaluated by incidence of adverse events/serious adverse events, vital signs, clinical laboratory evaluations, and 12-lead ECG.

Suicidality was monitored by the C-SSRS. Potential withdrawal symptoms after discontinuation of Compound **(1)** was assessed using the PWC-20.

### Pharmacokinetic Analysis

Compound **(1)** concentration-time data was evaluated using a nonlinear mixed-effects model. The model was used to estimate population PK parameters and identify any covariates that could have contributed to observed variability. Data from this study could be combined with data from other studies to support the analyses and could be reported separately.

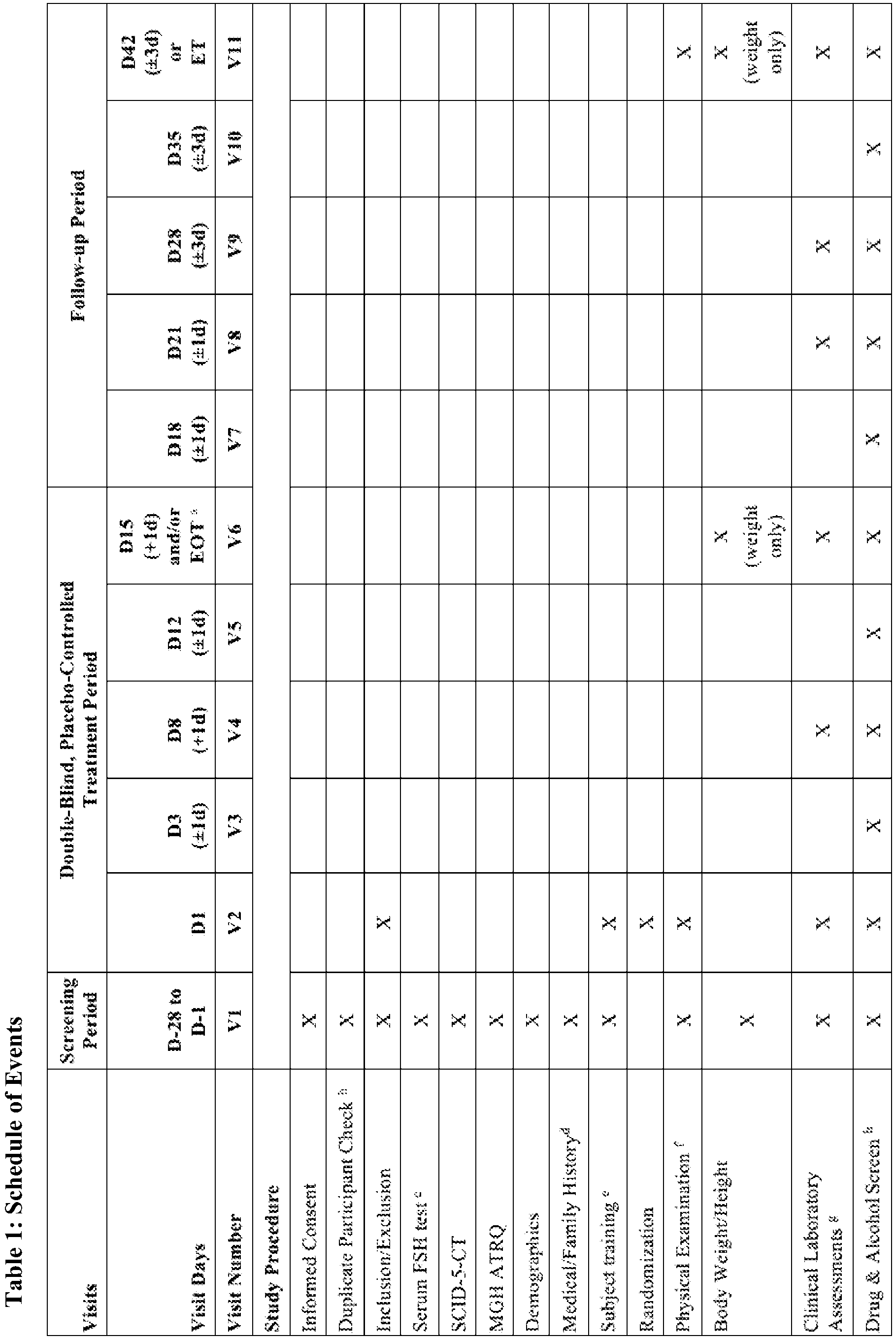

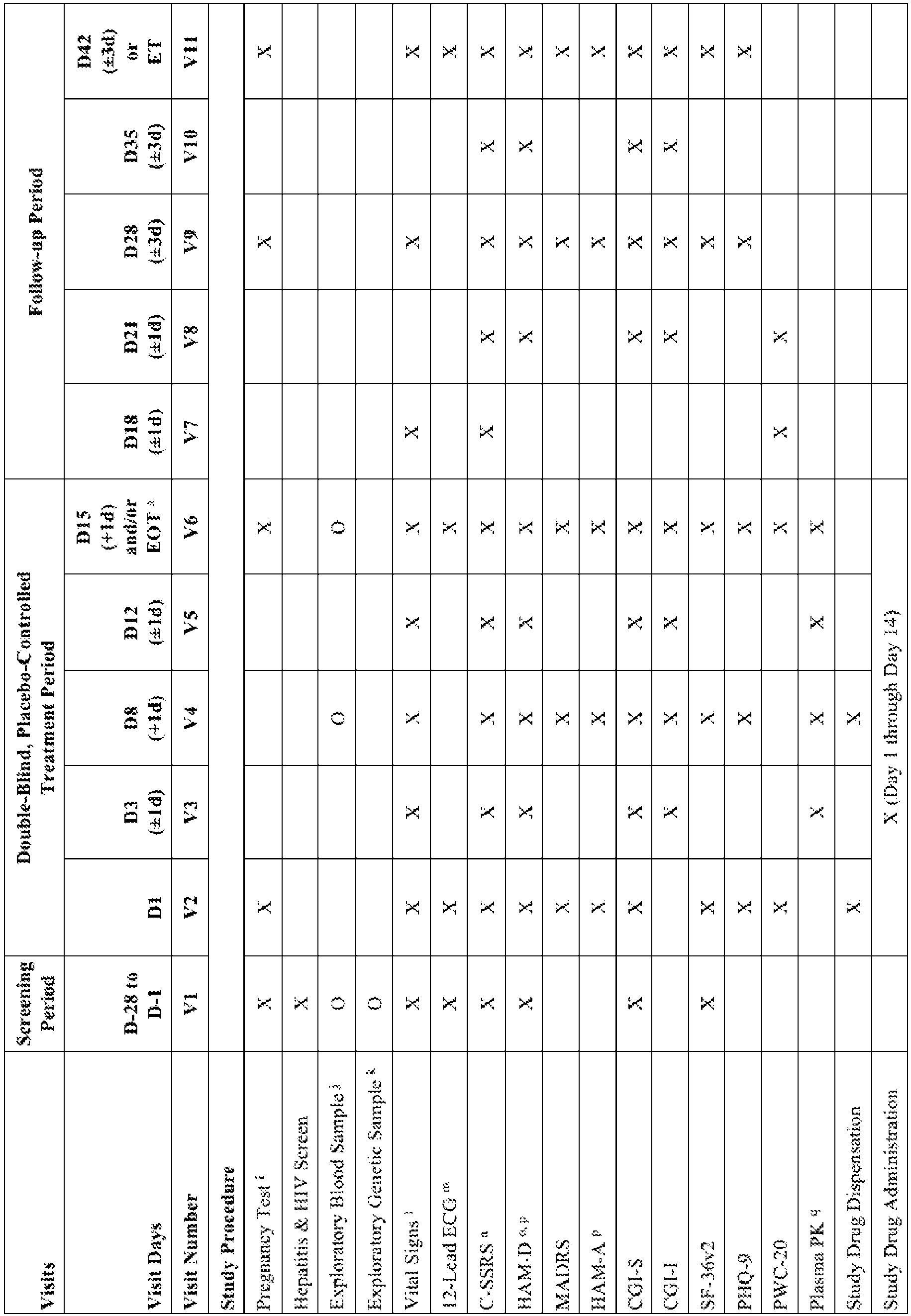

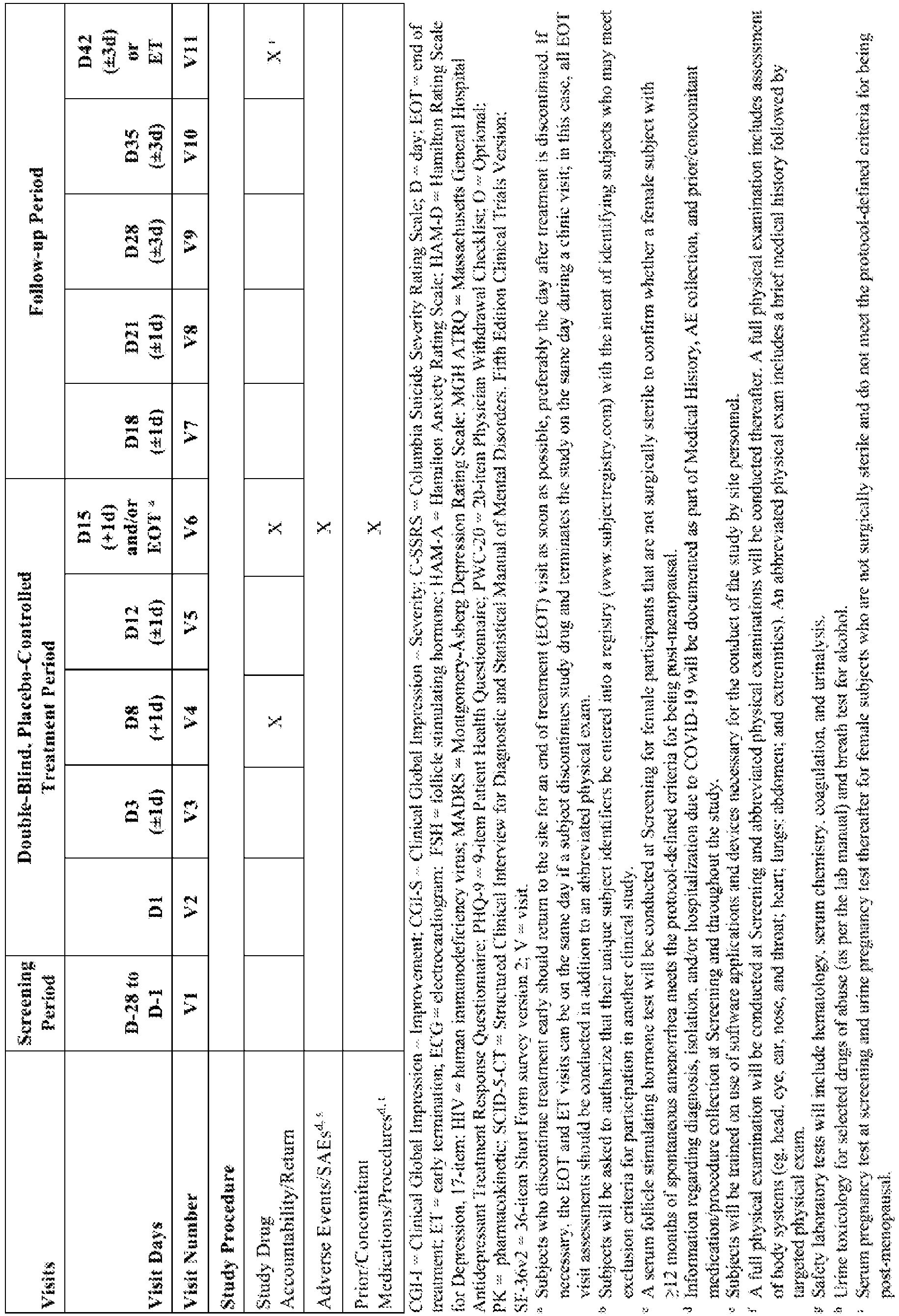

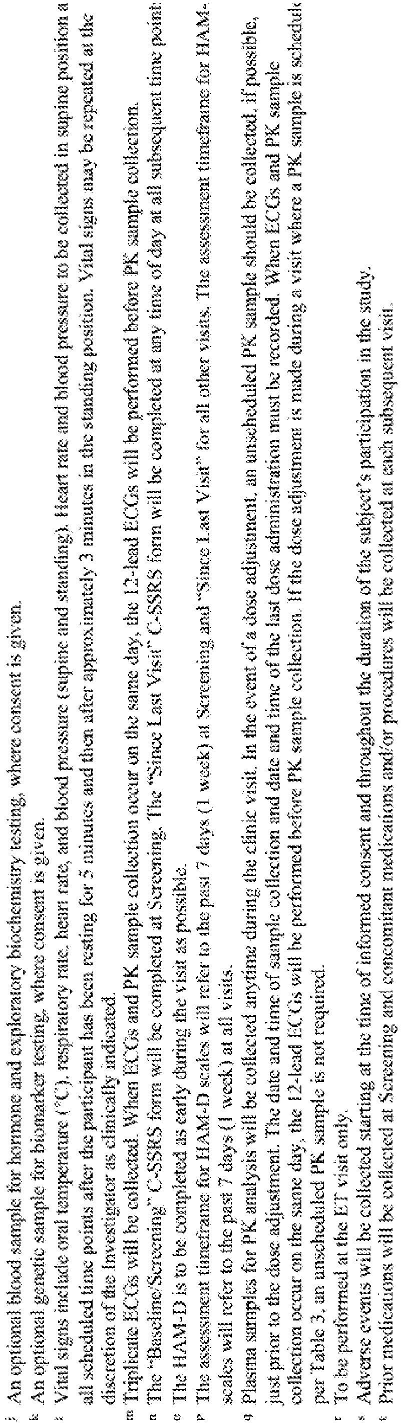

### Example 2. Efficacy Results of the Phase 3 Study of Compound (1) in Major Depressive Disorder (MDD) of Example 1.

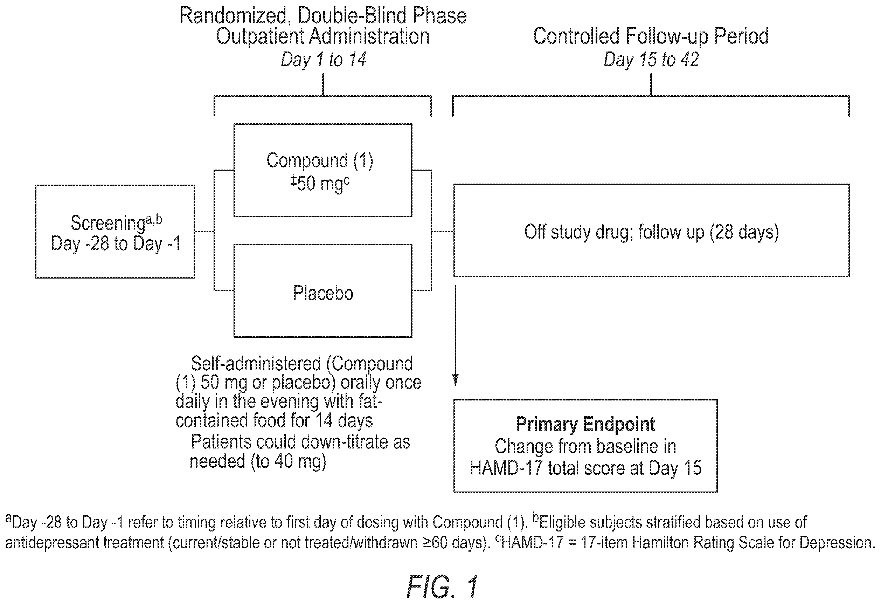

Compound **(1)** is an investigational two-week, once-daily oral drug for MDD that represents a potential new class of drug for the management of this common but serious mental health disorder. Compound **(1)** was evaluated in a double-blind, randomized placebo-controlled Phase 3 study (NCT04442490) in Major Depressive Disorder (MDD) as described in Example 1. The study evaluated the efficacy, safety, tolerability and pharmacokinetics of Compound **(1)** in adult patients diagnosed with MDD (HAM-D total score ≥24). FIG. 1 exemplifies the study design.

### Dosage

Compound **(1)** was self-administered in 50 mg daily doses once daily in the evening with fat-containing food for 14 days. Patients could down-titrate as needed to 40 mg.

### 2.1 Disposition and Demographics

The mean baseline HAMD-17 score at entry into the study was 26.8 (2.60) in the Compound **(1)** 50 mg treatment group (n=268) and 26.9 (2.67) in the placebo group (n=269). Table 2 shows disposition and subject progression. Table 3 shows the demographics of the study.

**Table 2. Disposition and Subject Progression.**

| | | **Compound (1) 50 mg (n = 271)** | **Placebo (n = 272)** |
|---|---|---|---|
| Dosed | | 268 | 269 |
| Completed study - n (%) | | 242 (90.3) | 235 (87.4) |
| Discontinued study - n (%) | | 26 (9.7) | 34 (12.6) |
| Reasons for discontinuation | | | |
| | Adverse events - n (%) | 6 (2.2) | 2 (0.7) |
| | Withdrawal by patient - n (%) | 10 (3.7) | 18 (6.7) |
| | Lost to Follow-up - n (%) | 7 (2.6) | 7 (2.6) |
| | Non-compliance study drug - n (%) | 1 (0.4) | 3 (1.1) |
| | Physician decision - n (%) | 1 (0.4) | 2 (0.7) |
| | Other - n (%) | 1 (0.4) | 2 (0.7) |

**Table 3. Baseline Demographics and Clinical Characteristics, Safety Population**

| | | **Compound (1) 50 mg (N=268)** | **Placebo (N=269)** |
|---|---|---|---|
| **Age, years, mean (SD)** | | 39.4 (12.30) | 40.1 (12.64) |
| **Years since initial diagnosis, mean (SD)** | | 10.6 (10.1) | 11.2 (10.8) |
| **Female sex, n (%)** | | 186 (69.4) | 166 (61.7) |

| **Race, n (%)** | | | |
|---|---|---|---|
| White | | 169 (63.1) | 206 (76.6) |
| Black/African American | | 75 (28.0) | 46 (17.1) |
| Asian | | 13 (4.9) | 4 (1.5) |
| Multiracial | | 7 (2.6) | 5 (1.9) |
| American Indian or Alaskan native | | 1 (0.4) | 3 (1.1) |
| Native Hawaiian or other Pacific Islander | | 1 (0.4) | 1 (0.4) |
| Other | | 2 (0.7) | 4 (1.5) |

| **Ethnicity, n (%)** | | | |
|---|---|---|---|
| Hispanic or Latino | | 58 (21.6) | 54 (20.1) |
| Not Hispanic or Latino | | 210 (78.4) | 215 (79.9) |
| **BMI, kg/m², mean (SD)** | | 29.6 (6.3) | 30.3 (6.2) |
| **HAMD-17 total score, mean (SD)** | | 26.8 (2.6) | 26.9 (2.7) |
| **MADRS total score, mean (SD)** | | 35.2 (4.8) | 35.0 (4.7) |

| **C-SSRS assessment, n (%)** | | | |
|---|---|---|---|
| | **No suicidal ideation/behavior** | 158 (58.9) | 134 (49.8) |
| | **Suicidal ideation** | 106 (39.6) | 133 (49.4) |
| | **Suicidal behavior** | 4 (1.5) | 2 (0.7) |
| **History of any antidepressant use, n (%)** | | 183 (68.3) | 190 (70.6) |
| **Concurrent antidepressant use (any stable dose)*, n (%)** | | 79 (29.5) | 81 (30.1) |
| **Number of depressive episodes experienced, mean (SD)** | | 5.3 (5.5) | 5.3 (6.7) |

| | | | |
|---|---|---|---|
| BMI = body mass index; HAMD-17 = 17-item Hamilton Rating Scale for Depression; MADRS = Montgomery Åsberg Depression Rating Scale; SD = standard deviation; *Defined as stable for at least 60 days; based on list of medications provided by World Health Organization Drug Dictionary anatomical therapeutic chemical level 3, N06A | | | |

Demographic and baseline characteristics were generally well balanced between Compound (**1**)-50mg group (Compound (**1**)-50 mg) and placebo groups; mean (SD) age: 39.4 (12.3) vs 40.1 (12.6); percentage of patients who were female: 69.4% vs 61.7%; white race: 63.1% vs. 76.6%; on pre-existing antidepressant therapy: 29.5% vs 30.1%; mean (SD) HAMD-17 total score: 26.8 (2.6) vs 26.9 (2.7). Patients had a diagnosis of MDD for approximately 11 years (mean (SD) Compound (**1**)-50 mg 10.6 (10.1), placebo 11.2 (10.8)).

Table 4 summarizes the primary and select secondary statistical outcomes at various timepoints.

**Table 4. Primary and Select Secondary Statistical Outcomes**

| **Outcome** | **Day 3** | **Day 8** | **Day 12** | **Day 15** |
|---|---|---|---|---|
| HAMD-17: LSmean TRT diff (p value) | -3.0(<0.0001) | -2.6(<0.0001) | -2.5 (0.0003) | -1.7 (0.0141) |
| CGI-Severity: LSmean TRT diff (p value) | -0.4(<0.0001) | -0.4(0.0001) | -0.3(0.0014) | -0.2(0.1193) |
| CGI-Improvement Response: Odds ratio (p value) | 1.8 (0.0032) | 1.9 (0.0005) | 1.6 (0.0101) | 1.5 (0.0191) |
| MADRS: LSmean TRT diff (p value) | * | -3.4 (0.0003) | * | -2.4 (0.0238) |
| HAM-A: LSmean TRT diff (p value) | * | -1.7 (0.0011) | * | -1.4 (0.0199) |

| | | | | |
|---|---|---|---|---|
| LS = least squares: TRT diff = Treatment difference; CGI-Improvement Response is odds ratio for 'much' or very much' improved; Grey box indicates time point not collected as per protocol. *not measured per protocol. | | | | |

### 2.2 HAMD-17 Total Score

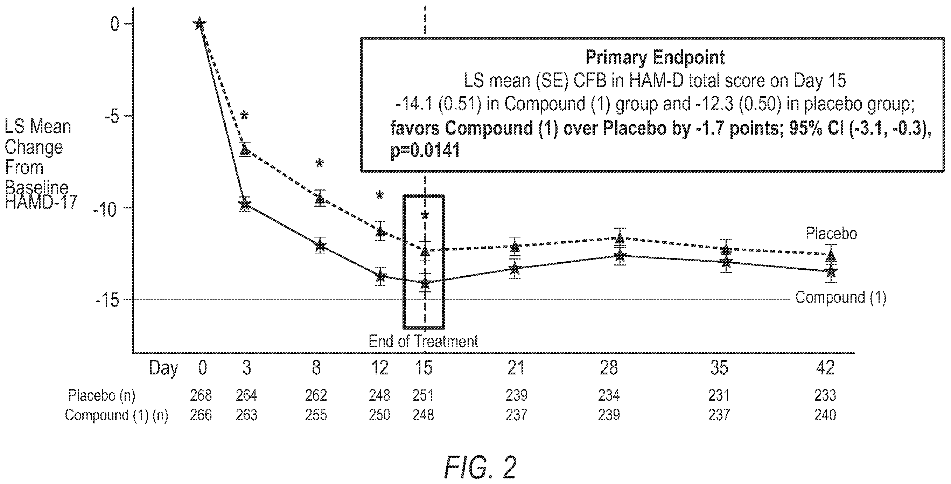

The study met its primary endpoint at Day 15, with statistically significant and clinically relevant reduction in the 17-Item Hamilton Depression Rating Scale (HAMD-17) total score on Day 15 (See FIG. 2).

At the Day 15 primary endpoint, Compound (**1**)-50 mg showed a statistically significant reduction in depressive symptoms as measured by HAMD-17 (p= 0.0141) compared to placebo; with rapid and significant effects observed as early as Day 3, and including Days 8 and 12. LS mean (SE) change from baseline (CFB) in HAMD-17 total score on Day 15 was -14.1 (0.51) (Compound **(1))** vs. -12.3 (0.50) (placebo); Δ -1.7 points; 95% CI (-3.1, -0.3), p=0.0141. The treatment effect was evident at all measured timepoints with nominal significance at Day 3 (LS mean (SE) CFB -9.8 (0.38) (Compound **(1))** vs. -6.8 (0.38) (placebo)); Δ -3.0 points 95% CI (-4.0 to -2.0, p <0.0001, Day 8 (LS mean (SE) CFB -12.0 (0.45) (Compound **(1))** vs. -9.5 (0.45) (placebo)) [Δ -2.6, p<0.0001], and Day 12 [Δ -2.5, p = 0.0003]. LSM treatment differences favored Compound (1). Day 42 had LS mean (SE) CFB - 13.5 (0.55) (Compound **(1))** vs. -12.6 (0.55) (placebo) Δ -0.9.

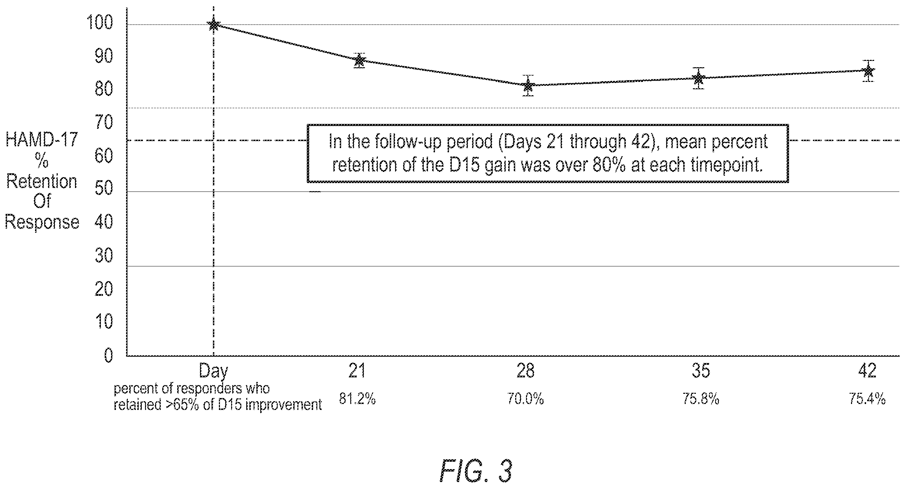

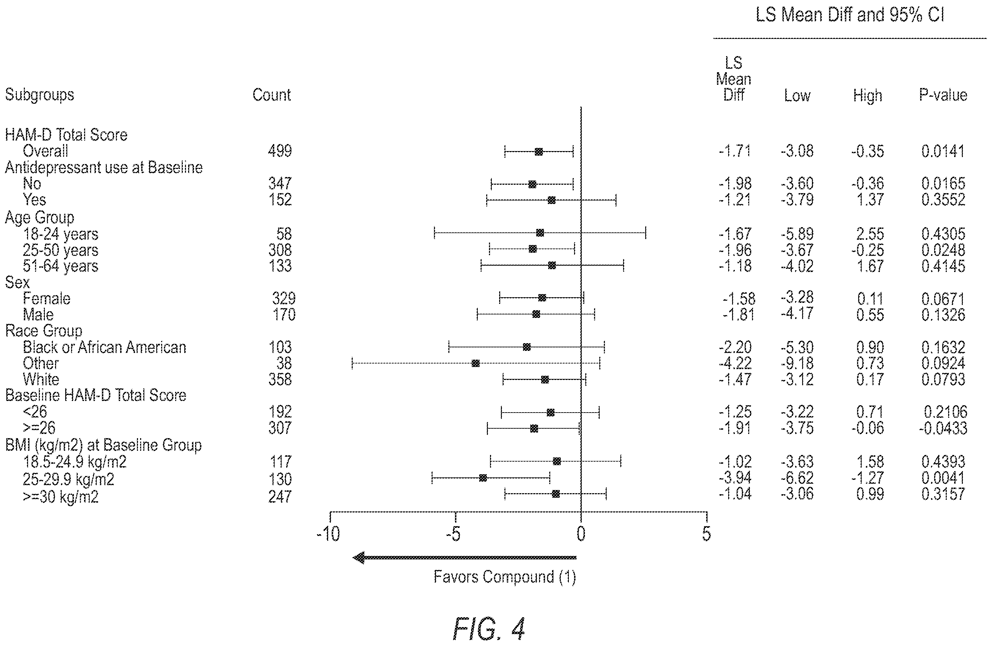

As shown in FIG. 3, among Compound (**1**)-treated HAMD-17 Day 15 responders, patients on average maintained 86.1% of their Day 15 improvement at Day 42. Patients with a Response at Day 15 to Compound **(1)** retained 86.1% of their HAMD-17 improvement at Day 42 (4 weeks after dosing ended). In addition, 75.4% of patients retained a pre-specified level of at least 65% of their Day 15 improvement at Day 42. Numeric evidence of treatment effect at Day 15 was present in favor of Compound **(1)** across all subgroups and subscales of HAMD-17 (See FIG. 4), for example antidepressant use at baseline, age, sex, race, baseline HAMD-17, and body mass index. A similar maintenance of response was also observed with the MADRS scale, where people who responded to Compound **(1)** at Day 15 maintained 87.6% of that response at Day 42. Additionally, Response and Remission rates are consistent with the weighted average Response and Remission rates across the placebo-controlled LANDSCAPE and NEST program clinical trials, including MDD and PPD.

### 2.3 Clinical Global Impression (CGI)

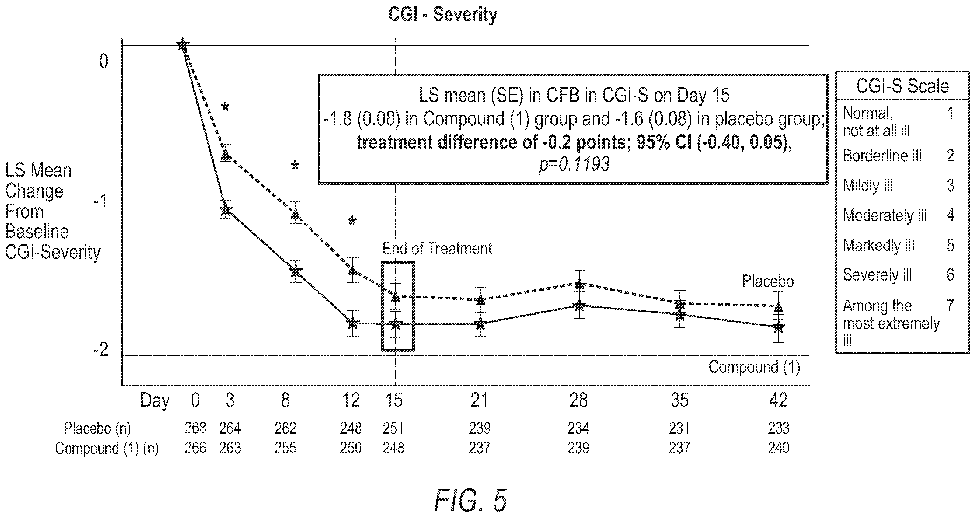

Following testing of the primary endpoint, the statistical analysis plan proceeded with testing of CFB in Clinical Global Impression-Severity of Illness (CGI-S) and CGI-Improvement (CGI-I). Results for CGI-S demonstrated a numerical advantage for Compound **(1)** at all measured timepoints (see FIG. 5), however were not statistically significant at Day 15 under the pre-specified analysis model. Results were similar for CGI-S between Compound **(1)** 50 mg and placebo (LS mean [SE] -1.8 [0.08] vs -1.6 [0.08]; LS mean difference -0.2, 95% CI -0.4 to 0.0; *P* = .12), so formal testing of key secondary endpoints stopped.

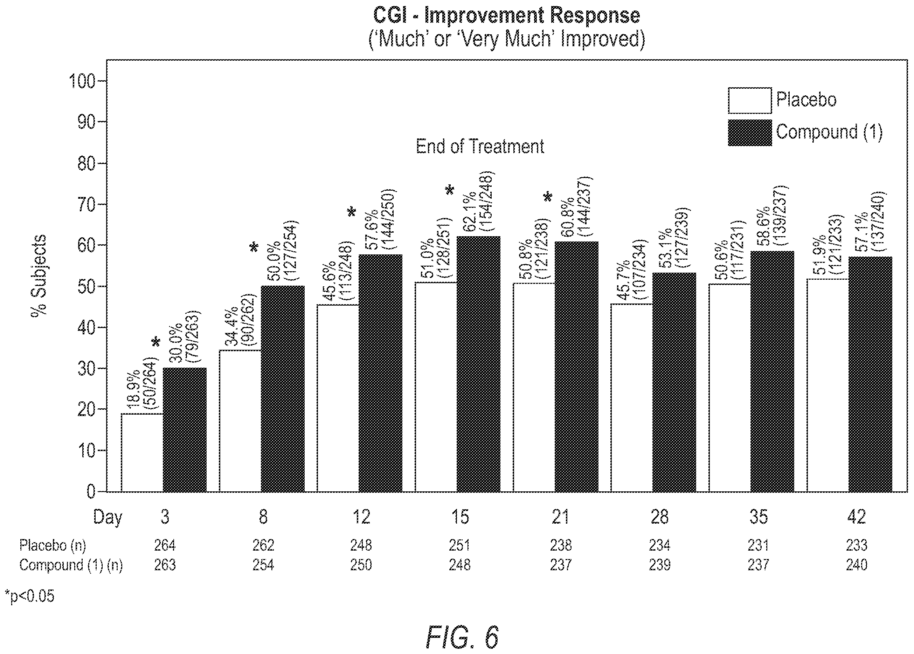

FIG. 6 shows one of the other secondary endpoints, CGI-I Response ("much" or "very much" improved), showing that rates of CGI-I Response at Day 15 were numerically greater in those receiving Compound (1) vs placebo.

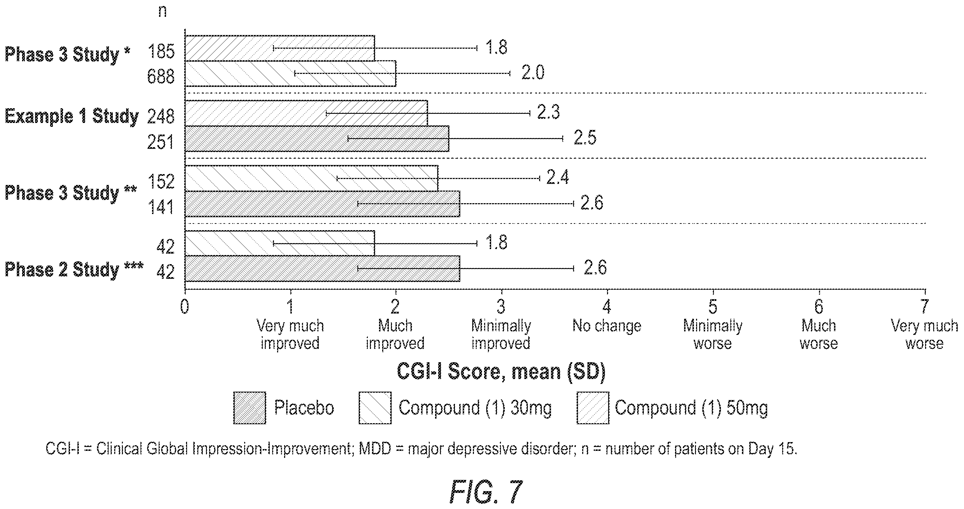

As shown in FIG. 7, at Day 15, clinician-assessed CGI-I scores indicated that treatment with Compound (1) greatly improved the patient's overall clinical condition, in this example as well as other clinical studies conducted by Applicant. *is a Phase 3 clinical trial study conducted by Applicant (ClinicalTrials.gov identifier/NCT number: NCT03864614). **is a Phase 3 clinical trial study conducted by Applicant (ClinicalTrials.gov identifier/NCT number: NCT0672175). ***is a Phase 2 clinical trial study conducted by Applicant (ClinicalTrials.gov identifier/NCT number: NCT03000530). The study design and results, if applicable, of the clinical trials identified by the NCT numbers are each incorporated by reference herein in its entirety.

### 2.4 MADRS

The clinical trial of Example 1 met its primary endpoint with patients receiving Compound (**1**)-50 mg demonstrating significant improvement in depressive symptoms compared with placebo at Day 15 as assessed by change from baseline (CFB) in HAMD-17 total score. While the HAMD-17 focuses predominately on the somatic symptoms of depression, including anxiety, the Montgomery-Åsberg Depression Rating Scale (MADRS) addresses core mood symptoms such as sadness, tension, lassitude, and pessimistic and suicidal thoughts (Table 5). Despite the differences in content and number of items between HAMD-17 and MADRS, studies indicate that the two scales are correlated.

**Table 5. Items Assessed by HAMD-17 and MADRS**

| **HAMD-17 Total Score Items** | **MADRS Total Score Items** |
|---|---|
| Depressed mood | Apparent sadness |
| Guilt | Reported sadness |
| Suicide | Inability to feel |
| Insomnia (early) | Pessimistic thoughts |
| Insomnia (middle) | Suicidal thoughts |
| Insomnia (late) | Reduced sleep |
| Work and Activities | Lassitude |
| Psychomotor retardation | Inner tension |
| Psycomotor agitation | Reduced appetite |
| Anxiety (psychic) | Concentration difficulties |
| Anxiety (somatic) | |
| Somatic symptoms (gastrointestinal) | |
| Somatic symptoms (general) | |
| Genital symptoms | |
| Hypochondriasis | |
| Weight loss | |
| Insight | |

CFB in MADRS total score was assessed at Days 8, 15 (secondary endpoint), 28, and 42.

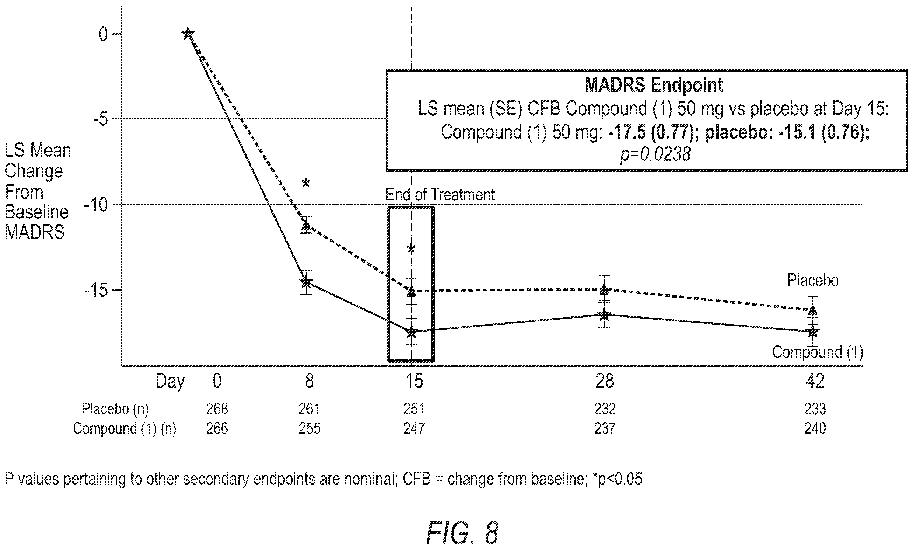

Consistent with the primary endpoint, patients treated with Compound **(1)** vs placebo demonstrated significant improvement in depression rating at Day 15 measured by CFB in MADRS total score (LS mean [SE] -17.5 [0.77] vs -15.1 [0.76]; LS mean difference -2.4, 95% CI -4.4 to -0.3; *P* = .02. Patients receiving Compound **(1)** demonstrated improvement in depressive symptoms compared with placebo as assessed by CFB in MADRS total score (LSM [SE] Compound (**1**)-50 mg vs placebo; see FIG. 8), with nominal significance at Days 8 and 15:
- Day 8, -14.6 (0.69) vs -11.2 (0.68);
- Day 15, -17.5 (0.77) vs -15.1 (0.76);
- Day 28, -16.4 (0.80) vs -14.9 (0.80); and
- Day 42, -17.5 (0.83) vs -16.2 (0.82).

The placebo adjusted CFB in MADRS demonstrated numerical advantage at all measured timepoints (Day 8, 15, 28, 42).

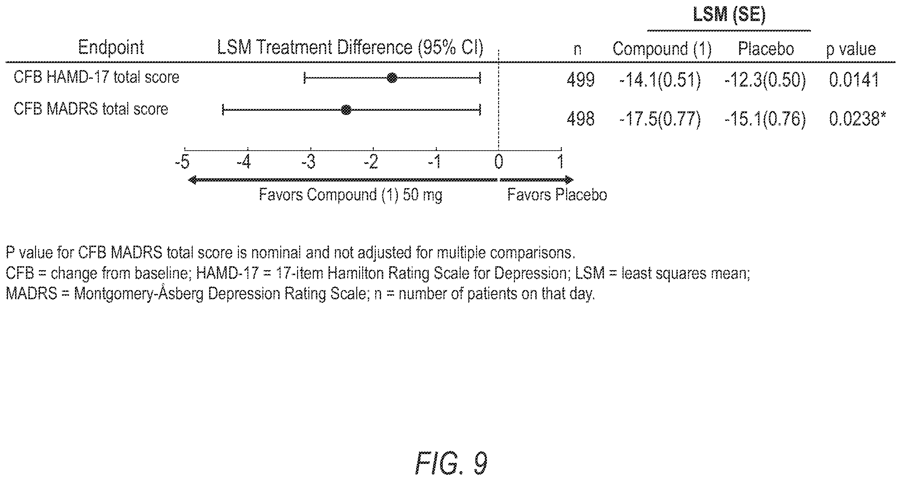

At Day 15, LSM CFB in MADRS total score (SE) for patients receiving Compound (**1**)-50 mg was -17.5 (0.77) compared with -15.1 (0.76) for patients receiving placebo (treatment difference -2.4 points; p=0.0238). The LSM treatment difference (Compound **(1)-**50 mg - placebo) in CFB in MADRS total score was: Day 8 (-3.4, p=0.0003), Day 15 (-2.4, p=0.0238), Day 28 (-1.5, NS), and Day 42 (-1.3, NS). LSM treatment difference in CFB in HAMD-17 and MADRS total scores both favored patients who received Compound **(1)** (see FIG. 9).

In summary, patients receiving Compound (**1**)-50 mg demonstrated improvement in depressive symptoms compared with placebo as assessed by MADRS total score. Patients receiving Compound (**1**)-50 mg showed rapid improvements in depression severity and symptoms as early as Day 3 (HAMD-17 total score) and Day 8 (first MADRS total score assessment on treatment), with benefits sustained through the follow-up period. Improvements as assessed by both scales continued through Day 42.

### 2.5 Response and Retention

The clinical study patients were followed for 28 days after treatment, with clinic visits at Days 21, 28, 35, and 42. Key secondary endpoints included LSM HAMD-17 total score and MADRS total score CFB at all timepoints measured and HAMD-17 and MADRS Response (≥50% improvement from baseline score) at Days 15 and 42.

Durability of treatment effect was assessed over the post-treatment period in patients treated with Compound (**1**)-50 mg, based on the efficacy observed at Day 15. Percent retention of the Day 15 HAMD-17 total score and MADRS total score Responses over time were examined in Day 15 responders only. Except for the primary endpoint, all p-values reported here are nominal and not adjusted for multiple comparisons. This analysis included patients with valid baseline HAMD-17 total score and at least 1 valid post-baseline HAMD-17 total score.

### Results

Overall, patients who received Compound **(1)** achieved faster treatment Response and Remission of symptoms compared with placebo-treated patients.

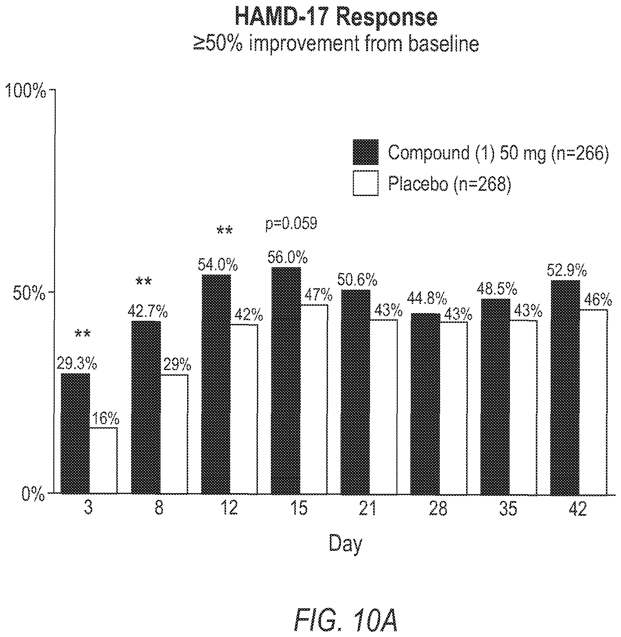

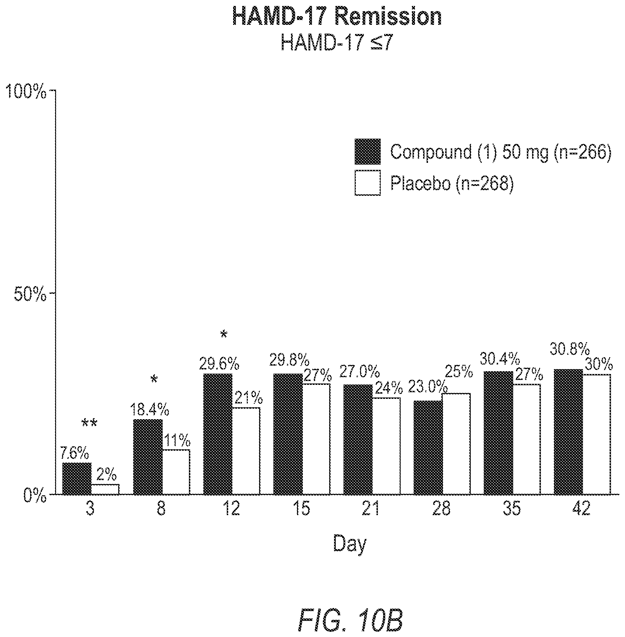

As shown in FIG. 10A, at Day 15, 139/248 Compound (**1**)-50 mg -treated patients (56.0%) showed a Response (≥ 50% reduction in HAMD-17 total score from baseline) as assessed by HAMD-17 total score. Among these, patients on average maintained 81.7% of their Day 15 improvement at Day 28 and 86.1% of their Day 15 improvement at Day 42. FIG. 10B shows Remission (HAMD-17≤7) as assessed by HAMD-17 total score.

As shown in FIG. 10A higher proportion of Compound (**1**)-treated vs placebo-treated patients achieved treatment Response (≥ 50% reduction in HAMD-17 total score from baseline) by Day 3 (29.3% vs 16%, odds ratio 2.14, 95% CI 1.4-3.3; *P* < .001), and the treatment difference persisted throughout the treatment period. At the first post-treatment follow-up study visit on Day 15, a numerically higher proportion of Compound (**1**)-treated patients achieved treatment Response compared with placebo-treated patients (56.0% vs 47.0% , odds ratio 1.40, 95% CI 1.0-2.0, *P* = 0.06). At the end of the follow-up period (Day 42), the proportion of patients with a HAMD-17 Response was similar between Compound **(1)** and placebo groups.

Symptom Remission (HAMD-17≤7; FIG. 10B) was observed more often by Day 3 in the Compound **(1)** group compared with the placebo group (7.6% vs 2%, odds ratio 3.57, 95% CI 1.4-9.1, *P* = 0.008), with an advantage for Compound **(1)** throughout the treatment period. At Day 15 and Day 42, symptom Remission slightly favored Compound **(1)** vs placebo. By Day 21, 83/226 (81.0%) Compound (**1**)-treated patients retained ≥65% of the HAMD-17 improvement at Day 15, which was reduced to 173/231 (74.9%) by Day 42.

These results suggest that a small proportion of patients lost a robust Treatment Response shortly after cessation of treatment, but broadly the Treatment Response was maintained in the post-treatment period.

As shown in Tables 6 and 7, results for Treatment Response and Remission were either similar or numerically improved in patients receiving Compound **(1)** 50 mg with concomitant antidepressants (ADTs) vs Compound **(1)** 50 mg monotherapy.

**Table 6. HAMD-17 Response by ADT use at baseline**

| | | **ADT use at baseline=Yes** | | **ADT use at baseline=No** | |
|---|---|---|---|---|---|
| **Visit** | **Statistics** | **Compound (1) 50 mg n/N (%)** | **Placebo n/N (%)** | **Compound (1) 50 mg n/N (%)** | **Placebo n/N (%)** |
| **Day 3** | Response | 22/78 (28.2) | 14/80 (17.5) | 55/185 (29.7) | 29/184 (15.8) |
| | Odds ratio (95% CI) | 1.9 (0.87, 3.98) | | 2.3 (1.36, 3.79) | |
| | *P*-value | .1103 | | .0017 | |
| **Day 8** | Response | 34/74 (45.9) | 24/80 (30.0) | 75/181 (41.4) | 53/182 (29.1) |
| | Odds ratio (95% CI) | 1.9 (1.01, 3.73) | | 1.7 (1.08, 2.58) | |
| | *P*-value | .0483 | | .0210 | |
| **Day 12** | Response | 42/74 (56.8) | 31/76 (40.8) | 93/176 (52.8) | 72/172 (41.9) |
| | Odds ratio (95% CI) | 1.9 (0.98, 3.58) | | 1.5 (1.02, 2.35) | |
| | *P*-value | .0595 | | .0409 | |
| **Day 15** | Response | 43/75 (57.3) | 37/77 (48.1) | 96/173 (55.5) | 81/174 (46.6) |
| | Odds ratio (95% CI) | 1.4 (0.74, 2.71) | | 1.4 (0.92, 2.12) | |
| | *P*-value | .2884 | | .1178 | |
| **Day 21** | Response | 42/73 (57.5) | 31/75 (41.3) | 78/164 (47.6) | 72/164 (43.9) |
| | Odds ratio (95% CI) | 1.8 (0.95, 3.53) | | 1.1 (0.73, 1.70) | |
| | *P*-value | .0713 | | .6150 | |
| **Day 28** | Response | 37/72 (51.4) | 32/73 (43.8) | 70/167 (41.9) | 68/161 (42.2) |
| | Odds ratio (95% CI) | 1.4 (0.72, 2.64) | | 1.0 (0.65, 1.55) | |
| | *P*-value | .3306 | | .9704 | |
| Day 35 | Response | 42/73 (57.5) | 33/72 (45.8) | 73/164 (44.5) | 67/159 (42.1) |
| | Odds ratio (95% CI) | 1.4 (0.76, 2.75) | | 1.1 (0.72, 1.70) | |
| | *P*-value | .2617 | | .6493 | |
| Day 42 | Response | 43/74 (58.1) | 33/72 (45.8) | 84/166 (50.6) | 74/161 (46.0) |
| | Odds ratio (95% CI) | 1.7 (0.91, 3.34) | | 1.2 (0.81, 1.90) | |
| | *P*-value | .0962 | | .3234 | |

| | | | | | |
|---|---|---|---|---|---|
| N refers to total number of patients on the study visit day. ADT = antidepressant therapy; CI = confidence interval. | | | | | |

**Table 7. HAMD-17 Remission by ADT use at baseline**

| | | **ADT use at baseline=Yes** | | **ADT use at baseline=No** | |
|---|---|---|---|---|---|
| **Visit** | **Statistics** | **Compound (1) 50 mg n/N (%)** | **Placebo n/N (%)** | **Compound (1) 50 mg n/N (%)** | **Placebo n/N (%)** |
| **Day 3** | Response | 6/78 (7.7) | 0/80 (0) | 14/185 (7.6) | 6/184 (3.3) |
| | Odds ratio (95% CI) | NE | | 2.4 (0.91, 6.58) | |
| | *P*-value | NA | | .0776 | |
| **Day 8** | Response | 13/74 (17.6) | 11/80 (13.8) | 34/181 (18.8) | 18/182 (9.9) |
| | Odds ratio (95% CI) | NE | | 2.1 (1.14, 3.91) | |
| | *P*-value | NA | | .0172 | |
| **Day 12** | Response | 23/74 (31.1) | 16/76 (21.1) | 51/176 (29.0) | 37/172 (21.5) |
| | Odds ratio (95% CI) | NE | | 1.5 (0.92, 2.44) | |
| | *P*-value | NA | | .1021 | |
| **Day 15** | Response | 23/75 (30.7) | 20/77 (26.0) | 51/173 (29.5) | 48/174 (27.6) |
| | Odds ratio (95% CI) | NE | | 1.1 (0.70, 1.76) | |
| | *P*-value | NA | | .6642 | |
| **Day 21** | Response | 22/73 (30.1) | 18/75 (24.0) | 42/164 (25.6) | 39/164 (23.8) |
| | Odds ratio (95% CI) | NE | | 1.0 (0.62, 1.65) | |
| | *P*-value | NA | | .9736 | |
| **Day 28** | Response | 17/72 (23.6) | 16/73 (21.9) | 38/167 (22.8) | 42/161 (26.1) |
| | Odds ratio (95% CI) | NE | | 0.86 (0.52, 1.41) | |
| | *P*-value | NA | | .5532 | |
| **Day 35** | Response | 26/73 (35.6) | 21/72 (29.2) | 46/164 (28.0) | 42/159 (26.4) |
| | Odds ratio (95% CI) | NE | | 1.1 (0.66, 1.74) | |
| | *P*-value | NA | | .7697 | |
| **Day 42** | Response | 24/74 (32.4) | 19/72 (26.4) | 50/166 (30.1) | 50/161 (31.1) |
| | Odds ratio (95% CI) | NE | | 1.0 (0.61, 1.55) | |
| | *P*-value | NA | | .9214 | |

| | | | | | |
|---|---|---|---|---|---|
| N refers to total number of patients on study visit day. ADT = antidepressant therapy; CI = confidence interval; NA = not applicable: NE = not estimable. | | | | | |

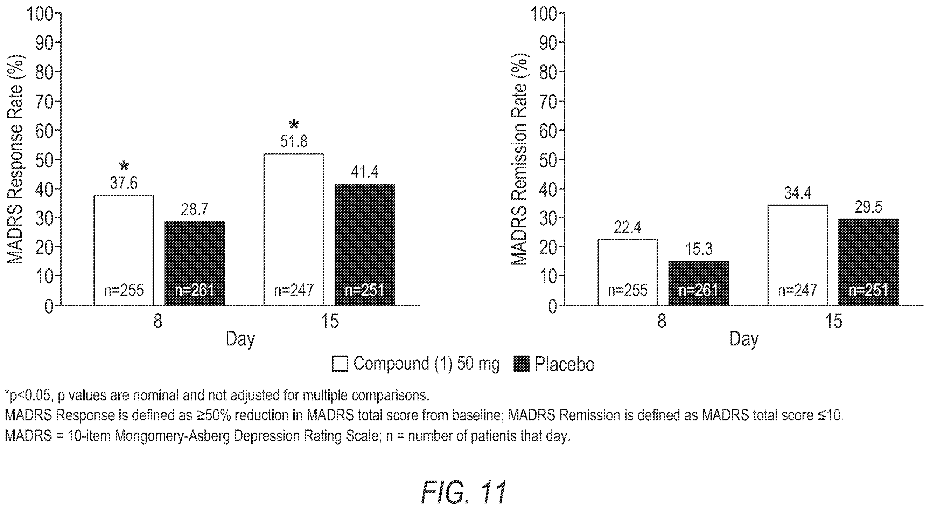

MADRS Response (≥ 50% reduction in MADRS total score from baseline) rates during the treatment period significantly favored Compound **(1)** compared with placebo. MADRS Remission (MADRS≤10) rates numerically favored Compound **(1)** during this same period (FIG. 11).

At Day 15, 128/248 Compound (**1**)-50 mg -treated patients (51.6%) showed a Response (≥ 50% reduction in MADRS total score from baseline) as assessed by MADRS total score. A similar maintenance of Response was also observed with the MADRS total score as was with HAMD-17 total score. Of the 128 Responders (i.e., having ≥ 50% reduction in MADRS total score from baseline), patients on average maintained 85.2% of their Day 15 improvement at Day 28 and 87.6% of that improvement at Day 42.

Maintenance of Response to Compound (**1**)-50 mg, as assessed by retention of both HAMD-17 total score and MADRS total score Response at Day 15, was observed at all timepoints measured through Day 42.

### 2.6 Anxiety (HAM-A and HAMD-17 Anxiety/Somatization subscale)

Approximately two-thirds of patients with MDD also experience symptoms of anxiety, including up to 20% who have a comorbid anxiety disorder (Mittal D, et al. Psychiatr Serv. 2006;57(12):1731-1737; Clayton AH, et al. Am J Psychiatry. 1991;148(11):1512-1517.; Stein MB, et al. J Affect Disord. 1995;34(2):79-84).

Secondary endpoints focusing on anxiety-related symptoms included CFB in HAM-A total score and the HAMD-17 Anxiety/Somatization subscale.

The impact of Compound **(1)** on symptoms of anxiety, as assessed by HAM-A and HAMD-17 Anxiety/Somatization subscale, is reported here.

### Methods

HAM-A total score was assessed on Days 8, 15, 28, and 42. HAMD-17 total score and HAMD-17 Anxiety/Somatization subscale were assessed on Days 3, 8, 12, 15, 21, 28, 35, and 42.

Statistics are from a mixed effects model. Except for CFB in HAMD-17 at Day 15 (primary endpoint), all p-values reported here are nominal and not adjusted for multiple comparisons.

### Results

The Compound (**1**)-50 mg group demonstrated a numerical improvement in depressive symptoms compared with placebo at all measured timepoints through Day 42 as assessed by HAMD-17 total score, with nominal significance at Days 3 (LS mean difference - 3.0, p <0.0001), 8 (LS mean difference -2.6, p <0.0001), and 12 (LS mean difference -2.5, p=0.0003). Among Compound (**1**)-50 mg -treated HAMD-17 Day 15 responders (≥50% improvement in HAMD-17 total score from baseline), patients on average maintained 86.1% of their Day 15 improvement at Day 42 (4 weeks after dosing ended).

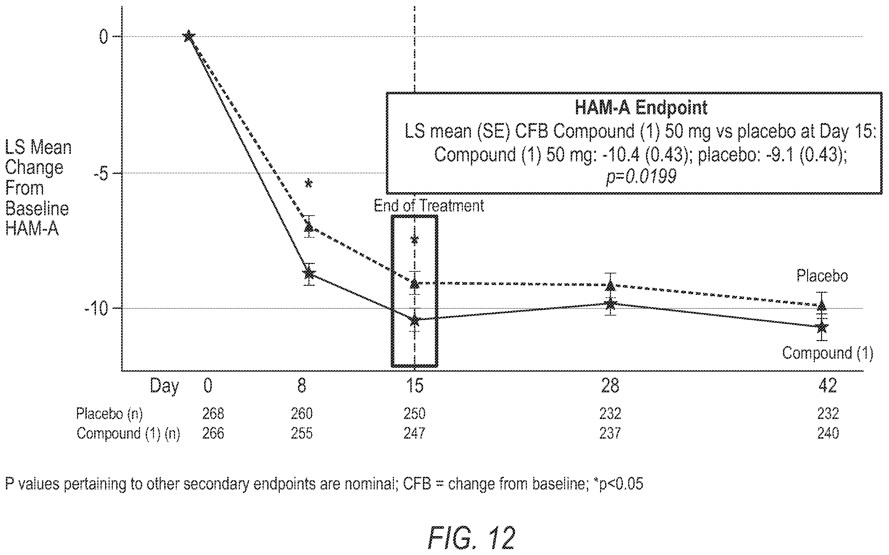

A similar trend was seen on the HAM-A total score with numerical improvements in anxiety symptoms compared with placebo at all measured timepoints (Day 8, 15, 28, 42) through Day 42, with nominal significance at Day 8 (LS mean difference -1.7, p=0.0011), and 15 (LS mean difference -1.4, p=0.0199) (see FIG. 12).

The CFB in HAM-A total score showed numerical improvements in symptoms of anxiety in patients treated with Compound **(1)** compared with those who received placebo at all measured timepoints, with nominal significance at Day 8 (LS mean (SE) CFB -8.7 (0.40) (Compound **(1))** vs. -7.0 (0.39) (Placebo)) and Day 15 (LS mean (SE) CFB -10.4 (0.43) (Compound **(1))** vs. placebo (-9.1 (0.43) (Placebo)) (see FIG. 12).

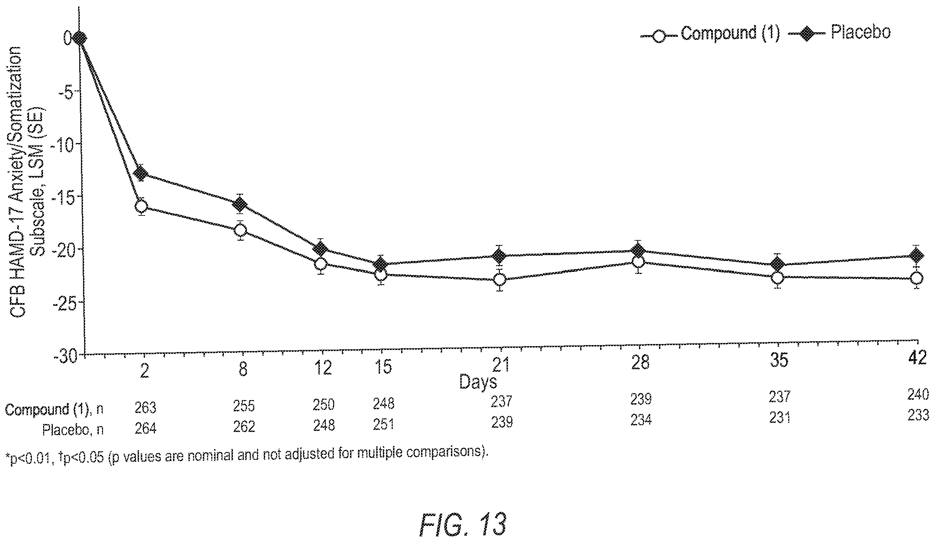

The CFB in HAMD-17 Anxiety/Somatization subscale score showed numerical improvements in symptoms of anxiety in patients treated with Compound **(1)** compared with those who received placebo at all measured timepoints, with nominal significance at Day 3 and Day 8. (see FIG. 13).

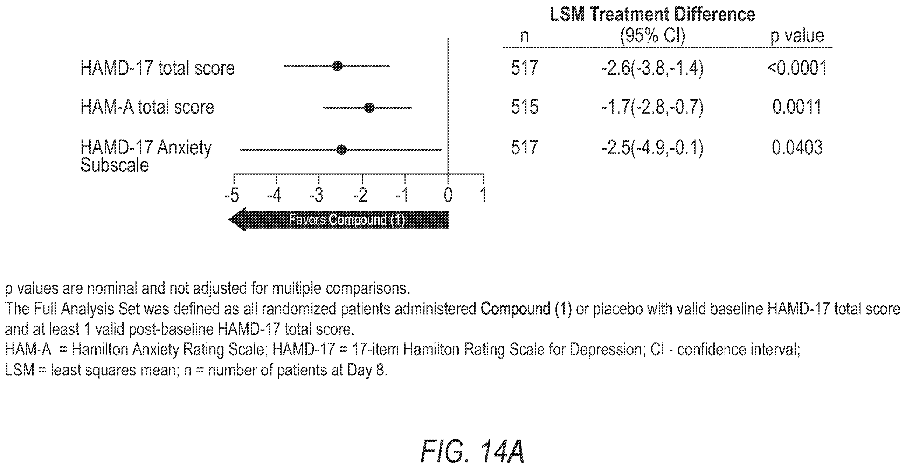

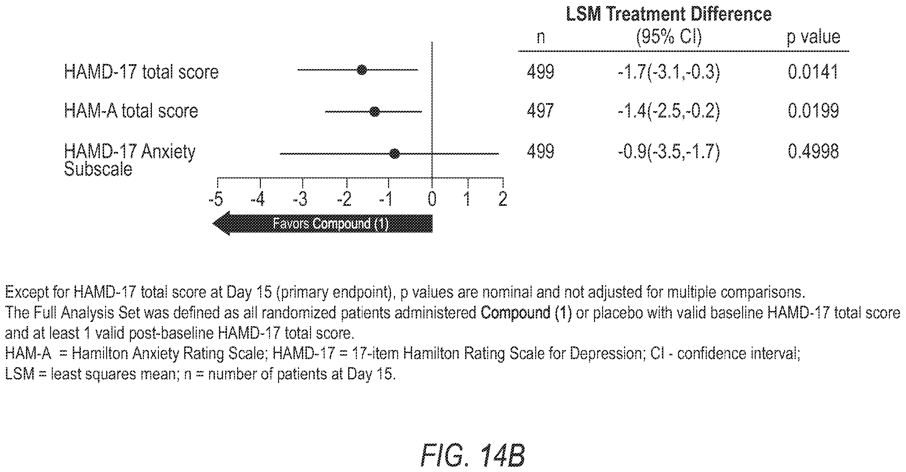

The CFB in HAM-A total score and HAMD-17 Anxiety/Somatization subscale score over time showed a trend similar to what was observed for HAMD-17 total score. At Days 8 and 15, LSM treatment difference (Compound **(1)** minus placebo) for HAMD-17 total score, HAMD-17 Anxiety/Somatization subscale score, and HAM-A total score all favored Compound **(1)** (FIG. 14A and 14B).

### Conclusions

Patients receiving Compound **(1)** demonstrated rapid improvements in symptoms of anxiety (as assessed by HAM-A) as early as Day 8, with numerical improvements maintained through the end of the study (Day 42). The HAMD-17 Anxiety/Somatization subscale showed similar trends, with notable significant effects at Days 3 and 8.

Similar results in the symptoms of depression and anxiety have been observed across the LANDSCAPE clinical development program (Gunduz-Bruce H, et al. N Engl J Med. 2019;381(10):903-911; Mittal A, et al. Poster presented at the American Academy of Neurology Annual Meeting. Toronto, Canada. April 25-May 1, 2020; Cutler AJ, et al. Poster presented at the American Society of Clinical Psychopharmacology Annual Meeting. Virtual Congress. June 1-4, 2021; Deligiannidis KM, et al. JAMA Psychiatry. 2021;78(9):951-959), confirming the impact of Compound **(1)** on the anxiety symptoms that may co-exist with those of depression in patients with MDD.

### 2.6.1 Major Depressive Disorder (MDD) with Elevated Anxiety

It is well-established that MDD with elevated anxiety as a symptom is associated with: more severe illness, more difficulty tolerating antidepressants (potentially impacting adherence), higher rates of non-response to treatment, and greater need for additional interventions and resources.

In the US, up to 78% of patients with MDD are classified as "MDD with anxious distress" using the DSM-5 specifier, and up to 70% are classified as "MDD with elevated anxiety" using a HAM-A total score ≥20. These patients have more severe depression, are less responsive to common ADT, and have poorer treatment outcomes.

The 14-item Hamilton Anxiety Rating Scale (HAM-A) assesses the effect of treatment on anxiety symptoms in patients with anxiety disorders (Bourin M. Therapie. 2000;55(1):147-153; Maier W, et al. J Affect Disord. 1988;14(1):61-68). HAM-A, which measures both psychic and somatic anxiety, can be a reliable and valid measure of the severity of anxiety in patients with MDD. The HAM-A psychic anxiety items can be used to assess elevated anxiety in patients with MDD. The HAMD-17 total score is the gold standard for assessing the severity of depression in clinical trials for MDD (Boessen R, et al. J Affect Disord. 2013;145(3):363-369; Bagby RM, et al. Am J Psychiatry. 2004;161(12):2163-2177; Bech P, et al. Acta Psychiatr Scand. 1981;63(3):290-299). The HAMD-17 Anxiety/Somatization subscale (6 items) is frequently used to investigate the specific effect of treatment on anxiety-related symptoms in patients with MDD (Huang CJ, et al. Int J Neuropsychopharmacol. 2019;22(10):609-615; McClintock SM, et al. Int J Methods Psychiatr Res. 2011;20:e69-e82). The HAMD-17 Anxiety/Somatization subscale anxiety (*e.g*., anxiety (psychic)) items can also be used to assess elevated anxiety in patients with MDD.

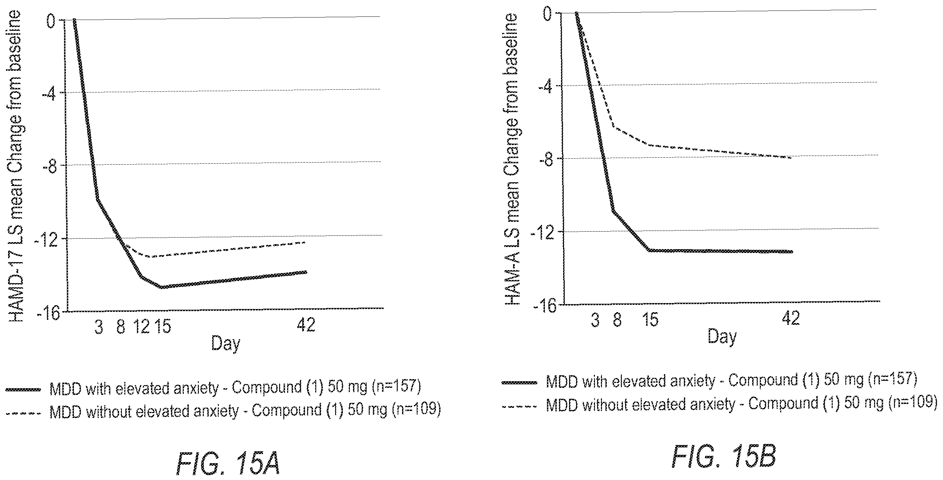

FIGs. 15A and 15B show that Compound **(1)** significantly improved depression (as evidenced by HAMD-17 CFB; FIG 15A) and anxiety (as evidenced by HAM-A CFB; FIG. 15B) symptoms in patients of the Example 1 study having MDD with elevated anxiety (patients with MDD who had baseline HAM-A≥20).

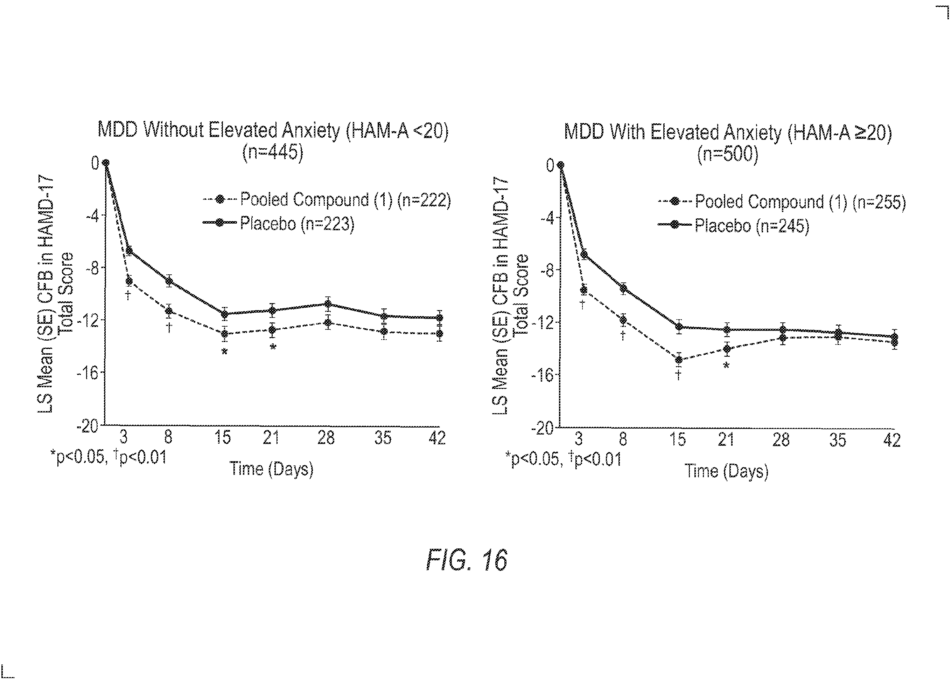

FIG. 16 shows pooled HAMD-17 CFB in MDD without elevated anxiety (patients with MDD who had baseline HAM-A<20) and MDD with elevated anxiety patients with MDD who had baseline HAM-A≥20) patients treated with Compound (1) vs placebo. The pooled data combines data from the clinical trial of Example 1, a Phase 3 clinical trial study conducted by Applicant (ClinicalTrials.gov identifier/NCT number: NCT0672175) and a Phase 2 clinical trial study conducted by Applicant (ClinicalTrials.gov identifier/NCT number: NCT03000530). The study design and results, if applicable, of the clinical trials identified by the NCT numbers are each incorporated by reference herein in its entirety.

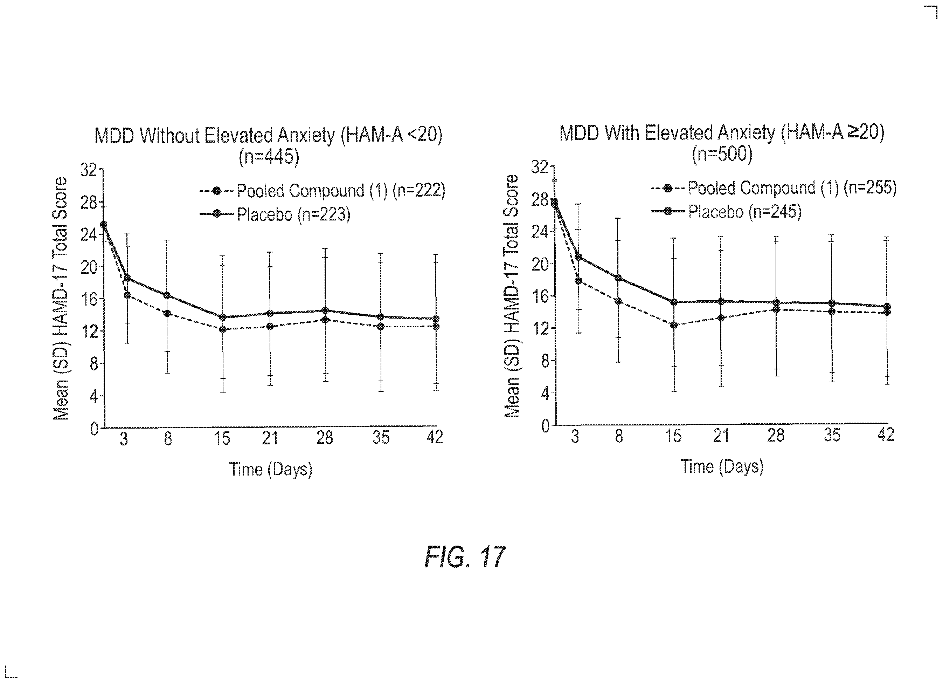

FIG. 17 shows pooled mean HAMD-17 total score in MDD without elevated anxiety (patients with MDD who had baseline HAM-A<20) and MDD with elevated anxiety patients with MDD who had baseline HAM-A≥20) patients treated with Compound (1) vs placebo. The pooled data combines data from the clinical trial of Example 1, a Phase 3 clinical trial study conducted by Applicant (ClinicalTrials.gov identifier/NCT number: NCT0672175) and a Phase 2 clinical trial study conducted by Applicant (ClinicalTrials.gov identifier/NCT number: NCT03000530).

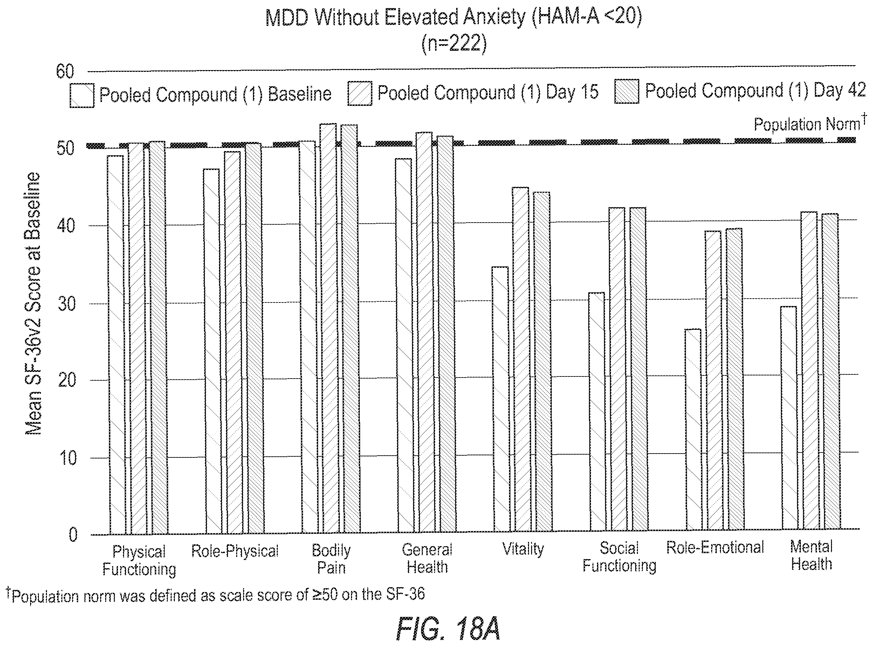

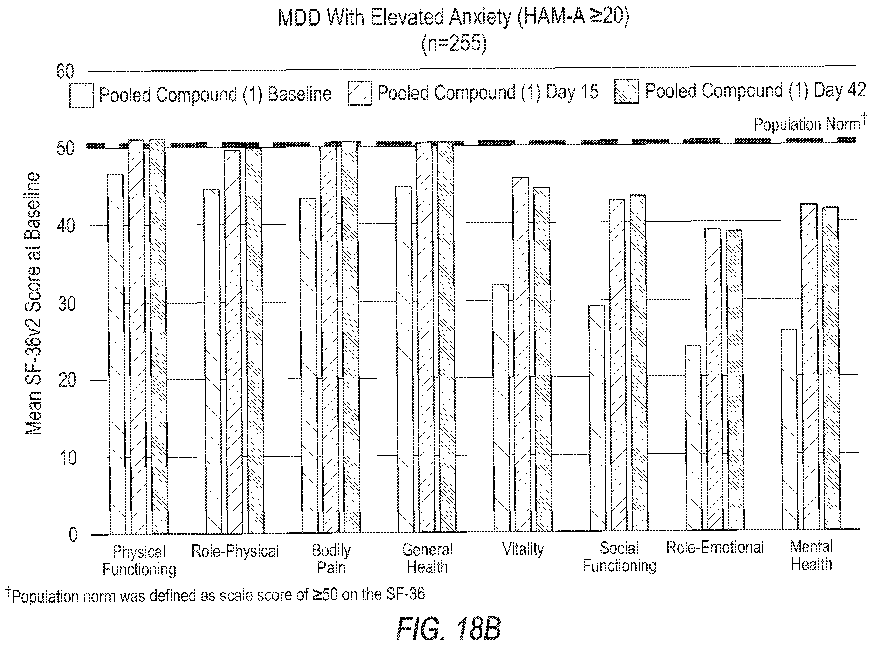

FIGs. 18A-18B show pooled mean SF-36v2 scores in MDD without elevated anxiety (patients with MDD who had baseline HAM-A<20; FIG. 18A) and MDD with elevated anxiety patients with MDD who had baseline HAM-A≥20; FIG. 18B) patients treated with Compound (1) vs placebo at baseline, Day 15 and Day 42. The pooled data combines data from the clinical trial of Example 1, a Phase 3 clinical trial study conducted by Applicant (ClinicalTrials.gov identifier/NCT number: NCT0672175) and a Phase 2 clinical trial study conducted by Applicant (ClinicalTrials.gov identifier/NCT number: NCT03000530).

Additional References for Section 2.6 include: Althaus AL, et al. Neuropharmacology. 2020;181:108333; Martinez Botella G, et al. J Med Chem. 2017;60(18):7810-7819; Hoffmann E, et al. Clin Pharmacokinet. 2020;59(1):111-120; and Clayton A, et al. New Medication Symposium. 34th European College of Neuropsychopharmacology Hybrid Congress. Lisbon, Portugal. October 2-5, 2021.

### 2.7 HAMD-17 Subscale Scores

As indicated above, the study of Example 1 met its primary endpoint and Compound (1) demonstrated significant improvement in depressive symptoms compared with placebo at Day 15 LSM CFB in HAMD-17 total score (treatment difference -1.7 points; p=0.0141). This section details HAMD-17 subscale outcomes capturing the core symptoms of depression and anxiety.

Least squares mean CFB in HAMD-17 subscale (Core Depression, Bech-6, Maier, and Anxiety) scores at Day 15 were calculated, but the study was not powered to make statistical comparisons of these subscales.

### Results

Similar to the primary endpoint at Day 15, patients receiving Compound (**1**)-50 mg demonstrated numerical improvements in symptoms compared with placebo across all HAMD-17 subscales: Core Depression (LSM difference -2.0 points; p=0.1906), Bech-6 (LSM difference -3.2 points; p=0.0759), Maier (LSM difference -3.0 points; p=0.0586), and Anxiety (LSM difference -0.9 points; p=0.4998).

Patients receiving Compound (**1**)-50 mg demonstrated numerical improvements in the core depressive and anxiety symptoms across HAMD-17 subscale scores, supporting the primary endpoint results.

### 2.8 SF-36 Scores

The study of Example 1 evaluated health-related quality of life (HRQoL) of Compound (**1**)-50 mg Compound (**1**)-50 mg vs placebo.

The Short Form-36 (SF-36v2) assessed patient-reported HRQoL for 8 domains (Physical-Functioning [PF]; Role-Physical [RP]; Bodily Pain [BP]; General Health [GH]; Vitality [V]; Social-Functioning [SF]; Role-Emotional [RE]; Mental Health [MH]). Safety and tolerability were evaluated by adverse events (AEs).

### Results

Baseline scores for SF-36v2 were similar for placebo (PF:49.20; RP:46.60; BP:47.38; GH:47.04; V:33.33; SF:30.22; RE:26.06; MH:28.28) and Compound (**1**)-50 mg (PF:49.40; RP:47.42; BP:47.07; GH:46.97; V:32.93; SF:29.85; RE:25.15; MH:27.40) groups. SF-26v2 improvements were observed in the Compound (**1**)-50 mg group CFB at Day 15: PF (3.41), RP (3.45), BP (5.26), GH (4.51), V (13.12), SF (12.89), RE (14.19), and MH (14.45).

At Day 15, Compound (**1**)-50 mg demonstrated a statistically significant (p=0.0029) and clinically meaningful increase in SF-36v2 least-squares mean vitality score versus placebo (3.1).

Patients receiving Compound **(1)**-50 mg reported improvements in multiple SF-36v2 domains, particularly vitality at Day 15, indicating significant HRQoL improvements.

### 2.9 Monotherapy vs Concomitant SOC ADT

The study of Example 1 permitted the use of stable dose, preexisting standard-of-care (SOC) antidepressant therapy (ADT). This section reports the safety and efficacy of Compound **(1)**-50 mg -treatment as monotherapy vs with concomitant SOC ADT.

### Results

The proportion of patients using antidepressants at baseline was balanced between Compound **(1)**-50 mg and placebo (29.3% vs 30.2%). Regardless of treatment, ADT use at baseline did not significantly impact HAMD-17 total score at Day 15 (LSM difference monotherapy: -1.98; p=0.0165 vs combination: -1.21; p=0.3552).

At Day 15, HAMD-17 total score Response rates for Compound (1) vs placebo were: overall (56.0% vs 47.0%), monotherapy (55.5% vs 46.6%), and concomitant SOC ADT (57.3% vs 48.1%).

TEAEs were reported in 111/189 (58.7%) vs 87/188 (46.3%) of patients receiving Compound **(1)**-50 mg - vs placebo-monotherapy and in 50/79 (63.3%) vs 33/81 (40.7%) of patients receiving SOC ADT.

Patients receiving Compound **(1)**-50 mg demonstrated improvement in depressive symptoms regardless of concurrent SOC ADT use with no evidence of increased adverse events.

### 2.10 Subgroup Analysis

This section contains baseline demographic and characteristic subgroup efficacy analyses.

Patients (N=543) were randomized to receive Compound **(1)**-50 mg (n=271) or placebo (n=272). At Day 15, LSM treatment difference in all analyzed subgroups favored Compound **(1)**-50 mg vs placebo:
- ADT use (No: -1.98; Yes: -1.21)
- Age (18-24 years: -1.67; 25-50 years: -1.96; 51-64 years: -1.18),
- Sex (female: -1.58; male: -1.81), race (Black: -2.20; White: -1.47; Other: -4.22)
- Baseline HAMD-17_{TS} (<26: -1.25; ≥26: -1.91), and
- Baseline BMI (kg/m²) (18.5-24.9: -1.02; 25-29.9: -3.94; ≥30: -1.04).

Treatment differences in all subgroups analyzed (based on baseline demographics and characteristics) favored Compound **(1)** - 50 mg vs placebo.

### Example 3. Detailed Safety Results of the Phase 3 Study of Example 1

### Safety Results

TEAEs were consistent with known Compound **(1)** profile, with ~60% (*e.g.,* about 60.1%) patients receiving Compound **(1)-**50 mg and ~45% (*e.g.,* about 44.6%) receiving placebo reporting ≥1 TEAE.

Most TEAEs reported by Compound **(1)-**50 mg patients were mild or moderate in severity. Severe TEAEs (6 in 5 subjects) of sedation, dizziness, vertigo, somnolence, psychotic disorder, anxiety assessed by principal investigator (PI) as related to IP in the Compound **(1)-**50 mg treatment group; 1 severe event of elevated transaminases assessed by PI as related to IP in the placebo treatment group. Two Compound **(1)-**50 mg patients and two placebo patients experienced serious adverse events (SAEs); none related to sedation. No deaths, no loss of consciousness, weight gain, sexual dysfunction, or euphoria reported. Most common TEAEs leading to study drug (Compound **(1))** discontinuation were dizziness and sedation. Table 8 is an overview of TEAEs through Day 42.

**Table 8. Overview of TEAEs through Day 42.**

| | | **Placebo (n = 269)** | **Compound (1) 50 mg (n = 268)** |
|---|---|---|---|
| At least 1 TEAE, n (%) | | 120 (44.6) | 161 (60.1) |
| | Mild | 76 (28.3) | 86 (32.1) |
| | Moderate | 41 (15.2) | 67 (25.0) |
| | Severe | 3 (1.1) | 8 (3.0) |
| SAE, n (%) | | 2 (0.7) | 2 (0.7) |
| Dose reduction to TEAE, n (%) | | 1 (0.4) | 23 (8.6) |
| Discontinuation of treatment due to TEAEs, n (%) | | 4 (1.5) | 9 (3.4) |
| Withdrawal from study due to TEAEs, n (%) | | 2 (0.7) | 6 (2.2) |
| Death, n | | 0 | 0 |

The most common (>5%) events were somnolence, dizziness, headache and sedation in patients receiving Compound **(1)-**50 mg and diarrhea and headache in patients receiving placebo. The most common TEAEs observed on Compound **(1)-**50 mg are consistent with the safety profile of Compound **(1)** seen to date. Table 6 shows all of the TEAEs incidence through Day 42.

### Additional safety findings.

Suicidality: No signal of increased suicidal ideation/behavior as assessed by the C-SSRS was observed throughout the study in patients receiving Compound **(1)-**50 mg or patients receiving placebo.

Withdrawal symptoms: No signals for withdrawal symptoms assessed by the PWC-20 at Day 18 or 21; scores were similar after discontinuation of Compound **(1)-**50 mg or placebo. Change from first PWC-20 assessment (SD) at Day 18; Compound **(1)** vs placebo: - 1.2 (4.3) vs. -1.2 (4.3); Day 21; Compound **(1)** vs placebo -0.3 (4.6) vs -0.5 (4.4).

Concomitant antidepressant therapy (ADT): No clinically meaningful differences in the safety profile for Compound **(1)-**50 mg monotherapy compared with those receiving Compound **(1)-**50 mg in combination with pre-existing ADT.

**Table 9. All TEAEs (>2%) incidence through Day 42.**

| **Preferred Terms (PTs)** | **Compound (1) 50 mg (n = 268)** | **Placebo (n = 269)** |
|---|---|---|
| Somnolence | 41 (15.3%) | 8 (3.0%) |
| Dizziness | 37 (13.8%) | 6 (2.2%) |
| Headache | 29 (10.8%) | 21 (7.8%) |
| Sedation | 20 (7.5%) | 1 (0.4%) |
| Nausea | 11 (4.1%) | 12 (4.5%) |
| Dry Mouth | 10 (3.7%) | 10 (3.7%) |
| Diarrhoea | 8 (3.0%) | 14 (5.2%) |
| Tremor | 8 (3.0%) | 0 (0%) |
| Alanine aminotransferase increased | 3 (1.1%) | 6 (2.2%) |

### Safety Takeaways

Compound **(1)** was generally well-tolerated and demonstrated a safety profile consistent with previous clinical studies. Compound **(1)** was generally well tolerated and demonstrated a safety profile consistent with previous clinical studies; trial completion rate was 90.3% in the Compound **(1)** group.

The incidence of treatment emergent adverse events (TEAEs) in the Compound **(1)** group was 60.1% vs 44.6% in the placebo (PBO) group. The majority of the TEAEs were mild to moderate in intensity. TEAEs occurring in at least 5% of Compound **(1)-**treated patients included somnolence 15.3% (3.0% PBO), dizziness 13.8% (2.2% PBO), headache 10.8% (7.8% PBO), and sedation 7.5% (0.4% PBO). No loss of consciousness or adverse effects of weight gain, sexual dysfunction, or euphoria were reported.

No deaths occurred in the study; two patients (0.7%) each in the Compound **(1)** and placebo groups reported serious adverse events. The incidence of TEAEs leading to study drug discontinuation was 3.4% and 1.5% in the Compound **(1)** and placebo groups, respectively.

No signals for withdrawal symptoms or increased suicidal ideation or behavior were identified as assessed by the 20-item Physician Withdrawal Checklist and the Columbia-Suicide Severity Rating Scale, respectively.

### 3.1 Detailed Safety Results

The study of Example 1 was designed to evaluate the efficacy, safety, and tolerability of Compound **(1)-**50 mg Compound **(1)-**50 mg compared with placebo in patients with MDD. Given that the AEs associated with current monoaminergic ADTs used as treatment for MDD (*e.g.,* SSRIs, SNRIs, TCAs) may result in treatment discontinuation, the unique safety profile of Compound **(1)** is of great interest and is reported here.

Safety and tolerability were evaluated by incidence and severity of adverse events (AEs), serious adverse events (SAEs), changes from baseline in clinical laboratory evaluations, vital signs, and 12-lead electrocardiography. Suicidality was monitored by the Columbia Suicide Severity Rating Scale (C-SSRS). The Physician Withdrawal Checklist-20 total score (PWC-20_{TS}) was used to monitor for the presence of potential withdrawal symptoms following discontinuation of Compound **(1)-**50 mg.

### Results

Patients (N=543) were randomized 1:1 to receive Compound **(1)-**50 mg (n=271) or placebo (n=272). The Safety Set included all patients who received at least one dose of Compound **(1)-**50 mg (n=268) or placebo (n=269). Overall, 242 (90.3%) patients in the Compound **(1)-**50 mg group and 235 (87.4%) in the placebo group completed the study. Demographic and baseline clinical characteristics were balanced between the treatment groups. The proportion of patients using antidepressants at baseline was 29.5% vs 30.1% in the Compound **(1)** and placebo groups, respectively.

Compound **(1)-**50 mg was generally well-tolerated, with a safety profile consistent with previous clinical studies.

The proportion of patients who reported a treatment emergent adverse event (TEAE) was 60.1% (161/268) in the Compound **(1)-**50 mg group and 44.6% (120/269) in the placebo group. The majority of the TEAEs were mild to moderate, with 8 (3.0%) and 3 (1.1%) patients having severe events in the Compound **(1)-**50 mg and placebo groups, respectively. The most common TEAEs (≥5% in any treatment group) included somnolence (15.3% vs. 3.0%), dizziness (13.8% vs. 2.2%), headache (10.8% vs. 7.8%), sedation (7.5% vs. 0.4%), and diarrhea (3.0% vs. 5.2%) in the Compound **(1)-**50 mg and placebo groups, respectively.

No AEs of loss of consciousness, weight gain, sexual dysfunction, or euphoria were reported.

Of patients who received treatment and discontinued study drug due to a TEAE(s), 3.4% (9/268) were in the Compound **(1)-**50 mg group and 1.5% (4/269) were in the placebo group. Two patients (0.7%) in each treatment group experienced serious adverse events (SAEs). One patient in each group experienced a treatment-related SAE that occurred during the treatment period. The patient in the Compound **(1)-**50 mg group who experienced SAEs of psychotic disorder and slow speech had a complicated medical history and varied aspects of the underlying psychiatric disease and behavior, notable for posttraumatic stress disorder, anxiety, multiple psychiatric hospitalizations due to suicidality, auditory and visual hallucinations, drug and alcohol abuse, and medication non-compliance. The patient in the placebo group had liver function levels increased (alanine aminotransferase, aspartate aminotransferase, blood alkaline phosphatase, gamma-glutamyltransferase). There was no signal of increased suicidal ideation/behavior as assessed by the C-SSRS observed throughout the study in patients receiving Compound **(1)-**50 mg or patients receiving placebo (0% worsening in suicidal ideation or behavior for either Compound **(1)-**50 mg or placebo groups at Day 15). No meaningful differences were observed in the incidence and type of adverse events between patients receiving Compound **(1)** with or without existing antidepressant therapy (Table 10).

**Table 10. Treatment-Emergent Adverse Events (>2%) by Antidepressant Use at Baseline: Safety Population**

| | **ADT use at baseline=Yes** | | **ADT use at baseline=No** | |
|---|---|---|---|---|
| | **Compound (1) 50 mg (N=79)** | **Placebo (N=81)** | **Compound (1) 50 mg (N=189)** | **Placebo (N=188)** |
| **Any TEAE** | 50 (63.3) | 33 (40.7) | 111 (58.7) | 87 (46.3) |
| **Dizziness** | 10 (12.7) | 2 (2.5) | 27 (14.3) | 4 (2.1) |
| **Headache** | 9 (11.4) | 2 (2.5) | 20 (10.6) | 19 (10.1) |
| **Somnolence** | 9 (11.4) | 1 (1.2) | 32 (16.9) | 7 (3.7) |
| **Sedation** | 7 (8.9) | 1 (1.2) | 13 (6.9) | 0 |
| **Tremor** | 3 (3.8) | 0 | 5 (2.6) | 0 |
| **Diarrhea** | 4 (5.1) | 6 (7.4) | 4 (2.1) | 8 (4.3) |
| **Dry mouth** | 4 (5.1) | 2 (2.5) | 6 (3.2) | 8 (4.3) |
| **Nausea** | 1 (1.3) | 3 (3.7) | 10 (5.3) | 9 (4.8) |
| **Suicidal ideation** | 3 (3.8) | 0 | 2 (1.1) | 0 |
| **Insomnia** | 2 (2.5) | 3 (3.7) | 1 (0.5) | 2 (1.1) |
| **Abnormal dreams** | 2 (2.5) | 1 (1.2) | 3 (1.6) | 1 (0.5) |
| **Restlessness** | 2 (2.5) | 0 | 1 (0.5) | 0 |
| **Upper respiratory tract infection** | 2 (2.5) | 1 (1.2) | 1 (0.5) | 1 (0.5) |
| **Urinary tract infection** | 2 (2.5) | 1 (1.2) | 0 (0) | 3 (1.6) |
| **Gastroenteritis** | 2 (2.5) | 0 | 2 (1.1) | 1 (0.5) |
| **Alanine aminotransferase increased** | 2 (2.5) | 1 (1.2) | 1 (0.5) | 5 (2.7) |
| **Disturbance in attention** | 0 | 0 | 5 (2.6) | 0 |
| **Flatulence** | 0 | 0 | 4 (2.1) | 1 (0.5) |

| | | | | |
|---|---|---|---|---|
| Data are represented as n (%). | | | | |

Compound **(1)** led to greater numerical improvements in insomnia symptoms, as reflected by greater reductions from baseline compared with placebo in HAMD-17 insomnia individual item scores at Day 15.

Insomnia was assessed based on HAMD-17 individual items for early insomnia (difficulty falling asleep), middle insomnia (waking during the night and unable to immediately fall back to sleep), and late insomnia (waking during early hours of the morning before completing sleep cycle).

Mean reduction from baseline was numerically greater vs placebo (nominal *P<*0.05*) for: Early insomnia: -1.0 vs -0.7; Middle insomnia: -1.2 vs -0.8; and Late insomnia: -0.8 vs -0.5.

There was no signal for increased suicidal ideation or suicidal behavior with Compound **(1)** (Table 11).

**Table 11. Shift from Baseline in Suicidal Ideation/Behavior (C-SSR Evaluation): Safety Population.**

| | **Assessment** | **Baseline** | **Day 8** | **Day 15** | **Day 42** |
|---|---|---|---|---|---|
| | | **N=268** | **N = 255** | **N = 248** | **N = 240** |
| **Compound (1)** | **No suicidal ideation/behavior, n (%)** | 158 (58.9) | 237 (92.9) | 229 (92.3) | 223 (92.9) |
| | **Suicidal ideation, n (%)** | 106 (39.6) | 18 (7.1) | 19 (7.7) | 17 (7.1) |
| | **Suicidal behavior, n (%)** | 4 (1.5) | 0 | 0 | 0 |

| | | **N=268** | **N = 255** | **N = 248** | **N = 240** |
|---|---|---|---|---|---|
| **Placebo** | **No suicidal ideation/behavior, n (%)** | 134 (49.8) | 231 (88.2) | 236 (94.0) | 208 (89.3) |
| | **Suicidal ideation, n (%)** | 133 (49.4) | 31 (11.8) | 15 (6.0) | 25 (10.7) |
| | **Suicidal behavior, n (%)** | 2 (0.7) | 0 | 0 | 0 |

| | | | | | |
|---|---|---|---|---|---|
| N = number of patients in the specific treatment group with non-missing C-SSRS evaluation at both baseline and specific visit. | | | | | |

No signals for withdrawal symptoms were observed as assessed by the PWC-20 (PWC-20 total score [standard deviation] at Day 15 was 7.3 [6.57] and 8.0 [6.57] for Compound (1)-50 mg and placebo respectively). The mean decrease (indicating improvement) in the PWC-20 total score was similar between the treatment groups, suggesting no withdrawal effect after completion of treatment or discontinuation of Compound **(1)** (Table 12).

**Table 12. Summary of PWC-20 Total Score by Study Visit: Safety Population.**

| | | **Compound (1) 50 mg N = 268** | **Placebo N = 269** |
|---|---|---|---|
| **Day 18** | **n** | 193 | 190 |
| | **CFFA, mean (SD)** | -1.2 (4.32) | -1.2 (4.32) |
| **Day 21** | **n** | 186 | 181 |
| | **CFFA, mean (SD)** | -0.3 (4.37) | -0.5 (4.36) |

| | | | |
|---|---|---|---|
| CFFA = change from the first assessment on or after the last dose within 1 day of last dose. A decrease in PCW-20 total score indicates improvement; n = number of patients that day. | | | |

### Conclusions

There were no clinically meaningful differences in the safety profile for Compound (1)-50 mg monotherapy compared with those receiving Compound **(1)-**50 mg in combination with pre-existing antidepressant therapy. No deaths were reported during the study.

Consistent with the results of previous clinical studies, Compound **(1)** was generally safe and well tolerated in patients with MDD. Approximately 3% of patients treated with Compound **(1)** discontinued treatment due to a TEAE. No TEAEs of weight gain, sexual dysfunction, or euphoria were reported in the study, suggesting improvement of depressive symptoms can be achieved without these AEs that are commonly reported with standard-of-care monoaminergic antidepressants. No evidence of withdrawal symptoms or increased suicidal ideation/behavior were identified.

### Example 4. A Multicenter, Randomized, Double-Blind, Parallel-Group, Placebo-Controlled Study Evaluating The Efficacy, Safety, And Pharmacokinetics Of Compound (1) In The Treatment Of Adult Female Subjects With Severe Postpartum Depression

### ClinicalTrials.gov Identifier: NCT02978326

*Primary Efficacy Objective:* To determine if treatment with Compound **(1)** reduces depressive symptoms in subjects with severe postpartum depression (PPD) compared to placebo as assessed by the change from baseline in the 17-item Hamilton Rating Scale for Depression (HAM-D) total score at Day 15.

*Secondary Efficacy Objectives:*
- To determine if treatment with Compound **(1)** Capsules 30 mg QD reduces depressive symptoms in subjects with severe PPD compared to placebo as assessed by the change from baseline in the HAM-D total score at all other time points.
- To determine if treatment with Compound **(1)** Capsules 30 mg QD reduces depressive symptoms compared to placebo as assessed by HAM-D Response, HAM-D Remission, change from baseline in Montgomery-Åsberg Depression Rating Scale (MADRS) total score, Clinical Global Impression - Improvement (CGI-I) Response, and changes from baseline in HAM-D subscales and individual item scores at Day 15 and all other time points.
- To determine if treatment with Compound **(1)** Capsules 30 mg QD reduces anxiety symptoms compared to placebo as assessed by changes from baseline in Hamilton Anxiety Rating Scale (HAM-A) total score Day 15 and all other time points.

*Safety Objective:* To evaluate the safety and tolerability of Compound **(1)** compared to placebo as assessed by the incidence of adverse events, vital sign measurements, clinical laboratory evaluations, electrocardiogram (ECG) parameters, and the Columbia Suicide Severity Rating Scale (C-SSRS).

*Other Objectives:*
- To assess Healthcare Resource Utilization (HCRU) at baseline and Day 45.
- To determine if treatment with Compound **(1)** Capsules 30 mg QD reduces subject-reported depressive symptoms compared to placebo as assessed by the change from baseline in the Edinburgh Postnatal Depression Scale (EPDS) total score and the Patient Health Questionnaire (PHQ-9) total score at Day 15 and other time points.
- To determine if treatment with Compound **(1)** Capsules 30 mg QD improves maternal behaviors compared to placebo as assessed by the change from baseline in the Barkin Index of Maternal Functioning (BIMF) total and subscale scores at Day 15 and other time points.
- To determine if treatment with Compound **(1)** Capsules 30 mg QD improves the general health status compared to placebo as assessed by the change from baseline in the Short Form-36 (SF-36) total score at Day 15 and other time points.

*Pharmacokinetic Objective:* To assess the pharmacokinetic (PK) profile of Compound **(1)** in plasma samples and the concentration of Compound **(1)** in breast milk, when possible, following administration of Compound **(1).**

### Study Design and Methodology

This was a multicenter, randomized, double-blind, parallel-group, placebo-controlled study of the efficacy, safety, and pharmacokinetics of Compound **(1)** in adult subjects diagnosed with severe PPD.

The study was be conducted in 2 parts. One subject was enrolled and dosed in Part A before it was closed to enrollment; the current amendment describes Part B only.

### Screening Period:

The Screening Period began with the signature of the informed consent form (ICF). The diagnosis of depression was be determined using the Structured Clinical Interview for Diagnostic and Statistical Manual of Mental Disorders-5 (DSM-5) Axis I Disorders (SCID-I). Eligibility was be determined by applying the inclusion/exclusion criteria. A full medical and family history was be taken including recording of all major depression episodes, other Axis I and Axis II disorders, and postpartum depression episodes in immediate female family members.

### Treatment Period:

Once subjects were confirmed as eligible for the study, they were randomized to active study drug or placebo on a 1:1 basis.

Randomized subjects received 30 mg QD of study drug (Compound **(1)** Capsules or placebo). Those subjects who could not tolerate 30 mg QD will received 20 mg QD for the remainder of the Treatment Period. Subjects who experienced intolerable adverse events (AEs) at the 20 mg QD dose level could be discontinued from study treatment at the discretion of the Investigator. Subjects were instructed to take the study drug with food. Study drug was self-administered by subjects in the evening (8:00 pm ±30 min) on an outpatient basis for the entire 14-day Treatment Period. Study drug administration was monitored via a follow-up call from the site each evening (within approximately 1 hour following the scheduled evening dose) on Days 1 to 14.

Subjects were not allowed to initiate psychotropic medications or other medications that may potentially have an impact on efficacy or safety endpoints within 30 days prior to informed consent until completion of the Day 15 assessments. Psychotropic medications initiated at least 30 days prior to informed consent had to remain at a stable dose until completion of the Day 15 assessments.

Efficacy and safety assessments were performed periodically during the study, and blood samples could be collected for analysis of Compound **(1)** and metabolites of Compound **(1)** as outlined in the Schedule of Events in Table 13. Blood samples will be collected and outcome measures will be obtained at pre-specified times over the 14-day Treatment Period. In addition, breast milk for assessment of Compound **(1)** concentrations could be collected if consent was received from the subject.

### Follow-up Period:

The Follow-up Period assessments was conducted on an outpatient basis on Day 21±1 day and Day 45±3 days after the initiation of study drug administration.

### Number of Subjects:

Approximately 140 subjects were randomized in a 1:1 ratio for approximately 70 subjects per treatment group. Additional subjects could be enrolled in order to ensure there were 130 evaluable subjects. Evaluable subjects were defined as those randomized subjects receiving study drug with valid baseline and at least 1 post-baseline HAM-D assessment.

### Inclusion Criteria:

The following inclusion criteria had to be met for individuals to be eligible for the study.
1. Subject has signed an ICF prior to any study-specific procedures being performed.
2. Subject is an ambulatory female between 18 and 45 years of age, inclusive.
3. Subject is in good physical health and has no clinically significant findings, as determined by the Investigator, on physical examination, 12-lead ECG, or clinical laboratory tests.
4. Subject agrees to adhere to the study requirements.
5. Subject either must have ceased lactating at screening or, if still lactating or actively breastfeeding at screening, must agree to temporarily cease giving breast milk to her infant(s) from just prior to receiving study drug through Day 21, allowing for a 7-day washout after the last dose of study drug.
6. Subject must have a negative pregnancy test at screening and Day 1 prior to the start of study drug administration.
7. Subject has had a major depressive episode that began no earlier than the third trimester and no later than the first 4 weeks following delivery, and meets criteria for major depressive episode per DSM-5, diagnosed by Structured Clinical Interview for (DSM-5) Axis I Disorders (SCID-I).
8. Subject has a HAM-D total score of ≥26 at screening and Day 1 (prior to randomization).
9. Subject is ≤6 months postpartum.
10. Subject is willing to delay start of other antidepressant or anxiety medications and any new pharmacotherapy regimens, including as-needed benzodiazepine anxiolytics, until after the Treatment Period ends and all Day 15 assessments have been completed.
11. Subject has no detectable hepatitis B surface antigen (HBsAg), no detectable anti-hepatitis C virus (HCV), detectable anti-HCV but negative viral load, and no detectable human immunodeficiency virus (HIV) antibody at screening.
12. Subject agrees to use 1 of the following methods of contraception during participation in the study and for 30 days following the last dose of study drug, unless they are surgically sterile:
   - Combined (estrogen and progestogen containing) oral, intravaginal, or transdermal hormonal contraception associated with inhibition of ovulation.
   - Oral, injectable, or implantable progestogen-only hormonal contraception associated with inhibition of ovulation.
   - Intrauterine device.
   - Intrauterine hormone-releasing system.
   - Bilateral tubal occlusion.
   - Vasectomized partner.

### Exclusion Criteria

Subjects were excluded if they meet any of the following exclusion criteria.
1. Subject has a recent history or active clinically significant manifestations of metabolic, hepatic, renal, hematological, pulmonary, cardiovascular, gastrointestinal, musculoskeletal, dermatological, urogenital, neurological, or eyes, ears, nose, and throat disorders, or any other acute or chronic condition that, in the Investigator's opinion, would limit the subject's ability to participate in or complete this clinical study.
2. Subject has a known allergy to Compound **(1)** Capsule or its excipients.
3. Subject has active psychosis per Investigator assessment.
4. Subject has attempted suicide associated with the current episode of PPD.
5. Subject has a medical history of seizures
6. Subject has a medical history of bipolar disorder, schizophrenia, and/or schizoaffective disorder.
7. Subject has a history of active alcoholism or drug addiction (including benzodiazepines) in the 12 months prior to screening.
8. Subject has had exposure to another investigational medication or device within 30 days prior to screening.
9. Subject has prior participation in any brexanolone or Compound **(1)** clinical study.
10. Subject who presents for the study while currently receiving psychotropic medications that are used with the intent to treat depressive symptoms such as antidepressants, atypical antipsychotics, etc., which have not been taken at the same dose for at least 30 days prior to Day 1. (Subjects presenting for the study who have stopped taking these medications within the 30 days prior to the start day of study drug may be eligible if they will be off of the medications for longer than 5 half-lives until the start day of study drug).
11. Use of any known strong inhibitors of cytochrome P450 (CYP)3A4 within 14 days or 5 half-lives (whichever is longer) or consumed grapefruit juice, grapefruit, Seville oranges, or products containing these within 14 days prior to receiving the first dose of study drug and throughout the study.
12. Use of any CYP inducers, such as rifampin, carbamazepine, ritonavir, enzalutamide, efavirenz, nevirapine, phenytoin, phenobarbital or St John's Wort, within 14 days or 5 half-lives (whichever is longer) prior to the first dose of study drug and throughout the study.
13. Subject has a positive urine drug test at the screening visit.
14. Subject plans to undergo elective surgery during participation in the study

### Investigational Product, Dosage, and Mode of Administration:

Compound **(1)** Capsules were available as hard gelatin capsules containing a white to off-white powder. In addition to the specified amount of Compound **(1)** Drug Substance, active Compound **(1)** Capsules contained croscarmellose sodium, mannitol, silicified microcrystalline cellulose, and sodium stearyl fumarate as excipients. Capsules were available in 10-mg, 20-mg, and 30-mg strengths in order to provide treatment doses of 20 mg and 30 mg. Subjects were administered 2 capsules per dose.

### Reference Therapy, Dosage, and Mode of Administration:

Matched placebo capsules containing only the above-listed capsule excipients were provided. Subjects were administered 2 placebo capsules per day, to maintain blinding.

### Duration of Participation:

Up to 76 days (14 days of treatment).

### Randomization:

Subjects were randomized to receive Compound **(1)** or matching placebo in a 1:1 ratio. Subjects, clinicians, and the study team were blinded to treatment allocation. Randomization was performed centrally via an interactive response technology (IRT) system.

### Dose Adjustment for Safety/Tolerability Reasons:

During the Treatment Period, subjects were able to receive study drug as long as there were no dose-limiting safety/tolerability concerns. Dose adjustment criteria are described above.

### Criteria for Evaluation:

### Primary Efficacy Endpoint

The primary efficacy endpoint was the change from baseline in HAM-D total score at the end of the Treatment Period (Day 15). The HAM-D total score was calculated as the sum of the 17 individual item scores.

### Secondary Efficacy Endpoints

Secondary endpoints included:
- Change from baseline in the HAM-D total score at all time points other than Day 15;
- HAM-D Response defined as a 50% or greater reduction from baseline in HAM-D total score;
- HAM-D Remission defined as a HAM-D total score of ≤7;
- Change from baseline in MADRS total score at Day 15 and other time points;
- CGI-I Response defined as "very much improved" or "much improved";
- Change from baseline in HAM-A total score at Day 15 and other time points;
- Changes from baseline in HAM-D subscales and individual item scores at Day 15 and other time points

Safety Endpoints: Safety and tolerability of study drug was evaluated by frequency of adverse events; severity, relatedness, and seriousness of adverse events; clinical laboratory measures, vital signs, ECGs; and concomitant medication usage. Suicidality was monitored using the C-SSRS.

Concomitant medications: The doses of all psychotropic medications were recorded throughout the study. No changes and/or additions to antidepressant or anxiolytic medicine were allowed during the Treatment Period.

Measures of plasma concentration: Plasma samples were collected to assay for concentrations of Compound **(1).** Pharmacokinetic concentration data was characterized with population PK modeling techniques to estimate individual measures of exposure to Compound **(1).** Breast milk could be collected, and samples could be analyzed for Compound **(1)** as an optional assessment if consent was received from the subject.

### Other Endpoints

Additional measures of affective symptoms and function related to the current episode of PPD severity were collected before, during, and after the Treatment Period, including the EPDS, PHQ-9, BIMF, and SF-36. Healthcare resource utilization data, including baseline diagnosis history, baseline antidepressant treatment history, and healthcare visits, inpatient visits, and medication use, were collected at screening and on Day 45.

Total and subscale scores including changes from baseline were calculated where applicable. Changes from baseline to the end of the Treatment Period (Day 15) and other time points were evaluated as other efficacy endpoints. In addition to the above scores, total score categories and individual item scores were evaluated as other endpoints

### Statistical Methods:

### General:

For the purpose of all safety, efficacy, and other analyses where applicable, baseline was defined as the last measurement prior to the start of blinded study drug administration.

Continuous endpoints were summarized with number (n), mean, standard deviation, median, minimum, and maximum. In addition, change from baseline values were calculated at each time point and summarized descriptively. For categorical endpoints, descriptive summaries included counts and percentages.

### Analysis Sets and Methods:

The All Randomized Set, defined as all subjects who have been randomized, was used for subject disposition, demographics, and baseline characteristic summaries. Subjects were classified according to randomized treatment.

The Safety Set, defined as all subjects administered study drug, was used to provide descriptive summaries of safety data. Subjects were summarized according to treatment received.

The Efficacy Set, defined as all subjects in the All Randomized Set who complete at least 1 day of study drug and have a valid baseline and at least 1 post-baseline efficacy assessment, were used to analyze efficacy data. Efficacy data was analyzed using appropriate descriptive statistics and pre-specified statistical methods, as well as other data presentation methods where applicable; subject listings were provided for all efficacy data. Subjects were analyzed according to randomized treatment.

The PK Set consisted of all subjects in the Safety Set with plasma concentration determinations for Compound **(1),** and was used for population PK modeling.

The change from baseline in HAM-D total score was analyzed using a mixed effects model for repeated measures (MMRM); the model included center, treatment, baseline HAM-D total score, assessment time point, and time point-by-treatment as explanatory variables. All post-baseline time points were included in the model. The primary comparison was between Compound **(1)** and placebo at the 15-day time point. Model-based point estimates (*e.g.,* least squares [LS] means), 95% confidence intervals, and p-values were reported. An unstructured covariance structure was used to model the within-subject errors. Continuous secondary and other variables were analyzed using similar methods.

Binary efficacy endpoints, including responder and remission endpoints, were summarized and analyzed using the generalized estimating equation method.

Adverse events were coded using Medical Dictionary for Regulatory Activities (MedDRATM) Version 19.1 or higher. The overall incidence of adverse events was displayed by System Organ Class, preferred term, and treatment. The incidence of adverse events was also presented by maximum severity and relationship to study drug. Vital signs, clinical laboratory measures, ECG, concomitant medication usage, and C-SSRS data was summarized by treatment, where applicable. Out-of-range safety endpoints could be categorized as low or high, where applicable. Safety data was summarized and examined for possible relationships between subject characteristics and plasma Compound **(1)** concentrations, as appropriate. Suicidality data collected using the C-SSRS at baseline and at each visit during the active Treatment Period was listed for all subjects. The C-SSRS listings included behavior type and/or category for suicidal ideation and suicidal behavior of the C-SSRS.

### Sample Size Calculation:

Assuming a 2-sided test at an alpha level of 0.05, a sample size of approximately 65 subjects per treatment group would provide 90% power to detect a placebo-adjusted treatment difference of approximately 4 points in the primary endpoint, change from baseline in HAM-D total score at Day 15 assuming standard deviation (SD) of 7 points.

Assuming a 10% dropout and a 1:1 randomization ratio, approximately 72 randomized subjects per treatment group were going to be required to obtain 130 evaluable subjects. Evaluable subjects are defined as those randomized subjects who received study drug and have a valid baseline and at least 1 post-baseline HAM-D assessment. Additional subjects could be randomized if the dropout rate is higher than 10%.

Table 13 shows the Schedule of Events for this clinical study.

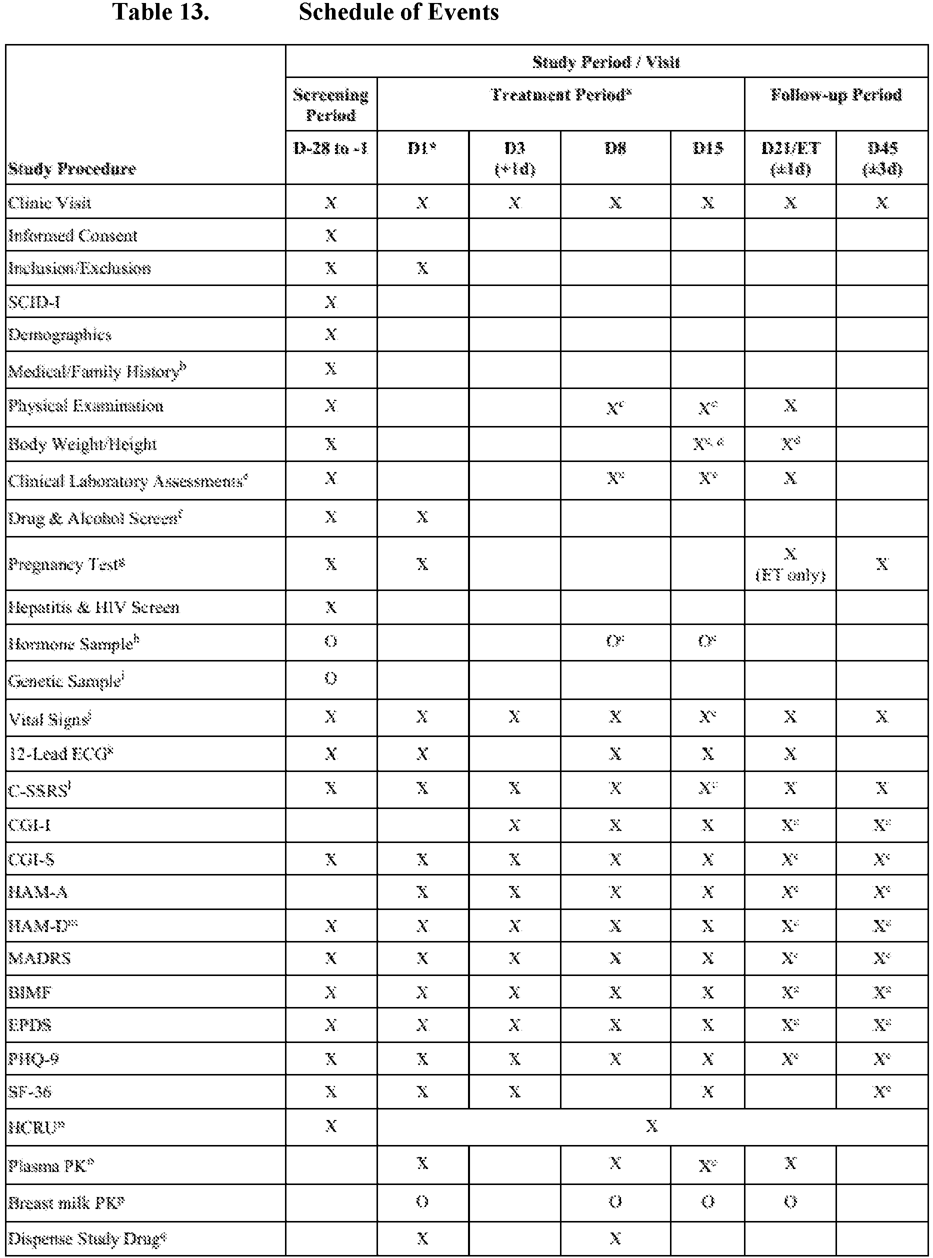

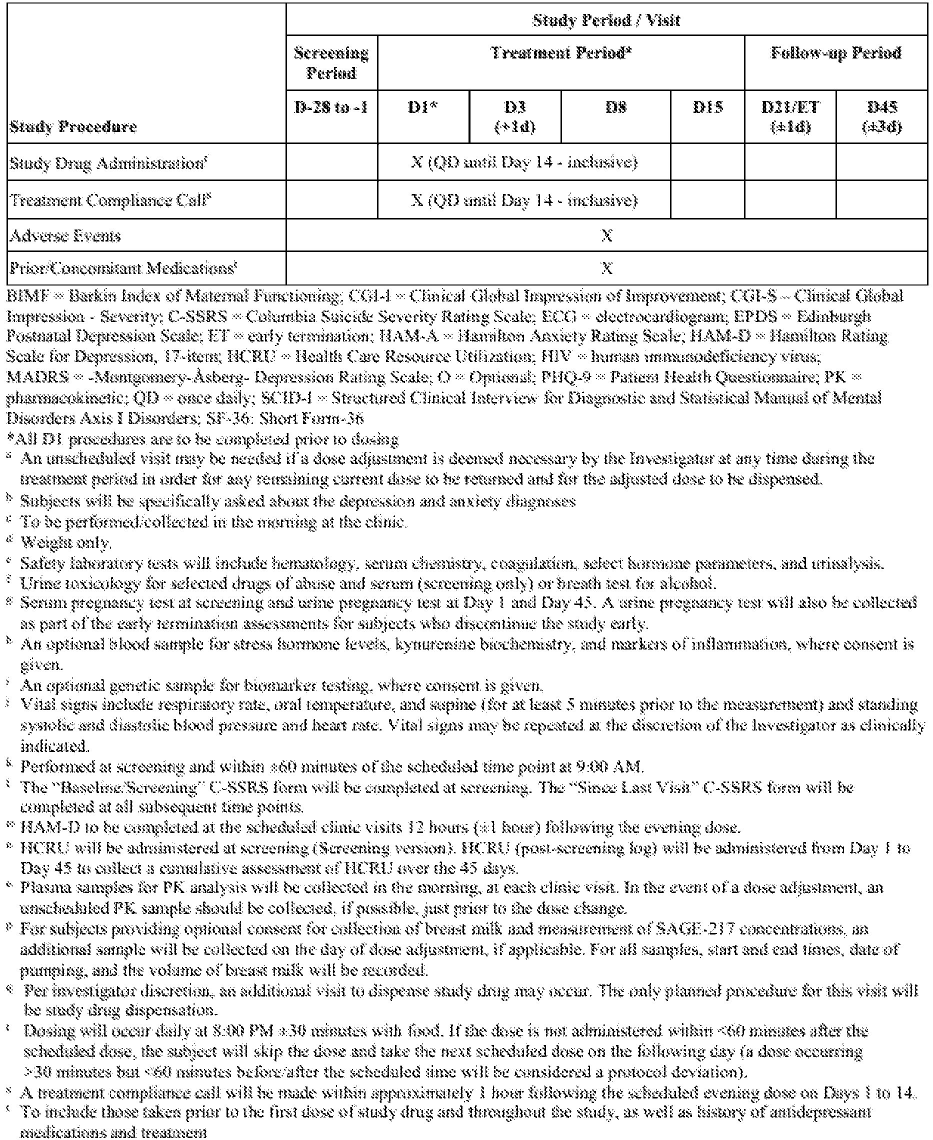

**Example 5. Rapid and sustained improvement in concurrent symptoms of depression and anxiety in a post hoc analysis of Compound (1) treatment in postpartum depression (PPD).**

### Introduction

Postpartum depression (PPD) is one of the most common medical complications during and after pregnancy. In the United States, estimates of women with self-reported symptoms of PPD vary each year by state from 9.7%-23.5%, with an overall prevalence of 13.2%. PPD symptoms can be associated with a significant impairment in mother-infant bonding and maternal function, including breastfeeding and caring for the child, with implications for the child's health and development. Multiple environmental and biological risk factors have been proposed to play a role in the development of PPD.

Compound **(1)** is an oral neuroactive steroid under investigation as a once-daily, 2-week therapy for major depressive disorder and PPD. Compound **(1)** is a positive allosteric modulator of GABAA receptors, and activity at these receptors may play a role in restoring adaptive signaling in the brain. Dysregulated adaptive signaling in neuronal networks has been implicated as a key mechanism in depression.

In a double-blind, randomized, placebo-controlled Phase 3 trial in adult women with PPD (NCT02978326; Clinical trial of Example 4), Compound **(1)** achieved the primary endpoint of a statistically significant reduction in symptoms of depression compared to placebo assessed by the 17-item clinician-rated Hamilton Rating Scale for Depression (HAMD-17) at Day 15. This example, with post hoc analyses, examined concurrent improvement of depressive and anxiety symptoms at Day 15 and Day 45 (trial follow-up). The study protocol is shown in Example 4.

### Methods

As indicated in Example 4, women (N = 151), ages 18-45, ≤6 months postpartum, diagnosed with PPD (defined as a major depressive episode with onset in the 3rd trimester or ≤ 4 weeks postpartum), and a baseline HAMD-17 total score ≥26, were randomized to study drug in an outpatient setting.

Patients were randomized 1:1 to receive oral Compound **(1)** 30 mg or placebo once daily for 14 days, with 4 weeks follow-up. The change from baseline (CFB) vs placebo in HAMD-17 at Day 15 was the primary endpoint. Treatment emergent adverse events (TEAEs) were assessed throughout the study.

Secondary endpoints included the CFB in the HAMD-17 at all other measured time points aside from the primary endpoint on Day 15, CFB in the Hamilton Rating Scale for Anxiety (HAM-A), and CFB in the Montgomery-Åsberg Depression Rating Scale (MADRS).

Post hoc analyses explored concurrent anxiety and depression improvement using a combination of scales at Days 15 and 45: **(1)** HAMD-17 ≤7+HAM-A ≤7, or (2) MADRS ≤10+HAM-A ≤7. Depression improvement was defined as either a HAMD-17 total score ≤7, or a MADRS total score ≤10. Improvement in anxiety symptoms was defined as a HAM-A total score ≤7.

CFB in HAMD-17 total score was evaluated using the least-squares mean from a mixed effect model for repeated measures. Concurrent improvement rates were assessed using Fisher's exact test, while estimates for odds ratios (ORs) and 95% confidence intervals (CIs) for ORs were derived using generalized estimating equations models for repeated measures, adjusting for baseline covariates (Table 14).

Sustained concurrent improvement was defined as meeting concurrent improvement criteria on both Day 15 and Day 45 and was assessed using Fisher's exact test. Secondary endpoints and post hoc analyses were not adjusted for multiplicity.

### Results

The Compound **(1)** and placebo arms included 76 and 74 patients, respectively, who were randomized and included in the efficacy analyses. Baseline demographics and patient characteristics were well balanced between the two treatment arms and have been described in detail previously.

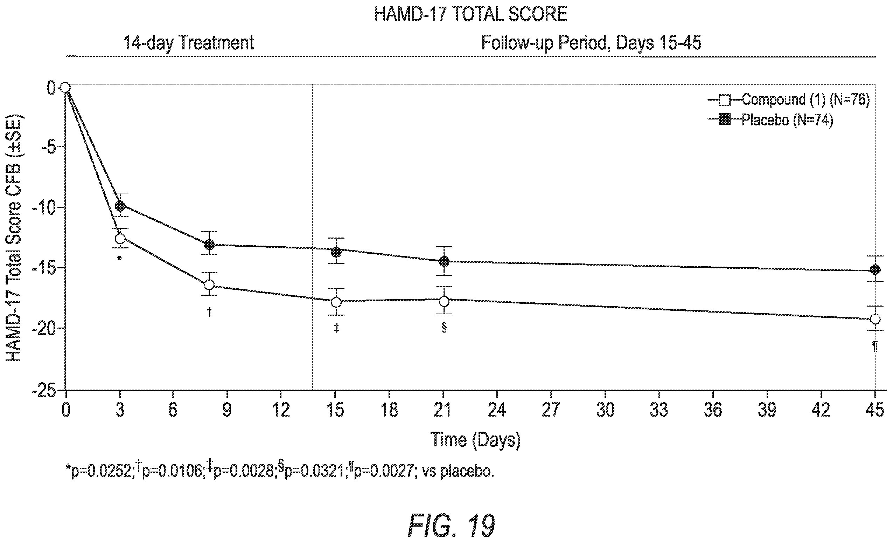

Compound **(1)** demonstrated statistically significant Day 15 CFB versus placebo in HAMD-17 total score (primary endpoint: -17.8 vs -13.6, p=0.0029) (FIG. 19) LS mean difference -2.4, 95% CI -4.4 to -0.3; *P* = .02.

Compound **(1)** was generally well tolerated, as previously described. The most common TEAEs occurring in ≥5% of patients who received Compound **(1)** were somnolence, headache, dizziness, upper respiratory tract infection, diarrhea, and sedation. One subject experienced a serious adverse event (SAE) in the Compound **(1)** arm that resolved after dose reduction, and one subject experienced an SAE in the placebo arm. There were no reports of loss of consciousness or syncope in either arm.

The likelihood of achieving concurrent improvement of depressive and anxiety symptoms (at Day 15 and Day 45) as well as sustained concurrent improvement were significantly higher for Compound **(1)-**treated patients (Table 14).

**Table 14. Treatment differences in concurrent symptoms (Compound (1) vs placebo)**

| **Improvement: HAMD-17 ≤7 and HAM-A ≤7** | **OR* (95% CI**)** | **p-value** |
|---|---|---|
| Day 15 | 2.75 (1.30, 5.84) | 0.0083 |
| Day 45 | 3.45 (1.66, 7.16) | 0.0009 |
| Sustained Concurrent Improvement | 6.21 (2.22, 17.41 | <0.001 |

| **Improvement: MADRS ≤10 and HAM-A ≤7** | **OR (95% CI)** | **p-value** |
|---|---|---|
| Day 15 | 2.35 (1.15, 4.80) | 0.0188 |
| Day 45 | 3.09 (1.50, 6.36) | 0.0009 |
| Sustained Concurrent Improvement | 3.71 (1.54, 8.95 | <0.001 |

| | | |
|---|---|---|
| * Odds Ratio; ** Confidence Interval | | |

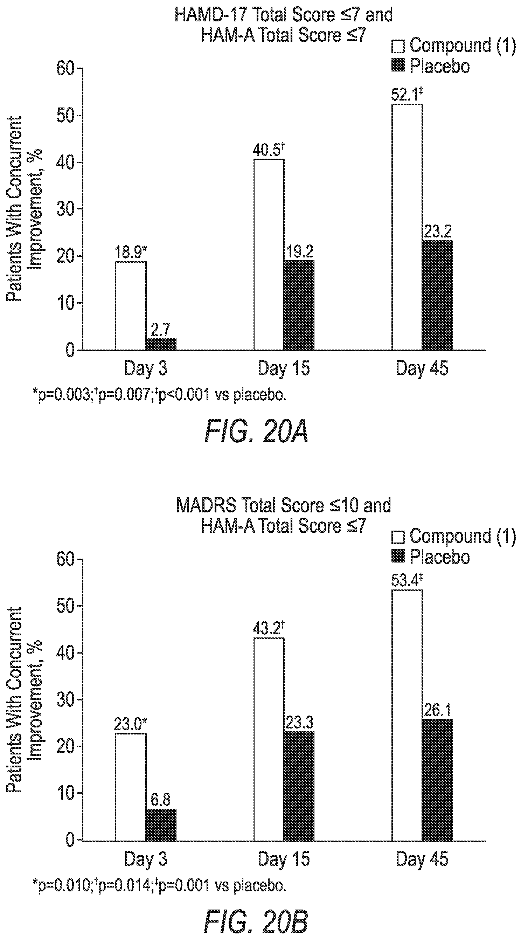

A significantly higher proportion of Compound **(1)-**treated patients achieved concurrent improvement of depressive and anxiety symptoms at Day 3 (p=0.003), Day 15 (p=0.007), and Day 45 (p<0.001) using the HAMD-17 and HAM-A scale combination (model adjusted p-values) (FIG. 20A).

Similarly, a significantly higher proportion of Compound **(1)-**treated patients achieved concurrent improvement of depressive and anxiety symptoms at Day 3 (p=0.010), Day 15 (p=0.014), and Day 45 (p=0.001) using the MADRS and HAM-A scale combination (model adjusted p-values) (FIG. 20B).

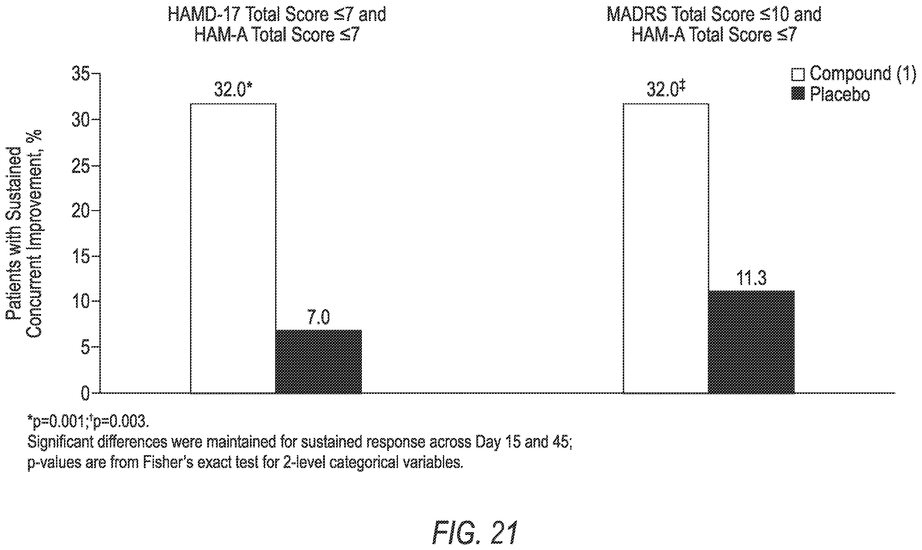

A significantly higher proportion of Compound **(1)-**treated patients achieved sustained concurrent improvement of anxiety and depression at Day 15 and Day 45, using HAMD-17/HAM-A (p<0.001). Similarly, a significantly higher proportion of Compound **(1)-**treated patients achieved sustained concurrent improvement of anxiety and depression from Day 15 to Day 45, using MADRS/HAM-A (p=0.003) (FIG. 21).

### Conclusions

Compound **(1)** achieved the primary endpoint of a statistically significant reduction in HAMD-17 total score at Day 15. Compound **(1)** demonstrated rapid (Day 3 and Day 15) and sustained (Day 45) improvements in core depression symptoms.

In post hoc analyses, sustained concurrent improvement of depression and anxiety symptoms were seen from Day 15 to Day 45.

Together these findings support the development of neuroactive steroids for the treatment of core depressive symptoms, as well as anxiety in patients with PPD.

### Example 6. Rapid Improvement And Sustained Effect Of Compound (1) On Depressive Symptoms, Residual Symptoms, And Patient-Reported Outcomes In Postpartum Depression

### Brief Summary

This study assessed the effects of Compound **(1)** on depressive symptoms, anxiety- and sleep-related symptoms, and patient-reported outcomes (PROs) in women with postpartum depression (PPD). This was post hoc analysis of the study of Example 4 (NCT02978326), a phase 3, double-blind, randomized, outpatient, placebo-controlled trial that was conducted from January 2017 to December 2018, at 33 US sites. Participants included women aged 18 to 45 years, ≤ 6 months postpartum, with PPD and baseline 17-item Hamilton Rating Scale for Depression (HAMD-17) score ≥ 26. Participants were randomized 1:1 to receive placebo (n = 76) or Compound **(1)** 30 mg (n = 77) administered each evening for two weeks. A significantly higher proportion of patients in the Compound **(1)** group achieved HAMD-17 Response (*P* =.0042) and Remission (*P* = .0088) at day 15 vs placebo. Single-digit number needed to treat (NNTs) were found for HAMD-17 Response and Remission starting at day 15 and sustained through day 45. Compound **(1)** demonstrated a statistically significant decrease in least squares mean HAMD-17 anxiety/somatization change from baseline compared with placebo starting at day 3 (*P* = .0073) and through day 45 (*P* = .0033). A significantly higher proportion of Compound **(1)-**treated patients achieved concurrent improvement of anxiety and depression (all *P* < .05) as early as day 3, which was sustained at the group level on days 15 and 45. Compound **(1)** also demonstrated an improvement in patient-reported depressive symptoms compared with placebo. Therefore, Compound **(1)** provides a range of beneficial effects on depressive symptoms, as well as improvements in anxiety, insomnia, and other important PROs.

### Introduction

Postpartum depression (PPD) is one of the most common medical complications during and after pregnancy. (Bauman BL, et al. MMWR Morb Mortal Wkly Rep. 2020;69(19):575-581; Hamilton BE, et al. National Center for Health Statistics. Births: Provisional data for 2018. Vital Statistics Rapid Release; Report No 7. Published May 2019; DeSisto CL, et al. Prev Chronic Dis. 2014;11:E104; Centers for Disease Control and Prevention. Diabetes During Pregnancy. Updated June 12, 2018; Centers for Disease Control and Prevention. Data on Selected Pregnancy Complications in the United States. Updated February 28, 2019; Executive summary: hypertension in pregnancy. American College of Obstetricians and Gynecology. Obstet Gynecol. 2013;122(5):1122-1131; Callaghan WM, et al. Am J Obstet Gynecol. 2010;202(4):353.e1-e6). Expert opinions vary regarding the timing and onset of symptoms of PPD (ACOG Committee Opinion No. 757: Screening for perinatal depression. Obstet Gynecol. 2018; 132(5):e208---e212; Stewart DE, et al. Postpartum depression: literature review of risk factors and interventions. University Health Network Women's Health Program; 2003; American Psychiatric Association. Diagnostic and Statistical Manual of Mental Disorders (DSM-5). 5th ed. American Psychiatric Association; 2013; National Institutes of Mental Health. Perinatal depression). PPD is defined by the Diagnostic and Statistical Manual of Mental Disorders, Fifth Edition (DSM-5*)* as a major depressive episode with peripartum onset, occurring during pregnancy or within the first 4 weeks postpartum. In the United States, estimates of new mothers with self-reported symptoms of PPD each year vary by state from 9.7%-23.5%, with an overall prevalence of 13.2%. Symptoms of PPD can be associated with significant impairment in mother-infant bonding and maternal function, including breastfeeding, and caring for the child, all of which have implications for the child's health and development (Kerstis B, et al. Arch Womens Ment Health. 2016;19(1):87-94; Posmontier B. J Midwifery Womens Health. 2008;53(4):310-318; Barkin JL, et al. J Womens Health (Larchmt). 2016;25(7):707-713; Gagliardi L, et al. Arch Dis Child. 2012;97(4):355-357). Multiple environmental and biological risk factors have been proposed to play a role in the development of PPD, including a previous history of depression and more recently the COVID-19 pandemic (McLeam KT, et al. Arch Pediatr Adolesc Med. 2006;160(3):279-284; Moore Simas TA, et al. J Med Econ. 2020;23(2):174-183).

The pathophysiology of PPD is likely multifactorial (Robertson E, et al. Gen Hosp Psychiatry. 2004;26(4):289-295; Biaggi A, et al. J Afect Disord. 2016:191:62-77) with evidence supporting a role for disruption of perinatal γ-aminobutyric acid (GABA) signaling, the major inhibitory signaling pathway of the central nervous system (Yim IS, et al. Annu Rev Clin Psychol. 2015;11:99-137). Compound **(1),** an investigational oral neuroactive steroid under clinical study as a once-daily, 2-week therapy for major depressive disorder and PPD, is a positive allosteric modulator of GABA_{A} receptors, which may play a role in restoring adaptive signaling in the brain. Restoring homeostasis through an interplay between excitatory and inhibitory drive and modulatory signaling can be referred to as adaptive signaling. Dysregulated adaptive signaling in neuronal networks has been implicated as a key mechanism in depression. In vitro, Compound **(1)** was shown to bind to both synaptic and extrasynaptic GABA_{A} receptors, potentiating both phasic and tonic currents, respectively. Compound **(1)** has also demonstrated phasic GABA_{A} receptor activity in vitro that indicates it has a distinct binding sites from benzodiazepines.

The ROBIN study (NCT02978326; study of Example 4) was a randomized, double-blind, placebo-controlled, phase 3 trial designed to evaluate the efficacy and safety of Compound **(1)** in adult women with PPD. Primary end points and safety results from the ROBIN study have been published previously. Briefly, the study reported that Compound **(1)** achieved the primary end point of a statistically significant reduction from baseline in symptoms of depression compared to placebo assessed by the 17-item Hamilton rating scale for depression total score (HAMD-17) total score at day 15 (-17.8 vs -13.6, *P* = .0029 respectively). Treatment with Compound **(1)** was generally well tolerated; the most common treatment emergent adverse events (TEAEs) occurring in ≥ 5% of patients who received Compound **(1)** were somnolence, headache, dizziness, upper respiratory tract infection, diarrhea, and sedation.

Given the substantial negative impact of PPD on patients, here we evaluated the impacts of treatments on different aspects of patients' lives and well-being in this post hoc analysis. Therefore, we assessed the effects of Compound **(1)** on clinician-reported depressive outcomes, anxiety- and sleep-related symptoms, as well as patient-reported outcomes (PROs) of depression and health-related quality of life (QoL) in women with PPD.

### Methods

### Trial Design

The ROBIN trial design and patient inclusion/exclusion criteria are discussed in Example 4. The study enrolled women (n = 151), aged 18 to 45 years, ≤ 6 months postpartum, diagnosed with PPD (defined as a with onset in the third trimester or ≤ 4 weeks postpartum), and an HAMD-17 total score ≥ 26 at baseline. Patients were randomized 1:1 to receive oral Compound **(1)** 30 mg or placebo once daily for 14 days as outpatients, with follow-up to day 45. The primary end point was the change from baseline (CFB) versus placebo in HAMD-17 total score at day 15. TEAEs were assessed throughout the study.

### Post-hoc outcome measures

Secondary end points included CFB in the HAMD-17 at all other measured timepoints (aside from the primary end point on day 15), as well as in the Hamilton Rating Scale for Anxiety (HAM-A), and Montgomery-Åsberg Depression Rating Scale (MADRS). Safety and tolerability were evaluated by adverse events (AEs), vital signs, clinical laboratory evaluations, electrocardiogram parameters, and the Columbia-Suicide Severity Rating Scale (C-SSRS). Exploratory, patient-reported end points included depression measured by two commonly-used screening tools in the real-world setting including the 9-item Patient Health Questionnaire (PHQ-9, scores of 1-4 = minimal depression, 5-9 = mild depression, 10-14 = moderate depression, 15-19 = moderately severe depression; and 20-27 = severe depression), total Edinburgh Postnatal Depression Score (EPDS; a commonly-used screening tool for PPD, cutoff scores of < 7, < 10, and < 13 were evaluated), as well as the Medical Outcomes Study 36-Item Short Form Survey Instrument version 2 (SF-36v2, individual meaningful change for each domain defined as: physical component summary [PCS]: > 3.4; mental component summary [MCS]: > 4.6; physical functioning: > 4.3; role physical: > 3.4; bodily pain: > 6.2; general health: > 7.2; vitality: > 6.2; social functioning: > 6.9; role emotional: > 4.5; and mental health: > 6.2) as a global measure for generic health status.

### Response Definition and Analysis outcomes

The proportion of individual patients demonstrating HAMD-17 Response, Remission, sustained Response, or sustained Remission were examined between treatment groups, to calculate number needed to treat (NNT). Sustained Response or Remission was defined as continued categorization of a Responder/Remitter at both days 15 and 45. Tolerability and safety outcomes included number needed to harm (NNH) for discontinuation due to an AE, as well as individual safety events of interest. NNH was calculated for **(1)** AEs with incidence ≥ 2% with Compound **(1)** and higher rate than placebo, and (2) the proportion of patients in each treatment arm discontinuing the study drug due to an AE. 95% confidence intervals (CIs) were calculated based upon Wilson score intervals.

Post-hoc analyses were conducted using CFB in the HAMD-17 Anxiety/Somatization (A/S) Subscale and EPDS Anxiety Subscale (EPDS-3A), rates of HAMD-17 A/S and HAM-A Response (≥50% reduction in score), and rate of HAM-A Remission (score ≤ 7). Post-hoc analyses explored concurrent anxiety and depression improvement using a combination of scales at days 15 and 45: **(1)** HAMD-17≤ 7 + HAM-A ≤ 7, or (2) MADRS ≤ 10 + HAM-A ≤ 7. Depression improvement was defined as either an HAMD-17 total score ≤ 7, or a MADRS total score ≤ 10. Improvement in anxiety symptoms was defined as a HAM-A total score ≤ 7. Sustained improvement was defined as meeting concurrent Remission criteria on both days 15 and 45. Adjusted odds ratios (ORs) were calculated using generalized estimating equation models. Scores for the HAM-D insomnia subscale (HAM-D-Ins, which consists of 3 questions rating difficulty falling asleep and staying asleep at 3 time periods during the night), and MADRS individual insomnia item (MADRS-Ins, which consists of a single question about reduced duration or depth of sleep compared with normal), were evaluated in the post-hoc analyses.

### Statistical analysis

CFB in HAMD-17 total score was evaluated using the least squares mean from a mixed-effect model for repeated measures. Concurrent improvement rates were derived using the generalized estimating equation models for repeated measures, adjusting for baseline covariates, while estimates and 95% CIs for ORs were evaluated using Fisher's exact test. Least squares mean ± standard error and adjusted ORs were reported by treatment arm. Secondary and post-hoc end points were not adjusted for multiplicity.

### Results

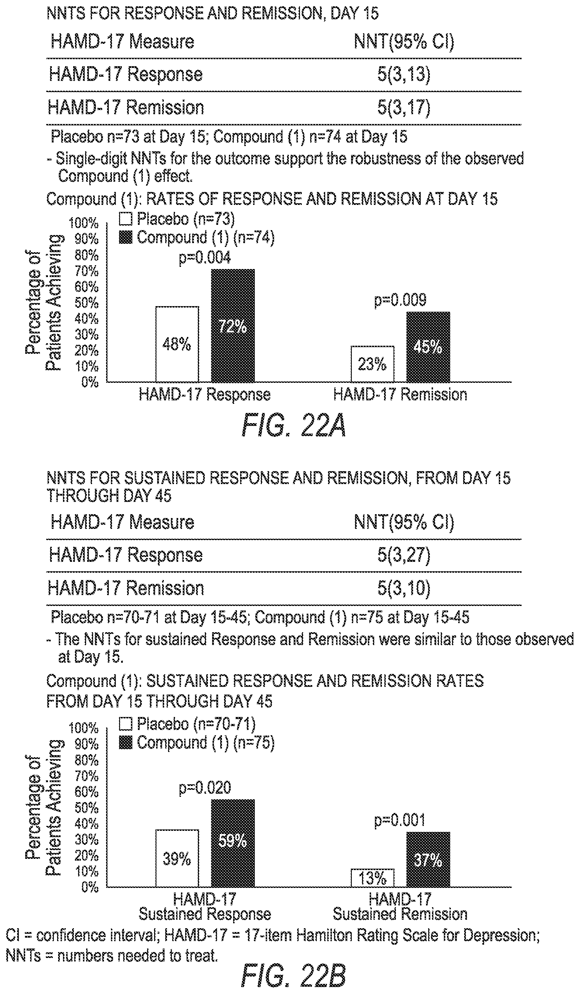

Of 275 women enrolled, 150 were randomized (placebo, n = 74; Compound **(1),** n = 76) and included in the efficacy analyses. Baseline demographics and patient characteristics were well balanced between the 2 treatment arms (Table 15). Compared with the placebo group, a significantly higher proportion of patients in the Compound **(1)** group achieved HAMD-17 Response (72% vs 48%; *P* = .0005) and Remission (45% vs 23%; *P* = .011) at day 15 (FIG. 22A). At day 45, there was a significantly greater rate of sustained HAMD-17 Response (59% vs 39%; *P* = .0200) and Remission (37% vs 13%, *P* =.0006) in the Compound **(1)** group compared with the placebo group (FIG. 22B). NNT for sustained Response and Remission were similar to those observed at day 15 (FIG. 22B). The NNH estimated of Compound **(1)** versus placebo were non-significant at the *P* < .05 threshold (Table 16).

At Day 15, 73.0% of Compound **(1)-**treated patients showed a Response as assessed by MADRS total score, compared to 47.9% in the placebo group. Also at Day 15, 54.1% of Compound **(1)-**treated patients showed Remission as assessed by MADRS total score, compared to 30.1% of patients in the placebo group.

**Table 15. Baseline Patient and Disease Characteristics**

| **Characteristic** | | **Placebo (n=74)** | **Compound (1) (n=76)** |
|---|---|---|---|
| Ethnicity | | | |
| | Hispanic/Latino | 18 (24.3) | 16 (21.1) |
| | Not Hispanic/Latino | 56 (75.7) | 60 (78.9) |
| Race | | | |
| | Black/African-American | 31 (41.9) | 31 (40.8) |
| | White | 40 (54.1) | 44 (57.9) |
| | Other | 3 (4.1) | 1 (1.3) |
| Age, years | | 27.4 (5.3) | 29.3 (5.4) |
| Height, cm | | 162.3 (7.1) | 165.0 (7.4) |
| Weight, kg | | 80.2 (23.6) | 85.1 (19.1) |
| BMI, kg/m² | | 30.3 (8.1) | 31.1 (6.2) |
| Baseline antidepressant use | | 13 (17.6) | 16 (21.1) |
| Baseline HAMD-17 total score | | 28.8 (2.3) | 28.4 (2.1) |
| Baseline MADRS total score | | 36.3 (4.7) | 34.9 (4.4) |
| Antidepressant Use | | | |
| | Baseline | 13 (17.6) | 16 (21.1) |
| Family history of PPD | | | |
| | Yes | 10 (13.5) | 10 (13.2) |
| | No | 64 (86.5) | 66 (86.8) |
| Onset of PPD | | | |
| | 3rdtrimester | 31 (41.9) | 33 (42.1) |
| | Within 4 weeks of delivery | 43 (58.1) | 44 (57.9) |
| Insomnia | | | |
| | HAM-D Sleep Subscale | 5.3 (1.0) | 5.2 (1.1) |
| | MADRS-Reduced Sleep | 4.5 (0.8) | 4.5 (0.7) |

| | | | |
|---|---|---|---|
| BMI = body mass index; SD = standard deviation | | | |

**Table 16. Discontinuation Rates due to AEs and NNH Estimate vs Placebo**

| OUTCOME, N (%) | | **PLACEBO N=73** | **COMPOUND (1) N=78** | **NNH (95% CI) VS. PLACEBO** |
|---|---|---|---|---|
| **Discontinuation due to AE** | | | | |
| | Subjects with an AE leading to treatment discontinuation | 0 | 1 (1.3%) | 78 (ns) |

| **Nervous system disorders** | | | | |
|---|---|---|---|---|
| | Somnolence | 8 (11.0%) | 12 (15.4%) | 23 (ns) |
| | Dizziness | 4 (5.5%) | 6 (7.7%) | 46 (ns) |
| | Sedation | 0 | 4 (5.1%) | 20 (ns) |

| **Gastrointestinal disorders** | | | | |
|---|---|---|---|---|
| | Diarrhoea | 2 (2.7%) | 5 (6.4%) | 28 (ns) |
| | Dry mouth | 0 | 3 (3.8%) | 26 (ns) |

| **Infections and infestations** | | | | |
|---|---|---|---|---|
| | Upper respiratory tract infection | 1 (1.4%) | 6 (7.7%) | 16 (ns) |
| | Dry mouth | 1 (1.4%) | 3 (3.8%) | 41 (ns) |

| **Musculoskeletal and connective tissue disorders** | | | | |
|---|---|---|---|---|
| | Pain in extremity | 1 (1.4%) | \| 2 (2.6%) | \| 84 (ns) |

| **General disorders and administration site conditions** | | | | |
|---|---|---|---|---|
| | Fatigue | 1 (1.4%) | \| 3 (3.8%) | 41 (ns) |

| | | | | |
|---|---|---|---|---|
| AE = adverse event; CI = confidence interval; NNH = number needed to harm; ns = not statistically significant at the *P* < .05 threshold | | | | |

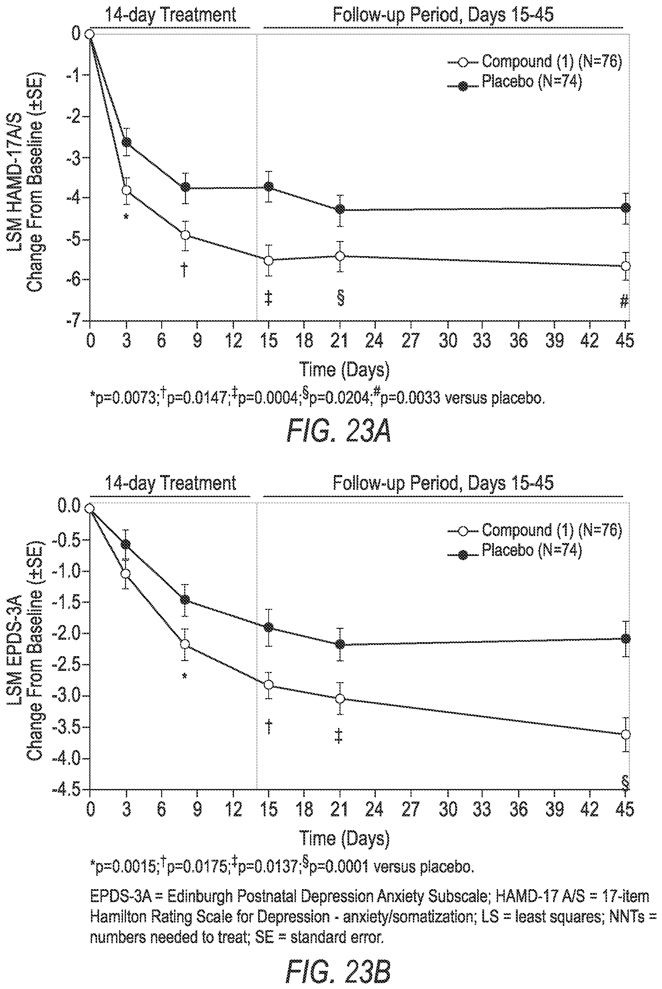

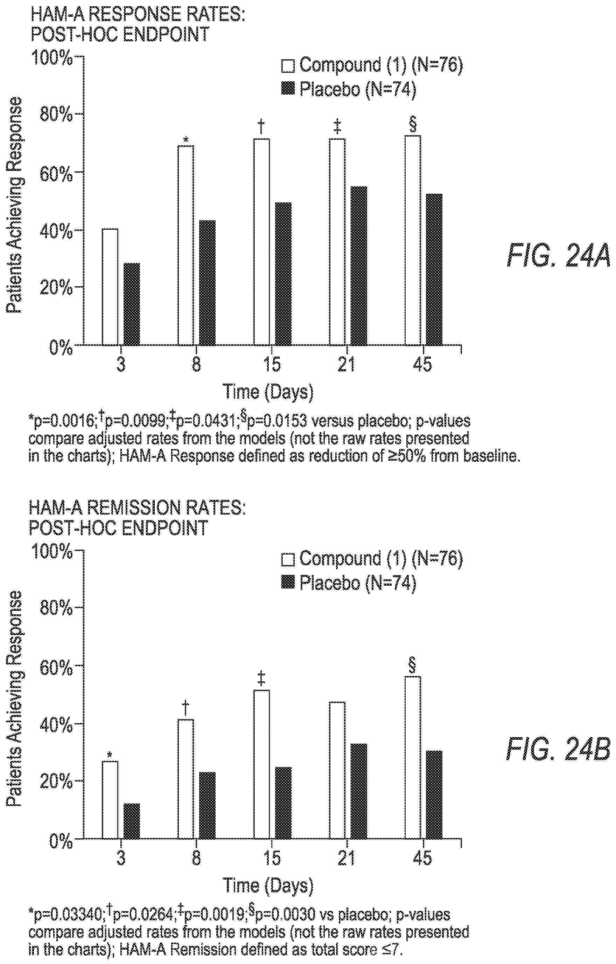

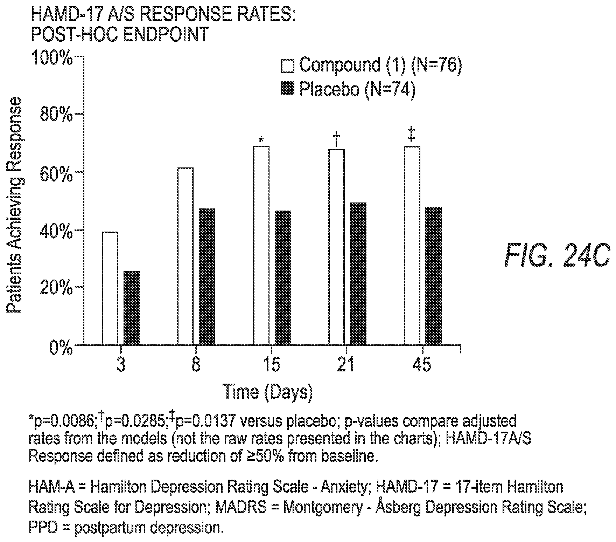

Changes from baseline in the HAMD-17 A/S and EPDS-3A scores are shown in FIG. 23A-23B. Compound **(1)** demonstrated a statistically significant decrease in least squares mean HAMD-17 A/S CFB compared with placebo starting at day 3 (-3.8 vs -2.7; *P* = .0073; FIG. 23A) and was sustained at all measured time points through day 45 (-5.7 vs -4.3; *P =* .0033). Compound **(1)** also demonstrated a statistically significant decrease in least square mean EPDS-3A change from baseline compared with placebo starting at Day 8 (-2.2 versus - 1.5; p=0.0315; FIG. 23B). Statistically significant differences were also sustained at all measured time points from Day 8 through Day 45 (-3.6 vs -2.1; *P* = .0001). Compound **(1)** demonstrated a significantly higher rate of HAM-A Remission compared with placebo at day 3 (27.0% vs 12.2%), HAM-A Response compared with placebo at day 8 (69.3% vs 43.2%), and HAMD-17 A/S Response compared with placebo at day 15 (68.9% vs 46.6%; FIG. 24A-24C).

A significantly higher proportion of Compound **(1)** -treated patients achieved concurrent improvement in remission of anxiety and depressive symptoms (all *P* < .05) as early as day 3, which was sustained at the group level on days 15 and 45 using the HAMD-17 and HAM-A scale combination (model adjusted *P*-values; FIG. 20A-20B; FIG. 21). Similarly, a significantly higher proportion of Compound **(1)** -treated patients achieved concurrent improvement of depressive and anxiety symptoms at day 3 (*P* = .010), day 15 (*P* = .014), and day 45 *(P* = .001) using the MADRS and HAM-A scale combination (model adjusted *P*-values; FIG. 20A-20B). A significantly higher proportion of Compound **(1)-**treated patients achieved sustained concurrent improvement of anxiety and depression at days 15 and 45 using HAMD-17/HAM-A (*P* < .001). Similarly, a significantly higher proportion of Compound **(1)-**treated patients achieved sustained concurrent improvement of anxiety and depression from days 15 to 45 using MADRS/HAM-A (*P* = .003; FIG. 21).

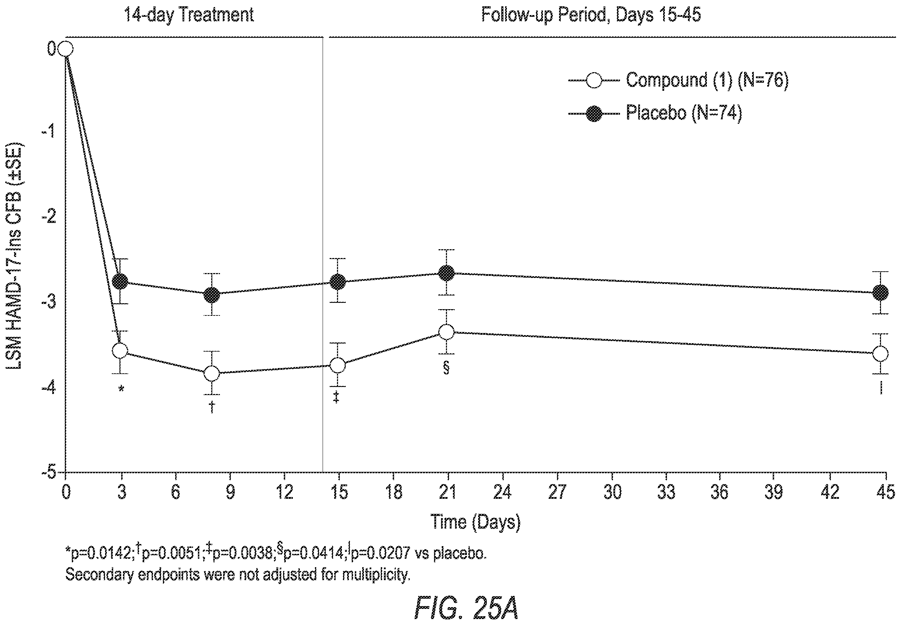

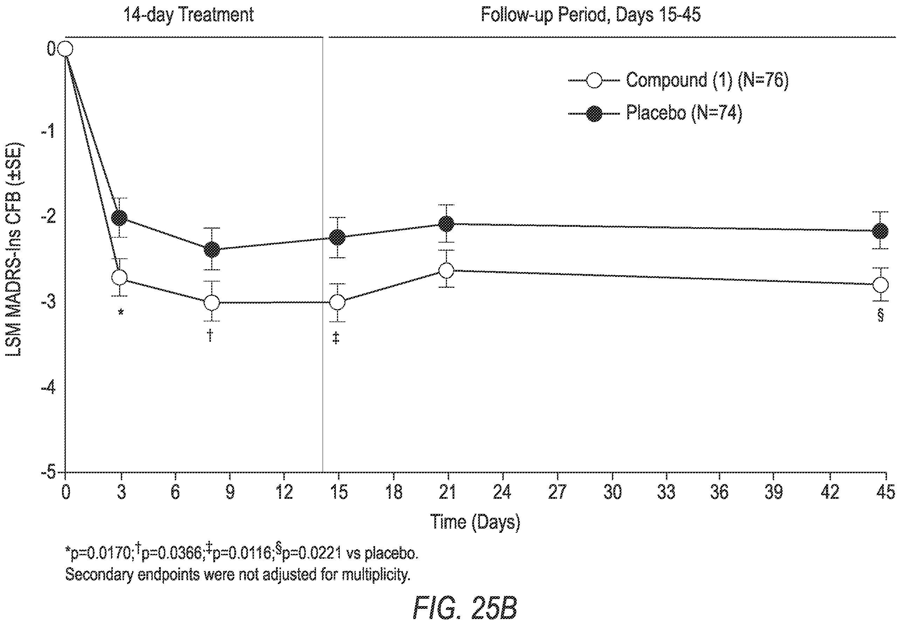

Patients treated with Compound **(1)** achieved significantly greater reductions in HAMD-17-Ins CFB versus placebo starting at day 3 (Compound **(1)-** placebo difference = - 0.841; *P* = .0142) and at all other time points measured during the study, including up to day 45 (30 days after cessation of treatment: *P* = .0207; FIG. 25A). FIG. 25B shows MADRS-Ins scorers CFB versus placebo.

Insomnia was assessed based on HAMD-17 individual items for early insomnia (difficulty falling asleep), middle insomnia (waking during the night and unable to immediately fall back to sleep), and late insomnia (waking during early hours of the morning before completing sleep cycle).

Mean reduction from baseline was numerically greater vs placebo (nominal *P*<0.05*) for: Early insomnia: -1.3 vs -0.9; Middle insomnia: -1.3 vs -0.9; and Late insomnia: -1.3 vs -0.8.

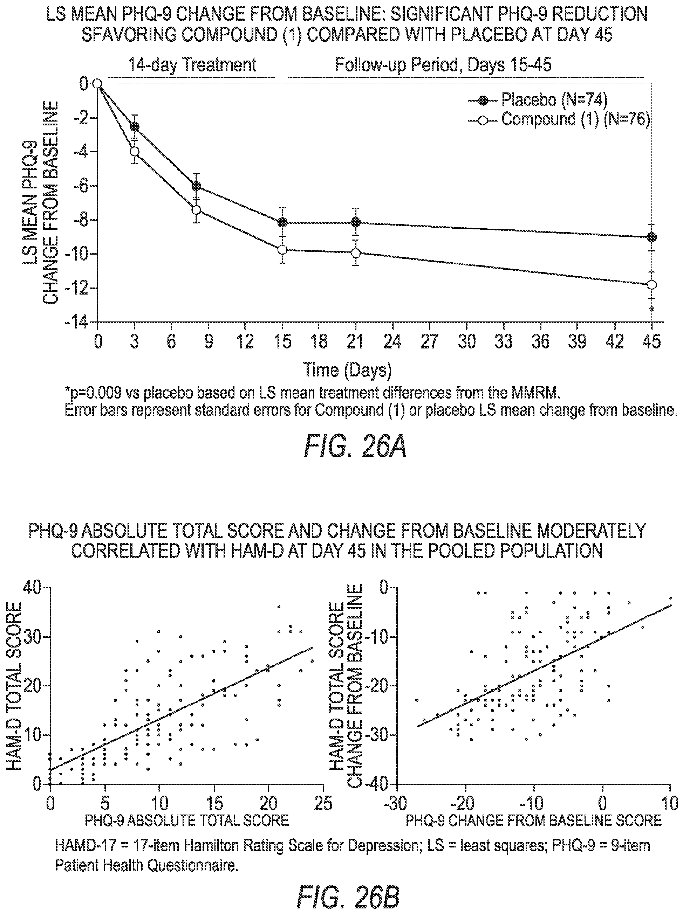

Compound **(1)** demonstrated a statistically significant decrease in least squares mean PHQ-9 depression total score compared with placebo (-11.8 vs -9.0, *P* = .009) at day 45 and numerically greater decrease in PHQ-9 total score at all post-baseline time points (FIG. 26A). HAM-D and PHQ-9 total scores were significantly and moderately (0.5-0.7) correlated at day 45 (r = .70, R2 = .49, *P* < .001; FIG. 26B).

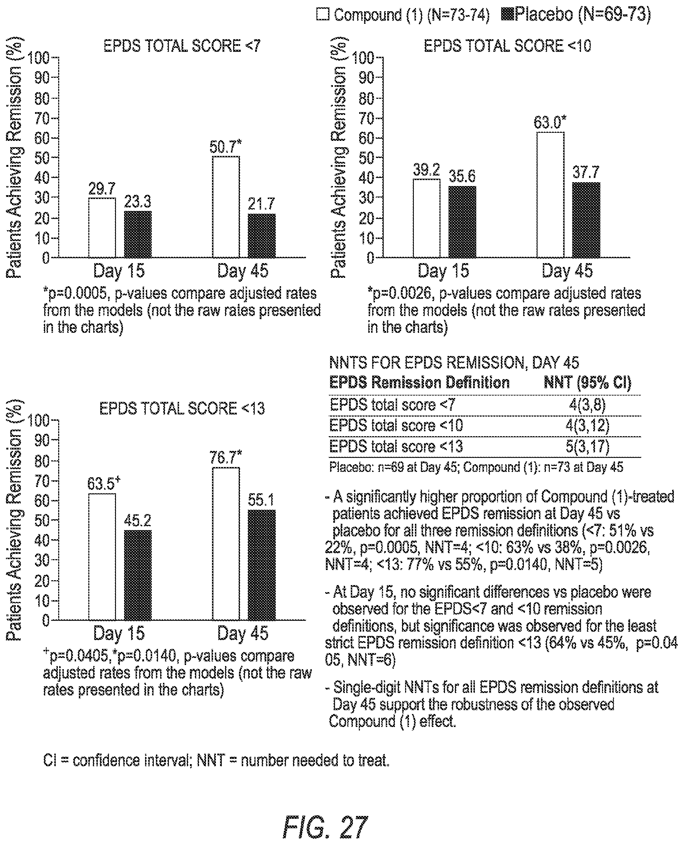

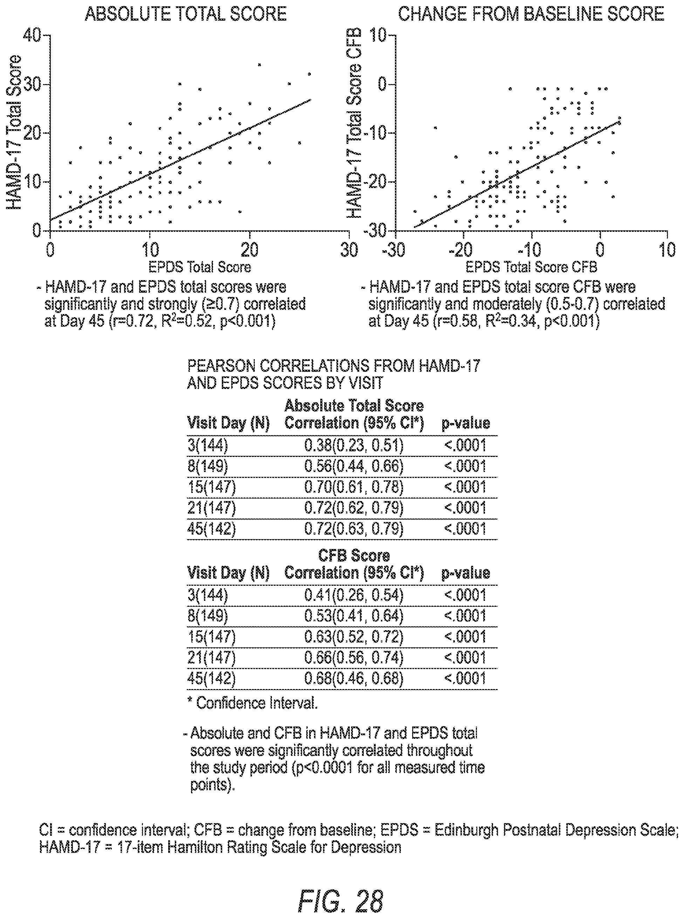

At day 15, there was no significant difference between patients treated with Compound **(1)** versus placebo for the EPDS < 7 and < 10 remission definitions, but significance was observed for the least strict EPDS remission definition < 13 (64% vs 45%, *P* = .0405, NNT = 6; FIG. 27). Single-digit NNTs for all EPDS remission definitions at day 45 support the robust observed Compound **(1)** effect (FIG. 27). Absolute and CFB in HAMD-17 and EPDS total scores were significantly correlated throughout the study period (*P* < .0001 for all measured time points; FIG. 28).

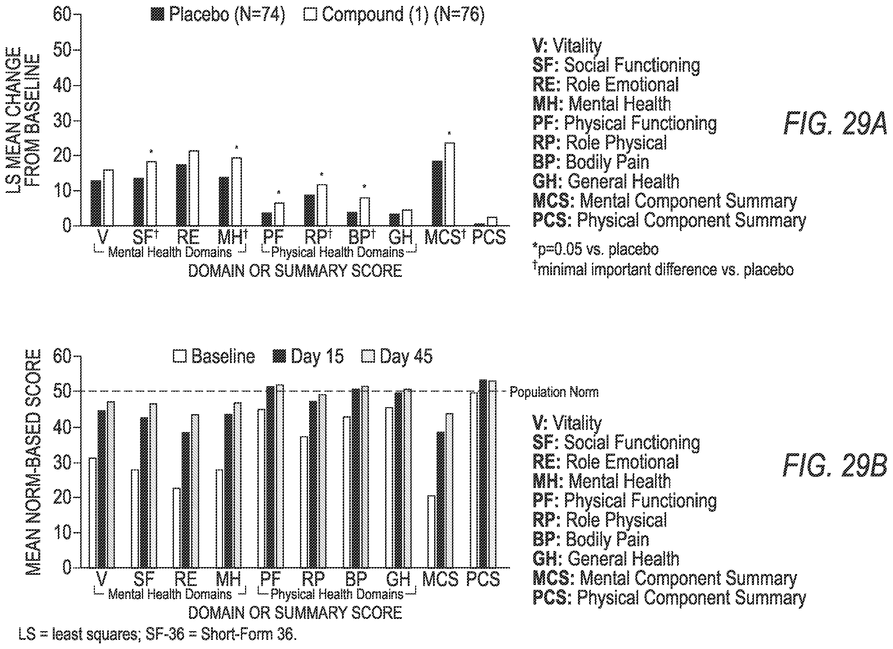

Compound **(1)** demonstrated statistically significant improvements compared with placebo at day 45 in 5 domains of the SF-36v2, including Social Functioning (SF; *P* =.008), Mental Health (MH; *P* =.008), Physical Functioning (PF; *P* = .038), Role Physical (RP; *P =* .0445), Bodily Pain (BP; *P* = .007), and Mental health Component Summary (MCS; *P* = .030) scores (FIG. 29A). Compound **(1)** surpassed the minimal important differences (MIDs) versus placebo at day 45 for the SF, MH, RP, and BP domains and the MCS score. At day 45, Compound **(1)** mean scores exceeded the US population normative levels for 3 domain scores (PF, BP, General Health) and 1 summary score (Physical Component Summary); RP and Vitality domain scores were within 1 MID from population normative levels (FIG. 29B).

In this study, Compound **(1)** was generally well tolerated. No loss of consciousness events or AEs signaling drug discontinuation/withdrawal or worsening of depression were reported. AEs occurring in ≥ 5% of Compound **(1)-**treated patients were somnolence, headache, dizziness, upper respiratory tract infection, diarrhea, and sedation. Overall, the robustness and consistency of responses observed in the clinical trial are noteworthy.

Indeed, across multiple clinical trials conducted by Applicant, Compound (1) has been generally well tolerated, with the most common treatment-emergent AEs (TEAEs; >5% in any Compound (1) arm) were headache, somnolence, dizziness, nausea, upper respiratory tract infection, sedation, fatigue, and diarrhea (Table 17). The majority of patients receiving Compound (1) and experiencing a TEAE reported TEAEs mild and moderate in severity: clinical trial of Example 4 94%, Phase 2 clinical trial conducted by Applicant 100%, previous Phase 3 clinical trial conducted by Applicant 98%,and clinical trial of Example 1 95.0%.

Table 17 shows the most common TEAEs across multiple clinical trials using Compound (1) for PPD or MDD treatment.

**Table 17. Most Common TEAEs (>5% in Compound (1) Arms)**

| | **PPD Treatment** | | **MDD Treatment** | | | | | | **Overall Ranges** | |
|---|---|---|---|---|---|---|---|---|---|---|
| | Clinical Trial of Example 4 | | Phase 2 Clinical Trial | | Phase 3 Clinical Trial | | Clinical Trial of Example 1 | | | |
| Preferred term, n (%) | Placebo (N=73) | Compound **(1)** 30 mg (N = 78) | Placebo (N=44) | Compound **(1)** 30 mg (N = 45) | Placebo (N=190) | Compound **(1)** 30 mg (N = 192) | Placebo (N=269) | Compound **(1)** 50 mg (N = 268) | Placebo (N=576), % | Compound **(1)** 30 or 50 mg (N = 583), % |
| Any TEAE | 38 (52.1) | 47 (60.3) | 20 (45.5) | 24 (53.3) | 93 (48.9) | 105 (54.7) | 120 (44.6) | 161 (60.1) | 44.6-52.1 | 53.3-60.3 |
| Headache | 9 (12.3) | 7 (9.0) | 7 (15.9) | 8 (17.8) | 14 (7.4) | 12 (6.3) | 21 (7.8) | 29 (10.8) | 7.4-15.9 | 6.3-17.8 |
| Somnolence | 8 (11.0) | 12 (15.4) | 1 (2.3) | 3 (6.7) | 8 (4.2) | 13 (6.8) | 8 (3.0) | 41 (15.3) | 2.3-11.0 | 6.7-15.4 |
| Dizziness | 4 (5.5) | 6 (7.7) | 1 (2.3) | 5 (11.1) | 7 (3.7) | 11 (5.7) | 6 (2.2) | 37 (13.8) | 2.2-5.5 | 5.7-13.8 |
| Nausea | 6 (8.2) | 3 (3.8) | 1 (2.3) | 5(11.1) | 9 (4.7) | 7 (3.6) | 12 (4.5) | 11 (4.1) | 2.3-8.2 | 3.6-11.1 |
| Sedation | 0 | 4 (5.1) | 2 (4.5) | 2 (4.4) | 6 (3.2) | 9 (4.7) | 1 (0.4) | 20 (7.5) | 0-4.5 | 4.4-7.5 |
| Fatigue | 1 (1.4) | 3 (3.8) | 0 | 2 (4.4) | 5 (2.6) | 13 (6.8) | 2 (0.7) | 3 (1.1) | 0-2.6 | 1.1-6.8 |
| URTI | 1 (1.4) | 6 (7.7) | 0 | 0 | 4 (2.1) | 6 (3.1) | 2 (0.7) | 3 (1.1) | 0-1.6 | 0-7.7 |
| Diarrhea | 2 (2.7) | 5 (6.4) | 3 (6.8) | 0 | 10 (5.3) | 12 (6.3) | 14 (5.2) | 8 (3.0) | 2.7-6.8 | 0-6.4 |

### Discussion

Results of these post-hoc analyses from the phase 3 ROBIN trial showed that Compound **(1)** was associated with rapid (day 3) and sustained (from day 15-45) reductions in depressive symptoms in women with PPD. These antidepressant effects of Compound **(1),** along with clinical trial findings for brexanolone injection, support the development of NAS GABA_{A}R PAMs as PPD therapies and potential fast-acting therapies to alleviate depressive symptoms. In addition, none of the monoaminergic antidepressants (*e.g.,* selective serotonin reuptake inhibitors, selective norepinephrine reuptake inhibitors or atypical antidepressants) currently have specific indications for PPD. Single-digit NNTs for both rapid (NNT = 5 at day 15) and sustained (NNT = 5 at day 45) outcomes support the potential for Compound **(1)** as a novel pharmacological therapy for the treatment of PPD. In addition to its effects on core symptoms of depression in this trial, further analyses showed that Compound **(1)** treatment also resulted in significantly greater reductions in anxiety symptoms and PROs compared with placebo, suggesting an overall treatment effect. Patients who received Compound **(1)** also demonstrated rapid (day 3) and sustained improvements (day 45) in insomnia-related symptoms compared with placebo. The improvements as measured by clinician-driven measures were mirrored somewhat by patient-self reports but with a delay in timing. The Compound **(1)** group showed improvements in health-related QoL compared with the placebo group at day 45, as assessed by the SF-36v2. Taken together, these results suggest that the rapid and sustained responses observed with Compound **(1)** treatment impact, not only core depressive symptoms, but also other important measures to patients including function and overall well-being.

### Study Limitations

The analyses of secondary and exploratory outcomes were performed post hoc and therefore were not controlled for multiplicity. Results may not be generalizable to patients outside the confines of a clinical trial due to the strict inclusion/exclusion criteria required by this registration study. Furthermore, reasons for clinical trial discontinuation can be complex, so that the NNH for discontinuation due to AEs in this study may not always generalize to overall tolerability in clinical practice. The brief duration of the study limits the sensitivity of calculating NNH for delayed AEs, and the relatively small sample sizes limit sensitivity of calculating NNH for uncommon AEs and sub-population effects. Finally, the NNH results for Compound **(1)** were non-significant at the *P* < .05 threshold as the sample size was small. Estimates for likelihood of help or harm were therefore not calculated for this population.

### Conclusions

The post-hoc analyses results reported here demonstrated that across outcomes, Compound **(1)-**treated patients with PPD experienced robust, sustained, and consistent improvements beginning as early as day 3 and sustained through day 45. In this study, Compound **(1)** treatment provides a range of beneficial effects on core depressive symptoms, as well as improvements in anxiety, insomnia, and other important PROs. Sustained concurrent improvement of depression and anxiety symptoms were seen from days 15 to 45. Taken together with the primary efficacy and safety findings reported previously in the ROBIN study, these findings support the development of NASs for the treatment of core depressive symptoms, as well as anxiety, in patients with PPD.

### References for Example 6

Bauman BL, Ko JY, Cox S, et al. Vital signs: postpartum depressive symptoms and provider discussions about perinatal depression - United States, 2018. MMWR Morb Mortal Wkly Rep. 2020;69(19):575-581.

Hamilton BE, Martin JA, Osterman MJK, et al. National Center for Health Statistics. Births: Provisional data for 2018. Vital Statistics Rapid Release; Report No 7. https://www.cdc.gov/nchs/data/vsrr/vsrr-007-508.pdf. Published May 2019.

DeSisto CL, Kim SY, Sharma AJ. Prevalence estimates of gestational diabetes mellitus in the United States, Pregnancy Risk Assessment Monitoring System (PRAMS), 2007-2010. Prev Chronic Dis. 2014;11:E104.

Centers for Disease Control and Prevention. Diabetes During Pregnancy. https://www.cdc.gov/reproductivehealth/maternalinfanthealth/diabetes-during-pregnancy.htm. Updated June 12, 2018.

Centers for Disease Control and Prevention. Data on Selected Pregnancy Complications in the United States. https://www.cdc.gov/reproductivehealth/maternalinfanthealth/pregnancy-complications-data.htm. Updated February 28, 2019.

Executive summary: hypertension in pregnancy. American College of Obstetricians and Gynecology. Obstet Gynecol. 2013;122(5):1122-1131.

Callaghan WM, Kuklina EV, Berg CJ. Trends in postpartum hemorrhage: United States, 1994-2006. Am J Obstet Gynecol. 2010;202(4):353.e1-e6.

ACOG Committee Opinion No. 757: Screening for perinatal depression. Obstet Gynecol. 2018;132(5):e208-e212.

Stewart DE, Robertson E, Dennis CL, et al. Postpartum depression: literature review of risk factors and interventions. University Health Network Women's Health Program; 2003. https://www.who.int/mental_health/prevention/suicide/lit_review_postpartum_depression.pdf

American Psychiatric Association. Diagnostic and Statistical Manual of Mental Disorders (DSM-5). 5th ed. American Psychiatric Association; 2013.

National Institutes of Mental Health. Perinatal depression. https: //www.nimh.nih.gov/health/publications/perinatal-depression/index.shtml.

Kerstis B, Aarts C, Tillman C, et al. Association between parental depressive symptoms and impaired bonding with the infant. Arch Womens Ment Health. 2016;19(1):87-94.

Posmontier B. Functional status outcomes in mothers with and without postpartum depression. J Midwifery Womens Health. 2008;53(4):310-318.

Barkin JL, Wisner KL, Bromberger JT, et al. Factors associated with postpartum maternal functioning in women with positive screens for depression. J Womens Health (Larchmt). 2016;25(7):707-713.

Gagliardi L, Petrozzi A, Rusconi F. Symptoms of maternal depression immediately after delivery predict unsuccessful breast feeding. Arch Dis Child. 2012;97(4):355-357.

McLearn KT, Minkovitz CS, Strobino DM, et al. Maternal depressive symptoms at 2 to 4 months post partum and early parenting practices. Arch Pediatr Adolesc Med. 2006;160(3):279-284.

Moore Simas TA, Huang MY, Packnett ER, et al. Matched cohort study of healthcare resource utilization and costs in young children of mothers with postpartum depression in the United States. J Med Econ. 2020;23(2):174-183.

Balbierz A, Bodnar-Deren S, Wang JJ, et al. Maternal depressive symptoms and parenting practices 3-months postpartum. Matern Child Health J. 2015;19(6):1212-1219.

Valla L, Wentzel-Larsen T, Smith L, et al. Association between maternal postnatal depressive symptoms and infants' communication skills: A longitudinal study. Infant Behav Dev. 2016;45(Pt A):83-90.

Koutra K, Chatzi L, Bagkeris M, et al. Antenatal and postnatal maternal mental health as determinants of infant neurodevelopment at 18 months of age in a mother-child cohort (Rhea Study) in Crete, Greece. Soc Psychiatry Psychiatr Epidemiol. 2013;48(8):1335-1345.

Robertson E, Grace S, Wallington T, et al. Antenatal risk factors for postpartum depression: a synthesis of recent literature. Gen Hosp Psychiatry. 2004;26(4):289-295.

Biaggi A, Conroy S, Pawlby S, et al. Identifying the women at risk of antenatal anxiety and depression: A systematic review. J Affect Disord. 2016;191:62-77.

Yim IS, Tanner Stapleton LR, Guardino CM, et al. Biological and psychosocial predictors of postpartum depression: systematic review and call for integration. Annu Rev Clin Psychol. 2015;11:99-137.

Payne JL, Maguire J. Pathophysiological mechanisms implicated in postpartum depression. Front Neuroendocrinol. 2019;52:165-180.

Duan C, Cosgrove J, Deligiannidis KM. Understanding peripartum depression through neuroimaging: a review of structural and functional connectivity and molecular imaging research. Curr Psychiatry Rep. 2017;19(10):70.

Maguire J. Neuroactive steroids and GABAergic involvement in the neuroendocrine dysfunction associated with major depressive disorder and postpartum depression. Front Cell Neurosci. 2019;13:83.

Osborne LM, Gispen F, Sanyal A, et al. Lower allopregnanolone during pregnancy predicts postpartum depression: an exploratory study. Psychoneuroendocrinology. 2017;79:116-121.

Luisi S, Petraglia F, Benedetto C, et al. Serum allopregnanolone levels in pregnant women: changes during pregnancy, at delivery, and in hypertensive patients. J Clin Endocrinol Metab. 2000;85(7):2429-2433.

Nappi RE, Petraglia F, Luisi S, et al. Serum allopregnanolone in women with postpartum "blues". Obstet Gynecol. 2001;97(1):77-80.

Deligiannidis KM, Kroll-Desrosiers AR, Tan Y, et al. Longitudinal proneuroactive and neuroactive steroid profiles in medication-free women with, without and at-risk for perinatal depression: A liquid chromatography-tandem mass spectrometry analysis. Psychoneuroendocrinology. 2020;121:104827.

Deligiannidis KM, Fales CL, Kroll-Desrosiers AR, et al. Resting-state functional connectivity, cortical GABA, and neuroactive steroids in peripartum and peripartum depressed women: a functional magnetic resonance imaging and spectroscopy study. Neuropsychopharmacology. 2019;44(3):546-554.

Hoffmann E, Nomikos GG, Kaul I, et al. SAGE-217, A Novel GABAA Receptor Positive Allosteric Modulator: Clinical Pharmacology and Tolerability in Randomized Phase I Dose-Finding Studies. Clin Pharmacokinet. 2020; 59: 111-120.

Martinez Botella G, Salituro FG, Harrison BL, et al. Neuroactive steroids. 2. 3α-hydroxy-3β-methyl-21-(4-cyano-1H-pyrazol-1'-yl)-19-nor-5β-pregnan-20-one (SAGE-217): a clinical next generation neuroactive steroid positive allosteric modulator of the (y-aminobutyric acid)A receptor. J Med Chem. 2017;60(18):7810-7819.

Althaus AL, Ackley MA, Belfort GM, et al. Preclinical characterization of Compound (1) (SAGE-217), a selective neuroactive steroid GABAA receptor positive allosteric modulator. Neuropharmacology. 2020;181:108333.

Deligiannidis KM, Meltzer-Brody S, Gunduz-Bruce H, et al. Effect of Zuranolone vs Placebo in Postpartum Depression: A Randomized Clinical Trial. JAMA Psychiatry. 2021;78(9):951-959.

Hamilton M. A rating scale for depression. J Neurol Neurosurg Psychiatry. 1960;23(1):56-62.

Hamilton M. The assessment of anxiety states by rating. Br J Med Psychol. 1959;32(1):50-55.

Montgomery SA, Åsberg M. A new depression scale designed to be sensitive to change. Br J Psychiatry. 1979;134:382-389.

Posner K, Brown GK, Stanley B, et al. The Columbia-Suicide Severity Rating Scale: initial validity and internal consistency findings from three multisite studies with adolescents and adults. Am J Psychiatry. 2011;168(12):1266-1277.

Kroenke K, Spitzer RL, Williams JB. The PHQ-9: validity of a brief depression severity measure. J Gen Intern Med. 2001; 16(9):606-613.

Cox JL, Holden JM, Sagovsky R. Detection of postnatal depression. Development of the 10-item Edinburgh Postnatal Depression Scale. Br J Psychiatry. 1987;150:782-786.

Citrome L, Ketter TA. When does a difference make a difference? Interpretation of number needed to treat, number needed to harm, and likelihood to be helped or harmed. Int J Clin Pract. 2013;67(5):407-411.

Citrome L. Compelling or irrelevant? Using number needed to treat can help decide. Acta Psychiatr Scand. 2008;117(6):412-419.

Citrome L. Relative vs. absolute measures of benefit and risk: what's the difference? Acta Psychiatr Scand. 2010;121(2):94-102.

Meltzer-Brody S, Colquhoun H, Riesenberg R, et al. Brexanolone injection in post-partum depression: two multicentre, double-blind, randomised, placebo-controlled, phase 3 trials. Lancet. 2018;392(10152):1058-1070.

Kanes S, Colquhoun H, Gunduz-Bruce H, et al. Brexanolone (SAGE-547 injection) in post-partum depression: a randomised controlled trial. Lancet. 2017;390(10093):480-489.

Barkin JL, Wisner KL, Bromberger JT, et al. Development of the Barkin Index of Maternal Functioning. J Womens Health (Larchmt). 2010;19(12):2239-2246.

### Example 7. Abuse Potential of Oral Compound (1) in Nondependent, Recreational Users of Central Nervous System Depressants: A Randomized, Double-Blind, Placebo-Controlled, Crossover Study

### Introduction

Major depressive disorder (MDD) is characterized by symptoms of depressed mood and/or loss of interest in pleasurable activities that causes clinically significant distress or impairment in social, occupational, or other important areas of functioning (Diagnostic and Statistical Manual of Mental Disorders, 5th ed. American Psychiatric Association). For the acute phase of treatment for adults, the American Psychiatric Association recommends pharmacotherapy and/or depression-focused psychotherapy, a combination of medications and psychotherapy, or other somatic therapies (Association AP. Practice guideline for the treatment of patients with major depressive disorder (3rd)). The decision of which pharmacotherapy treatment to use is typically based on factors including anticipated common adverse effects, tolerability, and pharmacological properties.

In the STAR*D study, adults with MDD had a remission rate of approximately 37% with their first-line, standard-of-care antidepressant treatment, and even lower response rates for second- (31%), third- (14%), and fourth-line (13%) treatments (Rush AJ, et al. Am J Psych. 2006;163:1905-1917). Antidepressant therapies often involve a delay between treatment initiation and clinically meaningful therapeutic effects; this may account for some of decreased response rates (Carboni E, et al. Front Neurosci. 2021;15). Furthermore, although antidepressants are generally thought to have low abuse liability, a study of the European Monitoring Agency Adverse Drug Reactions database found that 10.8% of patients receiving bupropion misused or abused the drug or demonstrated dependence (Schifano F, Chiappini S. Front Pharmacol. 2018;9). Overall, the antidepressants currently used as standard-of-care are limited by a suboptimal response rate, delayed treatment effects, and the possible risk of misuse, abuse, or dependence in specific patient populations.

To address the need for antidepressants with improved response rates and a faster onset of action without concerns for possible abuse, newer compounds with novel mechanisms of action are being developed (Singh JB, et al. Biol Psychiatry. 2016;80:424-431). For example, intranasal esketamine is a fast-acting drug approved by the US Food and Drug Administration (FDA) to treat adults with treatment-resistant depression or adults with MDD having acute suicidal ideation or behavior when taken in conjunction with an oral antidepressant (Cristea IA, Naudet F. Lancet Psychiatry. 2019;6:975-977; Fu D-J, et al. J Clin Psychiatry. 2020;81(3):19m13191; Ionescu DF, et al. Int J Neuropsychopharmacol. 2020;24(1):22-31; SPRAVATO^{®} [prescribing Information]. Titusville, N.J., Janssen Pharmaceuticals, Inc. July 2020). Esketamine demonstrated rapid antidepressant effects; however, it is associated with transient dissociative and psychotic symptoms. Therefore, although esketamine offers promise as a faster-acting treatment for MDD, its potential association with transient dissociation and psychotic symptoms and the need for concomitant oral antidepressant therapy may limit its widespread use.

Positive allosteric modulators of the γ-aminobutyric acid-gated chloride channel receptor (GABAAR) are potential novel treatments for depression and postpartum depression (PPD) (Hoffmann E, et al. Clin Pharmacokinet. 2020;59:111-120; Meltzer-Brody S, et al. The Lancet. 2018;392(10152):1058-1070; Food and Drug Administration. FDA approves first treatment for post-partum depression. Mar 19, 2019). Compound **(1)** is a neuroactive steroid GABAAR positive allosteric modulator (PAM) (Althaus AL, et al. Neuropharmacology. 2020;181; Martinez Botella G, et al. J Med Chem. 2017;60:7810-7819) currently under investigation as a once-daily, 2-week oral therapy for MDD and postpartum depression (PPD). Unlike benzodiazepines, Compound **(1)** targets both synaptic and extrasynaptic GABAARs, resulting in sustained potentiation of tonic and phasic postsynaptic currents, respectively (Hoffmann E, et al. Clin Pharmacokinet. 2020;59:111-120; Althaus AL, et al. Neuropharmacology. 2020;181; Martinez Botella G, et al. J Med Chem. 2017;60:7810-7819; Nicholson MW, et al. Mol Psychiatry. 2018;23:1851-1867; Deligiannidis KM, et al. JAMA Psychiatry. 2021;78:951-959). This provides the patient both with a rapid and sustained response. Brexanolone, a GABAAR modulator, is approved for the treatment of PPD (Meltzer-Brody S, et al. The Lancet. 2018;392(10152):1058-1070; Food and Drug Administration. FDA approves first treatment for post-partum depression. Mar 19, 2019; Kanes S, et al. Lancet. Jul 29 2017;390(10093):480-489). Patients receiving brexanolone are monitored for excessive sedation or loss of consciousness, which may be a class effect (Food and Drug Administration. FDA approves first treatment for post-partum depression. Mar 19, 2019; Morrison KE, et al. Drugs of today (Barcelona, Spain: 1998). 2019;55(9):537).

Treatment with Compound **(1)** has demonstrated in multiple Phase 2 and 3 clinical trials a rapid (as early as Day 3) and consistent improvement in depressive symptoms in patients with moderate to severe MDD or PPD; three out of four completed double-blind placebo-controlled trials met their primary endpoint of significant change from baseline in Hamilton Depression Rating Scale (HAMD)-17 total score at Day 15 (Deligiannidis KM, et al. JAMA Psychiatry. 2021;78:951-959; Gunduz-Bruce H, et al. N Engl J Med. 2019;381(10):903-911; Mittal A, et al. Poster presented at: American Academy of Neurology Annual Meeting; June 20, 2014; Toronto, Canada; Clayton A. Oral presentation presented at: ECNP Annual Meeting; October 2-5, 2021; Lisbon, Portugal) Compound **(1)** was generally well tolerated, with a consistent safety profile; the adverse events reported in ≥5% of patients in the Compound **(1)** arm across studies were headache, somnolence, dizziness, nausea, upper respiratory infection, sedation, fatigue, and diarrhea (*Id.*)*.* In a Phase 3 study in PPD, Compound **(1)** demonstrated efficacy and was generally well tolerated (Deligiannidis KM, et al. JAMA Psychiatry. 2021;78:951-959). In a Phase 1 dose-finding study in healthy participants, Compound **(1)** was generally well tolerated (Hoffmann E, et al. Clin Pharmacokinet. 2020;59:111-120). A Phase 2 study in adults with MDD receiving Compound **(1)** demonstrated a significant reduction in depressive symptoms at Day 15 compared with patients taking placebo (P < 0.001) (Gunduz-Bruce H, et al. N Engl J Med. 2019;381(10):903-911). The pivotal Phase 3 WATERFALL (Examples 1-3 of the present application) study demonstrated results consistent with previous Compound **(1)** studies, including rapid onset of improvement in depressive symptoms and was generally well tolerated.

Considering that GABAA PAMs represent a potential for abuse or misuse and that most drugs acting within the central nervous system (CNS) require assessment for human abuse potential, additional studies are needed to assess Compound **(1)**'s potential for abuse and misuse (Arnaud A, et al. J Affect Disord. 2021;285:112-119; U.S. Department of Health and Human Services Food and Drug Administration, Center for Drug Evaluation and Research (CDER). Assessment of abuse potential of drugs: guidance for industry. January, 2017).

### Clinical Study

### Study Population

Healthy adults aged 18-55 years, weighing ≥50.0 kg with a body mass index of 18.0-34.0 kg/m2 were eligible for the study. Participants were required to be current recreational users of CNS depressants (defined as using CNS depressants for nontherapeutic reasons ≥10 times over their lifetime, including at least once in the 12 weeks prior to screening). Exclusion criteria included a history of substance or alcohol dependence within the past 2 years and/or any admission to a drug or alcohol rehabilitation program. The full list of inclusion and exclusion criteria is shown below.

### Inclusion Criteria

Participants had to provide a valid form of photo identification and also provide written informed consent prior to the initiation of any protocol-specific procedures, as well as be able to communicate sufficiently to allow completion of all study assessments. Female participants had to agree to use a highly effective method of contraception during participation and for 30 days following the last dose of Compound **(1),** unless the participant was postmenopausal or not at risk of becoming pregnant. Male participants had agreed to use an acceptable method of effective contraception for the duration of the study and for 5 days after receiving the last dose of Compound **(1),** unless they were not sexually active; additionally they had to be willing to abstain from sperm donation for the duration of the study and for 5 days after receiving the last dose of Compound **(1).**

### Exclusion Criteria

Participants who were heavy smokers (>20 cigarettes per day) and/or were unable to abstain from smoking or the use of prohibited nicotine-containing products for ≥1 hour before and 6 hours after Compound **(1)** administration were excluded. In addition, participants were not allowed to have used nicotine replacement therapy (any formulation) or varenicline therapy within 1 month prior to screening. Participants who donated blood or experienced acute loss of blood (>500 mL) within 60 days prior to screening were not eligible. Exposure to an investigational drug or device within the 30 days, or 5 half-lives (if known), whichever was longer, prior to screening was also an exclusion criteria. Previous exposure to Compound **(1)** or a history of allergy or hypersensitivity (including rash) to Compound **(1)** or sedative/hypnotic drugs, or formulation excipients excluded participants. Investigative site personnel or in their immediate families were not eligible. Participants were not allowed to have used a prohibited medication/supplement or recreational drug or have consumed alcohol or prohibited food(s) or to have regularly consumed excessive amounts of caffeine, defined as greater than 6 servings per day.

Additional exclusion criteria included a significant history and/or presence of specific conditions such as hepatic, renal, cardiovascular, pulmonary, neurological, psychiatric, gastrointestinal, hematological, immunologic, ophthalmologic, metabolic, or oncological disease. A history of clinically significant gastrointestinal disease and/or surgery that might impact drug absorption or metabolism (*e.g.,* gastrectomy, and/or gastric bypass) was also an exclusion criteria. In addition, participants with any clinically significant abnormal physical examination finding or clinically significant abnormal values for hematology, clinical chemistry, or uranalysis or on a 12-lead electrocardiogram (ECG) were also excluded. The corrected QT interval (QTcF) ≥450 msec in male participants or ≥470 msec in female participants was exclusionary. In addition, participants were excluded who had a history, presence, and/or current evidence of positive serological results for hepatitis B surface antigen, hepatitis C antibodies, or HIV antibodies 1 or 2. Participants could not have had a history of suicidal behavior within 2 years of screening or have answered yes to questions 3, 4, or 5 on the Columbia-Suicide Severity Rating Scale at screening or at any admission or be currently at risk of suicide.

### Study Design

The study was conducted in accordance with the principles of the Declaration of Helsinki and the International Conference on Harmonisation for Good Clinical Practice. The institutional review board of the participating study center approved the study and all participants provided written informed consent prior to the screening assessments.

### Dose Selection

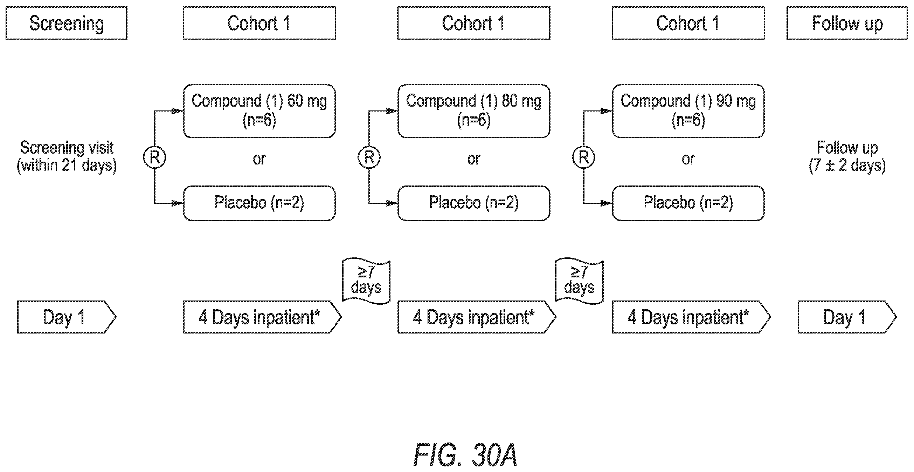

The first part of the study, dose selection, was a 3-cohort, randomized, double-blind, placebo-controlled, dose-escalation design to identify 2 doses higher than Compound **(1)**'s anticipated therapeutic dose (30 mg) to carry forward into the main study (FIG. 30A-30B) (U.S. Department of Health and Human Services Food and Drug Administration, Center for Drug Evaluation and Research (CDER). Assessment of abuse potential of drugs: guidance for industry. January, 2017; Griffiths RR, et al. Durg Alcohol Depend. 2003;70(3):S41-54; Schoedel K, Sellers E. Clin Pharmacol Ther. 2008;83(4):622-626). The 2 doses were selected based on their safety and tolerability in the dose selection phase, as assessed during sentinel dosing (described below) by the safety and tolerability endpoints (described below).

After a screening period of up to 21 days, eligible participants were randomized 3:1 to receive single escalating doses of Compound **(1)** (60, 80, or 90 mg) or matching placebo in 3 separate cohorts; Compound **(1)** was administered during 4 days of inpatient administration (FIG. 30A). Sentinel dosing was employed for each cohort, with one participant randomized to receive Compound **(1)** and one to receive placebo on Day 1. The remaining 6 participants (Compound **(1),** 5; placebo, 1) were dosed approximately 24 hours later after monitoring the safety and tolerability endpoints. Dose-escalation cohorts were separated by at least 7 days. If a dose was not well tolerated based on the safety data, doses in subsequent cohorts were to be modified, and Compound **(1)** dose was not allowed to exceed 90 mg. Participants underwent a 1-day, follow-up visit 7 (± 2) days after the end of the treatment and were not permitted to participate in the main study.

### Main Study

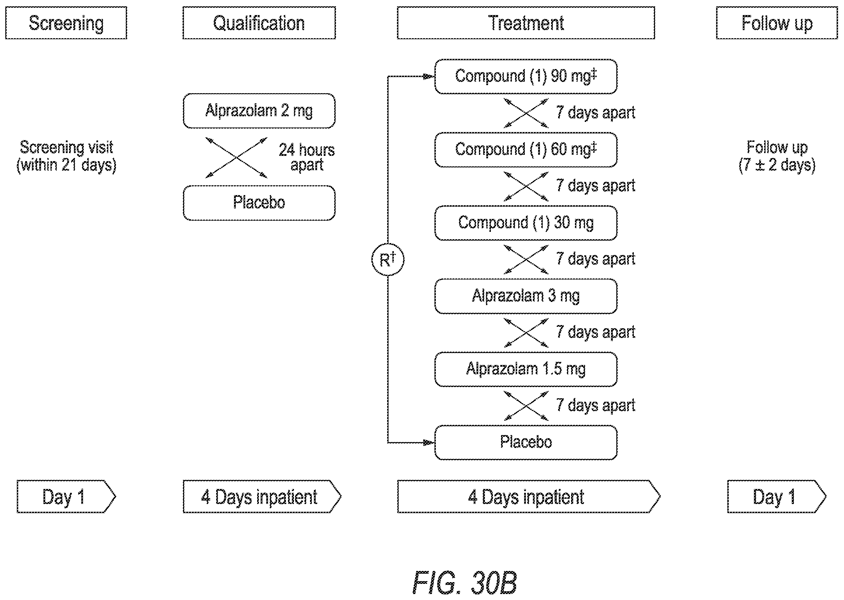

The main study consisted of 4 phases (FIG. 30B). The screening phase was a 1-day visit occurring within 21 days of the beginning of the qualification phase. The qualification phase was a 4-day, inpatient period to ensure participants could discriminate between alprazolam (active comparator) and placebo, and that they could tolerate the alprazolam 2-mg doses. Participants received single-oral doses of alprazolam 2 mg and placebo in a crossover manner 24 hours apart, and if they could discriminate between the two medications, they continued to the treatment phase. The treatment phase was a double-blind, active- and placebo-controlled, 6-way crossover design where participants were randomized equally to a single oral dose of 1 of 6 treatments: placebo, alprazolam (1.5 and 3 mg), and Compound **(1)** (30, 60, and 90 mg). The Compound **(1)** doses were based on the results from the first part, dose selection, and treatments were separated by 7 days. The follow-up phase was a 1-day visit occurring 7 (± 2) days after the treatment phase.

### Endpoints

For the first part, dose selection, endpoints included the observer-rated Modified Observer's Assessment of Alertness/Sedation (MOAA/S) scores along with safety and tolerability endpoints which included the Columbia-Suicide Severity Rating Scale (C-SSRS), frequency and severity of adverse events (AE) and serious adverse events (SAEs), and changes from baseline in clinical laboratory assessments, vital signs, and electrocardiogram (ECG).

For the main study, the primary pharmacodynamic (PD) endpoint was the maximum effect (Emax) for Drug Liking (at this moment) as assessed by a bipolar visual analog scale (VAS). Drug Liking was assessed using the question "At this moment, my liking for this drug is" where values could range from 0 (strong disliking) to 100 (strong liking), with a score of 50 being neutral.

The key secondary PD endpoints were Overall Drug Liking and Take Drug Again VAS Emax, which were both also assessed using similar bipolar scales ranging from 0 (strong disliking or definitely not, respectively) to 100 (strong liking or definitely so, respectively), and a score of 50 being neutral.

Other secondary endpoints included Emax and minimum effect (Emin) for High Effects, Good Drug Effects, Bad Drug Effects, Alert/Drowsiness, MOAA/S, Any Effect, Drug Similarity, and cognition (Reaction Time and Movement Time). Drug Similarity was measured as median values for Placebo Similarity, Benzodiazepines Similarity, and Opioids Similarity, and was assessed using the question "How similar is the drug you most recently received to [drug name]?" Example drugs were given for comparisons to benzodiazepines (ie, Ativan, Halcion, Klonopin, Librium, Valium, and Xanax) and opioids (ie, opium, morphine, hydrocodone, oxycodone, hydromorphone, oxymorphone, fentanyl, buprenorphine, meperidine, and tramadol); values ranged from 0 (not at all similar) to 100 (very similar).

Pharmacokinetic endpoints included the maximum observed plasma concentration of drug (Cmax), the time to Cmax (Tmax), and the area under the plasma concentration vs time curve from time 0-24 hours after dose administration (AUC0-24). Safety and tolerability endpoints included the frequency and severity of AEs and SAEs, the change from baseline in clinical laboratory assessments, vital signs, and ECG, and suicidality assessed by the Columbia-Suicide Severity Rating Scale (C-SSRS).

### Assessments

MOAA/S scores were assessed before dose administrations and at multiple time points (1, 2, 3, 4, 5, 6, 7, 8, 10, 12, and 24 hours) after dosing using a 6-point scale, ranging from 0 (no response after painful trapezius squeeze) to 5 (responds readily to name spoken in a normal tone; Table 18). Electrocardiogram measures and the C-SSRS were assessed during screening, admission, at Day 3, and/or at early termination, and AEs/SAEs were assessed on an ongoing basis during enrollment in the study. During the dose selection phase, vital signs were assessed at screening, admission, before dose administrations, and at multiple time points up to 24 hours after dosing (Table 19).

**Table 18. Pharmacodynamic Endpoints and Assessments During the Dose Selection and the Main Study's Qualification and Treatment Phases**

| **Study phase and measurements** | | **Scale polarity, range (neutral value)** | **Question** | **Response anchors** |
|---|---|---|---|---|
| **Primary endpoints** | | | | |
| | Drug Liking | Bipolar, 0-100 (50) | At this moment, my liking for this drug is | 0: Strong disliking |
| | | | | 50: Neither like nor dislike |
| | | | | 100: Strong liking |

| **Key secondary endpoints** | | | | |
|---|---|---|---|---|
| | Overall Drug Liking | Bipolar, 0-100 (50) | Overall, my liking for this drug is | 0: Strong disliking |
| | | | | 50: Neither like nor dislike |
| | | | | 100: Strong liking |
| | Take Drug Again | Bipolar, 0-100 (50) | I would take this drug again | 0: Definitely not |
| | | | | 50: Neutral |
| | | | | 100: Definitely so |

| **Secondary endpoints** | | | | |
|---|---|---|---|---|
| Alertness/Drowsiness VAS | | Bipolar, 0-100 (50) | At this moment, my mental state is | 0: Very drowsy |
| | | | | 50: Neither drowsy nor alert |
| | | | | 100: Very alert |
| | Any Drug Effects | Unipolar, 0-100 (NA) | At this moment, I feel any drug effect | 0: Not at all |
| | | | | 100: Extremely |
| | Bad Drug Effects | Unipolar, 0-100 (NA) | At this moment, I feel bad drug effects | 0: Not at all |
| | | | | 100: Extremely |
| | Good Drug Effects | Unipolar, 0-100 (NA) | At this moment, I feel good drug effects | 0: Not at all |
| | | | | 100: Extremely |
| | High | Unipolar, 0-100 (NA) | At this moment, I feel high | 0: Not at all |
| | | | | 100: Extremely |
| | Drug Similarity | Unipolar, 0-100 (NA) | How similar is the drug you most recently received to [drug name] | 0: Not at all similar |
| | | | | 100: Very similar |
| MOAA/S | | 0 to 5 | | 0: no response after painful trapezius squeeze |
| | | | | 1: responds only after painful trapezius squeeze |
| | | | | 2: responds only after mild prodding |
| | | | | 3: responds only after name is called loudly and/or repeatedly |
| | | | | 4: lethargic response to name spoken in normal tone |
| | | | | 5: responds readily to name spoken in normal tone |

| | | | | |
|---|---|---|---|---|
| MOAA/S, Modified Observer's Assessment of Alertness/Sedation; NA, not applicable; VAS, visual analog scale. | | | | |

**Table 19. Schedule of Assessments During the Dose Selection and the Main Study's Qualification and Treatment Phases**

| **Study phase and measurements** | | **Assessment timepoints** |
|---|---|---|
| **Dose selection phase, key safety measurements** | | |
| | Vital signs | Screening, admission, and predose and 1, 2, 3, 4, 5, 6, 7, 8, 10, 12, and 24 hours postdose |
| | MOAA/S | Predose, and 1, 2, 3, 4, 5, 6, 7, 8, 10, 12, and 24 hours postdose |
| | ECG | Screening, admission, and Day 3 and/or early termination |
| | C-SSRS | Screening, admission, and Day 3 and/or early termination |
| | AEs/SAEs | Ongoing from the time of consent throughout study participation |

| **Main study, qualification phase** | | |
|---|---|---|
| Drug Liking, Good Drug Effects, Bad Effects, and Any Drug Effects | | 0.5, 1, 1.5, 2, 3, 4, 6, 8, and 23 hours postdose |
| Take Drug Again and Overall Drug Liking | | 8 and 23 hours postdose |
| | High Effects and Alertness/Drowsiness | Predose and 0.5, 1, 1.5, 2, 3, 4, 6, 8, and 23 hours postdose |
| | Vital signs | Screening, admission, and predose and 1, 2, 4, 6, 8, 23 hours postdose and/or early termination |
| | MOAA/S | Predose and 0.5, 1, 2, 3, 4, 6, 8, and 23 hours postdose |
| | Safety measures (ECG, C-SSRS, and AEs/SAES) | Same as during dose selection phase |

| **Main study, treatment phase** | | |
|---|---|---|
| | Drug Liking, Good Drug Effects, Bad Drug Effects, Any Drug Effects | 0.5, 1, 1.5, 2, 3, 4, 6, 8, 10, 12, and 24 hours postdose |
| | Take Drug Again and Overall Drug Liking | 12 and 24 hours postdose |
| | Drug Similarity | 12 hours postdose |
| | High Effects and Alertness/Drowsiness | Predose, 0.5, 1, 1.5, 2, 3, 4, 6, 8, 10, 12, and 24 hours postdose |
| | MOAA/S | Predose and 0.5, 1, 2, 3, 4, 5, 7, 6, 8, 10, 12, and 24 hours postdose |
| | Reaction and Movement Time | Predose and 1, 2, 3, 4, 6, 8, and 12 hours postdose |
| | Plasma PK measurements | Predose, and at 0.5, 1, 2, 3, 4, 6, 8, 10, 12, and 24 hours postdose |
| Safety measures (vital signs, ECG, C-SSRS, AEs/SAEs) | | Same as dose selection phase |

| | | |
|---|---|---|
| AE, adverse event; C-SSRS, Columbia-Suicide Severity Rating Scale; ECG, electrocardiogram; MOAA/S, Modified Observer's Assessment of Alertness/Sedation; PK, pharmacokinetic; SAE, serious adverse event; VAS, visual analog scale. | | |

During the qualification and treatment phase of the main study, Drug Liking, Good Drug Effects, Bad Drug Effects, and Any Drug Effects were assessed at multiple times (0.5, 1, 1.5, 2, 3, 4, 6, 8, and 23 hours) after dosing. High Effects and Alertness/Drowsiness were assessed at these same multiple time points and predose. MOAA/S was assessed predose and at multiple times (0.5, 1, 2, 3, 4, 5, 6, 7, 8, 10, 12, and 24 hours) after dosing. Overall Drug Liking and Take Drug Again were assessed at 8 and 23 hours after dosing during the qualification phase and at 12 and 24 hours after dosing during the treatment phase. Drug Similarity was assessed 12 hours after dosing during the treatment phase. For both the qualification phase and treatment phase, ECG measures, C-SSRS, and AEs/SAEs were assessed on the same schedule as during the dose selection phase.

During the treatment phase, PK measures were assessed predose and at multiple time points, from 0.5 to 24 hours after dosing. Blood samples collected for plasma analyses were kept frozen (approximately -70°C to -80°C) until analysis. The quantification of the Compound **(1)** plasma concentration was performed by a bioanalytical laboratory (PRA Health Sciences, Lenexa, Kansas) using a validated liquid chromatography with a tandem mass spectroscopic method. The validated range of the assay was 1.00 to 500 ng/ml.

### Statistical Analysis

The dose selection safety set included all participants who received 1 dose of Compound **(1).** The main study qualification safety set included all participants who received ≥1 dose of alprazolam. For the treatment phase, the randomized set included all participants who were randomized and the safety set included all participants who received ≥1 dose of Compound **(1)** or alprazolam. The completer set included all participants in the randomized set who completed all treatment periods, had sufficient data for evaluation of the primary endpoint, and had ≥1 observation within 2 hours of Tmax for each treatment for Drug Liking VAS scores. The modified completer set included participants in the completer set and excluded those who demonstrated a similar response to all treatments and/or who demonstrated inappropriate responses to placebo relative to the positive. The PK set included all participants in the treatment phase who received ≥1 dose of Compound **(1)** and had ≥1 PK sample after dosing.

All randomized participants were included in the summaries of participant disposition. For the treatment phase, PD data were summarized using the modified completer set.

For the main study's primary endpoint (Drug Liking), the three main hypotheses we tested were a test of study validity (alprazolam vs placebo), relative abuse potential (Compound **(1)** vs alprazolam), and absolute abuse potential (Compound **(1)** vs placebo). Drug Liking was evaluated with a linear mixed-effects model containing treatment, period, sequence, and first order carryover effect as fixed effects and the participant as a random effect. The linear mixed model assumed the normality of the error terms, and the residuals from the model were investigated for normality using the Shapiro Wilk W test. The parameters were considered to be normally distributed if the probability value from the Shapiro-Wilk W test was ≥0.05. Parameters that did not meet this criteria were considered as non-normal in distribution, and a summary statistic and heat map by sequence and period was used to visually examine the first-order carryover effect. For parameters that were normally distributed, the significance of the first-order carryover effect was examined using an F-test as follows: if the p-value was >0.25, the first-order carryover was dropped from the linear mixed-effects model and a reduced model (i.e., fixed effects of treatment, period, and sequence) was fitted for the analysis; if the p-value was between 0.25 and 0.05 (inclusive of endpoints), then the first-order carryover was included; and if the p-value was <0.05, then the first-period analysis was carried out.

All PK analyses were based on the PK analysis set. Descriptive statistics included sample size, arithmetic mean, standard deviation, arithmetic coefficient of variation (CV), first quartile, median, and the minimum and maximum values for the third quartile. For summary statistics, PK concentration values below the limit of quantification were treated as zero. For calculating PK parameters, PK concentration values of zero that occurred prior to or after the first quantifiable concentration were imputed as zero or missing, respectively. The Compound **(1)** plasma PK parameters were estimated for each participant using noncompartmental analysis (Phoenix^{®} WinNonlin^{®} version 8.0 or later). The estimation of the terminal-elimination rate constant (λz) and λz-derived PK parameters were dependent upon inspection of PK data and a minimum of three time points in the terminal phase with adjusted R2 ≥0.8.

### Example 8. Results from a Randomized, Double-Blind, Placebo-Controlled, Crossover Study assessing Abuse Potential of Oral Compound (1) in Nondependent, Recreational Users of Central Nervous System Depressants:

This Example contains the results of the study described in Example 7.

The present study was conducted primarily to assess the human abuse potential of Compound **(1).** The study was conducted as a two-part study, consisting of a dose selection and main study. The study compared the abuse potential of single, oral doses of Compound **(1)** relative to alprazolam and placebo in nondependent, healthy recreational users of CNS depressants. The secondary objectives were to assess pharmacokinetic (PK) properties and the safety and tolerability of Compound **(1).**

### Patient disposition and baseline demographics

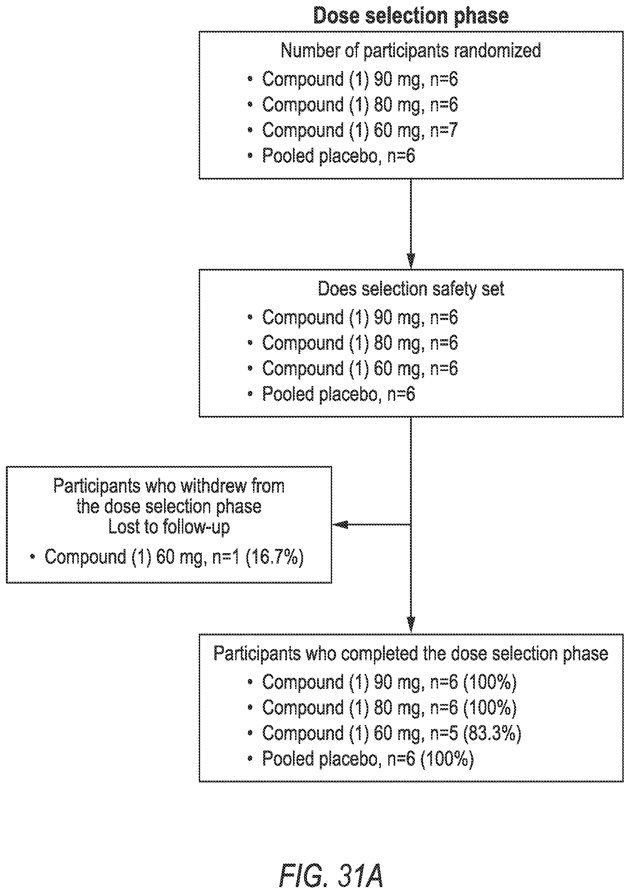

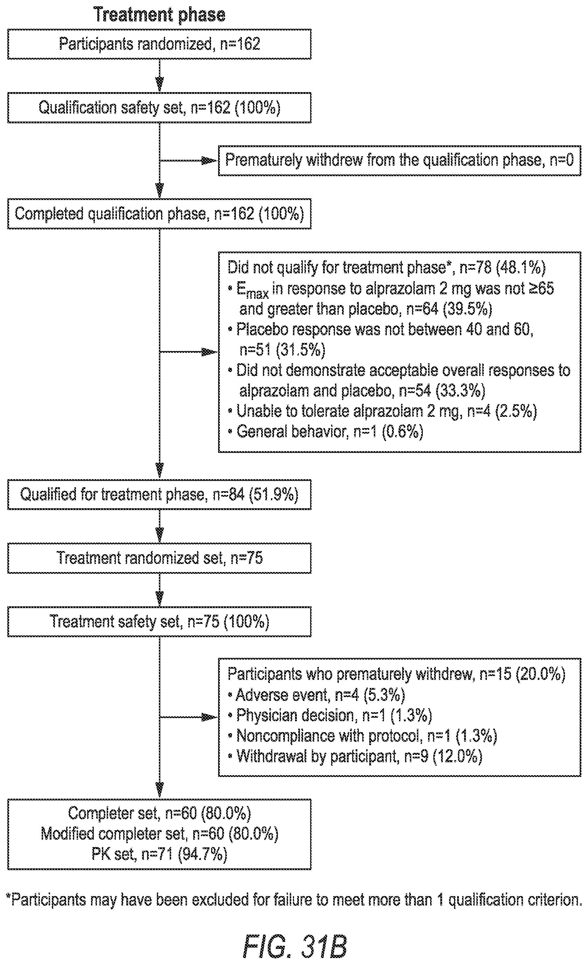

During dose selection, 24 participants received the study drug and 23 completed the dose selection phase; one participant who received Compound **(1)** 60 mg withdrew from the study and was lost to follow-up (FIG. 31A-31B). The 18 participants who received Compound **(1)** had a mean age of 30 years, and were mostly male (94%), Black (72%), and of non-Hispanic ethnicity (89%), and all had prior experience with sedative drugs and cannabinoids (Table 20).

In the main study, all 162 randomized participants completed the qualification phase. Of these, 60 completed the treatment phase and 71 comprised the PK set. In the treatment phase, participants had a mean age of 32 years, were mostly male (84%), Black (76%), of non-Hispanic ethnicity (95%), and all had prior experience with sedative drugs (Table 20).

**Table 20. Baseline Demographics and Characteristics**

| | | **Dose selection phase (N = 18)** | | | | **Treatment phase (N = 75)** |
|---|---|---|---|---|---|---|
| | | **Compound (1) 90 mg (n = 6)** | **Compound (1) 80 mg (n = 6)** | **Compound (1) 60 mg (n = 6)** | **Pooled placebo (n = 6)** | |
| **Age, years,** mean (SD) | | 30.4 (7.65) | 30.3 (10.42) | 29.7 (7.66) | 23.8 (3.13) | 32.1 (7.68) |
| **Sex, female,** n (%) | | 0 | 0 | 1 (16.7) | 1 (16.7) | 12 (16.0) |

| **Ethnicity,** n (%) | | | | | | |
|---|---|---|---|---|---|---|
| | Not Hispanic or Latino | 5 (83.3) | 5 (83.3) | 6 (100) | 5 (83.3) | 71 (94.7) |
| | Hispanic or Latino | 1 (16.7) | 1 (16.7) | 0 | 1 (16.7) | 4 (5.3) |

| **Race,** n (%) | | | | | | |
|---|---|---|---|---|---|---|
| | Black or African American | 6 (66.7) | 3 (50.0) | 6 (100) | 6 (100) | 57 (76.0) |
| | White | 1 (16.7) | 3 (50.0) | 0 | 0 | 17 (22.7) |
| | Multiple: Black or African American, White | 1 (16.7) | 0 | 0 | 0 | NA |
| | American Indian or Alaska Native | NA | NA | NA | NA | 1 (1.3) |
| **BMI, kg/m²,** mean (SD) | | 24.77 (3.71) | 22.88 (1.81) | 25.58 (3.78) | 22.27 (2.11) | 25.37 (3.39) |

| **Recreational drug use history*, n (%)** | | | | | | |
|---|---|---|---|---|---|---|
| | Xanax | NA | NA | NA | NA | 75 (100) |
| Marijuana/cannabis | | NA | NA | NA | NA | 72 (96.0) |
| | Oxycodone | NA | NA | NA | NA | 55 (73.3) |
| | Hydrocodone | NA | NA | NA | NA | 39 (52.0) |
| | MDMA | NA | NA | NA | NA | 37 (49.3) |
| | Cocaine | NA | NA | NA | NA | 46 (65.3) |
| | Valium | NA | NA | NA | NA | 23 (30.7) |
| | Klonopin | NA | NA | NA | NA | 13 (17.3) |
| | Adderall | NA | NA | NA | NA | 12 (16.0) |
| | Codeine | NA | NA | NA | NA | 10 (13.3) |
| | Psilocybin | NA | NA | NA | NA | 8 (10.7) |
| | Ambien | NA | NA | NA | NA | 7 (9.3) |
| | PCP | NA | NA | NA | NA | 7 (9.3) |
| | Ativan | NA | NA | NA | NA | 5 (6.7) |
| | LSD | NA | NA | NA | NA | 5 (6.7) |
| | Tramadol | NA | NA | NA | NA | 4 (5.3) |

| | | | | | | |
|---|---|---|---|---|---|---|
| *Recreational drug use history reported for the overall treatment set with occurrences of ≥5%. BMI, body mass index: LSD, lysergic acid diethylamide; MDMA, 3,4-methylenedioxymethamphetamine: NA, not applicable; PCP, phencyclidine; SD, standard deviation. | | | | | | |

### Dose selection

During the study's first part, dose selection, all participants had a MOAA/S score of 5 (responds readily to name spoken in a normal tone) at predose baseline and 1 hour after dosing. At 2 hours postdose the MOAA/S scores were elevated and did not return back to 5 until 8 hours postdose. All participants in the placebo group with available data had scores of 5 at all time points. Sedation increased over time following administration of all Compound **(1)** doses, with slightly greater increases observed with the 80- and 90-mg doses. At 2-7 hours postdose, 1 to 4 participants in at least 1 Compound **(1)** cohort reported mild sedation (MOAA/S score of 2-4 out of maximum sedation score of 0). During the 24 hours of monitoring, none of the groups scored <2, which would indicate poorly responsive or nonresponsive sedation. All scores returned to the baseline score of 5 by 8 hours postdose and remained so through day 3. Based on these results and other safety data (discussed below), the doses selected for the main study's treatment phase were 30, 60, and 90 mg.

### Primary endpoint, Drug Liking

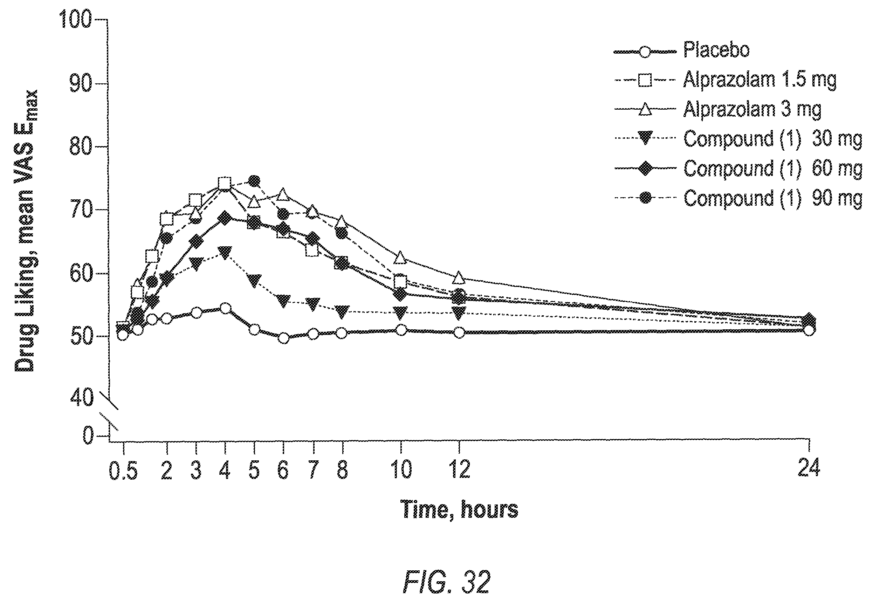

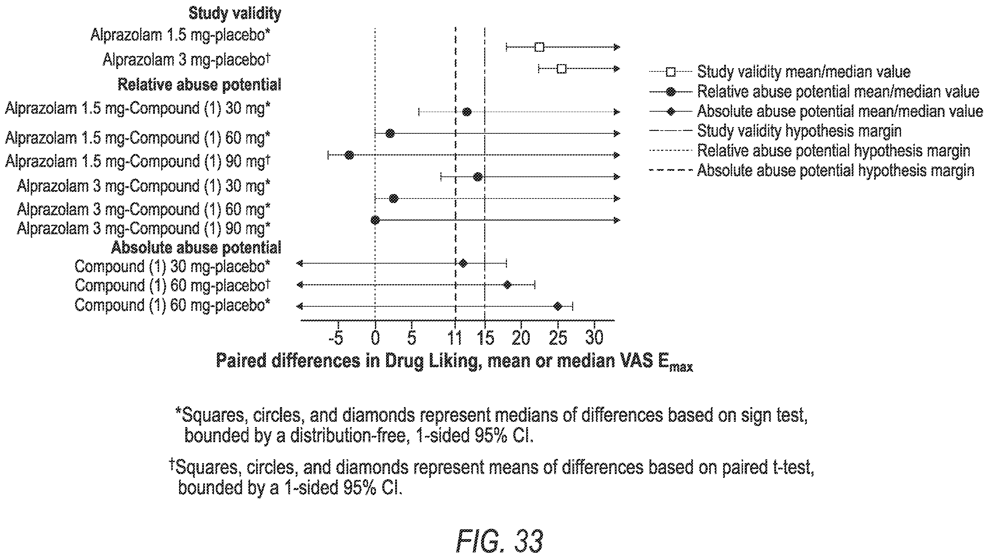

During the main study treatment phase, study validity was verified by alprazolam having significantly higher values than placebo on the primary endpoint of Drug Liking (Table 21). Compound **(1)** demonstrated significantly lower Drug Liking scores than alprazolam for Compound **(1)** 30 mg vs both alprazolam doses (P < 0.001 for both), Compound **(1)** 60 mg vs alprazolam (1.5 mg, P = 0.049; 3 mg, P = 0.002; Table 21; FIG. 32; FIG. 33). Compound **(1)** 90 mg was not significantly different vs alprazolam. Also, the assessment of absolute abuse potential demonstrated that all Compound **(1)** doses produced numerically higher Drug Liking scores than placebo.

### Key Secondary Endpoints

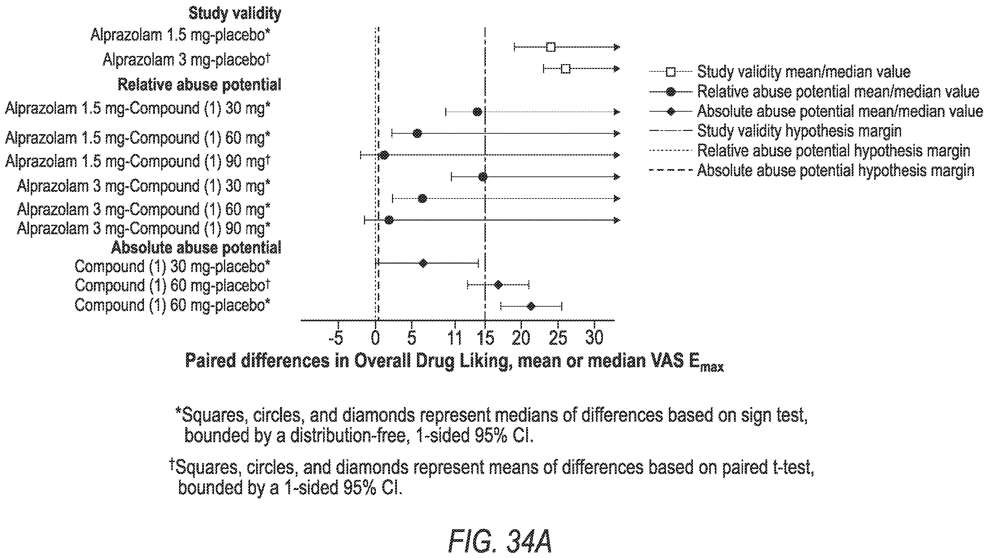

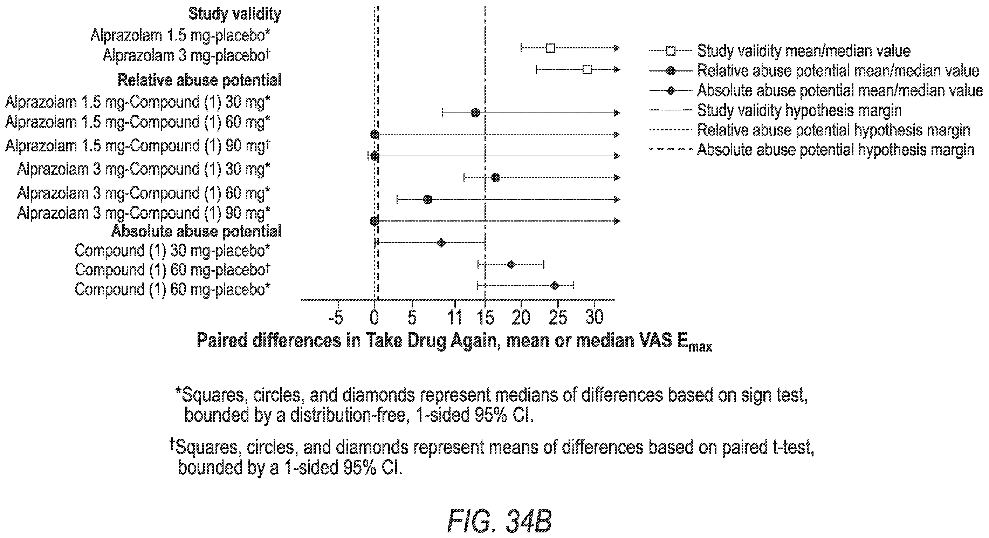

Study validity was demonstrated by alprazolam having significantly greater values than placebo for both Overall Drug Liking and Take Drug Again (Table 20). For Overall Drug Liking, Compound **(1)** 30 mg (P < 0.001) and 60 mg (P ≤ 0.005), but not 90 mg, demonstrated significantly lower scores than both alprazolam doses (Table 21; FIG. 34A). For Take Drug Again, Compound **(1)** 30 mg demonstrated significantly lower (P < 0.001) scores than both alprazolam doses and Compound (1) 60 mg was significantly lower (P = 0.003) than the alprazolam 3 mg dose (Table 21; FIG. 34B).

**Table 21. Differences in Drug Liking and Overall Drug Liking and Take Drug Again Emax Scores During Treatment Phase**

| **Treatments** | **Drug Liking Emax (95% CI)** | **Overall Drug Liking Emax (95% CI)** | **Take Drug Again Emax (95% CI)** |
|---|---|---|---|
| **Positive control vs placebo (study validity)** | | | |
| Alprazolam 1.5 mg - placebo | 22.50 (19.00, ∞)* | 24.00 (19.00, ∞)† | 24.00 (20.00, ∞)‡ |
| Alprazolam 3 mg - placebo | 25.52 (22.35, ∞)* | 26.00 (23.00, ∞)† | 29.00 (22.00, ∞)† |

| **Alprazolam vs Compound (1) (relative abuse potential)** | | | |
|---|---|---|---|
| Alprazolam 1.5 mg - Compound (1) 30 mg | 12.50 (6.00, ∞)* | 13.92 (9.56, ∞)* | 13.67 (9.27, ∞)* |
| Alprazolam 1.5 mg - Compound (1) 60 mg | 2.00 (0, ∞)‡ | 5.63 (2.20, ∞)† | 0 (0, ∞) |
| Alprazolam 1.5 mg - Compound (1) 90 mg | -3.55 (-6.43, ∞) | 1.12 (-2.20, ∞) | 0 (-1.00, ∞) |
| Alprazolam 3 mg - Compound **(1)** 30 mg | 14.00 (9.00, ∞)* | 14.62 (10.36, ∞)* | 16.43 (12.14, ∞)* |
| Alprazolam 3 mg - Compound **(1)** 60 mg | 2.50 (0, ∞)† | 6.33 (2.31, ∞)† | 7.15 (3.02, ∞)† |
| Alprazolam 3 mg - Compound **(1)** 90 mg | 0.00 (-4.00, ∞) | 1.82 (-1.47, ∞) | 0 (0, ∞) |

| **Compound (1) vs placebo (absolute abuse potential)** | | | |
|---|---|---|---|
| Compound **(1)** 30 mg - placebo | 12.00 (-∞, 18.00)§ | 6.50 (0, 14.00)‡ | 9.00 (0, 15.00)‡ |
| Compound **(1)** 60 mg - placebo | 18.12 (-∞, 21.84)§ | 16.75 (12.53, 20.97)* | 18.57 (14.06, 23.07)* |
| Compound **(1)** 90 mg - placebo | 25.00 (-∞, 27.00)§ | 21.27 (17.12, 25.41)* | 24.50 (14.00, 27.00)* |

| | | | |
|---|---|---|---|
| Friedman's test was used to assess overall treatment effects with an alpha value of *P* < 0.001. For comparisons, when paired differences between treatments were not symmetric; a sign test was used to assess differences between the 2 treatments; median of location shift, 1-sided 95% or 2-sided 90% CI of median, and p-value are presented. When paired differences between treatments were symmetric; a paired t-test was used to assess differences between the 2 treatments; mean, 1-sided 95% or 2-sided 90% CI of mean, and p-value are presented. **P* <0.001; †*P*<0.05; ‡*P* < 0.01; §Because Compound (1) 30 and 60 mg, but not 90 mg, was significantly different from the alprazolam, the p-values for comparisons of Compound (1) relative to placebo for absolute abuse potential are not shown; CI = confidence interval; Emax = maximum (peak) effect; SE = standard error; LS = least squares; VAS = visual analog scale. | | | |

### Secondary Endpoints

For High Effects and Good Drug Effects, both doses of alprazolam demonstrated significantly higher scores relative to placebo (P < 0.001; Table 21). For High Effects, Compound **(1)** 30 and 60, but not 90 mg, demonstrated significantly lower scores relative to both alprazolam doses. For Good Drug Effects, Compound **(1)** 30 mg demonstrated significantly lower scores than both alprazolam doses (P < 0.001 for both). In addition, the Compound **(1)** 60 mg group demonstrated was significantly lower Good Drug Effects scores than the alprazolam 3 mg group (P < 0.05), and Compound **(1)** 90 mg was not significantly different from either alprazolam dose.

For Bad Drug Effects, alprazolam 3 mg demonstrated a significantly greater score versus vs placebo (P <0.001; Table 22). Compound **(1)** 30 mg demonstrated a significantly lower Bad Drug Effects score than both alprazolam doses (P < 0.02), and 60 mg was only significantly lower than the higher alprazolam dose of 3 mg (P < 0.001).

**Table 22. Differences in High Effects, Good Drug Effects, and Bad Drug Effects Emax Scores During Treatment Phase.**

| **Treatments** | **High Effects Emax (SE; 95% CI)** | **Good Drug Effects Emax (95% CI)** | **Bad Drug Effects Emax (95% CI)** |
|---|---|---|---|
| **Positive control vs placebo (study validity)** | | | |
| Alprazolam 1.5 mg - placebo | 43.80 (4.13; 36.98, ∞)* | 49.00 (41.00, ∞)* | 0.00 (0, ∞) |
| Alprazolam 3 mg - placebo | 51.65 (4.14; 44.83, ∞)* | 53.47 (47.14, ∞)* | 1.00 (1.00, ∞)* |

| **Alprazolam vs Compound (1) (relative abuse potential)** | | | |
|---|---|---|---|
| Alprazolam 1.5 mg - Compound **(1)** 30 mg | 24.59 (4.13; 17.77, ∞)* | 24.62 (16.95, x0)* | 0 (0, 0) † |
| Alprazolam 1.5 mg - Compound **(1)** 60 mg | 7.57 (4.15; 0.71, ∞)† | 0.00 (-2.00, ∞) | 0 (0, 0) |
| Alprazolam 1.5 mg - Compound **(1)** 90 mg | -12.00 (4.13; -18.81, ∞) | -12.00 (-16.00, ∞) | -12.93 (-19.16, -6.70)* |
| Alprazolam 3 mg - Compound **(1)** 30 mg | 32.45 (4.15; 25.60, ∞)* | 33.48 (25.93, ∞)* | 1.00 (1.00, 11.00)* |
| Alprazolam 3 mg - Compound **(1)** 60 mg | 15.42 (4.11; 8.64, ∞)* | 7.50 (1.00, ∞)† | 1.00 (0.00, 6.00)* |
| Alprazolam 3 mg - Compound **(1)** 90 mg | -4.14 (4.12; -10.94, ∞) | -1.00 (-9.00, ∞) | -1.82 (-8.26, 4.62) |

| **Compound (1) vs placebo (absolute abuse potential)** | | | |
|---|---|---|---|
| Compound **(1)** 30 mg - placebo | 19.21 (4.17; 12.33, 26.08)* | 19.98 (12.24, 27.72)* | 0 (0, 0) |
| Compound **(1)** 60 mg - placebo | 36.23 (4.13; 29.41, 43.05)* | 40.53 (33.73, 47.34)* | 0 (0, 0)‡ |
| Compound **(1)** 90 mg - placebo | 55.80 (4.15; 48.95, 62.64)* | 59.00 (49.00, 71.00)* | 0 (0, 8.00)* |

| | | | |
|---|---|---|---|
| Friedman's test was used to assess overall treatment effects with an alpha value of *P* < 0.001. For comparisons, when paired differences between treatments were not symmetric; a sign test was used to assess differences between the 2 treatments; median of location shift, 1-sided 95% or 2-sided 90% CI of median, and p-value are presented. When paired differences between treatments were symmetric; a paired t-test was used to assess differences between the 2 treatments; mean, 1-sided 95% or 2-sided 90% CI of mean, and p-value are presented. * *P* < 0.001; †*P* < 0.05; ‡ *P <* 0.01; CI = confidence interval; Emax = maximum (peak) effect; SE = standard error; LS = least squares; VAS = visual analog scale. | | | |

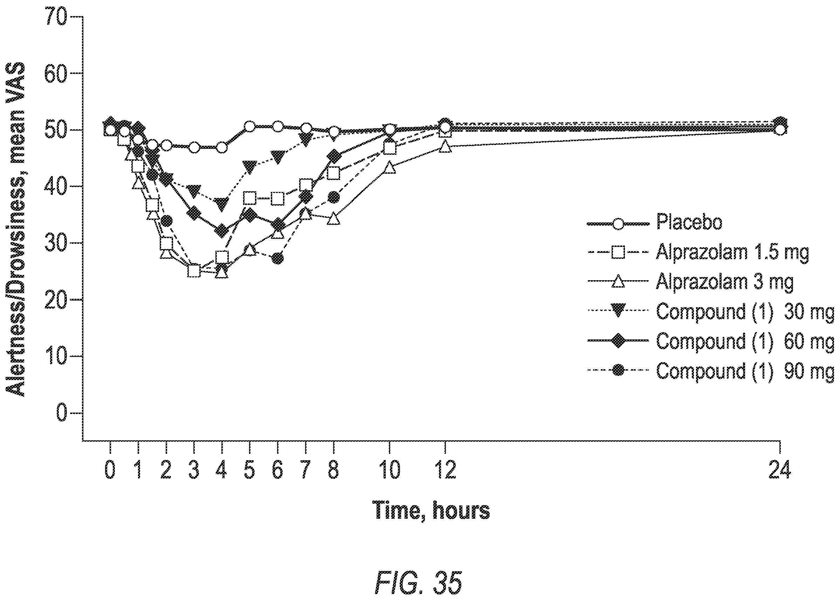

For Alert/Drowsiness, the mean scores for placebo ranged from 48-53 (FIG. 35). After dosing, the Compound **(1)** scores remained below the neutral value of 50 (neither drowsy nor alert) for approximately 6-7 hours postdose for 30 mg and for approximately 8-10 hours postdose for the 60 and 90 mg doses. For MOAA/S, both alprazolam and Compound **(1)** demonstrated increased scores over time, with the greatest scores observed between 2-4 hours postdose (data not shown).

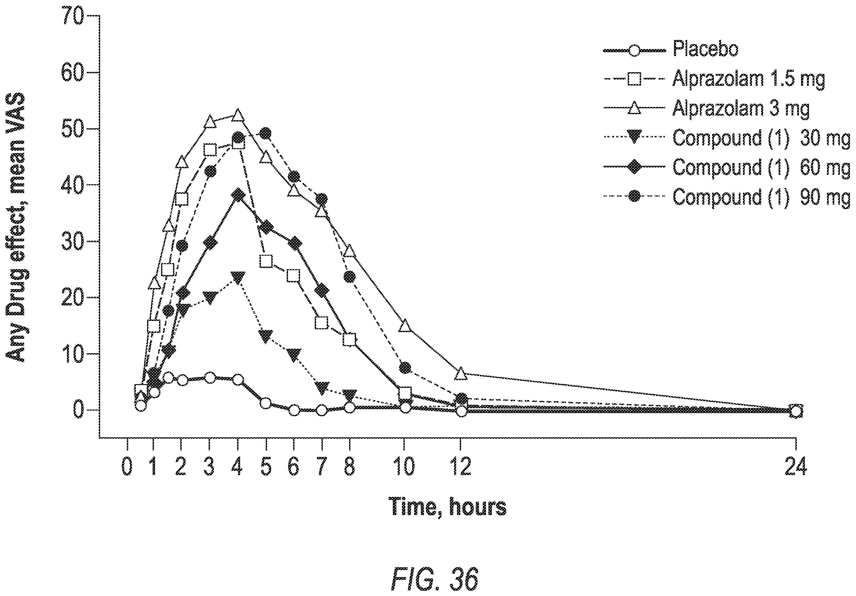

For Any Drug Effect, scores for placebo were <10 (0 = not feeling any drug effect) throughout the study, while scores for alprazolam and Compound **(1)** demonstrated a gradual increase with peak effects between 4-5 hours postdose (FIG. 36). Both alprazolam doses demonstrated significantly greater VAS scores compared with placebo (P < 0.001 for both). In addition, the scores with Compound **(1)** 30 mg were significantly lower than with both alprazolam doses (P < 0.001 for both). The Compound **(1)** 60 mg scores were significantly lower than alprazolam 3 mg (P = 0.002), and the Compound **(1)** 90 mg scores were not significantly different than either alprazolam dose.

For Drug Similarity measure of Placebo Similarity, placebo had the highest median score of 86.0, and all alprazolam and Compound **(1)** dosages had median scores of zero. For the Drug Similarity measure of Benzodiazepines Similarity, placebo had a median score of 0, and the Compound **(1)** 90 mg and alprazolam 1.5 mg groups had the greatest median scores of 92.5 for each. For the Drug Similarity measure of Opioids Similarity, placebo had a median score of 0, and the highest scores were for the alprazolam 3 mg (40.0) and Compound **(1)** 90 mg (39.0) groups.

For Reaction Time, all alprazolam and Compound **(1)** doses had significantly increased Emax scores compared to placebo (P ≤ 0.003 for all comparisons), with alprazolam 3 mg having the greatest increase (Table 23). For Movement Time, alprazolam 1.5 mg and 3 mg and Compound **(1)** 60 and 90 mg had significantly increased Emax scores compared to placebo (P < 0.001 for all comparisons). Alprazolam 3 mg demonstrated significantly increased scores vs all Compound **(1)** doses (P < 0.001 for all), but alprazolam 1.5 mg only demonstrated significantly increased scores vs Compound **(1)** 30 mg (P < 0.001).

**Table 23. Analysis of Reaction and Movement Time Emax.**

| | | **Reaction time, median difference, (CI)** | **p-value** | **Movement time, mean difference, (CI)** | **p-value** |
|---|---|---|---|---|---|
| **Alprazolam vs placebo** | | | | | |
| | 1.5 mg | 54.00 (46.00, ∞) | <0.001 | 56.50 (45.00, ∞) | <0.001 |
| | 3 mg | 127.00 (116.00, ∞) | <0.001 | 187.50 (123.00, ∞) | <0.001 |

| **Alprazolam vs Compound (1)** | | | | | |
|---|---|---|---|---|---|
| | 1.5 mg vs 30 mg | 46.50 (36.00, ∞) | <0.001 | 49.50 (24.00, ∞) | <0.001 |
| | 1.5 mg vs 60 mg | 18.00 (1.00, ∞) | 0.018 | 18.45 (-3.30, ∞) | 0.081 |
| | 1.5 mg vs 90 mg | -3.98 (-17.79, ∞) | 0.684 | 6.00 (-4.00, ∞)^{†} | 0.217 |
| | 3 mg vs 30 mg | 100.0 (82.00, ∞) | <0.001 | 144.50 (122.00, ∞) | <0.001 |
| | 3 mg vs 60 mg | 74.50 (59.00, ∞) | <0.001 | 116.00 (77.00, ∞)) | <0.001 |
| | 3 mg vs 90 mg | 60.50 (37.00, ∞) | <0.001 | 103.50 (83.00, ∞) | <0.001 |

| **Compound (1) vs placebo** | | | | | |
|---|---|---|---|---|---|
| | 30 mg vs placebo | 16.00 (6.00, 18.00) | 0.003 | 11.00 (-6.00, 25.00) | 0.519 |
| | 60 mg vs placebo | 42.00 (30.00, 50.00) | <0.001 | 51.50 (29.00, 72.00) | <0.001 |
| | 90 mg vs placebo | 66.50 (50.00, 81.00) | <0.001 | 53.50 (33.00, 102.00) | <0.001 |

| | | | | | |
|---|---|---|---|---|---|
| Friedman's test was used to assess overall treatment effects with a p-value <0.001 for both endpoints. For comparisons, when paired differences between treatments were not symmetric; a sign test was used to assess differences between 2 treatments. Median of location shift, 1-sided 95% or 2-sided 90% CI of median, and p-value are presented, unless otherwise indicated. When paired differences between treatments were symmetric; a paired t-test was used to assess differences between 2 treatments. Mean, 1-sided 95% or 2-sided 90%. CI = confidence interval; Emax =maximum (peak) effect. | | | | | |

### Pharmacokinetic Endpoints

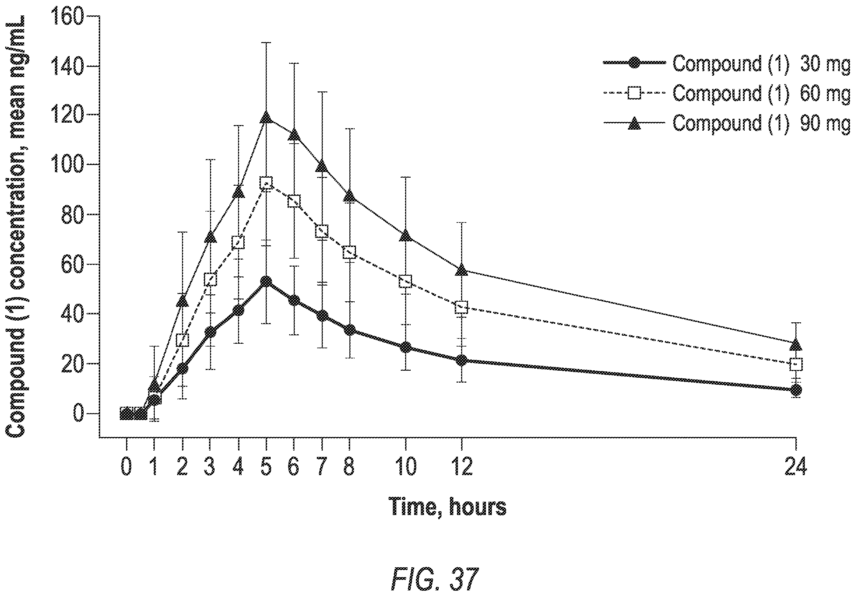

Higher Compound **(1)** doses were associated with greater Cmax values with the geometric mean values ranging from 55.5-123.1 ng/ml (Table 24; FIG. 37). In addition, higher Compound **(1)** doses were associated with greater AUC0-24, with values ranging from 519.9-1334 h×ng/ml. For both Cmax and AUC0-24, all CV values were less than 30% and consistent across treatments. For all three Compound (1) doses, the median Tmax value was 5.1 hours, and the T1/2 values ranged from 9.92-10.93 hours the three dosages.

**Table 24. Summary of PK Parameters Following a Single Oral Dose of Compound (1).**

| **PK Parameter** | **Compound (1)** | | |
|---|---|---|---|
| | **90 mg (n = 67)** | **60 mg (n = 66)** | **30 mg (n = 65)** |
| **Cmax,** ng/mL, geometric mean (CV) | 123.1 (24.3) | 97.6 (24.3) | 55.5 (26.7) |
| **Tmax,** hours, median (minimum, maximum) | 5.1 (3.1, 10.1) | 5.1 (3.1, 10.1) | 5.1 (1.1, 12.1) |
| **AUC0-24,** hr×ng/mL, geometric mean (CV) | 1334 (24.1) | 982 (23.2) | 520 (23.6) |
| **T1/2,** hours, geometric mean (CV) | 10.9 (29.7)* | 9.9 (21.2)* | 10.1 (31.2)* |

| | | | |
|---|---|---|---|
| *For T1/2, the number of participants for the 90 mg (n = 62), 60 mg (n = 60), and 30 mg (n = 66) dose cohorts was less than for the full PK set. AUC0-24 = area under the plasma concentration versus time curve from time 0 to 24 hours after dose administration; Cmax = maximum observed plasma concentration of drug; CV = percent coefficient of variation; T1/2 = estimate of the terminal elimination half-life of the drug; Tmax = time to maximum observed plasma concentration of drug. | | | |

### Safety

During dose selection, 21/24 (87.5%) participants reported 46 TEAE events; there were no deaths, SAEs, or discontinuations due to an AE (Table 25). The majority of TEAEs were mild in severity, with 79.2% (19/24) of participants reporting a total of 44 mild TEAEs. There were two moderate TEAEs; one instance of somnolence following administration of Compound **(1)** 90 mg and one instance of a toothache following administration of placebo. There were no clinically significant changes in mean clinical chemistry, hematology, or urinalysis.

During the treatment phase, 36.2% (25/69) of participants in the placebo group had ≥1 TEAE. In the Compound **(1)** groups, the proportion of participants with ≥1 TEAE increased with higher doses of (Table 26). The majority of participants with TEAEs (217/259) reported they were mild; there were no severe TEAEs or deaths. Four participants discontinued due to TEAEs: one participant in the Compound **(1)** 30 mg group had elevated blood pressure, one participant in the Compound **(1)** 60 mg had eye pain and ocular discomfort (each a TEAE), one participant in the Compound **(1)** 90 mg group had a confusional state, and one participant in the Compound **(1)** 90 mg group had facial swelling associated with a dental retainer. The confusional state was determined to be possibly related to treatment; all others were determined as not related to treatment. There were no clinically significant changes in mean clinical chemistry, hematology, or urinalysis.

**Table 25. Summary of Frequently Occurring (>2%) TEAEs during Dose Selection**

| **Preferred term n (%)** | | **Compound (1) dose** | | | | |
|---|---|---|---|---|---|---|
| | | **90 mg (n = 6)** | **80 mg (n = 6)** | **60 mg (n = 6)** | **All doses (n = 18)** | **Placebo (n = 6)** |
| **Mild TEAE** | | 5 (83.3) | 5 (83.3) | 5 (83.3) | 15 (83.3) | 4 (66.7) |
| **Moderate TEAE** | | 1 (16.7) | 0 | 0 | 1 (5.6) | 1 (16.7) |
| **Any TEAE** | | 6 (100) | 5 (83.3) | 5 (83.3) | 16 (88.9) | 5 (83.3) |
| | Somnolence* | 4 (66.7) | 5 (83.3) | 4 (66.7) | 13 (72.2) | 2 (33.3) |
| | Euphoric mood* | 4 (66.7) | 1 (16.7) | 0 | 5 (27.8) | 0 |
| | Feeling of relaxation* | 1 (16.7) | 2 (33.3) | 0 | 3 (16.7) | 1 (16.7) |
| | Dizziness postural | 0 | 2 (33.3) | 1 (16.7) | 3 (16.7) | 0 |
| | Yawning | 1 (16.7) | 0 | 0 | 1 (5.6) | 2 (33.3) |
| | Irritability* | 1 (16.7) | 1 (16.7) | 0 | 2 (11.1) | 0 |
| | Disturbance in attention | 0 | 1 (16.7) | 0 | 1 (5.6) | 0 |
| | Headache | 0 | 0 | 1 (16.7) | 1 (5.6) | 0 |
| | Paresthesia | 0 | 1 (16.7) | 0 | 1 (5.6) | 0 |
| | Tremor | 1 (16.7) | 0 | 0 | 1 (5.6) | 0 |
| | Feeling abnormal | 1 (16.7) | 0 | 0 | 1 (5.6) | 0 |
| | Feeling hot | 0 | 0 | 0 | 0 | 1 (16.7) |
| | Hunger | 0 | 0 | 0 | 0 | 1 (16.7) |
| | Asthenopia | 0 | 1 (16.7) | 0 | 1 (5.6) | 0 |
| | Vision blurred | 0 | 1 (16.7) | 0 | 1 (5.6) | 0 |
| | Lacrimation increased | 0 | 0 | 0 | 0 | 1 (16.7) |
| | Orthostatic HR response increase | 0 | 0 | 1 (16.7) | 1 (5.6) | 0 |
| | Blood bilirubin increased | 0 | 0 | 0 | 0 | 1 (16.7) |
| | Hyperhidrosis | 1 (16.7) | 0 | 0 | 1 (5.6) | 0 |
| | Toothache | 0 | 0 | 0 | 0 | 1 (16.7) |

| | | | | | | |
|---|---|---|---|---|---|---|
| Percentages are calculated based on the number of participants per treatment group. *Most common (occurring in > 1 participant) abuse-potential-related TEAEs. For the mild and moderate TEAE, if a participant has more than one TEAE within a category, they are only counted once according to the highest severity. HR = heart rate: TEAE = treatment-emergent adverse event. | | | | | | |

**Table 26. Treatment-Emergent Adverse Events Reported in >2% of All Participants**

| | | **Alprazolam dose** | | **Compound (1) dose** | | | |
|---|---|---|---|---|---|---|---|
| **Preferred term n (%)** | | **1.5 mg n=71** | **3 mg n=66** | **90 mg n=67** | **60 mg n=66** | **30 mg n=65** | **Placebo n=69** |
| **Mild TEAE** | | 48 (67.6) | 39 (59.1) | 39 (58.2) | 38 (57.6) | 31 (47.7) | 22 (31.9) |
| **Moderate TEAE** | | 8 (11.3) | 13 (19.7) | 10 (14.9) | 4 (6.1) | 4 (6.2) | 3 (4.3) |
| **Any TEAE** | | 56 (78.9) | 52 (78.8) | 49 (73.1) | 42 (63.6) | 35 (53.8) | 25 (36.2) |
| | Somnolence | 37 (52.1) | 41 (62.1) | 30 (44.8) | 22 (33.3) | 17 (26.2) | 11 (15.9) |
| | Fatigue | 15 (21.1) | 8 (12.1) | 9 (13.4) | 9 (13.6) | 6 (9.2) | 2 (2.9) |
| | Euphoric mood | 6 (8.5) | 13 (19.7) | 10 (14.9) | 7 (10.6) | 5 (7.7) | 0 |
| | Tremor | 0 | 0 | 12 (17.9) | 3 (4.5) | 0 | 0 |
| | Headache | 6 (8.5) | 7 (10.6) | 8 (11.9) | 4 (6.1) | 5 (7.7) | 5 (7.2) |
| | Hiccups | 0 | 6 (9.1) | 0 | 0 | 0 | 0 |
| | Dizziness* | 1 (1.4) | 5 (7.6) | 1 (1.5) | 2 (3.0) | 2 (3.1) | 1 (1.4) |
| | Lacrimation increased | 1 (1.4) | 3 (4.5) | 0 | 0 | 0 | 0 |
| | Feeling drunk | 3 (4.2) | 0 | 2 (3.0) | 1 (1.5) | 0 | 0 |
| | Confusional state | 0 | 1 (1.5) | 2 (3.0) | 0 | 0 | 0 |
| | Diarrhea | 1 (1.4) | 2 (3.0) | 0 | 1 (1.5) | 0 | 0 |
| | Fine motor skill dysfunction | 0 | 0 | 2 (3.0) | 0 | 0 | 0 |
| | Irritability | 0 | 0 | 2 (3.0) | 0 | 0 | 0 |
| | Swelling face | 0 | 0 | 2 (3.0) | 0 | 0 | 0 |
| | Back pain | 0 | 1 (1.5) | 0 | 0 | 0 | 2 (2.9) |
| | Upper respiratory tract infection | 2 (2.8) | 0 | 0 | 0 | 0 | 0 |

| | | | | | | | |
|---|---|---|---|---|---|---|---|
| Data shown are number (%) of participants with TEAE per group with percentages calculated based on the number of participants per treatment group. For the mild and moderate TEAE, if a participant has more than one TEAE within a category, they are only counted once according to the highest severity. TEAE = treatment-emergent adverse event. | | | | | | | |

### Discussion

This study evaluated the human abuse potential of Compound **(1)** against alprazolam, a schedule IV compound (Wilbraham D, et al. J Clin Pharmacol. 2020;60:495-504). Overall, the Compound **(1)** doses of 30 and 60 mg largely demonstrated significantly lower abuse potential than the active comparator, alprazolam 1.5 and 3 mg, for Drug Liking (primary endpoint), Overall Drug Liking and Take Drug Again (key secondary endpoints), and most other secondary endpoints. The exception to this trend was that for Take Drug Again Compound **(1)** 60 mg did not demonstrate significantly lower scores than the lower alprazolam dose of 1.5 mg. Compound **(1)** 90 mg demonstrated a similar, but not significantly lower, abuse potential as alprazolam. Supporting the study validity of these findings regarding Compound **(1),** both doses of alprazolam (active comparator) demonstrated significantly higher Drug Liking scores vs placebo, and significantly greater scores for the key secondary endpoints of Overall Drug Liking and Take Drug Again scores. Furthermore, tests of the absolute abuse potential of Compound **(1)-**as assessed with Drug Liking-demonstrated significantly higher scores than placebo. These findings demonstrated the validity of the study, the lower relative abuse potential of Compound **(1)** 30 and 60 mg, but not 90 mg, vs alprazolam, and the lower absolute abuse potential of Compound **(1)** vs placebo.

For sedation, as measured by the PD endpoint Alert/Drowsiness, both Compound **(1)** and alprazolam demonstrated an apparent dose-related response, with higher dosages having longer durations of sedation. Over the 24 hours analyzed, both Compound **(1)** and alprazolam demonstrate a reduction in Alertness/Drowsiness VAS scores, with a peak effect occurring approximately 3-4 hours after dose administration, upon which both drugs showed a trend back towards the neutral non-drowsy state. Overall, these results indicate that both Compound **(1)** and alprazolam demonstrated minimal negative effects and sedative effects, which appeared to be dose proportional and peaked around 3-4 hours after dose administration.

In this study, the objective measures of Reaction Time and Movement Time and the observer-rated MOAA/S assessed psychomotor effects and responsiveness associated with Compound **(1)** and compared with alprazolam. The Reaction Time and Movement Times were generally lower with Compound **(1)** than with alprazolam, and the lowest dose of Compound **(1)** (30 mg) was not significantly different than placebo for the Movement Time measure. In addition, for the dose selection phase, responsiveness as assessed with the MOAA/S scores was slightly lower with the Compound **(1)** 80 and 90 mg doses, but not low enough to prevent the use of the Compound **(1)** 90 mg dose for the main study. In the main study's treatment phase, both Compound **(1)** and alprazolam demonstrated lower responsiveness over time, with the greatest effects observed approximately between 2-4 hours postdose; Compound **(1)** doses returning back to neutral by approximately 8 to 12 hours post dose and the greatest alprazolam dose took approximately 24 hours. Overall Compound **(1)** demonstrated psychomotor effects and responsiveness that was comparable or improved relative to alprazolam.

During the main study, the incidence of TEAEs was generally higher for alprazolam vs Compound **(1).** In addition, the TEAEs that occurred were generally mild with only two moderate TEAEs being reported. For Compound **(1),** the incidence of TEAEs appeared to be dose-related; the pattern of TEAEs associated with Compound **(1)** 30 and 60 mg was similar, but a greater proportion of participants reported TEAEs following Compound **(1)** 60 mg. For Compound **(1)** 30 and 60 mg, the two most commonly reported TEAE included somnolence followed by fatigue. The proportion of participants reporting moderate TEAEs was highest and comparable for Compound **(1)** 90 mg and alprazolam 3 mg. Compound **(1)** 90 mg demonstrated relatively low occurrences of the abuse-potential-related TEAEs of fine motor skill dysfunction and irritability. Overall, Compound **(1)** appeared to demonstrate a favorable safety profile that was dose proportional and either comparable to or more favorable than alprazolam.

All three Compound **(1)** doses were readily absorbed following a single-dose administration, as was seen in prior studies (Hoffmann E, et al. Clin Pharmacokinet. 2020;59:111-120), and the concentration of Compound **(1)** exposure (i.e., Cmax and AUC0-24 ) rose with increasing dosages. The Tmax of approximately 5 hours (for all three Compound **(1)** doses) was longer than the value of 1 hour reported by a prior study of 108 healthy volunteers (Hoffmann E, et al. Clin Pharmacokinet. 2020;59:111-120). This Tmax value of approximately 5 hours corresponded closely to the 5-hour delay interval between dosing and the maximum Drug Liking VAS Emax value, suggesting a correspondence between the increasing plasma levels and reported subjective effects. In addition, the T1/2 values (10-11 hours for all Compound **(1)** doses) were comparable to each other but shorter than the T1/2 values (14-19 hours) reported by Hoffman and Colleagues (Hoffmann E, et al. Clin Pharmacokinet. 2020;59:111-120). These differences in Tmax and T1/2 were possibly due to the prior study's use of an aqueous solution Compound **(1)** formulation versus the present study's use of a capsule-based formulation (Hoffmann E, et al. Clin Pharmacokinet. 2020;59:111-120). For the present study, there was low intersubject variability in exposure parameters (CV values <30%), which, along with the dose-proportional relationships in exposure, are important PK/PD features of Compound **(1).**

Strengths of the current study include the inclusion of a dose selection part and a qualification phase with an assessment of study validity. Since recreational users of CNS depressants were expected to be able to reasonably tolerate a CNS-acting compound such as Compound **(1),** the dose selection phase ensured that an adequately high and tolerable dose of Compound **(1)** (i.e., up to 90 mg) was assessed in the main study. In addition, the double-blind qualification phase was beneficial, as it ensured study validity by verifying that participants were able to distinguish the subjective effects of the active comparator, alprazolam, from placebo.

Limitations of the study include the single-dose design and use of subjective measures of drug effects. In a real-world setting the abuse of CNS-acting drugs can typically involve multiple, repeated doses, and therefore the abuse potential and safety of Compound **(1)** may differ under these conditions; however, a strength of the single-dose design is that it allows for increased safety, experimental control, and aligns with the relevant FDA guidelines for human abuse studies (U.S. Department of Health and Human Services Food and Drug Administration, Center for Drug Evaluation and Research (CDER). Assessment of abuse potential of drugs: guidance for industry. January, 2017). The use of self-reported measures can potentially introduce error and bias; an attempt to counteract this was the use of a double-blind study design and the inclusion of observer-rated and objective measures such as the MOAA/S and Reaction and Movement Times.

This study of healthy, recreational, CNS-depressant users, demonstrated that single, oral doses of Compound **(1)** 30 and 60 mg demonstrated favorable human abuse potential relative to both doses (1.5 and 3 mg) of the active comparator alprazolam. Also Compound **(1)** 90 mg produced subjective effects that were similar to the schedule IV benzodiazepine alprazolam. Finally, Compound **(1)** demonstrated a safety profile that was consistent with prior studies (Hoffman); it was generally well tolerated with an encouraging safety and tolerability profile demonstrated by a majority of TEAEs reported by Compound **(1)** groups being mild, with no reported severe TEAEs nor deaths.

### EQUIVALENTS AND SCOPE

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

### OTHER EMBODIMENTS

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

Aspects and features of the present disclosure are set out in the following numbered clauses which contain the subject matter of the claims of the PCT application as filed.
1. A method of treating major depressive disorder (MDD) with elevated anxiety in a subject in need thereof, comprising administering a therapeutically effective amount of Compound **(1):** 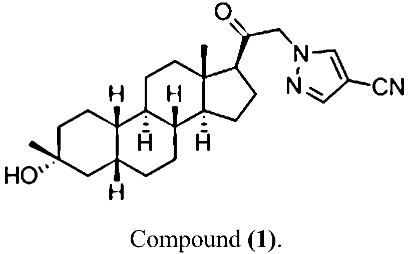
2. A method of treating major depressive disorder (MDD) with elevated anxiety in a subject in need thereof, comprising administering a therapeutically effective amount of a pharmaceutically acceptable salt of Compound **(1):** 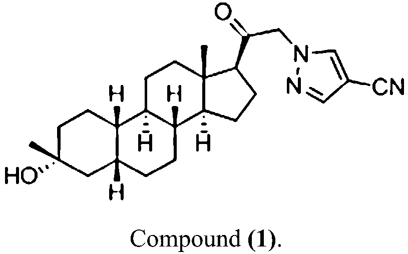
3. The method of clause 1 or 2, wherein Compound **(1),** or the pharmaceutically acceptable salt of Compound **(1),** is administered once a day for about 14 days or about 2 weeks.
4. The method of clause 1, wherein Compound **(1)** is administered at a dose of about 20 mg to about 55 mg.
5. The method of clause 1, wherein Compound **(1)** is administered at a dose of about 50 mg.
6. The method of clause 1, wherein Compound (1) is administered at a dose of about 30 mg or about 40 mg.
7. The method of clause 2, wherein the pharmaceutically acceptable salt of Compound **(1)** is administered at a dose equivalent to about 20 mg to about 55 mg of the free base compound.
8. The method of clause 2, wherein the pharmaceutically acceptable salt of Compound **(1)** is administered at a dose equivalent to about 50 mg of the free base compound.
9. The method of clause 2, wherein the pharmaceutically acceptable salt of Compound **(1)** is administered at a dose equivalent to about 30 mg or about 40 mg of the free base compound.
10. The method of any one of clauses 1-9, wherein Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally.
11. The method of clause 10, wherein Compound **(1),** or the pharmaceutically acceptable salt of Compound **(1),** is administered orally.
12. The method of any one of clauses 1-11, wherein Compound **(1),** or the pharmaceutically acceptable salt of Compound **(1),** is administered with food.
13. The method of any one of clauses 1-12, wherein Compound **(1),** or the pharmaceutically acceptable salt of Compound **(1),** is administered once a day at night.
14. The method of clause 1, wherein Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ.
15. The method of clause 1, wherein Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ.
16. The method of clause 1, wherein Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.
17. The method of clause 1, wherein Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.
18. The method of any one of clauses 1-17, wherein Compound **(1),** or the pharmaceutically acceptable salt of Compound **(1),** is re-administered to the subject in response to a recurrence of depression symptoms after completion of the initial treatment.
19. The method of clause 18, wherein there is at least a 6 week interval between the last dose of the initial treatment and the first dose of the re-administration.
20. The method of clause 18 or 19, wherein each of the initial treatment and re-administration occurs for about 14 days or about 2 weeks.
21. The method of any one of clauses 1-20, further comprising administration of a second therapeutic agent.
22. The method of any one of clauses 1-21, wherein the subject is treatment naive.
23. The method of any one of clauses 1-21, wherein the subject has been on a stable dose of an additional antidepressant for at least 30 days or for at least 60 days prior to the administration of Compound **(1),** or the pharmaceutically acceptable salt of Compound **(1).**
24. The method of any one of clauses 1-23, wherein MDD with elevated anxiety is characterized by a Hamilton Rating Scale for Anxiety (HAM-A) total score of 17 or greater or by a Hamilton Rating Scale for Depression (HAM-D) Anxiety/Somatization subscale score of 7 or greater, prior to the administration of Compound **(1),** or the pharmaceutically acceptable salt of Compound **(1).**
25. The method of any one of clauses 1-23, wherein MDD with elevated anxiety is characterized by a HAM-D total score of 24 or greater and a HAM-A total score of 17 or greater prior to the administration of Compound **(1),** or the pharmaceutically acceptable salt of Compound **(1).**
26. The method of any one of clauses 1-23, wherein MDD with elevated anxiety is characterized by a HAM-D total score of 24 or greater and a HAM-D Anxiety/Somatization subscale score of 7 or greater prior to the administration of Compound **(1),** or the pharmaceutically acceptable salt of Compound **(1).**
27. The method of any one of clauses 1-26, wherein the subject exhibits a reduction in HAM-D total score, HAM-A total score, HAM-D Anxiety/Somatization subscale score, or a combination thereof, from baseline.
28. The method of clause 27, wherein the subject exhibits a reduction of at least 14 points in HAM-D total score on Day 15 after administration of Compound **(1),** or the pharmaceutically acceptable salt of Compound **(1).**
29. The method of clause 27, wherein the subject exhibits a reduction of at least 12 points in HAM-A total score on Day 15 after administration of Compound **(1),** or the pharmaceutically acceptable salt of Compound **(1).**
30. A method of treating postpartum depression (PPD) with elevated anxiety in a subject in need thereof, comprising administering a therapeutically effective amount of Compound **(1):** 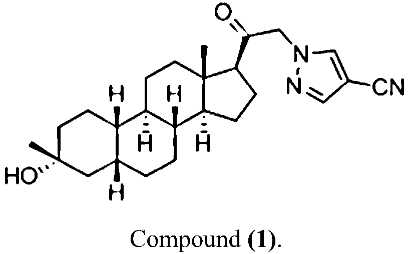
31. A method of treating postpartum depression (PPD) with elevated anxiety in a subject in need thereof, comprising administering a therapeutically effective amount of a pharmaceutically acceptable salt of Compound **(1):** 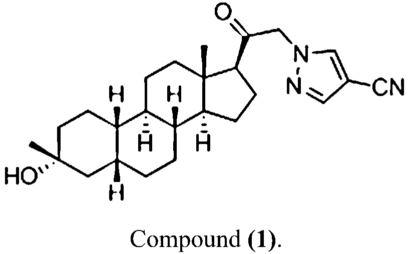
32. The method of clause 30 or 31, wherein Compound **(1),** or the pharmaceutically acceptable salt of Compound **(1),** is administered once a day for about 14 days or about 2 weeks.
33. The method of clause 30, wherein Compound **(1)** is administered at a dose of about 20 mg to about 55 mg.
34. The method of clause 30, wherein Compound **(1)** is administered at a dose of about 50 mg.
35. The method of clause 30, wherein Compound **(1)** is administered at a dose of about 30 mg or about 40 mg.
36. The method of clause 31, wherein the pharmaceutically acceptable salt of Compound **(1)** is administered at a dose equivalent to about 20 mg to about 55 mg of the free base compound.
37. The method of clause 31, wherein the pharmaceutically acceptable salt of Compound **(1)** is administered at a dose equivalent to about 50 mg of the free base compound.
38. The method of clause 31, wherein the pharmaceutically acceptable salt of Compound **(1)** is administered at a dose equivalent to about 30 mg or about 40 mg of the free base compound.
39. The method of any one of clauses 30-38, wherein Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally.
40. The method of clause 39, wherein Compound **(1),** or the pharmaceutically acceptable salt of Compound **(1),** is administered orally.
41. The method of any one of clauses 30-40, wherein Compound **(1),** or the pharmaceutically acceptable salt of Compound **(1),** is administered with food.
42. The method of any one of clauses 30-41, wherein Compound **(1),** or the pharmaceutically acceptable salt of Compound **(1),** is administered once a day at night.
43. The method of clause 30, wherein Compound **(1)** is in a crystalline form having an XRPD pattern comprising peaks between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ.
44. The method of clause 30, wherein Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ.
45. The method of clause 30, wherein Compound **(1)** is in a crystalline form having an XRPD pattern comprising peaks between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ.
46. The method of clause 30, wherein Compound **(1)** is in a crystalline form having an XRPD pattern comprising peaks between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.
47. The method of any one of clauses 30-46, wherein Compound **(1),** or the pharmaceutically acceptable salt of Compound **(1),** is re-administered to the subject in response to a recurrence of depression symptoms after completion of initial treatment.
48. The method of clause 47, wherein there is at least a 6 week interval between the last dose of the initial treatment and the first dose of the re-administration.
49. The method of clause 47 or 48, wherein each of the initial treatment and re-administration occurs for about 14 days or about 2 weeks.
50. The method of any one of clauses 30-49, further comprising administration of a second therapeutic agent.
51. The method of any one of clauses 30-50, wherein the subject is treatment naive.
52. The method of any one of clauses 30-50, wherein the subject has been on a stable dose of an additional antidepressant for at least 30 days or for at least 60 days prior to the administration of Compound **(1),** or the pharmaceutically acceptable salt of Compound **(1).**
53. The method of any one of clauses 30-52, wherein PPD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).**
54. The method of any one of clauses 30-52, wherein PPD with elevated anxiety is characterized by a HAM-D total score of 26 or greater and a HAM-A total score of 17 or greater prior to the administration of Compound **(1),** or the pharmaceutically acceptable salt of Compound **(1).**
55. The method of any one of clauses 30-52, wherein PPD with elevated anxiety is characterized by a HAM-D total score of 26 or greater and a HAM-D Anxiety/Somatization subscale score of 7 or greater prior to the administration of Compound **(1),** or the pharmaceutically acceptable salt of Compound **(1).**
56. The method of any one of clauses 30-55, wherein the subject exhibits a reduction in HAM-D total score, HAM-A total score, HAM-D Anxiety/Somatization subscale score, or a combination thereof, from baseline.
57. A method of treating major depressive disorder (MDD) with elevated anxiety in a subject in need thereof, comprising administering about 30 mg to about 50 mg of Compound (1): 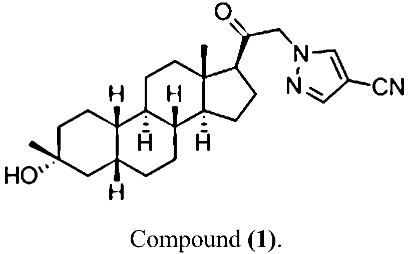
58. A method of treating major depressive disorder (MDD) with elevated anxiety in a subject in need thereof, comprising administering a pharmaceutically acceptable salt of Compound (1) at a dose equivalent to about 30 mg to about 50 mg of the free base compound: 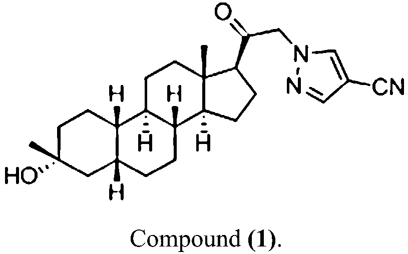
59. A method of treating postpartum depression (PPD) with elevated anxiety in a subject in need thereof, comprising administering about 30 mg to about 50 mg of Compound **(1):** 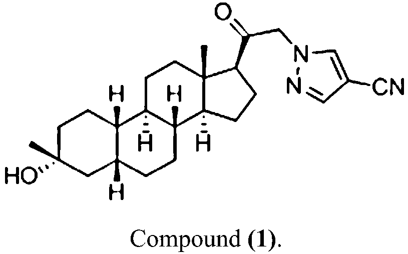
60. A method of treating postpartum depression (PPD) with elevated anxiety in a subject in need thereof, comprising administering a pharmaceutically acceptable salt of Compound **(1)** at a dose equivalent to about 30 mg to about 50 mg of the free base compound: 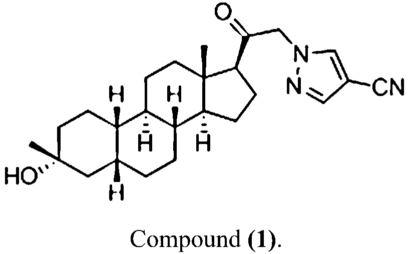
61. A method of treating major depressive disorder (MDD) with elevated anxiety in a subject in need thereof, comprising administering about 30 mg to about 50 mg of Compound **(1)** once a day for about 14 days or about 2 weeks: 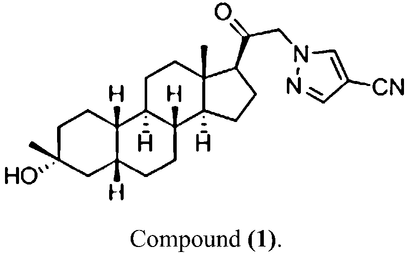
62. A method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 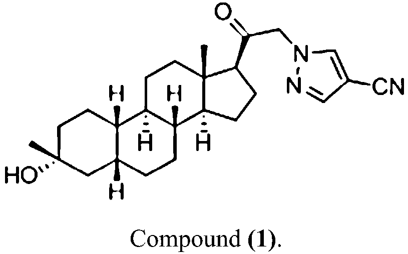
63. A method of treating postpartum depression (PPD) with elevated anxiety in a subject in need thereof, comprising administering about 30 mg to about 50 mg of Compound **(1)** once a day for about 14 days or about 2 weeks: 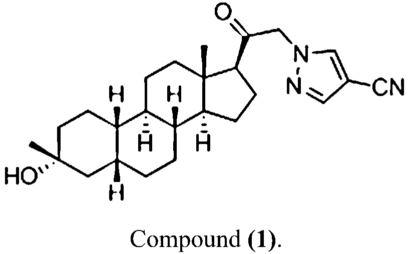
64. A method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 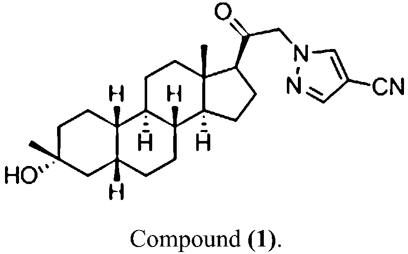
65. A method of treating major depressive disorder (MDD) with elevated anxiety in a subject in need thereof, comprising administering about 30 mg to about 50 mg of Compound **(1)** once a day for about 14 days or about 2 weeks: 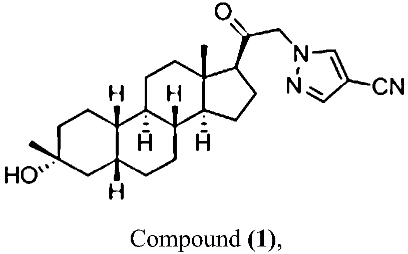 wherein the subject is treatment naïve.
66. A method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 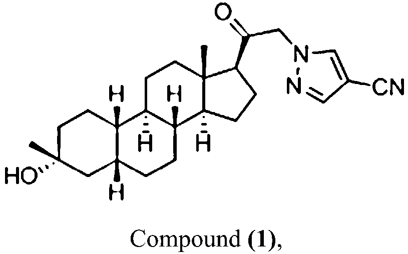 wherein the subject is treatment naive.
67. A method of treating postpartum depression (PPD) with elevated anxiety in a subject in need thereof, comprising administering about 30 mg to about 50 mg of Compound **(1)** once a day for about 14 days or about 2 weeks: 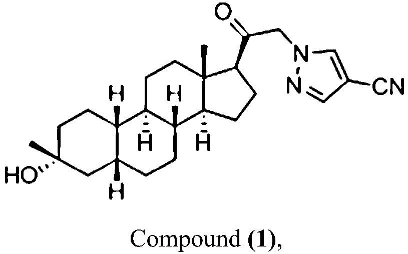 wherein the subject is treatment naïve.
68. A method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 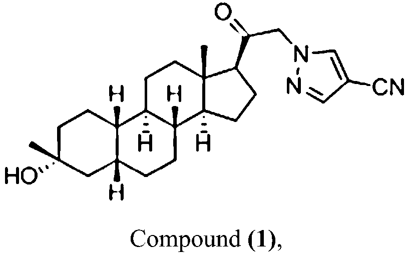 wherein the subject is treatment naive.
69. A method of treating major depressive disorder (MDD) with elevated anxiety in a subject in need thereof, comprising administering about 30 mg to about 50 mg of Compound **(1)** once a day for about 14 days or about 2 weeks: 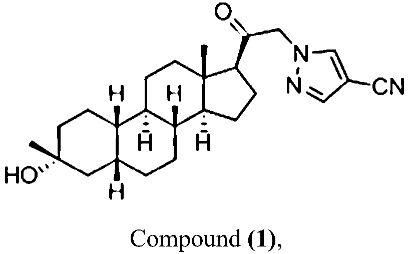 wherein the subject has been on a stable dose of an additional antidepressant for at least 60 days prior to the administration of Compound **(1).**
70. A method of treating major depressive disorder (MDD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 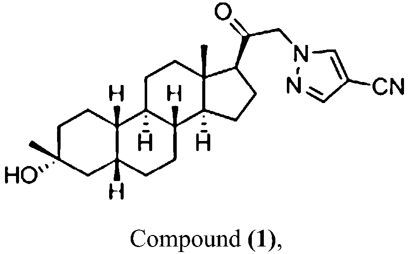 wherein the subject has been on a stable dose of an additional antidepressant for at least 60 days prior to the administration of the pharmaceutically acceptable salt of Compound **(1).**
71. A method of treating postpartum depression (PPD) with elevated anxiety in a subject in need thereof, comprising administering about 30 mg to about 50 mg of Compound **(1)** once a day for about 14 days or about 2 weeks: 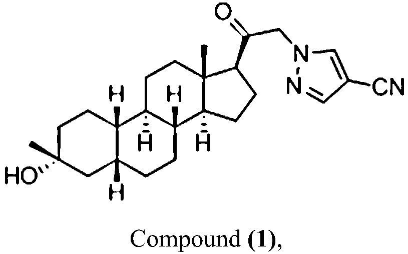 wherein the subject has been on a stable dose of an additional antidepressant for at least 30 days prior to the administration of Compound **(1).**
72. A method of treating postpartum depression (PPD) with elevated anxiety in a subject in need thereof, comprising administering a pharmaceutically acceptable salt of Compound (1) at a dose equivalent to about 30 mg to about 50 mg of the free base compound once a day for about 14 days or about 2 weeks: 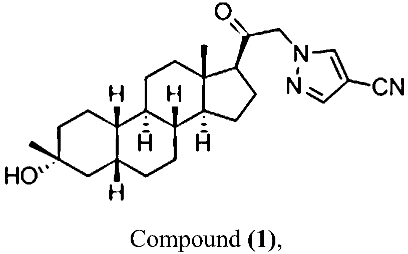 wherein the subject has been on a stable dose of an additional antidepressant for at least 30 days prior to the administration the pharmaceutically acceptable salt of Compound **(1).**
73. A method of treating postpartum depression (PPD) with elevated anxiety in a subject in need thereof, comprising administering about 30 mg to about 50 mg of Compound **(1)** once a day for about 14 days or about 2 weeks: 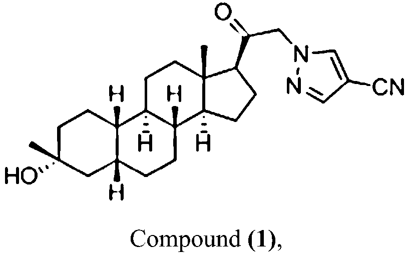 wherein the subject has been on a stable dose of an additional antidepressant for at least 30 days prior to the administration of Compound **(1).**
74. A method of treating postpartum depression (PPD) with elevated anxiety in a subject in need thereof, comprising administering a pharmaceutically acceptable salt of Compound **(1)** at a dose equivalent to about 30 mg to about 50 mg of the free base compound once a day for about 14 days or about 2 weeks: 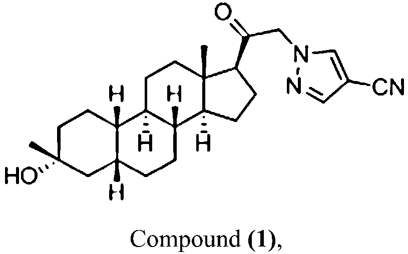 wherein the subject has been on a stable dose of an additional antidepressant for at least 60 days prior to the administration the pharmaceutically acceptable salt of Compound **(1).**
75. The method of any one of clauses 57-74, wherein Compound **(1)** is administered at a dose of about 50 mg or the pharmaceutically acceptable salt of Compound **(1)** is administered at a dose equivalent to about 50 mg of the free base compound.
76. The method of any one of clauses 57-74, wherein Compound **(1)** is administered at a dose of about 40 mg or the pharmaceutically acceptable salt of Compound **(1)** is administered at a dose equivalent to about 40 mg of the free base compound.
77. The method of any one of clauses 57-74, wherein Compound **(1)** is administered at a dose of about 30 mg or the pharmaceutically acceptable salt of Compound **(1)** is administered at a dose equivalent to about 30 mg of the free base compound.
78. The method of any one of clauses 57-77, wherein Compound **(1),** or the pharmaceutically acceptable salt of Compound **(1),** is administered orally, parenterally, intradermally, intrathecally, intramuscularly, subcutaneously, vaginally, as a buccal, sublingually, rectally, topically, as an inhalation, intranasaly, or transdermally.
79. The method of clause 77, wherein Compound **(1),** or the pharmaceutically acceptable salt of Compound **(1),** is administered orally.
80. The method of any one of clauses 57-79, wherein Compound **(1),** or the pharmaceutically acceptable salt of Compound **(1),** is administered with food.
81. The method of any one of clauses 57-80, wherein Compound **(1),** or the pharmaceutically acceptable salt of Compound **(1),** is administered once a day at night.
82. The method of any one of clauses 57-64, wherein the subject is treatment naive.
83. The method of any one of clauses 57-64, wherein the subject has been on a stable dose of an additional antidepressant for at least 30 days or for at least 60 days prior to the administration of Compound **(1),** or the pharmaceutically acceptable salt of Compound **(1).**
84. The method of any one of clauses 57-83, further comprising administration of a second therapeutic agent.
85. The method of any one of clauses 57-84, wherein Compound **(1),** or the pharmaceutically acceptable salt of Compound **(1),** is re-administered to the subject in response to a recurrence of depression symptoms after completion of an initial treatment.
86. The method of clause 85, wherein there is at least a 6 week interval between the last dose of the initial treatment and the first dose of the re-administration.
87. The method of clause 85, wherein the re-administration occurs for about 14 days or about 2 weeks.
88. The method of any one of clauses 57-87, wherein MDD with elevated anxiety or PPD with elevated anxiety is characterized by a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1).**
89. The method of any one of clauses 57-87, wherein MDD with elevated anxiety or PPD with elevated anxiety is characterized by a HAM-D total score of 24 or greater and a HAM-A total score of 17 or greater prior to the administration of Compound **(1),** or the pharmaceutically acceptable salt of Compound **(1).**
90. The method of any one of clauses 57-87, wherein MDD with elevated anxiety or PPD with elevated anxiety is characterized by a HAM-D total score of 24 or greater and a HAM-D Anxiety/Somatization subscale score of 7 or greater prior to the administration of Compound **(1),** or the pharmaceutically acceptable salt of Compound **(1).**
91. The method of any one of clauses 57-90, wherein the subject exhibits a reduction in HAM-D total score, HAM-A total score, HAM-D Anxiety/Somatization subscale score, or a combination thereof, from baseline.
92. The method of clause 91, wherein the subject exhibits a reduction of at least 14 points in HAM-D total score on Day 15 after administration of Compound **(1),** or the pharmaceutically acceptable salt of Compound **(1).**
93. The method of clause 91, wherein the subject exhibits a reduction of at least 12 points in HAM-A total score on Day 15 after administration of Compound **(1),** or the pharmaceutically acceptable salt of Compound **(1).**

## Claims

1. Compound **(1),** or a pharmaceutically acceptable salt of Compound **(1):** 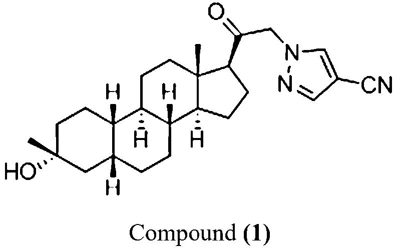 for use in a method of treating major depressive disorder (MDD) with elevated anxiety in a subject in need thereof, comprising administering a therapeutically effective amount of Compound **(1)** or a pharmaceutically acceptable salt of Compound **(1).**

2. The compound for use according to claim 1, wherein Compound **(1),** or the pharmaceutically acceptable salt of Compound **(1),** is administered once a day for 14 days or 2 weeks.

3. The compound for use according to claim 1 or claim 2, wherein Compound **(1)** is administered at a dose of:
(A) 20 mg to 55 mg;
(B) 50 mg; or
(C) 30 mg or 40 mg.

4. The compound for use according to claim 1 or claim 2, wherein the pharmaceutically acceptable salt of Compound **(1)** is administered at a dose equivalent to:
(A) 20 mg to 55 mg of the free base compound;
(B) 50 mg of the free base compound; or
(C) 30 mg or 40 mg of the free base compound.

5. The compound for use according to any one of claims 1-3, wherein Compound **(1)** is in a crystalline form having an XRPD pattern comprising peaks:
(A) between and including 9.7 to 10.1 degrees in 2θ, between and including 11.6 to 12.0 degrees in 2θ, between and including 13.2 to 13.6 degrees in 2θ, between and including 14.2 to 14.6 degrees in 2θ, between and including 14.6 to 15.0 degrees in 2θ, between and including 16.8 to 17.2 degrees in 2θ, between and including 20.5 to 20.9 degrees in 2θ, between and including 21.3 to 21.7 degrees in 2θ, between and including 21.4 to 21.8 degrees in 2θ, and between and including 22.4 to 22.8 degrees in 2θ;
(B) between and including 9.3 to 9.7 degrees in 2θ, between and including 10.6 to 11.0 degrees in 2θ, between and including 13.0 to 13.4 degrees in 2θ, between and including 14.7 to 15.1 degrees in 2θ, between and including 15.8 to 16.2 degrees in 2θ, between and including 18.1 to 18.5 degrees in 2θ, between and including 18.7 to 19.1 degrees in 2θ, between and including 20.9 to 21.3 degrees in 2θ, between and including 21.4 to 21.8 degrees in 2θ, and between and including 23.3 to 23.7 degrees in 2θ;
(C) between and including 9.7 to 10.1 degrees in 2θ, between and including 14.6 to 15.0 degrees in 2θ, between and including 16.8 to 17.2 degrees in 2θ, between and including 20.5 to 20.9 degrees in 2θ, and between and including 21.3 to 21.7 degrees in 2θ; or
(D) between and including 9.3 to 9.7 degrees in 2θ, between and including 10.6 to 11.0 degrees in 2θ, between and including 13.0 to 13.4 degrees in 2θ, between and including 18.7 to 19.1 degrees in 2θ, and between and including 21.4 to 21.8 degrees in 2θ.

6. Compound **(1),** or a pharmaceutically acceptable salt of Compound **(1):** 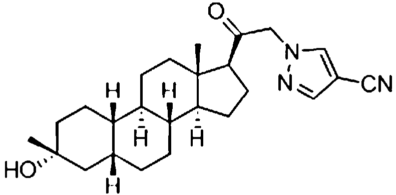 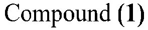 for use in a method of treating major depressive disorder (MDD) with elevated anxiety in a subject in need thereof,
wherein Compound **(1)** is administered at a dose of 30 mg to 50 mg, or the pharmaceutically acceptable salt of Compound **(1)** is administered at a dose equivalent to 30 mg to 50 mg of the free base compound.

7. The compound for use according to claim 6, wherein Compound **(1)** or the pharmaceutically acceptable salt of Compound **(1)** is to be administered once a day for 14 days or 2 weeks.

8. Compound **(1),** or a pharmaceutically acceptable salt of Compound **(1):** 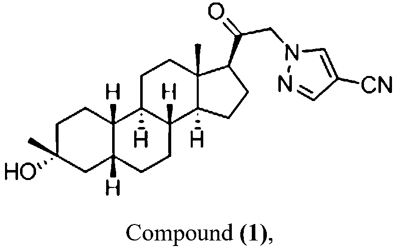 for use in a method of treating major depressive disorder (MDD) with elevated anxiety in a subject in need thereof,
wherein Compound **(1)** is administered at a dose of 30 mg to 50 mg once a day for 14 days or 2 weeks, or the pharmaceutically acceptable salt of Compound **(1)** is administered at a dose equivalent to 30 mg to 50 mg of the free base compound once a day for 14 days or 2 weeks; and
wherein the subject is treatment naive.

9. Compound **(1),** or a pharmaceutically acceptable salt of Compound **(1):** 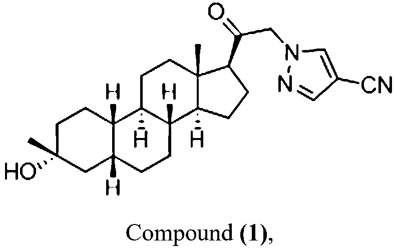 for use in a method of treating major depressive disorder (MDD) with elevated anxiety in a subject in need thereof,
wherein Compound **(1)** is administered at a dose of 30 mg to 50 mg once a day for 14 days or 2 weeks, or the pharmaceutically acceptable salt of Compound **(1)** is administered at a dose equivalent to 30 mg to 50 mg of the free base compound once a day for 14 days or 2 weeks; and
wherein the subject has been on a stable dose of an additional antidepressant for:
(A) at least 30 days prior to the administration of Compound **(1)** or the pharmaceutically acceptable salt of Compound **(1);** or
(B) at least 60 days prior to the administration of Compound **(1)** or the pharmaceutically acceptable salt of Compound **(1).**

10. The compound for use according to any one of claims 6-9, wherein:
(A) Compound **(1)** is administered at a dose of 50 mg or the pharmaceutically acceptable salt of Compound **(1)** is administered at a dose equivalent to 50 mg of the free base compound;
(B) Compound **(1)** is administered at a dose of 40 mg or the pharmaceutically acceptable salt of Compound **(1)** is administered at a dose equivalent to 40 mg of the free base compound; or
(C) Compound **(1)** is administered at a dose of 30 mg or the pharmaceutically acceptable salt of Compound **(1)** is administered at a dose equivalent to 30 mg of the free base compound.

11. The compound for use according to any one of claims 1-10, wherein Compound **(1),** or the pharmaceutically acceptable salt of Compound **(1),** is administered:
(A) orally, parenterally, intradermally, intrathecally, intramuscularly, subcutaneously, vaginally, as a buccal, sublingually, rectally, topically, as an inhalation, intranasally, or transdermally;
(B) orally;
(C) with food; and/or
(D) once a day at night.

12. The compound for use according to any one of claims 1-7 or 10-11, wherein:
(A) the subject is treatment naive; or
(B) the subject has been on a stable dose of an additional antidepressant for at least 30 days or for at least 60 days prior to the administration of Compound **(1),** or the pharmaceutically acceptable salt of Compound **(1).**

13. The compound for use according to any one of claims 1-12, wherein Compound **(1),** or the pharmaceutically acceptable salt of Compound **(1),** is re-administered to the subject in response to a recurrence of depression symptoms after completion of an initial treatment;
optionally, wherein there is at least a 6 week interval between the last dose of the initial treatment and the first dose of the re-administration; or
optionally, wherein the re-administration occurs for 14 days or 2 weeks.

14. The compound for use according to any one of claims 1-13, wherein MDD with elevated anxiety is **characterized by**:
(A) a Hamilton Rating Scale for Anxiety (HAM-A) total score of 17 or greater, or by a Hamilton Rating Scale for Depression (HAM-D) Anxiety/Somatization subscale score of 7 or greater, prior to the administration of Compound **(1),** or the pharmaceutically acceptable salt of Compound **(1);**
(B) a HAM-D total score of 24 or greater and a HAM-A total score of 17 or greater prior to the administration of Compound **(1),** or the pharmaceutically acceptable salt of Compound **(1);** or
(C) a HAM-D total score of 24 or greater and a HAM-D Anxiety/Somatization subscale score of 7 or greater prior to the administration of Compound **(1),** or the pharmaceutically acceptable salt of Compound **(1).**

15. The compound for use according to any one of claims 1-14, wherein the subject exhibits a reduction in HAM-D total score, HAM-A total score, HAM-D Anxiety/Somatization subscale score, or a combination thereof, from baseline;
optionally, wherein the subject exhibits a reduction of at least 14 points in HAM-D total score on Day 15 after administration of Compound **(1),** or the pharmaceutically acceptable salt of Compound **(1);** or
optionally, wherein the subject exhibits a reduction of at least 12 points in HAM-A total score on Day 15 after administration of Compound **(1),** or the pharmaceutically acceptable salt of Compound **(1).**

16. The compound for use according to any one of claims 1-3 or 5-15, wherein the compound is Compound **(I).**
